# EUROPEAN PATENT APPLICATION

(11) **EP 3 553 182 A1**
(43) Date of publication of application: **16.10.2019**
(21) Application number: 18305440.2
(22) Date of filing: 11.04.2018
(51) Int. Cl.: C12Q 1/6827, C12Q 1/6834, C12Q 1/6886

(54) **DETECTION METHOD OF SOMATIC GENETIC ANOMALIES, COMBINATION OF CAPTURE PROBES AND KIT OF DETECTION**

(71) Applicant: Université de Bourgogne, 21000 Dijon (FR); CGFL (Centre Georges François Leclerc), 21000 Dijon (FR)
(72) Inventor: BOIDOT, Romain, 21310 ARCEAU (FR); VEGRAN, Frédérique, 21310 ARCEAU (FR); GHIRINGHELLI, François, 21000 DIJON (FR)
(74) Representative: Jacobacci Coralis Harle

(57) **Abstract**

This invention relates to a detection method of somatic genetic anomalies potentially present in a tissue sample cancerous, taken from a subject, wherein said detection method comprises the following steps:
(a) shearing of the genomic DNA extracted from said tissue sample, to obtain sheared genomic DNA;
(b) preparing a library by hybridization capture from the sheared genomic DNA obtained at step (a), by the implementation of at least 43 capture probes having at least 110 nucleotides in length and at least 75% sequence homology with respectively SEQ ID NO:1 to SEQ ID NO:43, said capture probes being able to hybridize on fragments from at least 5 target regions selected from : ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA contained in the library issued at step (b), and
(d) analyzing of the sequences of said sheared genomic DNA obtained at step (c), for detecting somatic genetic anomalies in said tissue sample.

The present invention also refers to a combination of capture probes and a kit comprising said combination.

## Description

### FIELD OF THE INVENTION

The present invention relates to field of cancer detection.

Especially, the present invention relates to a detection method of genetic anomalies potentially present in a tumor using specific capture probes, a kit of detection of these genetic anomalies, and a combination of capture probes.

### DESCRIPTION OF RELATED ART

Cancer is one of the leading causes of death in the world. For instance, in 2010, nearly 1.5 million people (essentially women) developed breast cancer worldwide. It is estimated that 5 to 10% of these cases were hereditary. In addition, more than 1.5 million new cases and more than 0.5 million deaths were reported during 2010 in the United States alone.

Treatment currently performed for cancer is mainly symptomatic therapy that mostly consists of surgical therapy with a combination of radiation therapy and chemotherapy. Owing to advancements in medical technology, cancer is now almost a curable disease if it can be detected early.

Indeed, it is desirable not only to detect cancer, but also to diagnose: a cancer developed in a location invisible to the naked eye, an extent of cancer, a malignancy or postoperative course of cancer, a recurrence, a metastasis, and the like.

The widespread use of Next Generation Sequencing (NGS), also known as massively parallel sequencing, has opened up new avenues for cancer research and diagnosis. NGS will bring huge amounts of new data on cancer, and especially cancer genetics.

NGS represents an effective way to capture a large amount of genomic information about a cancer. Most NGS technologies revolve around sequencing by synthesis. Each DNA fragment to be sequenced is bound to an array, and then DNA polymerase adds labeled nucleotides sequentially. A high-resolution camera captures the signal from each nucleotide becoming integrated and notes the spatial coordinates and time. The sequence at each spot can then be inferred by a computer program to generate a contiguous DNA sequence, referred to as a read.

Multiple technological enhancements have allowed NGS to be more readily implemented in a clinical workflow. Samples now no longer need to be handled differently from standard diagnostic specimens, and recent advances have even enabled increasingly complex genomic data to be derived from a patient's peripheral blood. The concept of precision medicine goes hand in hand with an understanding of the cancer genome as determined by NGS.

Before the development of NGS, tumor genotyping was performed only on specific genomic loci that were known to be frequently mutated in cancer, which are known as "hotspots", in general by Sanger sequencing. These approaches were best suited to recurrent activating mutations in oncogenes, such as in the *KRAS* gene in colon and lung cancer.

However, these approaches were insufficient to identify alterations in tumor suppressors (in which an alteration anywhere in the gene may impact its function) or the increasingly complex area of "long tail" hotspot alterations in oncogenes. In addition, these approaches only allow a very limited exploration of the human genome.

Thus, current assay options involve approaches that may capture known cancer genes ("gene panels"), whole-exome, whole-genome and/or whole-transcriptome approaches. There are several trade-offs to increasing the portion of the genome that is sequenced. The first is a loss of coverage for the same amount of sequencing. Coverage, or depth, is defined as the average number of mappable reads at a given locus in your panel. Lower coverage limits the ability to confidently call a variant of low allele fraction to be biologically real and not a technical artifact. A second is that whole-genome and whole-exome sequencing require germline sequencing to enhance the identification of true somatic variants, which may uncover incidental clinically relevant inherited disorders.

To achieve these last approaches, the large majority of the cancer detection kits on the market are based on amplicon sequencing.

Amplicon sequencing enriches target genes by PCR with a set of primers for exons of selected genes prior to NGS analysis. Especially, the set of primers will amplify different and large DNA fragments. These protocols have the advantage of less required input DNA and less turnaround time than hybrid capture methods, which is critical for clinical application, but potentially PCR amplification can bias the observed allele fraction. It also pulls information out of a lower percentage of starting material, further increasing the chance of bias in calling copy number variations. The informatics analysis is relatively easy, as any read that does not map to a locus between primers can be disregarded.

A downside of this simplicity is that the assay is inherently unable to detect unexpected fusions, because either the 5' or 3' primer would fail to bind the translocated DNA. Also, this sequencing method requires DNA which is in a perfect state. In addition, the specific primers of these different fragments require similar hybridization and elongation conditions. These conditions being difficult to meet, the amplicon sequencing generates a large amount of artefacts.

Hence, there is a need to develop a new detection method of somatic genetic anomalies present in a tumor which enables to detect various types of cancer with high sensitivity at one time, and preferably for different patients.

Especially, there is a need to develop a new detection method which allows to provide a personalized medicine for each patient.

### SUMMARY OF THE INVENTION

The present invention meets these needs since the inventors, surprisingly and unexpectedly, discovered that a specific combination of capture probes embedded in hybrid capture enables to obtain a high sensitive measurement method for detecting somatic gene anomalies and this, for various type of cancer and at one time.

Moreover, the inventors have discovered that the specific combination of capture probes embedded in hybrid capture is capable of not only detecting cancer, but also diagnosing cancer having developed in a location invisible to the naked eye, the extent of cancer, the malignancy or postoperative course of cancer, recurrence, metastasis, and the like.

The present invention thus provides a detection method of somatic genetic anomalies potentially present in a tissue sample (potentially cancerous), taken from a subject, wherein said detection method comprises the following steps:
(a) shearing of a genomic DNA extracted from said tissue sample, to obtain sheared genomic DNA;
(b) preparing a library by hybridization capture from the sheared genomic DNA obtained at step (a), by the implementation of at least 43 capture probes having at least 110 nucleotides in length and at least 75 % sequence homology with respectively SEQ ID NO:1 to SEQ ID NO:43, said capture probes being able to hybridize on fragments from at least 5 target regions selected from : ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA contained in the library issued at step (b), and
(d) analyzing of the sequences of said sheared genomic DNA obtained at step (c), for detecting somatic genetic anomalies in said tissue sample.

The present invention enables a high sensitivity of the analysis of at least 5, preferably 15, full coding sequence (and closed intron sequences) genes with a high reliability. This is the consequence of a very low background signal, a specific design targeting theranostics studied genes, and the use of internal controls. Finally, this design can be used on solid (tumor) and liquid (blood) samples.

The present invention concerns a combination of capture probes, comprising at least 43 capture probes comprising at least 110 nucleotides and at least 75 % sequence homology with respectively the probes of SEQ ID NO:1 to SEQ ID NO:43.

In general, the combination of capture probes comprises further at least 4 additional capture probes comprising at least 110 nucleotides and at least 75 % sequence homology with respectively the probes of SEQ ID NO:44 to SEQ ID NO:47 and/or the probes of SEQ ID NO:48 to SEQ ID NO:51 or combination thereof.

Preferably, the combination of capture probes comprises further at least 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

The present invention also refers to a kit of detection of somatic genetic anomalies potentially present in a tissue sample potentially cancerous taken from a subject, comprising the combination of capture probes as defined above.

The various embodiments of the present invention are especially described in the detailed specification hereafter. These embodiments may be considered separately or be combined with each other.

### DETAILED DESCRIPTION OF THE INVENTION AND PREFERRED EMBODIMENTS

The practice of the present invention will employ, unless otherwise indicated, conventional techniques of molecular biology, microbiology and recombinant DNA techniques, which are within the skill of the art. Such techniques are explained fully in the literature. See, e.g., Sambrook, Fritsch & Maniatis, Molecular Cloning: A Laboratory Manual, Fourth Edition (2012); Oligonucleotide Synthesis (M. J. Gait, ed., 1984); Nucleic Acid Hybridization (B. D. Hames & S. J. Higgins, eds., 1984); A Practical Guide to Molecular Cloning (B. Perbal, 1984); and a series, Methods in Enzymology (Academic Press, Inc.).

### A -DEFINITIONS

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof, such as "includes" and "including") are openended linking verbs. They are used to specify the presence of stated features, integers, steps or components or groups thereof, but do not preclude the presence or addition of one or more other features, integers, steps or components or groups thereof. As a result, a method, or a step in a method, that "comprises," "has," "contains," or "includes" one or more steps or elements possesses those one or more steps or elements, but is not limited to possessing only those one or more steps or elements.

Unless otherwise indicated, all numbers or expressions referring to quantities of ingredients, ranges, reaction conditions, etc. used herein are to be understood as modified in all instances by the term "about."

Also unless otherwise indicated, the indication of an interval of values « from X to Y » or "between X to Y", according to the present invention, means as including the values of X and Y.

As used in the specification and claims, the singular forms "a", "an" and "the" can include plural references unless the context clearly dictates otherwise. For example, the term "a cell" can include a plurality of cells, including mixtures thereof.

The cancer is the class of disease characterized by rapid and uncontrolled proliferation of cells within a tissue of a multi-tissue eukaryote. Cancers are generally thought to be genetic diseases of somatic cells, arising through sequential mutations that create oncogenes and inactivate tumor-suppressor genes.

The term "subject", as used herein, generally refers to a biological entity containing expressed genetic materials. The biological entity can be a plant, animal, or microorganism, including, e.g., bacteria, viruses, fungi, and protozoa. The subject can be tissues, cells and their progeny of a biological entity obtained *in vivo* or cultured *in vitro.* The subject can be a mammal. The mammal can be a human. The human may be diagnosed or suspected of being at high risk for a disease. The disease can be cancer. The human may not be diagnosed or suspected of being at high risk for a disease.

As used herein, a "sample" or "nucleic acid sample" can refer to any substance containing or presumed to contain nucleic acid. The sample can be a biological sample obtained from a subject. The nucleic acids can be RNA, DNA, e.g., genomic DNA, mitochondrial DNA, viral DNA, synthetic DNA, or cDNA reverse transcribed from RNA.

The term "genomic DNA" consists advantageously in DNA from the genome containing all coding (exon) and noncoding (intron and other) sequences.

The nucleic acids in a nucleic acid sample generally serve as templates for extension of a hybridized primer. In some embodiments, the biological sample is a liquid sample. The liquid sample can be whole blood, plasma, serum, ascites, cerebrospinal fluid, sweat, urine, tears, saliva, buccal sample, cavity rinse, or organ rinse. The liquid sample can be an essentially cell-free liquid sample (e.g., plasma, serum, sweat, plasma, urine, sweat, tears, saliva, sputum, cerebrospinal fluid). In other embodiments, the biological sample is a solid biological sample, e.g., feces or tissue biopsy, e.g., a tumor biopsy. A sample can also comprise *in vitro* cell culture constituents (including but not limited to conditioned medium resulting from the growth of cells in cell culture medium, recombinant cells and cell components). The sample can comprise a single cell, e.g., a cancer cell, a circulating tumor cell, a cancer stem cell, and the like.

"Nucleotides" and "nt" are used interchangeably herein to generally refer to biological molecules that can form nucleic acids. Nucleotides can have moieties that contain not only the known purine and pyrimidine bases, but also other heterocyclic bases that have been modified. Such modifications include methylated purines or pyrimidines, acylated purines or pyrimidines, alkylated riboses, or other heterocycles. In addition, the term "Nucleotide" includes those moieties that contain hapten, biotin, or fluorescent labels and may contain not only conventional ribose and deoxyribose sugars, but other sugars as well.

Modified nucleosides or nucleotides also include modifications on the sugar moiety, e.g., wherein one or more of the hydroxyl groups are replaced with halogen atoms or aliphatic groups, are functionalized as ethers, amines, or the like. Modified nucleosides or nucleotides can also include peptide nucleic acid (PNA). Peptide nucleic acid generally refers to oligonucleotides in which the deoxyribose backbone has been replaced with a backbone having peptide linkages. Each subunit generally has attached a naturally occurring or non-naturally occurring base. One exemplary PNA backbone is constructed of repeating units of N-(2-aminoethyl)glycine linked through amide bonds. PNA can bind both DNA and RNA to form PNA/DNA or PNA/RNA duplexes. The resulting PNA/DNA or PNA/RNA duplexes can be bound with greater affinity than corresponding DNA/DNA or DNA/RNA duplexes as evidence by their higher melting temperatures (Tm). The neutral backbone of the PNA also can render the Tm of PNA/DNA(RNA) duplexes to be largely independent of salt concentration in a reaction mixture. Thus the PNA/DNA duplex can offer an advantage over DNA/DNA duplex interactions which are highly dependent on ionic strength. Exemplary embodiments of PNA are described in US Patent Nos. 7,223,833 and 5,539,083.

"Nucleotides" can also include nucleotides comprising a Tm-enhancing base (e.g., a Tm-base enhancing nucleotide). Exemplary Tm-enhancing base nucleotides include, but are not limited to nucleotides with Superbases™, locked nucleic acids (LNA) or bridged nucleic acids (BNA). BNA and LNA generally refer to modified ribonucleotides wherein the ribose moiety is modified with a bridge connecting the 2' oxygen and 4' carbon. Generally, the bridge "locks" the ribose in the 3'-endo (North) conformation, which is often found in the A- form duplexes. The term "locked nucleic acid" (LNA) generally refers to a class of BNAs, where the ribose ring is "locked" with a methylene bridge connecting the 2'-0 atom with the 4'-C atom. LNA nucleosides containing the six common nucleobases (T, C, G, A, U and mC) that appear in DNA and RNA are able to form base-pairs with their complementary nucleosides according to the standard Watson-Crick base pairing rules. Accordingly, Tm- enhancing base nucleotides such as BNA and LNA nucleotides can be mixed with DNA or RNA bases in an oligonucleotide whenever desired. The locked ribose conformation enhances base stacking and backbone pre-organization. Base stacking and backbone pre- organization can give rise to an increased thermal stability (e.g., increased Tm) and discriminative power of duplexes. LNA can discriminate single base mismatches under conditions not possible with other nucleic acids. Locked nucleic acid is disclosed for example in WO 99/14226.. Nucleotides can also include modified nucleotides as described in European Patent Application No. EP1995330.

Other modified nucleotides can include 5-Me-dC-CE phosphoramidite, 5-Me-dC- CPG, 2-Amino-dA-CE phosphoramidite, N4-Et-dC-CE Phosphoramidite, N4-Ac-N4-Et-dC- CE Phosphoramidite, N6-Me-dA-CE Phosphoramidite, N6-Ac-N6-Me-dA-CE

Phosphoramidite, Zip nucleic acids (ZNA®, described in US Patent Application Ser. No. 12/086,599), 5'-Trimethoxystilbene Cap Phosphoramidite, 5'-Pyrene Cap Phosphoramidite, 3'-Uaq Cap CPG. (Glen Research).

Yet other modified nucleotides can include nucleotides with modified nucleoside bases such as, e.g., 2-Aminopurine, 2,6-Diaminopurine, 5-Bromo-deoxyuridine, deoxyuridine, Inverted dT, inverted ddT, ddC, 5-Methyl deoxyCytidine, deoxylnosine, 5-Nitroindole, 2'-0-Methyl RNA bases, Hydroxmethyl dC, Iso-dG and Iso-dC (Eragen Biosciences, Inc), 2'-Fluoro bases having a fluorine modified ribose.

The terms "polynucleotides", "nucleic acid", "nucleotides" and "oligonucleotides" can be used interchangeably. They can refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers.

A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The term "target polynucleotide," "target region", or "target", as use herein, generally refers to a polynucleotide of interest under study. In certain embodiments, a target polynucleotide contains one or more sequences that are of interest and under study. A target polynucleotide can comprise, for example, a genomic sequence. The target polynucleotide can comprise a target sequence whose presence, amount, and/or nucleotide sequence, or changes in these, are desired to be determined.

The target polynucleotide can be a region of gene associated with a disease. In general, the region is an exon. In some embodiments, the gene is a druggable target. The term "druggable target", as used herein, generally refers to a gene or cellular pathway that is modulated by a disease therapy. The disease can be cancer. Accordingly, the gene can be a known cancer-related gene. Especially, the cancer-related gene is selected from the group consisting of ALK, BRAF, EGFR, EIF1AY, ERBB2, ERBB4, KDR, KIT, KRAS, MAP2K1, MET, NRAS, PDGFRA, SLC28A1, TP53.

The term "genomic sequence", as used herein, generally refers to a sequence that occurs in a genome. Because RNAs are transcribed from a genome, this term encompasses sequence that exist in the nuclear genome of an organism, as well as sequences that are present in a cDNA copy of an RNA (e.g., an mRNA) transcribed from such a genome.

The terms "anneal", "hybridize" or "bind," can refer to two polynucleotide sequences, segments or strands, and can be used interchangeably and have the usual meaning in the art. Two complementary sequences (e.g., DNA and/or RNA) can anneal or hybridize by forming hydrogen bonds with complementary bases to produce a double-stranded polynucleotide or a double-stranded region of a polynucleotide.

As used herein, the term "complementary" generally refers to a relationship between two antiparallel nucleic acid sequences in which the sequences are related by the base-pairing rules: A pairs with T or U and C pairs with G. A first sequence or segment that is "perfectly complementary" to a second sequence or segment is complementary across its entire length and has no mismatches. A first sequence or segment is "substantially complementary" to a second sequence of segment when a polynucleotide consisting of the first sequence is sufficiently complementary to specifically hybridize to a polynucleotide consisting of the second sequence.

The term "duplex," or "duplexed," as used herein, can describe two complementary polynucleotides that are base-paired, e.g., hybridized together.

As used herein, the term "Tm" generally refers to the melting temperature of an oligonucleotide duplex at which half of the duplexes remains hybridized and half of the duplexes dissociates into single strands. See Sambrook and Russell (2001; Molecular Cloning: A Laboratory Manual, 3rd ed., Cold Spring Harbor Press, Cold Spring Harbor N.Y., ch. 10).

As used herein, "amplification" of a nucleic acid sequence generally refers to *in vitro* techniques for enzymatically increasing the number of copies of a target sequence. Amplification methods include both asymmetric methods (in which the predominant product is single-stranded) and conventional methods (in which the predominant product is double-stranded). A "round" or "cycle" of amplification can refer to a PCR cycle in which a double stranded template DNA molecule is denatured into single-stranded templates, forward and reverse primers are hybridized to the single stranded templates to form primer/template duplexes, primers are extended by a DNA polymerase from the primer/template duplexes to form extension products. In subsequent rounds of amplification the extension products are denatured into single stranded templates and the cycle is repeated.

The terms "template", "template strand", "template DNA" and "template nucleic acid" can be used interchangeably herein to refer to a strand of DNA that is copied by an amplification cycle.

The term "denaturing," as used herein, generally refers to the separation of a nucleic acid duplex into two single strands.

The term "extending", as used herein, generally refers to the extension of a primer hybridized to a template nucleic acid by the addition of nucleotides using an enzyme, e.g., a polymerase.

A "primer "is generally a nucleotide sequence (e.g., an oligonucleotide), generally with a free 3'-OH group, that hybridizes with a template sequence (such as a target polynucleotide, or a primer extension product) and is capable of promoting polymerization of a polynucleotide complementary to the template. A primer can be, for example, a sequence of the template (such as a primer extension product or a fragment of the template created following R ase cleavage of a template -DNA complex) that is hybridized to a sequence in the template itself (for example, as a hairpin loop), and that is capable of promoting nucleotide polymerization. Thus, a primer can be an exogenous (e.g., added) primer or an endogenous (e.g., template fragment) primer.

The terms "determining", "measuring", "evaluating", "assessing," "assaying," and "analyzing" can be used interchangeably herein to refer to any form of measurement, and include determining if an element is present or not. These terms can include both quantitative and/or qualitative determinations. Assessing may be relative or absolute. "Assessing the presence of can include determining the amount of something present, as well as determining whether it is present or absent.

The term "free in solution," as used here, can describe a molecule, such as a polynucleotide, that is not bound or tethered to a solid support.

The term "genomic fragment", as used herein, can refer to a region of a genome, e.g., an animal or plant genome such as the genome of a human, monkey, rat, fish or insect or plant. A genomic fragment may or may not be adaptor ligated. A genomic fragment may be adaptor ligated (in which case it has an adaptor ligated to one or both ends of the fragment, to at least the 5' end of a molecule), or non-adaptor ligated.

"Pre-amplification", as used herein, generally refers to non-clonal amplification of nucleic acids. For example, pre-amplification of a nucleic acid library is generally performed prior to clonal amplification of the library and/or loading onto a sequencer.

The term "ligase", as used herein, generally refers to an enzyme that is commonly used to join polynucleotides together or to join the ends of a single polynucleotide.

The term "ligation", as used herein, generally refers to the joining of two ends of polynucleotides or the joining of ends of a single polynucleotide by the formation of a covalent bond between the ends to be joined. The covalent bond can be a phosphodiester bond.

The term "ATP-dependent ligation", as used herein, generally refers to ligation by an ATP-dependent ligase. An exemplary mechanism of ATP-dependent ligation is described herein.

"Donor" and "acceptor" nucleic acid species generally refer to two distinct populations of nucleic acid molecules to be joined in a ligation reaction. The "donor" species generally refers to a population of nucleic acid molecules which may accept a nucleoside monophosphate(NMP) at either a 5' or 3' end. The "acceptor" species generally refers to a second population of nucleic acid molecules containing a 3 Or 5' OH group which may be ligated to the "donor" species via the NMP at either the 5' or 3' end of the donor species.

The donor and acceptor species can be any nucleic acid species. They can be, for example, polynucleotides isolated from a biological source. The biological source can be a subject. Exemplary biological sources and subjects are described herein. They can be oligonucleotides. Methods for preparing oligonucleotides of specific sequence are known in the art, and include, for example, cloning and restriction of appropriate sequences and direct chemical synthesis. Chemical synthesis methods may include, for example, the phosphotriester method described by Narang et al, 1979, Methods in Enzymology 68:90, the phosphodiester method disclosed by Brown et al, 1979, Methods in Enzymology 68: 109, the diethylphosphoramidate method disclosed in Beaucage et al, 1981, Tetrahedron Letters 22: 1859, and the solid support method disclosed in U.S. Pat. No. 4,458,066. They can be RNA or DNA. The DNA can be partially or fully denatured DNA. The DNA can be single stranded (ss)DNA. Partially denatured can be "frayed" at ends such that a "frayed" end can comprise 1, 2, 3, 4, 5, or more than 5 non-annealed nucleotides.

The donor and/or acceptor nucleic acid species can be of any size, ranging from, e.g., 1-50 nt, 10-100 nt, 50-200 nt, 100-400 nt, 200-600 nt, 500-1000 nt, 800-2000 nt, or greater than 2000 nt. In some embodiments, the donor and/or acceptor nucleic acid species is over 120 nt long.

The donor or acceptor nucleic acid species can include, e.g., genomic nucleic acids, adaptor sequences, and/or barcode sequences. The donor or acceptor nucleic acid species can include oligonucleotides. The donor or acceptor nucleic acid species can comprise a detectable label or affinity tag.

The detectable label can be any molecule that enables detection of a molecule to be detected. Non-limiting examples of detectable labels include, e.g., chelators, photoactive agents, radioactive moieties (e.g., alpha, beta and gamma emitters), fluorescent agents, luminescent agents, paramagnetic ions, or enzymes that produce a detectable signal in the presence of certain reagents (e.g., horseradish peroxidase, alkaline phosphatase, glucose oxidase).

Exemplary fluorescent compounds include, e.g., fluorescein isothiocyanate, rhodamine, phycoerytherin, phycocyanin, allophycocyanin, o-phthaldehyde, fluorescamine, and commercially available fluorophores such as Alexa Fluor 350, Alexa Fluor 488, Alexa Fluor 532, Alexa Fluor 546, Alexa Fluor 568, Alexa Fluor 594, Alexa Fluor 647, DyLight dyes such as DyLight 488, DyLight 594, DyLight 647, and BODIPY dyes such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine and Texas Red. Such compounds are commercially available (see, e.g., Molecular Probes, Inc.).

The affinity tag can be selected to have affinity to a capture moiety. The affinity tag can comprise, by way of non-limiting example only, biotin, desthiobiotin, histidine, polyhistidin, myc, hemagglutinin (HA), FLAG, a fluorescence tag, a tandem affinity purification (TAP) tag, a glutathione S transferase (GST) tag, or derivatives thereof. The capture moiety can comprise, e.g., avidin, streptavidin, Neutravidin™, nickel, or glutathione or other molecule capable of binding the affinity tag.

In some embodiments, the acceptor species and the donor species can be the same species. For example, in some embodiments a user may desire to circularize a linear nucleic acid or to form concatemers of a single nucleic acid species.

The term "reaction mixture" as used herein generally refers to a mixture of components necessary to effect a desired reaction. The mixture may further comprise a buffer (e.g., a Tris buffer). The reaction mixture may further comprise a monovalent salt. The reaction mixture may further comprise a cation, e.g., Mg and/or Mn. The concentration of each component is well known in the art and can be further optimized by an ordinary skilled artisan. In some embodiments, the reaction mixture also comprises additives including, but not limited to, non-specific background/blocking nucleic acids (e.g., salmon sperm DNA), non-specific background/blocking proteins (e.g., bovine serum albumin, non-fat dry milk) biopreservatives (e.g. sodium azide), PCR enhancers (e.g. Betaine, Trehalose, etc.), and inhibitors (e.g. RNAse inhibitors). In some embodiments, a nucleic acid sample is admixed with the reaction mixture.

A "primer binding site" can refer to a site to which a primer hybridizes in an oligonucleotide or a complementary strand thereof.

The term "separating", as used herein, can refer to physical separation of two elements (e.g., by size, affinity, degradation of one element etc.).

The term "sequencing", as used herein, can refer to a method by which the identity of at least 10 consecutive nucleotides (e.g., the identity of at least 20, at least 50, at least 100, at least 200, or at least 500 or more consecutive nucleotides) of a polynucleotide are obtained.

The term "adaptor-ligated", as used herein, can refer to a nucleic acid that has been ligated to an adaptor. The adaptor can be ligated to a 5' end or a 3' end of a nucleic acid molecule, or can be added to an internal region of a nucleic acid molecule.

The term "bridge PCR" can refer to a solid-phase polymerase chain reaction in which the primers that are extended in the reaction are tethered to a substrate by their 5' ends. During amplification, the amplicons form a bridge between the tethered primers. Bridge PCR (which may also be referred to as "cluster PCR") is used in Illumina's Solexa platform. Bridge PCR and Illumina's Solexa platform are generally described in a variety of publications, e.g., Gudmundsson et al (Nat. Genet. 2009 41 : 1122-6), Out et al (Hum. Mutat. 2009 30: 1703-12) and Turner (Nat. Methods 2009 6:315-6), U.S. Pat. No. 7,115,400, and publication application publication nos. US20080160580 and US20080286795.

The term "barcode sequence" as used herein, generally refers to a unique sequence of nucleotides that can encode information about an assay. A barcode sequence can encode information relating to the identity of an interrogated allele, identity of a target polynucleotide or genomic locus, identity of a sample, a subject, or any combination thereof. A barcode sequence can be a portion of a primer, a reporter probe, or both. A barcode sequence may be at the 5'-end or 3'-end of an oligonucleotide, or may be located in any region of the oligonucleotide. A barcode sequence may or may not be part of a template sequence. Barcode sequences may vary widely in size and composition; the following references provide guidance for selecting sets of barcode sequences appropriate for particular embodiments: Brenner, U.S. Pat. No. 5,635,400; Brenner et al, Proc. Natl. Acad. Sci., 97: 1665-1670 (2000); Shoemaker et al, Nature Genetics, 14: 450-456 (1996); Morris et al, European patent publication 0799897A1; Wallace, U.S. Pat. No. 5,981,179. A barcode sequence may have a length of about 4 to 36 nucleotides, about 6 to 30 nucleotides, or about 8 to 20 nucleotides.

The terms "somatic genetic anomalies" relate to an alteration or mutation in DNA that occurs after conception. Somatic mutations can occur in any of the cells of the body except the germ cells (sperm and egg) and therefore are not passed on to children. These alterations can cause cancer or other diseases.

The term "mutation", as used herein, generally refers to a change of the nucleotide sequence of a genome. Mutations can involve large sections of DNA (e.g., copy number variation). Mutations can involve whole chromosomes (e.g., aneuploidy). Mutations can involve small sections of DNA. Examples of mutations involving small sections of DNA include, e.g., point mutations or single nucleotide polymorphisms, multiple nucleotide polymorphisms, insertions (e.g., insertion of one or more nucleotides at a locus), multiple nucleotide changes, deletions (e.g., deletion of one or more nucleotides at a locus), and inversions (e.g., reversal of a sequence of one or more nucleotides).

The term "locus", as used herein, can refer to a location of a gene, nucleotide, or sequence on a chromosome. An "allele" of a locus, as used herein, can refer to an alternative form of a nucleotide or sequence at the locus. A "wild-type allele" generally refers to an allele that has the highest frequency in a population of subjects. A "wild-type" allele generally is not associated with a disease. A "mutant allele" generally refers to an allele that has a lower frequency than a "wild-type allele" in the general population and may be associated with a disease. A "mutant allele" may not have to be associated with a disease. The term "interrogated allele" generally refers to the allele that an assay is designed to detect.

The term "single nucleotide polymorphism", or "SNP", as used herein, generally refers to a type of genomic sequence variation resulting from a single nucleotide substitution within a sequence. "SNP alleles" or "alleles of a SNP" generally refer to alternative forms of the SNP at particular locus. The term "interrogated SNP allele" generally refers to the SNP allele that an assay is designed to detect.

The term "copy number variation" or "CNV" refers to differences in the copy number of genetic information. In many aspects it refers to differences in the per genome copy number of a genomic region. For example, in a diploid organism the expected copy number for autosomal genomic regions is 2 copies per genome. Such genomic regions should be present at 2 copies per cell. For a recent review see Zhang et al. Annu. Rev. Genomics Hum. Genet. 2009. 10:451 - 81. CNV is a source of genetic diversity in humans and can be associated with complex disorders and disease, for example, by altering gene dosage, gene disruption, or gene fusion. They can also represent benign polymorphic variants. CNVs can be large, for example, larger than 1 Mb, but many are smaller, for example between 100 bases and 1 Mb. More than 38,000 CNVs greater than 100 bases (and less than 3 Mb) have been reported in humans. Along with SNPs these CNVs account for a significant amount of phenotypic variation between individuals. In addition to having deleterious impacts, e.g. causing disease, they may also result in advantageous variation.

In certain cases, an oligonucleotide used in the method described herein may be designed using a reference genomic region, i.e., a genomic region of known nucleotide sequence, e.g., a chromosomal region whose sequence is deposited at NCBI's Genbank database or other database, for example.

The term "genotyping", as used herein, generally refers to a process of determining differences in the genetic make-up (genotype) of an individual by examining the individual's DNA sequence using biological assays and comparing it to another individual's sequence or a reference sequence.

A "plurality" generally contains at least 2 members. In certain cases, a plurality may have at least 10, at least 100, at least 100, at least 10,000, at least 100,000, at least 1000000, at least 10000000, at least 100000000, or at least 1000000000 or more members.

The term "separating", as used herein, generally refers to physical separation of two elements (e.g., by cleavage, hydrolysis, or degradation of one of the two elements).

The terms "label" and "detectable moiety" can be used interchangeably herein to refer to any atom or molecule which can be used to provide a detectable signal, and which can be attached to a nucleic acid or protein. Labels may provide signals detectable by fluorescence, radioactivity, colorimetry, gravimetry, X-ray diffraction or absorption, magnetism, enzymatic activity, and the like.

### B- METHOD OF DETECTION

Aspects of the invention relate to a method, a combination of capture probes and kits comprising said combination of capture probes that improve the monitoring and treatment of a subject suffering from a disease, especially cancer.

The cancer can be a tumor, a leukemia such as acute leukemia, acute t-cell leukemia, acute lymphocytic leukemia, acute myelocytic leukemia, myeloblastic leukemia, promyelocytic leukemia, myelomonocytic leukemia, monocytic leukemia, erythroleukemia, chronic leukemia, chronic myelocytic (granulocytic) leukemia, or chronic lymphocytic leukemia, polycythemia vera, lymphomas such as Hodgkin's lymphoma, follicular lymphoma or non-Hodgkin's lymphoma, multiple myeloma, Waldenstrom's macroglobulinemia, heavy chain disease, solid tumors, sarcomas, carcinomas such as, e.g., fibrosarcoma, myxosarcoma, liposarcoma, chondrosarcoma, osteogenic sarcoma, lymphangio sarcoma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic, carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma, glioma, craniopharyngioma, ependymoma, pinealoma, hemangioblastoma, acoustic neuroma, oligodendroglioma, meningioma, melanoma, neuroblastoma, retinoblastoma, endometrial cancer, non-small cell lung cancer.

The subject can be suspected to or known to harbor a solid tumor, or can be a subject who previously harbored a solid tumor.

Especially, the present invention relates to a detection method of somatic genetic anomalies potentially present in a tissue sample, taken from a subject, wherein said detection method comprises the following steps:
(a) shearing of a genomic DNA extracted from said tissue sample, to obtain sheared genomic DNA;
(b) preparing a library by hybridization capture from the sheared genomic DNA obtained at step (a), by the implementation of at least 43 capture probes having at least 110 nucleotides in length and at least 75 % sequence homology with respectively SEQ ID NO:1 to SEQ ID NO:43, said capture probes being able to hybridize on fragments from at least 5 target regions selected from : ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA from the library issued at step (b), and
(d) analyzing of the sequences of said sheared genomic DNA obtained at step (c), for detecting somatic genetic anomalies in said tissue sample that may be cancerous.

Several embodiments of the sequencing methods and assays for determining mutational status of a sample are described herein.

### Sample

According to the invention, the starting sample can be a biological sample obtained from a subject. The biological samples may be a tumor sample and normal cells from the subject. In particular embodiments, the sample is a solid biological sample, e.g., a tumor sample.

In general, nucleic acid is isolated from the tumor sample and normal cells using any methods known in the art. The nucleic acid is DNA, and in particular genomic DNA..

The genomic DNA from the tumor sample and normal cells can be used to prepare a subject-specific tumor genomic DNA library and/or normal genomic DNA library. DNA libraries (and in particular genomic DNA library) can be used for sequencing by a sequencing platform

In some embodiments, the solid biological sample is processed prior to the probe-based assay. Processing can comprise fixation in a formalin solution, followed by embedding in paraffin (e.g., is a FFPE sample). Processing can alternatively comprise freezing of the sample prior to conducting the probe- based assay. In some embodiments, the sample is neither fixed nor frozen. The unfixed, unfrozen sample can be, by way of example only, stored in a storage solution configured for the preservation of nucleic acid. Exemplary storage solutions are described herein. In some embodiments, non-nucleic acid materials can be removed from the starting material using enzymatic treatments (for example, with a protease).

The sample can optionally be subjected to homogenization, sonication, French press, dounce, freeze/thaw, which can be followed by centrifugation. The centrifugation may separate nucleic acid-containing fractions from non- nucleic acid-containing fractions.

In some embodiments, the sample is a liquid biological sample. Exemplary liquid biological samples are described herein. In some embodiments, the liquid biological sample is a blood sample (e.g., whole blood, plasma, or serum). In some embodiments, a whole blood sample is subjected to acellular components (e.g., plasma, serum) and cellular components by use of a Ficoll reagent described in detail Fuss et al, Curr Protoc Immunol (2009) Chapter 7:Unit7.1, which is incorporated herein by reference.

Nucleic acid can be isolated from the biological sample using any means known in the art. For example, nucleic acid can be extracted from the biological sample using liquid extraction (e.g, Trizol, DNAzol) techniques. Nucleic acid can also be extracted using commercially available kits (e.g., Qiagen DNeasy kit, QIAamp kit, Qiagen Midi kit, QIAprep spin kit, Promega Maxwell kits...).

### Next generation sequencing platforms

The detection method of the invention comprises a sequencing step of sheared genomic DNA contained in a library obtained by hybridization capture with a specific combination of capture probes.

The sequencing platform can be a next-generation sequencing (NGS) platform. In some embodiments, the method further comprises sequencing the nucleic acid libraries using NGS technology. NGS technology can involve sequencing of clonally amplified DNA templates or single DNA molecules in a massively parallel fashion (e.g. as described in Volkerding et al. Clin Chem 55:641-658 [2009]; Metzker M Nature Rev 1 1:31-46 [2010]). In addition to high-throughput sequence information, NGS provides digital quantitative information, in that each sequence read is a countable "sequence tag" representing an individual clonal DNA template or a single DNA molecule.

The next-generation sequencing platform can be a commercially available platform. Commercially available platforms include, e.g., platforms for sequencing-by-synthesis, ion semiconductor sequencing, pyrosequencing, reversible dye terminator sequencing, sequencing by ligation, single-molecule sequencing, sequencing by hybridization, and nanopore sequencing. Platforms for sequencing by synthesis are available from, e.g., Illumina, 454 Life Sciences, Helicos Biosciences, and Qiagen. Illumina platforms can include, e.g., Illumina's Solexa platform, Illumina's Genome Analyzer, MiSeq, NextSeq500, Novaseq, HiSeq and are described in Gudmundsson et al (Nat. Genet. 2009 41 : 1122-6), Out et al (Hum. Mutat. 2009 30: 1703- 12) and Turner (Nat. Methods 2009 6:315-6), U.S. Patent Application Pub nos. US20080160580 and US20080286795, U.S. Patent Nos. 6306597, 7115400, and 7232656. 454 Life Science platforms include, e.g., the GS Flex and GS Junior, and are described in U.S. Patent No. 7,323,305. Platforms from Helicos Biosciences include the True Single Molecule Sequencing platform. Platforms for ion semiconductor sequencing include, e.g., the Ion Torrent Personal Genome Machine (PGM) and are described in U.S. Patent No. 7948015. Platforms for pryosequencing include the GS Flex 454 system and are described in U.S. Patent Nos. 7211390; 7244559; 7264929. Platforms and methods for sequencing by ligation include, e.g., the SOLiD sequencing platform and are described in U.S. Patent No. 5750341. Platforms for single-molecule sequencing include the SMRT system from Pacific Bioscience and the Helicos True Single Molecule Sequencing platform.

For instance, the DNA sequencing technology can utilize the Ion Torrent sequencing platform, which pairs semiconductor technology with a sequencing chemistry to directly translate chemically encoded information (A, C, G, T) into digital information (0, 1) on a semiconductor chip. Without wishing to be bound by theory, when a nucleotide is incorporated into a strand of DNA by a polymerase, a hydrogen ion is released as a byproduct. The Ion Torrent platform detects the release of the hydrogen atom as a change in pH. A detected change in pH can be used to indicate nucleotide incorporation. The Ion Torrent platform comprises a high-density array of micro-machined wells to perform this biochemical process in a massively parallel way. Each well holds a different library member, which may be clonally amplified. Beneath the wells is an ion-sensitive layer and beneath that an ion sensor. The platform sequentially floods the array with one nucleotide after another. When a nucleotide, for example a C, is added to a DNA template and is then incorporated into a strand of DNA, a hydrogen ion will be released. The charge from that ion will change the pH of the solution, which can be identified by Ion Torrent's ion sensor. If the nucleotide is not incorporated, no voltage change will be recorded and no base will be called. If there are two identical bases on the DNA strand, the voltage will be double, and the chip will record two identical bases called. Direct identification allows recordation of nucleotide incorporation in seconds. Library preparation for the Ion Torrent platform generally involves ligation of two distinct adaptors at both ends of a DNA fragment.

The DNA sequencing technology utilizes an Illumina sequencing platform, which generally employs cluster amplification of library members onto a flow cell and a sequencing-by-synthesis approach. Cluster-amplified library members are subjected to repeated cycles of polymerase-directed single base extension. Single-base extension can involve incorporation of reversible-terminator dNTPs, each dNTP labeled with a different removable fluorophore. The reversible-terminator dNTPs are generally 3' modified to prevent further extension by the polymerase. After incorporation, the incorporated nucleotide can be identified by fluorescence imaging. Following fluorescence imaging, the fluorophore can be removed and the 3' modification can be removed resulting in a 3' hydroxyl group, thereby allowing another cycle of single base extension. Library preparation for the Illumina platform generally involves ligation of two distinct adaptors at both ends of a DNA fragment.

Therefore, the present invention presents the advantage of being usable with the NGS available on the market. These next-generation sequencing platforms are known from the skilled person in the art and will not be further described herein.

The different steps of the present detection method will be described below.

### Step (a): Shearing of the genomic DNA

### Shearing/Fragmentation (i):

According to one aspect of the invention, the genomic DNA extracted (started DNA) from the biological sample is sheared/ fragmented/sized to the target sequences having a desired length.

The extracted genomic DNA may be sheared by any means known in the art, such as for example, by mechanical shearing, by nebulization, or by sonication.

In particular, three approaches are available to shear/fragment nucleic acid chains: physical, enzymatic, and chemical. DNA fragmentation is typically done by physical methods (i.e., acoustic shearing and sonication) or enzymatic methods (i.e., non-specific endonuclease cocktails and transposase tagmentation reactions).

For instance, acoustic shearing with a Covaris instrument (Covaris, Woburn, MA) is typically done to obtain DNA fragments in the 100-5000 bp range, while Covaris g-TUBEs are employed for the 6-20 Kbp range necessary for mate-pair libraries. Enzymatic methods include digestion by DNase I or Fragmentase, a two enzyme mix (New England Biolabs, Ipswich MA). An alternative enzymatic method for fragmenting DNA is Illumina's Nextera tagmentation technology (Illumina, San Diego, CA) in which a transposase enzyme simultaneously fragments and inserts adapter sequences into dsDNA. This method has several advantages, including reduced sample handling and preparation time.

### Subsequent steps of the pre-library preparation (ii) to (vi):

With the exception of Illumina's Nextera prep, steps before library preparation (b) generally entail: (*i*) shearing/fragmentation above-mentioned, (*ii*) end-repair, (*iii*) phosphorylation of the 5' prime ends, (*iv*) A-tailing of the 3' ends to facilitate ligation to sequencing adapters, *(v)* ligation of adapters, and (*vi*) some number of pre-PCR cycles to enrich for product that has adapters ligated to both ends.

For instance, the pre-PCR (sub-step vi) may be performed by using the following conditions: 1 cycle of 2 min at 98°C, 10 cycles of 30 sec at 98°C, followed by 30 sec at 60°C and 1 min at 72°C. The final elongation is 5 min at 72°C.

The primary differences in an Ion Torrent workflow are the use of blunt-end ligation to different adapter sequences.

In general, once the starting DNA has been sheared, the fragment ends are blunted and 5' phosphorylated using a mixture of three enzymes: T4 polynucleotide kinase, T4 DNA polymerase, and Klenow Large Fragment. Next, the 3' ends are A-tailed using either Taq polymerase or Klenow Fragment (exo-). Taq is more efficient at A-tailing, but Klenow (exo-) can be used for applications where heating is not desired, such as preparing mate-pair libraries. During the adapter ligation reaction the optimal adapter: fragment ratio is ∼10:1, calculated on the basis of copy number or molarity. Too much adapter favors formation of adapter dimers that can be difficult to separate and dominate in the subsequent PCR amplification. Bead or column-based cleanups can be performed after end repair and A-tail reactions, but after ligation we find bead-based cleanups are more effective at removing excess adapter dimers.

To facilitate multiplexing, different barcoded adapters can be used with each sample. Alternatively, barcodes can be introduced at the PCR amplification step by using different barcoded PCR primers to amplify different samples. High quality reagents with barcoded adapters and PCR primers are readily available in kits from many vendors.

An alternative method is the Nextera DNA Sample Prep Kit (Illumina), which prepares genomic DNA libraries by using a transposase enzyme to simultaneously fragment and tag DNA in a single-tube reaction termed "tagmentation". The engineered enzyme has dual activity; it fragments the DNA and simultaneously adds specific adapters to both ends of the fragments. These adapter sequences are used to amplify the insert DNA by PCR. The PCR reaction also adds index (barcode) sequences. The preparation procedure improves on traditional protocols by combining DNA fragmentation, end-repair, and adaptor-ligation into a single step. This protocol is very sensitive to the amount of DNA input compared with mechanical fragmentation methods. In order to obtain transposition events separated by the appropriate distances, the ratio of transposase complexes to sample DNA is critical. Because the fragment size is also dependent on the reaction efficiency, all reaction parameters, such as temperatures and reaction time, are critical and must be tightly controlled.

Preferably, the sheared genomic DNA obtained at step (a) has a length higher than or equal to 300 bp, preferably ranging from 300 to 600 bp, and ideally ranging from 300 to 350 bp

As used herein, a length higher than or equal to 300 bp includes the following values or any intervals between these values: 300; 310; 320; 330; 340; 350; 360; 370; 380; 390; 400; 410; 420; 430; 440; 450; 460; 470; 480; 490; 500; 510; 520; 530; 540; 550; 560; 570; 580; 590; 600; 610; 620; 630; etc.

### Step (b) Library preparation

According to an embodiment of the invention, the preparation of the library comprises
(b1) the hybridization of the sheared genomic DNA obtained at step (a) with a specific set of capture probes, so as to enrich the sheared genomic DNA contained in the library, and
(b2) the library amplification so as to increase the sheared genomic DNA contained in the library.

As used herein, the terms "library" is used herein and can refer to a plurality of the sheared genomic DNA obtained from a biological sample.

### Step b1 Hybridization of the sheared genomic DNA obtained at step (a)

This step is performed by using hybrid capture technology.

Especially, in hybrid capture, sheared DNA sequences obtained at step (a) are hybridized to the combination of capture probes according to the invention.

The combination of capture probes comprises at least 43 capture probes having at least 110 nucleotides in length and at least 75 % sequence homology with respectively SEQ ID NO:1 to SEQ ID NO:43, said capture probes being able to hybridize on fragments from at least 5 target regions selected from ALK (NM_004304.4), BRAF (NM_004333.4), EGFR (NM_005228.3), EIF1AY (NM_004681.3), ERBB2 (NM_004448.2), ERBB4 (NM_005235.2), KDR, KIT (NM_000222.2), KRAS(NM_033360.2), MAP2K1 *MAP2K1* (NM_002755.3), MET *MET* (NM_001127500.1), NRAS (NM_002524.4), PDGFRA *PDGFRA* (NM_006206.4), SLC28A1 (NM_004213.4), TP53 (NM_000546.5).

Each of the target regions is herein specified by their 3-letter gene symbol and their NCBI Reference Sequence.

In general, the combination of capture probes comprises further at least 4 additional capture probes comprising at least 110 nucleotides and at least 75 % sequence homology with respectively the probes of SEQ ID NO:44 to SEQ ID NO:47 and/or the probes of SEQ ID NO:48 to SEQ ID NO:51 or combination thereof.

Preferably, the combination of capture probes comprises further at least 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

In particular, the library preparation at step (b) comprises the implementation of:
- 43 capture probes having at least 75%, especially 80%, preferably 85%, in particular 90% and ideally 95%, and possibly 100% sequence homology with the capture probes of SEQ ID NO:1 to SEQ ID NO:43, respectively and/or;
- additional capture probes having at least 75%, especially 80%, preferably 85%, in particular 90% and ideally 95% and possibly 100% sequence homology with the capture probes of SEQ ID NO:44 to SEQ ID NO:47 and/or;
- additional capture probes having at least 75%, especially 80%, preferably 85%, in particular 90% and ideally 95% and possibly 100% sequence homology with the capture probes with the capture probes of SEQ ID NO:48 to SEQ ID NO:51 and/or;
- additional capture probes having at least 75%, especially 80%, preferably 85%, in particular 90% and ideally 95% and possibly 100% sequence homology with the capture probes with the capture probes of SEQ ID NO:52 to SEQ ID NO:1229, respectively.

In general, the library preparation at step (b) comprises the implementation of capture probes comprising from 110 to 150 nucleotides in length, preferably from 120 to 140 nucleotides in length and ideally from 125 to 135 nucleotides in length from SEQ ID NO: 1 to SEQ ID NO: 1229.

According to an embodiment of the invention, the combination of capture probes according to the invention is biotinylated. The biotin is bound to streptavidin beads and then non-bound DNA is washed away. This has the advantage of more reliable detection of copy number changes.

In general, the library preparation can comprise any method known in the art or as described herein which is suitable with the combination of capture probes according to the invention.

Preferably, the hybridization with said capture probes takes place at a temperature of at least 55°C, preferably at least at 58°C and preferably at a temperature ranging from 60°C to 70°C or from 58 to 65°C.

In general, the sheared genomic DNA captured at step (b) has a length higher than or equal to, 300 bp, preferably ranging from 300 to 600 bp, and ideally ranging from 300 to 350 bp.

After DNA shearing and purification with magnetic beads, sheared genomic DNA is end repaired for instance, thanks to specific polymerase enzyme at 20°C for 30 minutes. Next, DNA sample may be purified with magnetic beads and adenylated at 3' end thanks to a specific enzyme at 37°c for 30 minutes.

Afterwards, once genomic DNA fragments purified with magnetic beads, they may be ligated with adaptor oligo (specific of sequencing technology chosen) thanks to for instance a DNA ligase at 20°C for 15 minutes. Then, DNA fragments are purified for instance with magnetic beads and amplified by PCR with a very high fidelity polymerase (such as : 98°C 2 min, 6 to 10 cycles of 98°C 30 sec + 65°C 30 sec + 72°C 1 min, and 72°C 10 min).

After purification with magnetic beads, the totality of amplified genomic DNA may be hybridized with the probe cocktail (for instance, 95°C 5 min, then 65°C overnight).

Then, hybridized genomic DNA fragments may be captured thanks to streptavidin-coated magnetic beads. Finally, captured libraries may be amplified with indexing primers by PCR with a very high fidelity polymerase (such as: 98°C 2 min, 10 to 16 cycles of 98°C 30 sec + 57°C 30 sec + 72°C 1 min, and 72°C 10 min). Next, DNA sample may be purified with magnetic beads. Quantity and quality of indexed libraries are assessed by Tapestation, Qubit, Bioanalyzer or qPCR.

Step (b2) Library amplification Library amplification is advantageously made by PCR technology.

To facilitate multiplexing, different barcoded adapters can be used with each sample. Alternatively, barcodes can be introduced at the PCR amplification step by using different barcoded PCR primers to amplify different samples. High quality reagents with barcoded adapters and PCR primers are readily available in kits from many vendors.

### Step (c) Sequencing

Next, sheared genomic DNA, contained in the library issued at step (b), is sequenced.

In general, the target-enriched libraries are sequenced using any methods known in the art or as described herein in part "Next generation sequencing platforms". Sequencing can reveal the presence of mutations in one or more cancer-related genes in the set.

In some embodiments a subset of 5, 6, 7, 8, 9, 10, 15 genes harboring the mutations are selected for further monitoring by assessment of cell-free DNA in a fluid sample isolated from the subject at later time points.

In some embodiments a subset of no more than 5 genes harboring the mutations are selected for further monitoring by assessment of cell-free DNA in a fluid sample isolated from the subject at later time points.

### Step (d) analyzing of the sequences of said sheared genomic DNA obtained at step (c)

Analyzing step (d) mainly aims at detecting somatic genetic anomalies potentially present in said cancer tissue sample, taken from the subject.

The length of the sequence read can vary depending on the particular sequencing technology utilized. NGS platforms can provide sequence reads that vary in size from tens to hundreds, or thousands of base pairs. In some embodiments of the method described herein, the sequence reads are about 20 bases long, about 25 bases long, about 30 bases long, about 35 bases long, about 40 bases long, about 45 bases long, about 50 bases long, about 55 bases long, about 60 bases long, about 65 bases long, about 70 bases long, about 75 bases long, about 80 bases long, about 85 bases long, about 90 bases long, about 95 bases long, about 100 bases long, about 110 bases long, about 120 bases long, about 130, about 140 bases long, about 150 bases long, about 200 bases long, about 250 bases long, about 300 bases long, about 350 bases long, about 400 bases long, about 450 bases long, about 500 bases long, about 600 bases long, about 700 bases long, about 800 bases long, about 900 bases long, about 1000 bases long, or more than 1000 bases long.

Partial sequencing of DNA fragments present in the sample can be performed, and sequence tags comprising read that map to a known reference genome can be counted. Only sequence reads that uniquely align to the reference genome can be counted as sequence tags. In one embodiment, the reference genome is the human reference genome NCBI38/hg19 sequence, which is available on the world wide web at genome.ucsc.edu/. Other sources of public sequence information include GenBank, dbEST, dbSTS, EMBL (the European Molecular Biology Laboratory), and the DDBJ (the DNA Databank of Japan). The reference genome can also comprise the human reference genome NCBI38/hg19 sequence and an artificial target sequences genome, which includes polymorphic target sequences. In yet another embodiment, the reference genome is an artificial target sequence genome comprising polymorphic target sequences.

Mapping of the sequence tags can be achieved by comparing the sequence of the tag with the sequence of the reference genome to determine the chromosomal origin of the sequenced nucleic acid (e.g. cell free DNA) molecule, and specific genetic sequence information is not needed. A number of computer algorithms are available for aligning sequences, including without limitation BLAST (Altschul et al., 1990), BLITZ (MPsrch) (Sturrock & Collins, 1993), FASTA (Person & Lipman, 1988), BOWTIE (Langmead et al, Genome Biology 10:R25.1-R25.10 [2009]), or ELAND (Illumina, Inc., San Diego, CA, USA). In one embodiment, one end of the clonally expanded copies of the DNA molecule is sequenced and processed by bioinformatic alignment analysis for the Illumina Genome Analyzer, which uses the Efficient Large-Scale Alignment of Nucleotide Databases (ELAND) software. Additional software includes SAMtools {SAMtools, Bioinformatics, 2009, 25(16):2078-9), and the Burroughs-Wheeler block sorting compression procedure which involves block sorting or preprocessing to make compression more efficient.

The sequencing platforms described herein generally comprise a solid support immobilized thereon surface-bound oligonucleotides which allow for the capture and immobilization of sequencing library members to the solid support. Surface bound oligonucleotides generally comprise sequences complementary to the adaptor sequences of the sequencing library.

The analyzing of the sequences, at step (d), can be defined by two parameters: depth and coverage rate.

Depth is the number of reads that include a given nucleotide in the reconstructed sequence (in x). Deep sequencing refers to the general concept of aiming for high number of replicate reads of each region of a sequence.

The coverage rate is the zone covered by at least a read (in %).

Preferably, at the analyzing of the sequences (d), the average depth is of at least 200x, preferably ranging from 200 to 10000x, in particular ranging from 1000x to 1500x, and ideally ranging from 1000x to 1200x.

According to an embodiment, at the analyzing of the sequences (d), the coverage rate is of at least 90% at 200x, preferably ranging from 90% to 99.9% at 500x, in particular ranging from 95% to 99,9% at 500×, and ideally ranging from 98% to 99,9% at 500×.

The sequences of said sheared genomic DNA, obtained at step (c), are analyzed for detecting possible somatic genetic anomalies in said tissue sample.

This analysis can be achieved by well-known bioinformatics technics / pipelines, to identify somatic genetic anomalies (potentially tumorous / cancerous) in said sheared genomic DNA from the library in comparison to known somatic genetic anomalies or to normal genomes. These somatic genetic anomalies can consist in somatic single-base mutations (SSM), somatic insertion/deletion mutations (SIM) and larger structural changes (rearrangements and chromosome segment copy number changes).

### COMBINATION OF PROBES

The present invention concerns also a combination of capture probes, as specified hereabove, i.e. comprising at least 43 capture probes comprising at least 110 nucleotides in length and at least 75 % sequence homology with respectively the probes of SEQ ID NO:1 to SEQ ID NO:43.

In general, the combination of capture probes comprises further at least 4 additional capture probes comprising at least 110 nucleotides and at least 75 % sequence homology with respectively the probes of SEQ ID NO:44 to SEQ ID NO:47 and/or the probes of SEQ ID NO:48 to SEQ ID NO:51 or combination thereof.

Preferably, the combination of capture probes comprises further at least 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

Of course, all the characteristics described above for the detection method according to the invention are incorporated herein for the description of the combination of probes.

### KIT OF DETECTION

The present invention also refers to a kit of detection of somatic genetic anomalies potentially present in a tissue sample potentially cancerous taken from a subject, comprising the combination of capture probes as defined above.

Of course, all the characteristics described above for the detection method according to the invention or for the combination of probes are incorporated herein for the description of the detection kit.

In one embodiment, the kit according to the invention comprises further at least: nuclease-free water, 10× End Repair Buffer, dNTP Mix, T4 DNA Polymerase, Klenow DNA Polymerase, T4 Polynucleotide Kinase, 10x Klenow Polymerase Buffer, dATP, Exo(-) Klenow, 5x T4 DNA Ligase Buffer, SureSelect Adaptor Oligo Mix, T4 DNA Ligase, SureSelect Primer, SureSelect Illumina Indexing Pre-Capture PCR Reverse Primer, 5× Herculase II Reaction Buffer, 100 mM dNTP Mix, Herculase II Fusion DNA Polymerase, SureSelect Hyb 1, SureSelect Hyb 2, SureSelect Hyb 3, SureSelect Hyb 4, SureSelect Indexing Block 1, SureSelect Block 2, SureSelect Illumina Indexing Block 3, RNase Block solution, Capture Library, Dynabeads MyOne Streptavidin T1, Agencourt AMPure XP beads, plastic 96-well plates, or combination thereof.

In some embodiments, kits of the invention further include a packaging material. As used herein, the term "packaging material" can refer to a physical structure housing the components of the kit. The packaging material can maintain sterility of the kit components, and can be made of material commonly used for such purposes (e.g., paper, corrugated fiber, glass, plastic, foil, ampules, etc.). Kits can also include a buffering agent, a preservative, or a protein/nucleic acid stabilizing agent.

### DESCRIPTION OF THE FIGURES

The present invention will be hereafter illustrated without being limited thereto by means of the following examples. It will be referred to the following figures in the examples:

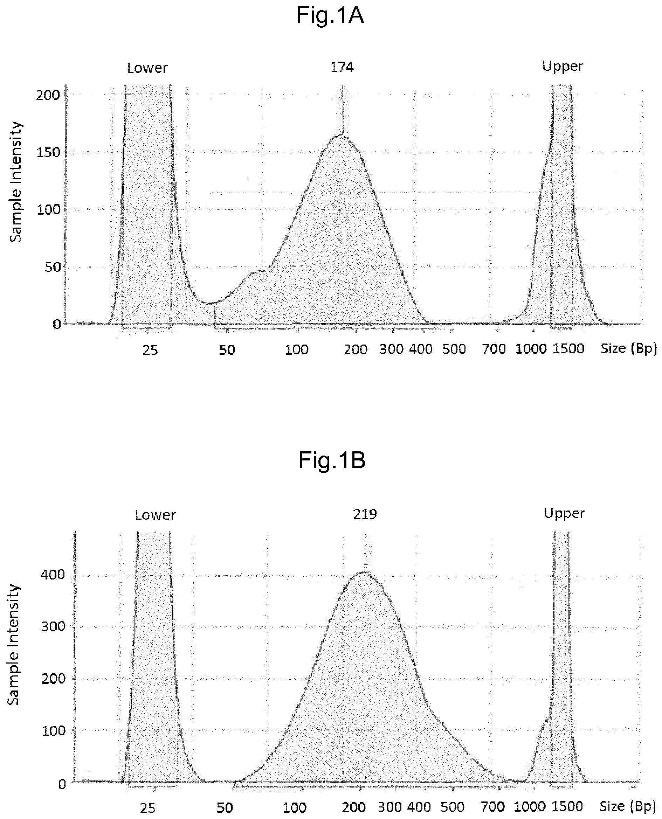

FIG. 1: FIG.1A shows the result obtained with the shearing conditions for the detection kit from FFPE tumor by using the instructions of the manufacturer's protocol and FIG.1B shows the result obtained with the shearing conditions for the detection kit from blood DNA by using the instructions of the manufacturer's protocol;

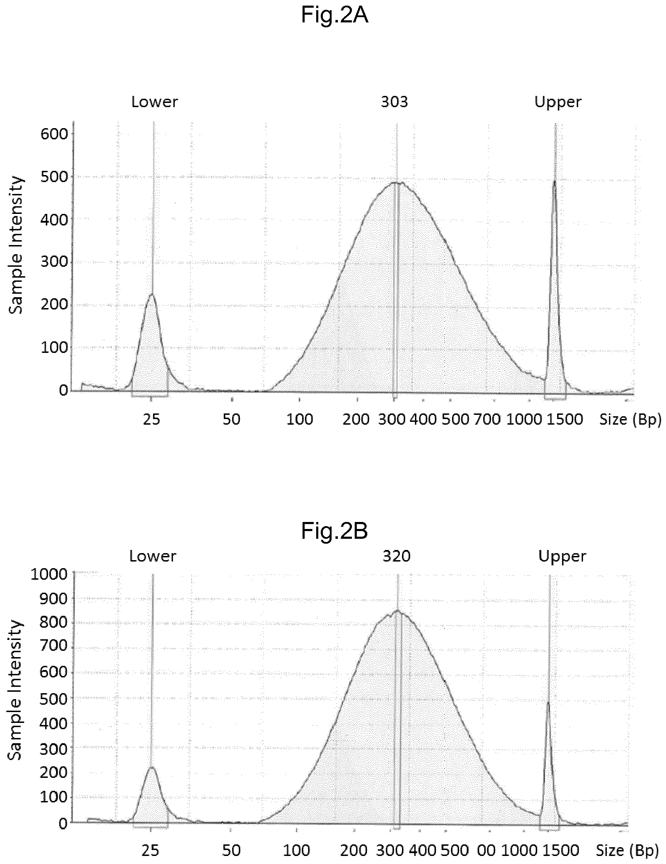

FIG. 2: FIG.2A shows the result obtained with the shearing conditions for the detection kit from FFPE tumor according to the invention by using the protocol described at paragraph 1.c below and FIG.2B shows the result obtained with the shearing conditions for the detection kit from blood DNA according to the invention by using the protocol described at paragraph 1.c below.

### EXAMPLES

### 1- Material and method

### a- Samples

For this assay, 100 samples (tumors, plasmas and commercial samples) were tested. Especially, samples B15 to B17 were obtained from archives of our laboratory, samples CHA, VIA, LOB, RIG, LEM, GAR, TOR, PEN, LUC, LED, POQ, 17BM00026 were obtained from plasma samples obtained in our hospital with STRECK tubes and commercial sample (V600E, G719S and G13D) was obtained from Horizon Discovery.

DNA was extracted from formalin-fixed paraffin-embedded tumors. Tumors were characterized by a pathologist to determine the tumor cell content and sent to the molecular biology platform for DNA extraction. Seven 15*µ*m tumor slices were extracted using the Maxwell 16 FFPE Plus LEV DNA purification kit (Promega, Madison, USA) according to the manufacturerordingcordingadison, USA) accord assessed by spectrophotometry with absorbance at 230, 260, and 280 nm. DNA was quantified using a fluorimetric assay with a Qubit device.

For plasma samples, 4 ml of plasma obtained from STRECK tubes were extracted with the QIAamp circulating nucleic acid kit (Qiagen, Hilden, Germany), by following the manufacturer's protocol.

### b- Materials/Equipment

For DNA shearing, about 200 ng of DNA were diluted in TE buffer and sonicated thanks to a S220 Covaris Sonicator (Covaris, Woburn, Mass, USA). Libraries were performed with a custom SureSelect XT kit (Agilent). Pre-capture libraries are concentrated with a vacuum concentrator Plus (Eppendorf, Hambourg, Germany). A MixMate device (Eppendorf), and a water bath are also used for capture.

Quality of libraries was assessed thanks to a Tapestation System (Agilent, Santa Clara, CA, USA). Libraries were sequenced on a MiSeq system (Illumina, San Diego, CA, USA) or a NextSeq500 system (Illumina).

Reagents used for the library preparation were Nuclease-free water, 10× End Repair Buffer, dNTP Mix, T4 DNA Polymerase, Klenow DNA Polymerase, T4 Polynucleotide Kinase, 10x Klenow Polymerase Buffer, dATP, Exo(-) Klenow, 5x T4 DNA Ligase Buffer, SureSelect Adaptor Oligo Mix, T4 DNA Ligase, SureSelect Primer, SureSelect Illumina Indexing Pre-Capture PCR Reverse Primer, 5× Herculase II Reaction Buffer, 100 mM dNTP Mix, Herculase II Fusion DNA Polymerase, SureSelect Hyb 1, SureSelect Hyb 2, SureSelect Hyb 3, SureSelect Hyb 4, SureSelect Indexing Block 1, SureSelect Block 2, SureSelect Illumina Indexing Block 3, RNase Block solution. All these reagents were purchased in the SureSelectXT kit. The Custom Capture Library kit is the object of the present invention. Dynabeads MyOne Streptavidin T1, Agencourt AMPure XP beads and plastic 96-well plates were also used for the preparation.

| **Tested probes according to the invention** | SEQ ID NO : 1 - 1229 |
|---|---|
| Probes according to the prior art (comparative examples) | The prior characterization of samples was performed by dedicated custom allelic discrimination kit (Applied Biosystems, Foster City, CA, USA), fragment analysis and Sanger sequencing (Applied Biosystems), TruSeq Custom Amplicon kit (Illumina), Tumor Hotspot MASTR Plus (Multiplicom, Niel, Belgium), or SureSelect XT Human All exon v5 (Agilent) |

### c- Hybrid-capture amplification assay

For these assays, the protocol used is described below. All indicated volumes are for 1 sample (well). These volumes must be adjusted with the number of samples handled at the same time.

### ✔ Assessment of gDNA (genomic DNA) Yield

Quantify freshly extracted gDNA before preparation of the library. Use the fluorometric yield assessment method to quantify intact gDNA (avoid using a spectrophotometer for gDNA yield assessment). Measure the (260/280 nm) ratio and ensure that it is between 1.8 and 2. NOTE: about 200 ng of gDNA are required for the experiment, but a lesser quantity can be used (for example for DNA extracted form plasma)

### ✔ gDNA fragmentation

Dilute about 200 ng DNA in a final volume of 50 µl of TE buffer. Next, sonicate in the Covaris device with the following program: Duty Factor = 10%, Peak Incident Power = 175, Cycles per Burst = 200, bath temperature = 4°C to 8°C, treatment time = 120 sec (for FFPE samples) or 180 sec (for fresh tumor samples) to obtain DNA fragment from 300 to 350 bp.

It is possible to check that your DNA fragments are from 300 to 350 bp thanks to a Bioanalyzer (Agilent) or a Tapestation (Agilent).

### ✔ End Repair

Mix well 35.2µl of Nuclease free water, 10µl 10x End Repair Buffer, 1.6µl dNTP, 1µl T4 DNA Polymerase, 2µl Klenow DNA polymerase, T4 Polynucleotide Kinase, and 50µl of sheared DNA. Incubate 30 min at 20°C and then hold à +4°C.

### ✔ Magnetic purification

Once magnetic beads (AMPure XP) come at room temperature for 30 min, add 180 µl of homogeneous beads to end repaired DNA samples. Mix well and incubate for 5 min at room temperature.

Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 32 µl of nuclease free water to each sample well. After sealing and mixing incubate 2 min at room temperature and replace the plate on the magnetic stand for 3 min. Transfer the supernatant (30µl) containing the purified DNA to a new plate well.

Purified DNA can be stored at -20°C.

### ✔ 3' adenylation

Mix well 11µl of Nuclease free water, 10µl 10x Klenow polymerase Buffer, 1µl dATP, 3µl Exo(-) Klenow, and 30 µl of purified end repaired DNA. Incubate 30 min at 37°C and then hold à +4°C. (If using thermocycler, do not use heated lid).

### ✔ Magnetic purification

Once magnetic beads (AMPure XP) come at room temperature for 30 min, add 90 µl of homogeneous beads to end repaired DNA samples. Mix well and incubates for 5 min at room temperature.

Then, put the plate on a magnetic stand for 5 min and carefully discard the supernatant without moving the plate from the stand.
Still without moving the plate from the stand, wash the beads twice with 200 µl of fresh prepared 70% ethanol for 1 min.

Completely dry the beads (possibly at 37°C for 3 to 5 min), and add 15 µl of nuclease free water to each sample well. After sealing and mixing, incubate 2 min at room temperature and replace the plate on the magnetic stand for 3 min.

Transfer the supernatant (13µl) containing the purified DNA to a new plate well, and proceed immediately the ligation of paired-end adaptor

### ✔ Paired-end adaptor ligation

Mix well 15.5µl of Nuclease free water, 10µl 5x T4 DNA ligase Buffer, 10µl of 1/10^{th} SureSelect Adaptor oligo, 1.5µl T4 DNA Ligase, and 13 µl of purified 3' adenyled DNA. Incubate 15 min at 20°C and then hold à +4°C (if using thermocycler, do not use heated lid).

The plate can be stored at -20°C.

### ✔ Magnetic purification

Once magnetic beads (AMPure XP) come at room temperature for 30 min, add 90 µl of homogeneous beads to end repaired DNA samples. Mix well and incubate for 5 min at room temperature.

Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 32 µl of nuclease free water to each sample well. After sealing and mixing incubate 2 min at room temperature and replace the plate on the magnetic stand for 3 min.

Transfer the supernatant (30µl) containing the purified DNA to a new plate well.

Purified DNA can be stored at -20°C.

### ✔ Amplification of adaptor-ligated library

Mix well 6µl of Nuclease free water, 1.25µl SureSelect primer, 1.25µl SureSelect Illumina indexing Pre-capture PCR reverse primer, 10µl 5x Herculase II Reaction buffer, 0.5µl 100mM dNTP Mix, 1 µl Herculase II Fusion DNA Polymerase and 30 µl of purified adaptor-ligated DNA.

Perform the following PCR program: 98°C for 2 min, 10 cycles of 98°C 30 sec + 65°C 30 sec + 72°C 1 min, 72°C 10 sec and then hold à +4°C.

### ✔ Magnetic purification

Once magnetic beads (AMPure XP) come at room temperature for 30 min, add 90 µl of homogeneous beads to end repaired DNA samples. Mix well and incubate for 5 min at room temperature. Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 30 µl of nuclease free water to each sample well. After sealing and mixing incubate 2 min at room temperature and replace the plate on the magnetic stand for 3 min. Transfer the supernatant (30µl) containing the purified DNA to a new plate well.

Purified DNA can be stored at -20°C.

It is possible to check that your DNA fragments are from 400 to 450 bp thanks to a Bioanalyzer (Agilent) or a Tapestation (Agilent).

### ✔ Hybridization of purified DNA with the capture library

Place the plate containing the purified amplified adaptor-ligated libraries into a vacuum concentrator and dehydrate libraries for 33 min at room temperature (a small volume of liquid, about 3 µl, should be visible).

During this time, prepare hybridization buffer containing: 6.63µl SureSelect Hyb 1, 0.27µl SureSelect Hyb 2, 2.65µl SureSelect Hyb 3, and 3.45µl SureSelect Hyb 4, mix well and keep at room temperature until use.

Next, prepare SureSelect block mix containing: 2.5µl SureSelect indexing block 1, 2.5µl SureSelect block 2, and 0.6µl SureSelect Illumina indexing block 3. Mix well and keep on ice until use.

Add 5.6µl of SureSelect block mix to the well containing DNA and mix by pipetting. Transfer the sealed plate in a thermocycler and incubate at 95°C for 5 min and hold at 65°C for at least 5 min (with the lid at 105°C).

During this time, prepare 5µl of a 10% dilution of RNase block solution on ice, and prepare the capture hybridization mix by mixing well 13µl hybridization buffer mixture prepared above, 5µl of 10% RNase block solution, and 2µl capture library. Keep at room temperature until use.

Once the at least 5 min at 65°C are finished, kept the plate in the thermocycler at 65°C and add 20µl of the capture hybridization mix to each well. Mix well by pipetting and seal the plate.

Incubate the plate overnight at 65°C with a heated lid.

### ✔ Capture of the hybridized DNA

Tomorrow morning, prewarm SureSelect wash buffer 2 at 65°C. Next, completely resuspend Dynabeads MyOne Streptavidin T1 magnetic beads (on vortex). Then, transfer 50µl of these beads into wells of a new PCR plate.

Add 200µl of SureSelect binding buffer to the 50µl beads and mix by pipetting. Then, put the plate on a magnetic stand for 5 min and carefully discard the supernatant without moving the plate from the stand. Wash again 2 more times with SureSelect binding buffer.

Finally, resuspend beads in 200µl SureSelect binding buffer.

By maintaining hybridization plate at 65°C, transfer the entire volume of each hybridization mixture to the plate containing 200µl of washed streptavidin beads, and mix well by pipetting.

Seal the plate and mix vigorously on a MixMate device at 1800 rpm for 30 min at room temperature.

After brief spinning, put the plate on a magnetic stand for 5 min and carefully discard the supernatant without moving the plate from the stand. Remove the plate from the stand and resuspend beads in 200µl of SureSelect wash buffer 1, mix by pipetting. Incubate 15 min at room temperature, briefly spin and put back the plate on a magnetic stand for 5 min. and carefully discard the supernatant without moving the plate from the stand. Remove the plate from the stand, resuspend the beads in 200µl of pre-warm (65°C) wash buffer 2, and mix by pipetting. Seal and incubate the plate in a waterbath at 65°C for 10 min.

Then, put the plate on a magnetic stand for 5 min and carefully discard the supernatant without moving the plate from the stand. Repeat this wash 2 more times.

After the third wash, remove all the supernatant and add 30µl of nuclease-free water to each sample, mix by pipetting, and keep the plate on ice until use.

### ✔ Amplification of the captured libraries

Mix well 18.5µl of Nuclease free water, 5µl 5x Herculase II Reaction buffer, 0.5µl 100mM dNTP Mix, 1 µl Herculase II Fusion DNA Polymerase, 1µl SureSelect Illumina indexing post-capture forward PCR primer and add 31 µl of this preparation to each sample. Next, add 5µl of the appropriate indexing primer to each sample well. Finally, transfer 14µl of the captured DNA into the reaction mix, mix by pipetting, seal the plate and transfer the plate in a thermocycler.

Run the following PCR program: 98°C for 2 min, 16 cycles of 98°C 30 sec + 57°C 30 sec + 72°C 1 min, 72°C 10 min and then hold à +4°C.

### ✔ Magnetic purification

Let magnetic beads (AMPure XP) come to room temperature for 30 min. Next, add 90 µl of homogeneous beads to end repaired DNA samples. Mix well and incubate for 5 min at room temperature. Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 30 µl of nuclease free water to each sample well. After sealing and mixing incubate 2 min at room temperature and replace the plate on the magnetic stand for 3 min. Transfer the supernatant (30µl) containing the purified library to a new plate well.

Purified library can be stored at -20°C for a long time.

### ✔ Quality and quantity assessment of library

Check the quality of library by a fragment analyzer. The library size should be around 500 bp.

Assess the quantity of DNA by using electrophoresis quantification or fluorimetric quantification.

Once library quantified, pool libraries and prepare for injection by following instruction of your sequence device.

### ✔ Sequencing

The sequencing can be performed with 120 to 150 bases for read 1, 9 cycles for index reads, and 120 to 150 bases for read 2.

### 2- Results

### a- Shearing conditions

By referring to Fig.1A to FIG.2B which show the shearing conditions according to the prior art and according to the invention, it can be seen that the shearing conditions according to the invention enables a good fragment pool down and a good quality of sequencing as compared

### b- Sensibility of the detection method of somatic genetic anomalies present in the samples

The results of the sensibility of the combination of capture probes according to the invention are illustrated in the tables below.

| **Origin** | **Sample number** | **Variations observed with classical methods** | **Variations observed with our capture protocol** |
|---|---|---|---|
| Tumor | B08.36 | *EGFR* : Deletion exon 19 | *EGFR* : Deletion exon 19 |
| Tumor | B08.227 | *EGFR* : Deletion exon 19 | *EGFR* : Deletion exon 19 |
| | | *EGFR* : Insertion exon 20 | *EGFR* : Insertion exon 20 |
| Tumor | B09.74 | *EGFR* : L858R | *EGFR* : L858R |
| Tumor | B10.165 | *EGFR* : Deletion exon 19 | *EGFR* : Deletion exon 19 |
| Tumor | B11.437 | *EGFR* : Deletion exon 19 | *EGFR* : Deletion exon 19 |
| Tumor | B12.621 | *EGFR* : Deletion exon 19 | *EGFR* : Deletion exon 19 |
| Tumor | B12.648 | *EGFR* : Deletion exon 19 | *EGFR* : Deletion exon 19 |
| Tumor | B13.425 | *EGFR* : Deletion exon 19 | *EGFR* : Deletion exon 19 |
| Tumor | B13.560 | *EGFR* : Deletion exon 19 | *EGFR* : Deletion exon 19 |
| | | | *EGFR* : S752F |
| Tumor | B14.488 | *EGFR* : Deletion exon 19 | *EGFR* : Deletion exon 19 |
| Tumor | B14.626 | *EGFR* : L858R | *EGFR* : L858R |
| Tumor | B15.133 | *EGFR* : Deletion exon 19 | *EGFR* : Deletion exon 19 |
| Tumor | B15.169 | *EGFR* : L858R | *EGFR* : L858R |
| | | *EGFR* : T790M | *EGFR* : T790M |
| Tumor | B15.305 | *EGFR* : Deletion exon 19 | *EGFR* : Deletion exon 19 |
| Tumor | B15.812 | *EGFR* : Insertion exon 20 | *EGFR* : Insertion exon 20 |
| | | | *EGFR* : H773Y |
| Tumor | B15.853 | *EGFR* : Deletion exon 19 | *EGFR* : Deletion exon 19 |
| Tumor | B15.878 | *EGFR* : Deletion exon 19 | *EGFR* : Deletion exon 19 |
| Tumor | B16.237 | *KRAS* : G12V | *KRAS* : G12V |
| Tumor | B16.298 | *EGFR* : Deletion exon 19 | *EGFR* : Deletion exon 19 |
| Tumor | B16.357 | *EGFR* : L858R | *EGFR* : L858R |
| Tumor | B17.12 | *KRAS* : G12S | *KRAS* : G12S |
| Tumor | B16.1251 | *KRAS* : G12V | *KRAS* : G12V |
| Tumor | B17.16 | *KRAS* : G12V | *KRAS* : G12V |
| Tumor | B17.14 | *KRAS* : G12C | *KRAS* : G12C |
| Tumor | B17.21 | *BRAF* : V600E | *BRAF* : V600E |
| Tumor | B 17.22 | *EGFR* : Deletion exon 19 | *EGFR* : Deletion exon 19 |
| Tumor | B17.27 | KIT: L576P | *KIT* : L576P |
| | | | ***KRAS* : G12D** |
| Tumor | B17.32 | *KRAS* : G12D | *KRAS* : G12D |
| Tumor | B17.36 | *KRAS* : G12V | *KRAS* : G12V |
| | | | ***EGFR* : H304Y** |
| Tumor | B12.55 | *EGFR* : L858R | *EGFR* : L858R |
| | | | ***EGFR* : D612G** |
| Tumor | B14.415 | *EGFR* : Deletion exon 19 | *EGFR* : Deletion exon 19 |
| Tumor | B14.750 | *EGFR* : L858R | *EGFR* : L858R |
| Tumor | B15.346 | *EGFR* : Deletion exon 19 | *EGFR* : Deletion exon 19 |
| Tumor | B15.849 | *EGFR* : Deletion exon 19 | *EGFR* : Deletion exon 19 |
| Tumor | B16.996 | *KRAS* : G12V | *KRAS* : G12V |
| Tumor | B16.1053 | *KRAS* : Q61 H | *KRAS* : Q61H |
| Tumor | B16.1056 | *NRAS* : Q61 H | *NRAS* : Q61H |
| Tumor | B16.1078 | *KIT* : V654A | *KIT* : V654A |
| | | *KIT* : Y823D | *KIT* : Y823D |
| Tumor | B16.1120 | *BRAF* : V600E | *BRAF* : V600E |
| Tumor | B16.1124 | *KRAS* : G12C | *KRAS* : G12C |
| Tumor | B16.1126 | *EGFR* : L858R | *EGFR* : L858R |
| Tumor | B16.1127 | *KRAS* : G12C | *KRAS* : G12C |
| Tumor | B16.1129 | *BRAF* : V600E | *BRAF* : V600E |
| Tumor | B16.1130 | *KRAS* : G13D | *KRAS* : G13D |
| Tumor | B16.1137 | *BRAF* : V600E | *BRAF* : V600E |
| Tumor | B16.1138 | *KRAS* : G13D | *KRAS* : G13D |
| Tumor | B16.1148 | *NRAS* : Q61 L | *NRAS* : Q61L |
| Tumor | B16.418 | *EGFR* : G719A | *EGFR* : G719A |
| Tumor | B16.430 | *EGFR* : L858R | *EGFR* : L858R |
| Tumor | B16.655 | *EGFR* : G719A | *EGFR* : G719A |
| Tumor | B16.990 | *ERBB*2 : insertion exon 20 | *ERBB*2 : insertion exon 20 |
| Tumor | B16.501 | *EGFR* : Deletion exon 19 | *EGFR* : Deletion exon 19 |
| Tumor | B16.1141 | *EGFR* : insertion exon 20 | *EGFR* : insertion exon 20 |
| Tumor | B16.1143 | *KRAS* : G12C | *KRAS* : G12C |
| Tumor | B16.1145 | *KRAS* : G12V | *KRAS* : G12V |
| Tumor | B16.1149 | *KRAS* : G13D | *KRAS* : G13D |
| Tumor | B16.1153 | *KRAS* : K117N | *KRAS* : K117N |
| Tumor | B16.1155 | *KRAS* : G13C | *KRAS* : G13C |
| Tumor | B16.1157 | *KRAS* : G12C | *KRAS* : G12C |
| Tumor | B17.41 | *KRAS* : G12A | *KRAS* : G12A |
| Tumor | B17.47 | *EGFR* : L858R | *EGFR* : L858R |
| Tumor | B17.48.1 BP | *KRAS* : G13C | *KRAS* : G13C |
| Tumor | B17.48.4DP | *KRAS* : Q61 L | *KRAS* : Q61 L |
| Tumor | B17.58 | *KRAS* : G12S | *KRAS* : G12S |
| Tumor | B17.59 | *BRAF* : V600E | *BRAF* : V600E |
| Tumor | B17.64 | *KRAS* : G12D | *KRAS* : G12D |
| Tumor | B17. 65 | *BRAF* : V600E | *BRAF* : V600E |
| Tumor | B17.60 | *EGFR* : L858R | *EGFR* : L858R |
| Tumor | B17.61 | *KRAS :* A146P | *KRAS* : A146P |
| Tumor | B17.67 | *BRAF* : V600K | *BRAF* : V600K |
| Tumor | B17.05 | No mutation | No mutation |
| Tumor | B17.11 | No mutation | No mutation |
| Tumor | B17.13 | No mutation | No mutation |
| Tumor | B17.15 | No mutation | No mutation |
| Tumor | B17.19 | No mutation | No mutation |
| Tumor | B17.20 | No mutation | No mutation |
| Tumor | B17.23 | No mutation | No mutation |
| Tumor | B17.05 | No mutation | No mutation |
| Tumor | B17.11 | No mutation | No mutation |
| Tumor | B16.1152 | No mutation | No mutation |
| Tumor | B15.342 | No mutation | ***EGFR* : E709G** |
| | | | ***EGFR* : G719C** |
| Tumor | B17.18 | No mutation | ***BRAF* : G596R** |
| Tumor | B15.820 | No mutation | ***EGFR* : T751 P** |
| Tumor | N16.296 | No mutation | ***EGFR* : S768I** |
| | | | ***EGFR* : V769L** |
| Tumor | B14.632 | No mutation | ***EGFR* : L858R** |
| Plasma | CHA | *KRAS* : G12C | **No mutation** |
| Plasma | VIA | *KRAS* : *G12S* | *KRA S* : G12S |
| Plasma | LOB | *EGFR* : G719A | *EGFR* : G719A |
| Plasma | RIG | No mutation | No mutation |
| Plasma | LEM | No mutation | No mutation |
| Plasma | GAR | *KRAS* : A146T | *KRAS* : A146T |
| Plasma | TOR | No mutation | No mutation |
| Plasma | PEN | No mutation | No mutation |
| Plasma | LUC | No mutation | No mutation |
| Plasma | LED | *KRAS* : G12C | *KRAS* : G12C |
| Plasma | POQ | *EGFR* : Deletion exon 19 | *EGFR* : Deletion exon 19 |
| | | | ***EGFR* : T790M** |
| Plasma | 17BM00026 | *EGFR* : Deletion exon 19 | *EGFR* : Deletion exon 19 |
| | | | ***EGFR* : T790M** |

| **Origin** | **Expected variations** | **Observed percentage run 1** | **Observed percentage run 2** | **Observed percentage run 3** |
|---|---|---|---|---|
| Commercial | BRAF : V600E | 9% | 9% | 7% |
| | EGFR : G719S | 18% | 20% | 20% |
| | KRAS : G13D | 23% | 26% | 26% |

It could be observed from the above tables that the combination of capture probes according to the invention enables to detect genetic anomalies with a high precision.

## Claims

1. Detection method of somatic genetic anomalies potentially present in a tissue sample, potentially cancerous, taken from a subject, wherein said detection method comprises the following steps:
(a) shearing of the genomic DNA extracted from said tissue sample, to obtain sheared genomic DNA;
(b) preparing a library by hybridization capture from the sheared genomic DNA obtained at step (a), by the implementation of at least 43 capture probes having at least 110 nucleotides in length and at least 75% sequence homology with respectively SEQ ID NO:1 to SEQ ID NO:43, said capture probes being able to hybridize on fragments from at least 5 target regions selected from : ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA contained in the library issued at step (b), and
(d) analyzing of the sequences of said sheared genomic DNA obtained at step (c), for detecting somatic genetic anomalies in said tissue sample.

2. Detection method according to claim 1, wherein the library preparation at step (b) comprises the implementation of further at least 4 additional capture probes comprising at least 110 nucleotides and at least 75 % sequence homology with respectively the probes of SEQ ID NO:44 to SEQ ID NO:47 or the probes of SEQ ID NO:48 to SEQ ID NO:51 or combination thereof.

3. Detection method according to claim 1 or to claim 2, wherein the library preparation at step (b) comprises the implementation of further at least 100 additional capture probes, preferably at least 200, in particular at least 300, and ideally at least 1178 additional capture probes comprising at least 110 nucleotides in length and at least 75 % sequence homology with SEQ ID NO: 52 to SEQ ID NO: 1229.

4. Detection method according to any one of the preceding claims, wherein the library preparation at step (b) comprises the implementation of:
- 43 capture probes having at least 75%, preferably 85%, in particular 90% and ideally 95%, sequence homology with the capture probes of SEQ ID NO:1 to SEQ ID NO:43;
- additional capture probes having at least 75%, preferably 85%, in particular 90% and ideally 95% and possibly 100% sequence homology with the capture probes of SEQ ID NO:44 to SEQ ID NO:47 and/or;
- additional capture probes having at least 75%, preferably 85%, in particular 90% and ideally 95% and possibly 100% sequence homology with the capture probes with the capture probes of SEQ ID NO:48 to SEQ ID NO:51 and/or;
- additional capture probes having at least 75%, preferably 85%, in particular 90% and ideally 95% and possibly 100% sequence homology with the capture probes with the capture probes of SEQ ID NO:52 to SEQ ID NO:1229, respectively.

5. Detection method according to any one of the preceding claims, wherein the library preparation at step (b) comprises the implementation of said capture probes comprising from 110 to 150 nucleotides in length, preferably from 120 to 140 nucleotides in length and ideally from 125 to 135 nucleotides in length.

6. Detection method according to any one of the preceding claims, wherein said capture probes are able to hybridize on fragments from at least 8, preferably from at least 10 and ideally from at least 15 target regions selected from : ALK, BRAF, EGFR, EIF1AY, ERBB2, ERBB4, KDR, KIT, KRAS, MAP2K1, MET, NRAS, PDGFRA, SLC28A1, TP53.

7. Detection method according to any one of the preceding claims, wherein the library preparation at step (b) comprises:
(b1) the hybridization of the sheared genomic DNA obtained at step (a) with the capture probes, so as to enrich the sheared genomic DNA contained in the library,
(b2) the library amplification.

8. Detection method according to claim 7, wherein step (b1) takes place at a temperature of at least 55°C, preferably at least at 58°C and preferably at a temperature ranging from 60°C to 70°C or from 58 to 65°C.

9. Detection method according to any one of the preceding claims, wherein the sheared genomic DNA obtained at step (a) and captured at step (b) have a length higher than or equal to 300 bp, preferably ranging from 300 to 600 pb, and ideally ranging from 300 to 350 pb.

10. Detection method according to any one of the preceding claims, wherein, at the analyzing of the sequences (d), the average depth is of at least 200x, preferably ranging from 200 to 10000x, in particular ranging from 500x to 1500x, and ideally ranging from 500x to 1200x.

11. Detection method according to any one of the preceding claims, wherein, at the analyzing of the sequences (d), the coverage rate is of at least 90% at 200x, preferably ranging from 90% to 99,9% at 500x, in particular ranging from 95% to 99,9% at 500x, and ideally ranging from 98% to 99,9% at 500x.

12. Detection method according to any one of the preceding claims, wherein the somatic genetic anomalies are linked to the cancer selected from at least one of these followings cancers: a tumor, a leukemia such as acute leukemia, acute t-cell leukemia, acute lymphocytic leukemia, acute myelocytic leukemia, myeloblastic leukemia, promyelocytic leukemia, myelomonocytic leukemia, monocytic leukemia, erythroleukemia, chronic leukemia, chronic myelocytic (granulocytic) leukemia, or chronic lymphocytic leukemia, polycythemia vera, lymphomas such as Hodgkin's lymphoma, follicular lymphoma or non-Hodgkin's lymphoma, multiple myeloma, Waldenstrom's macroglobulinemia, heavy chain disease, solid tumors, sarcomas, carcinomas such as, e.g., fibrosarcoma, myxosarcoma, liposarcoma, chondrosarcoma, osteogenic sarcoma, lymphangio sarcoma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic, carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma, glioma, craniopharyngioma, ependymoma, pinealoma, hemangioblastoma, acoustic neuroma, oligodendroglioma, meningioma, melanoma, neuroblastoma, retinoblastoma, endometrial cancer, non-small cell lung cancer, glioblastoma or lung cancer.

13. Combination of capture probes, comprising at least 43 capture probes comprising at least 110 nucleotides and at least 75 % sequence homology with respectively the probes of SEQ ID NO:1 to SEQ ID NO:43.

14. Combination of capture probes according to claim 13, comprising further at least:
- 4 additional capture probes comprising at least 110 nucleotides and at least 75 % sequence homology with respectively the probes of SEQ ID NO:44 to SEQ ID NO:47 or the probes of SEQ ID NO:48 to SEQ ID NO:51 or combination thereof; and/or
- 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

15. Kit of detection of somatic genetic anomalies potentially present in a tissue sample potentially cancerous taken from a subject, comprising the combination of capture probes as defined in claim 13 or claim 14.


SEQUENCE LISTING

<110>  Université de Bourgogne
       Centre Georges-François Leclerc

<120>  Detection method of somatic genetic anomalies, combination of
       capture probes and kit of detection

<130>  CB111EP

<160>  1229

<170>  PatentIn version 3.5

<210>  1
<211>  120
<212>  DNA
<213>  Artificial Sequence

<220>
<223>  ALK-E25

<400>  1
cagtctttac tcacctgtag atgtctcggg ccatcccgaa gtctccaatc ttggccactc      60

ttccagggcc tggacaggtc aagaggcagt ttctggcagc aatgtctctg ggaagaaagg     120


<210>  2
<211>  120
<212>  DNA
<213>  Artificial Sequence

<220>
<223>  ALK-E24

<400>  2
ctttgactca ccggtggatg aagtggtttt cctccaaata ctgacagcca caggcaatgt      60

cccgagccac gtgcagaagg tccagcatgg ccagggagga gggctggctc tgtggggaga     120


<210>  3
<211>  120
<212>  DNA
<213>  Artificial Sequence

<220>
<223>  ALK-E23

<400>  3
acaactgcag caaagactgg ttctcactca ccgggcgagg gcgggtctct cggaggaagg      60

acttgaggtc tccccccgcc atgagctcca gcaggatgaa ccggggcagg gattgcaggc     120


<210>  4
<211>  120
<212>  DNA
<213>  Artificial Sequence

<220>
<223>  ALK-E23

<400>  4
cactcaccgg gcgagggcgg gtctctcgga ggaaggactt gaggtctccc cccgccatga      60

gctccagcag gatgaaccgg ggcagggatt gcaggctcac cccaatgcag cgaacaatgt     120


<210>  5
<211>  120
<212>  DNA
<213>  Artificial Sequence

<220>
<223>  EGFR-E18

<400>  5
gtccccccca gcttgtggag cctcttacac ccagtggaga agctcccaac caagctctct      60

tgaggatctt gaaggaaact gaattcaaaa agatcaaagt gctgggctcc ggtgcgttcg     120


<210>  6
<211>  120
<212>  DNA
<213>  Artificial Sequence

<220>
<223>  EGFR-E18

<400>  6
ttacacccag tggagaagct cccaaccaag ctctcttgag gatcttgaag gaaactgaat      60

tcaaaaagat caaagtgctg ggctccggtg cgttcggcac ggtgtataag gtaaggtccc     120


<210>  7
<211>  120
<212>  DNA
<213>  Artificial Sequence

<220>
<223>  EGFR-E19

<400>  7
ctctgtcata gggactctgg atcccagaag gtgagaaagt taaaattccc gtcgctatca      60

aggaattaag agaagcaaca tctccgaaag ccaacaagga aatcctcgat gtgagtttct     120


<210>  8
<211>  120
<212>  DNA
<213>  Artificial Sequence

<220>
<223>  EGFR-E20

<400>  8
caggaagcct acgtgatggc cagcgtggac aacccccacg tgtgccgcct gctgggcatc      60

tgcctcacct ccaccgtgca gctcatcacg cagctcatgc ccttcggctg cctcctggac     120

<210>  9
<211>  120
<212>  DNA
<213>  Artificial Sequence

<220>
<223>  EGFR-E20

<400>  9
accgtgcagc tcatcacgca gctcatgccc ttcggctgcc tcctggacta tgtccgggaa      60

cacaaagaca atattggctc ccagtacctg ctcaactggt gtgtgcagat cgcaaaggta      120


<210>  10
<211>  120
<212>  DNA
<213>  Artificial Sequence

<220>
<223>  EGFR-E21

<400>  10
tttcagggca tgaactactt ggaggaccgt cgcttggtgc accgcgacct ggcagccagg      60

aacgtactgg tgaaaacacc gcagcatgtc aagatcacag attttgggct ggccaaactg      120


<210>  11
<211>  120
<212>  DNA
<213>  Artificial Sequence

<220>
<223>  EGFR-E21

<400>  11
ctggcagcca ggaacgtact ggtgaaaaca ccgcagcatg tcaagatcac agattttggg      60

ctggccaaac tgctgggtgc ggaagagaaa gaataccatg cagaaggagg caaagtaagg      120


<210>  12
<211>  120
<212>  DNA
<213>  Artificial Sequence

<220>
<223>  MET-E14

<400>  12
ctgtcttata tgtagtccat aaaacccatg agttctgggc actgggtcaa agtctcctgg      60

ggcccatgat agccgtcttt aacaagctct ttctttctct ctgttttaag atctgggcag      120


<210>  13
<211>  120
<212>  DNA
<213>  Artificial Sequence

<220>
<223>  MET-E14

<400>  13
ttttccagaa ggtatatttc agtttattgt tctgagaaat acctatacat atacctcagt      60

gggttgtgac attgttgttt atttttggtt ttgcatttat atttttataa aaacctaaag     120


<210>  14
<211>  120
<212>  DNA
<213>  Artificial Sequence

<220>
<223>  MET-E17

<400>  14
ctcaggaatc actgacatag gagaagtttc ccaatttctg accgagggaa tcatcatgaa      60

agattttagt catcccaatg tcctctcgct cctgggaatc tgcctgcgaa gtgaagggtc     120


<210>  15
<211>  120
<212>  DNA
<213>  Artificial Sequence

<220>
<223>  MET-E17

<400>  15
tcccaatgtc ctctcgctcc tgggaatctg cctgcgaagt gaagggtctc cgctggtggt      60

cctaccatac atgaaacatg gagatcttcg aaatttcatt cgaaatgaga ctcatgtaag     120


<210>  16
<211>  120
<212>  DNA
<213>  Artificial Sequence

<220>
<223>  MET-E18

<400>  16
actctctttg actgcagaat ccaactgtaa aagatcttat tggctttggt cttcaagtag      60

ccaaaggcat gaaatatctt gcaagcaaaa agtttgtcca cagagacttg gctgcaagaa     120


<210>  17
<211>  120
<212>  DNA
<213>  Artificial Sequence

<220>
<223>  MET-E18

<400>  17
ctgtaaaaga tcttattggc tttggtcttc aagtagccaa aggcatgaaa tatcttgcaa      60

gcaaaaagtt tgtccacaga gacttggctg caagaaactg tatgtaagta tcagaatctc     120

<210>  18
<211>  120
<212>  DNA
<213>  Artificial Sequence

<220>
<223>  MET-E19

<400>  18
gtcctttctg taggctggat gaaaaattca cagtcaaggt tgctgatttt ggtcttgcca      60

gagacatgta tgataaagaa tactatagtg tacacaacaa aacaggtgca aagctgccag     120


<210>  19
<211>  120
<212>  DNA
<213>  Artificial Sequence

<220>
<223>  gene MET-E19

<400>  19
ataaagaata ctatagtgta cacaacaaaa caggtgcaaa gctgccagtg aagtggatgg      60

ctttggaaag tctgcaaact caaaagttta ccaccaagtc agatgtggta atgtattggt     120


<210>  20
<211>  120
<212>  DNA
<213>  Artificial Sequence

<220>
<223>  MET-E20

<400>  20
ctagtggtcc tttggcgtgc tcctctggga gctgatgaca agaggagccc caccttatcc      60

tgacgtaaac acctttgata taactgttta cttgttgcaa gggagaagac tcctacaacc     120


<210>  21
<211>  120
<212>  DNA
<213>  Artificial Sequence

<220>
<223>  BRAF-E15

<400>  21
tggatccaga caactgttca aactgatggg acccactcca tcgagatttc actgtagcta      60

gaccaaaatc acctattttt actgtgaggt cttcatgaag aaatatatct gaggtgtagt     120


<210>  22
<211>  120
<212>  DNA
<213>  Artificial Sequence

<220>
<223>  HER2-E20

<400>  22
caggaagcat acgtgatggc tggtgtgggc tccccatatg tctcccgcct tctgggcatc        60

tgcctgacat ccacggtgca gctggtgaca cagcttatgc cctatggctg cctcttagac       120


<210>  23
<211>  120
<212>  DNA
<213>  Artificial Sequence

<220>
<223>  HER2-E20

<400>  23
acggtgcagc tggtgacaca gcttatgccc tatggctgcc tcttagacca tgtccgggaa        60

aaccgcggac gcctgggctc ccaggacctg ctgaactggt gtatgcagat tgccaaggta       120


<210>  24
<211>  120
<212>  DNA
<213>  Artificial Sequence

<220>
<223>  PDGFRA-E12

<400>  24
ttatagaaac cgaggtatga aattcgctgg agggtcattg aatcaatcag cccagatgga        60

catgaatata tttatgtgga cccgatgcag ctgccttatg actcaagatg ggagtttcca       120


<210>  25
<211>  120
<212>  DNA
<213>  Artificial Sequence

<220>
<223>  PDGFRA-E12

<400>  25
cgctggaggg tcattgaatc aatcagccca gatggacatg aatatattta tgtggacccg        60

atgcagctgc cttatgactc aagatgggag tttccaagag atggactagt gcttggtaag       120


<210>  26
<211>  120
<212>  DNA
<213>  Artificial Sequence

<220>
<223>  PDGFRA-E14

<400>  26
aacagccacg gccagatcca gtgaaaaaca agctctcatg tctgaactga agataatgac        60

tcacctgggg ccacatttga acattgtaaa cttgctggga gcctgcacca agtcaggtgg       120

<210> 27
<211> 120
<212> DNA
<213> Artificial Sequence

<220>
<223> PDGFRA-E18

<400> 27
tttccatgca gtgtgtccac cgtgatctgg ctgctcgcaa cgtcctcctg gcacaaggaa 60

aaattgtgaa gatctgtgac tttggcctgg ccagagacat catgcatgat tcgaactatg 120


<210> 28
<211> 120
<212> DNA
<213> Artificial Sequence

<220>
<223> PDGFRA-E18

<400> 28
atctggctgc tcgcaacgtc ctcctggcac aaggaaaaat tgtgaagatc tgtgactttg 60

gcctggccag agacatcatg catgattcga actatgtgtc gaaaggcagt gtacgtcctc 120


<210> 29
<211> 120
<212> DNA
<213> Artificial Sequence

<220>
<223> KIT-E9

<400> 29
cttcccttta gatgctctgc ttctgtactg ccagtggatg tgcagacact aaactcatct 60

gggccaccgt ttggaaagct agtggttcag agttctatag attctagtgc attcaagcac 120


<210> 30
<211> 120
<212> DNA
<213> Artificial Sequence

<220>
<223> KIT-E9

<400> 30
atagattcta gtgcattcaa gcacaatggc acggttgaat gtaaggctta caacgatgtg 60

ggcaagactt ctgcctattt taactttgca tttaaaggta acaacaaagg tatatttctt 120


<210> 31
<211> 120
<212> DNA
<213> Artificial Sequence

<220>
<223> KIT-E11

<400>  31
tccccacaga aacccatgta tgaagtacag tggaaggttg ttgaggagat aaatggaaac      60

aattatgttt acatagaccc aacacaactt ccttatgatc acaaatggga gtttcccaga     120


<210>  32
<211>  120
<212>  DNA
<213>  Artificial Sequence

<220>
<223>  KIT-E11

<400>  32
gtacagtgga aggttgttga ggagataaat ggaaacaatt atgtttacat agacccaaca      60

caacttcctt atgatcacaa atgggagttt cccagaaaca ggctgagttt tggtcagtat     120


<210>  33
<211>  120
<212>  DNA
<213>  Artificial Sequence

<220>
<223>  KIT-E13

<400>  33
tttagcgagt gcccatttga cagaacggga agccctcatg tctgaactca aagtcctgag      60

ttaccttggt aatcacatga atattgtgaa tctacttgga gcctgcacca ttggaggtaa     120


<210>  34
<211>  120
<212>  DNA
<213>  Artificial Sequence

<220>
<223>  KIT-E14

<400>  34
ctatatatct caccttcttt ctaacctttt cttatgtgct tttagggccc accctggtca      60

ttacagaata ttgttgctat ggtgatcttt tgaatttttt gagaagaaaa cgtgattcat     120


<210>  35
<211>  120
<212>  DNA
<213>  Artificial Sequence

<220>
<223>  KIT-E14

<400>  35
ttatttgttc aaagcaggaa gatcatgcag aagctgcact ttataagaat cttctgcatt      60

caaaggagtc ttcctggtaa gactgattta cataaatagt tagctgttga caggcagttc     120

<210>  36
<211>  120
<212>  DNA
<213>  Artificial Sequence

<220>
<223>  KIT-E17

<400>  36
caacctaata gtgtattcac agagacttgg cagccagaaa tatcctcctt actcatggtc      60

ggatcacaaa gatttgtgat tttggtctag ccagagacat caagaatgat tctaattatg      120


<210>  37
<211>  120
<212>  DNA
<213>  Artificial Sequence

<220>
<223>  KIT-E17

<400>  37
acttggcagc cagaaatatc ctccttactc atggtcggat cacaaagatt tgtgattttg      60

gtctagccag agacatcaag aatgattcta attatgtggt taaaggaaac gtgagtaccc      120


<210>  38
<211>  120
<212>  DNA
<213>  Artificial Sequence

<220>
<223>  KRAS-E4

<400>  38
ttacctgtct tgtctttgct gatgtttcaa taaaaggaat tccataactt cttgctaagt      60

cctgagcctg ttttgtgtct actgttctag aaggcaaatc acatttattt cctactagga      120


<210>  39
<211>  120
<212>  DNA
<213>  Artificial Sequence

<220>
<223>  KRAS-E3

<400>  39
ggcaaataca caaagaaagc cctccccagt cctcatgtac tggtccctca ttgcactgta      60

ctcctcttga cctgctgtgt cgagaatatc caagagacag gtttctccat caattactac      120


<210>  40
<211>  120
<212>  DNA
<213>  Artificial Sequence

<220>
<223>  KRAS-E2

<400>  40
acaagattta cctctattgt tggatcatat tcgtccacaa aatgattctg aattagctgt      60

atcgtcaagg cactcttgcc tacgccacca gctccaacta ccacaagttt atattcagtc     120


<210>  41
<211>  120
<212>  DNA
<213>  Artificial Sequence

<220>
<223>  NRAS-E4

<400>  41
accatacctg tctggtcttg gctgaggttt caatgaatgg aatcccgtaa ctcttggcca      60

gttcgtgggc ttgttttgta tcaactgtcc ttgttggcaa atcacacttg tttcccacta     120


<210>  42
<211>  120
<212>  DNA
<213>  Artificial Sequence

<220>
<223>  NRAS-E3

<400>  42
ggcaaataca cagaggaagc cttcgcctgt cctcatgtat tggtctctca tggcactgta      60

ctcttcttgt ccagctgtat ccagtatgtc caacaaacag gtttcaccat ctataaccac     120


<210>  43
<211>  120
<212>  DNA
<213>  Artificial Sequence

<220>
<223>  NRAS-E2

<400>  43
ggcctcacct ctatggtggg atcatattca tctacaaagt ggttctggat tagctggatt      60

gtcagtgcgc ttttcccaac accacctgct ccaaccacca ccagtttgta ctcagtcatt     120


<210>  44
<211>  120
<212>  DNA
<213>  Artificial Sequence

<220>
<223>  MAP2K1-E2

<400>  44
gaagaagctg gaggagctag agcttgatga gcagcagcga aagcgccttg aggcctttct      60

tacccagaag cagaaggtgg gagaactgaa ggatgacgac tttgagaaga tcagtgagct     120

<210>  45
<211>  120
<212>  DNA
<213>  Artificial Sequence

<220>
<223>  HER4-E10

<400>  45
cagtttaaaa aattacctgt tatctctctg actgtccgaa agacgttcag tttctctggg      60

tctatggctt caattgcatt gtaagggtcc ctagaaaatc aagaagagat gtagccaaat      120


<210>  46
<211>  120
<212>  DNA
<213>  Artificial Sequence

<220>
<223>  HER4-E12

<400>  46
gtacttacta caattttcag cttttctgtt gtcccggatt actattctct ggttgattgt      60

gctgaagagt gttgtccagt taatggtatg ataataacac aggttgctgt tgtcagtaat      120


<210>  47
<211>  120
<212>  DNA
<213>  Artificial Sequence

<220>
<223>  HER4-E12

<400>  47
acacaggttg ctgttgtcag taatatagat gtttcctgcg ctgatttcct tcagggactg      60

gaactgtaga gaggtgatgc cctgttgctt gaggataagc aaggacaggc cactaaggag      120


<210>  48
<211>  120
<212>  DNA
<213>  Artificial Sequence

<220>
<223>  TP53-E4

<400>  48
gggggctggt gcaggggccg ccggtgtagg agctgctggt gcaggggcca cggggggagc      60

agcctctggc attctgggag cttcatctgg acctgggtct tcagtgaacc attgttcaat      120


<210>  49
<211>  120
<212>  DNA
<213>  Artificial Sequence

<220>
<223>  KDR-E11

<400>  49
taacttcaat tttatttcct ccctggaagt cctccacact tctccattct tcacaagggt        60

atgggtttgt cactgagaca gcttggctat aagaaagaga taacagcgca tattatgatt       120


<210>  50
<211>  120
<212>  DNA
<213>  Artificial Sequence

<220>
<223>  SLC28A1

<400>  50
tgtggctgtc tctggacacc tcccagcggc ctgagcaact ggtgtccttc gcaggaatct        60

gcgtgttcgt cgctctcctc tttgcctgct caaagcatca ttgcgcagtg agtgctagtt       120


<210>  51
<211>  120
<212>  DNA
<213>  Artificial Sequence

<220>
<223>  EIF1AY

<400>  51
ctcctcagag tatgctcagg taatcaaaat gttgggaaat ggacgattgg aagcattgtg        60

ttttgatggt gtaaagaggt tatgccatat cagagggaaa ttgagaaaaa aggtaggtgt       120


<210>  52
<211>  120
<212>  DNA
<213>  Artificial Sequence

<220>
<223>  EIF1AY

<400>  52
ttagcaaagt tttaactatg ggattatttt taacaaacaa ttgtgttttc tttttcttaa        60

agacaaacac aatgcatact tactgccgaa agcttgacaa gattaaaata agtccctcat       120


<210>  53
<211>  120
<212>  DNA
<213>  Artificial Sequence

<220>
<223>  EIF1AY

<400>  53
tatttttaac aaacaattgt gttttctttt tcttaaagac aaacacaatg catacttact        60

gccgaaagct tgacaagatt aaaataagtc cctcatgaca ccatcaaaga gaatatgcac       120

<210>  54
<211>  120
<212>  DNA
<213>  Artificial Sequence

<220>
<223>  EIF1AY

<400>  54
tctttttctt aaagacaaac acaatgcata cttactgccg aaagcttgac aagattaaaa      60

taagtccctc atgacaccat caaagagaat atgcactgtt gtaaagcctg agtattttac      120


<210>  55
<211>  120
<212>  DNA
<213>  Artificial Sequence

<220>
<223>  EIF1AY

<400>  55
tgcatactta ctgccgaaag cttgacaaga ttaaaataag tccctcatga caccatcaaa      60

gagaatatgc actgttgtaa agcctgagta ttttacttgg cagctatttt cattatttat      120


<210>  56
<211>  120
<212>  DNA
<213>  Artificial Sequence

<220>
<223>  EIF1AY

<400>  56
acaagattaa aataagtccc tcatgacacc atcaaagaga atatgcactg ttgtaaagcc      60

tgagtatttt acttggcagc tattttcatt atttatcata ttgcatttta tgaaaagatt      120


<210>  57
<211>  120
<212>  DNA
<213>  Artificial Sequence

<220>
<223>  EIF1AY

<400>  57
gacaccatca aagagaatat gcactgttgt aaagcctgag tattttactt ggcagctatt      60

ttcattattt atcatattgc attttatgaa aagattttta tataaacatg aagatcttga      120


<210>  58
<211>  120
<212>  DNA
<213>  Artificial Sequence

<220>
<223>  EIF1AY

<400>  58
tgttgtaaag cctgagtatt ttacttggca gctattttca ttatttatca tattgcattt      60

tatgaaaaga tttttatata aacatgaaga tcttgatgaa attattggca tttcaggaag     120


<210>  59
<211>  120
<212>  DNA
<213>  Artificial Sequence

<220>
<223>  EIF1AY

<400>  59
ttggcagcta ttttcattat ttatcatatt gcattttatg aaaagatttt tatataaaca      60

tgaagatctt gatgaaatta ttggcatttc aggaagtgct gaaatgttat tggaagtgat     120


<210>  60
<211>  120
<212>  DNA
<213>  Artificial Sequence

<220>
<223>  EIF1AY

<400>  60
catattgcat tttatgaaaa gatttttata taaacatgaa gatcttgatg aaattattgg      60

catttcagga agtgctgaaa tgttattgga agtgatgaaa ttattggcat ttcaggaagt     120


<210>  61
<211>  120
<212>  DNA
<213>  Artificial Sequence

<220>
<223>  EIF1AY

<400>  61
tgcatcttag ttcagtcggc tcttagagta gtaaccgcca gaaaggagtc ggaagaggtc      60

tcacgaggct gtcatcaccg ccatgcccaa gaataaaggt actgctgtaa gcctctggga     120


<210>  62
<211>  120
<212>  DNA
<213>  Artificial Sequence

<220>
<223>  EIF1AY

<400>  62
tcacgaggct gtcatcaccg ccatgcccaa gaataaaggt actgctgtaa gcctctggga      60

ctatacctcg gcttgctctg ccagtaaccc cgacgcctgt tccaggccgc agtgactgtt     120

<210>  63
<211>  120
<212>  DNA
<213>  Artificial Sequence

<220>
<223>  EIF1AY

<400>  63
tatgtagatt tttacattaa ttttttatct actcttttaa ttaattaggt aaaggaggta      60

aaaacaggcg caggggtaaa aatgagaatg aatctgaaaa aagagagttg gtgtttaaag      120


<210>  64
<211>  120
<212>  DNA
<213>  Artificial Sequence

<220>
<223>  EIF1AY

<400>  64
tttcagtcat tacattttaa caaatgtgga ccaagatctc aacctttttt tttatctcct      60

ctcctcagag tatgctcagg taatcaaaat gttgggaaat ggacgattgg aagcattgtg      120


<210>  65
<211>  120
<212>  DNA
<213>  Artificial Sequence

<220>
<223>  EIF1AY

<400>  65
ttttgatggt gtaaagaggt tatgccatat cagagggaaa ttgagaaaaa aggtaggtgt      60

gtaggttact tttcaataaa aatttgccgc aaaaaatgtc tctgctttaa atacatggtc      120


<210>  66
<211>  120
<212>  DNA
<213>  Artificial Sequence

<220>
<223>  EIF1AY

<400>  66
tttttatttt taaaataatc tgcagtaaat ttcttacagg tttggataaa tacatcagac      60

attatattgg ttggtctacg ggactatcag gtaaaaataa catttaaagt tgtggtatgt      120


<210>  67
<211>  120
<212>  DNA
<213>  Artificial Sequence

<220>
<223>  EIF1AY

<400>  67
attatattgg ttggtctacg ggactatcag gtaaaaataa catttaaagt tgtggtatgt      60

ctgtgtttaa gcagttgtta atgtttggaa ggtaactata ctagcatctt tgacccattc     120


<210>  68
<211>  120
<212>  DNA
<213>  Artificial Sequence

<220>
<223>  EIF1AY

<400>  68
gtgatatttt ggtataagac gttttgaaag ttatttgttt atttctaagg ataacaaagc      60

tgatgtaatt ttaaagtaca atgcagatga agctagaagc ctgaaggcat atggcgagct     120


<210>  69
<211>  120
<212>  DNA
<213>  Artificial Sequence

<220>
<223>  EIF1AY

<400>  69
tgatgtaatt ttaaagtaca atgcagatga agctagaagc ctgaaggcat atggcgagct      60

tccagaacat ggtaagatca aaatgatttt atctcctcat tatttgatat taatgtttgt     120


<210>  70
<211>  120
<212>  DNA
<213>  Artificial Sequence

<220>
<223>  EIF1AY

<400>  70
gactacaggt tatataatca gtatgtctgt tattttacct ttagctaaaa tcaatgaaac      60

agacacattt ggtcctggag atgatgatga aatccagttt gacgatattg gagatgatga     120


<210>  71
<211>  120
<212>  DNA
<213>  Artificial Sequence

<220>
<223>  EIF1AY

<400>  71
agacacattt ggtcctggag atgatgatga aatccagttt gacgatattg gagatgatga      60

tgaagacatt gatgatgtaa gtaaataaac ctaacaggta ttttgtttta tctatgcatt     120

<210>  72
<211>  120
<212>  DNA
<213>  Artificial Sequence

<220>
<223>  EIF1AY

<400>  72
cagtttacca ctgggagtct ttatatgcag tagaatgttg taataagagt tatttttcta      60

tggatctgat gttcttttca attacagatc taaattgaac caagtgtttt tacatgacaa      120


<210>  73
<211>  120
<212>  DNA
<213>  Artificial Sequence

<220>
<223>  EIF1AY

<400>  73
tggatctgat gttcttttca attacagatc taaattgaac caagtgtttt tacatgacaa      60

gttctctgag gatggttcta cagttgggat tttggccatc atcaaccaag aagagaaatt      120


<210>  74
<211>  120
<212>  DNA
<213>  Artificial Sequence

<220>
<223>  EIF1AY

<400>  74
aaaacaggcg caggggtaaa aatgagaatg aatctgaaaa aagagagttg gtgtttaaag      60

aggatggaca aggtaagact tttcaactta ggcttctttc attaatatgt tttgttgttg      120


<210>  75
<211>  120
<212>  DNA
<213>  Artificial Sequence

<220>
<223>  SLC28A1

<400>  75
gtgtgtgggt ggggtggggg tgcaatgctg agggtaatgg ctttctgtct cttgcctgca      60

ggggtctagc tcttgctgct ttcctgggcc tggtcctgtg gctgtctctg gacacctccc      120


<210>  76
<211>  120
<212>  DNA
<213>  Artificial Sequence

<220>
<223>  SLC28A1

<400>  76
atgctgaggg taatggcttt ctgtctcttg cctgcagggg tctagctctt gctgctttcc        60

tgggcctggt cctgtggctg tctctggaca cctcccagcg gcctgagcaa ctggtgtcct       120


<210>  77
<211>  120
<212>  DNA
<213>  Artificial Sequence

<220>
<223>  SLC28A1

<400>  77
ctcttgcctg caggggtcta gctcttgctg ctttcctggg cctggtcctg tggctgtctc        60

tggacacctc ccagcggcct gagcaactgg tgtccttcgc aggaatctgc gtgttcgtcg       120


<210>  78
<211>  120
<212>  DNA
<213>  Artificial Sequence

<220>
<223>  SLC28A1

<400>  78
ttgctgcttt cctgggcctg gtcctgtggc tgtctctgga cacctcccag cggcctgagc        60

aactggtgtc cttcgcagga atctgcgtgt tcgtcgctct cctctttgcc tgctcaaagc       120


<210>  79
<211>  120
<212>  DNA
<213>  Artificial Sequence

<220>
<223>  SLC28A1

<400>  79
agcggcctga gcaactggtg tccttcgcag gaatctgcgt gttcgtcgct ctcctctttg        60

cctgctcaaa gcatcattgc gcagtgagtg ctagttgtgg ggcccagggc tggaagtgtt       120


<210>  80
<211>  120
<212>  DNA
<213>  Artificial Sequence

<220>
<223>  SLC28A1

<400>  80
tcgcaggaat ctgcgtgttc gtcgctctcc tctttgcctg ctcaaagcat cattgcgcag        60

tgagtgctag ttgtggggcc cagggctgga agtgtttgcc tctctcttct gcccctaggc       120

<210>  81
<211>  120
<212>  DNA
<213>  Artificial Sequence

<220>
<223>  SLC28A1

<400>  81
tgggcgctcc ctgatttgtc tttcagctgg aaggtctggg acatggagaa cgacccctcg      60

agacgaagag agtccatctc tctcacacct gtggccaagg gtctggagaa catgggggct      120


<210>  82
<211>  120
<212>  DNA
<213>  Artificial Sequence

<220>
<223>  SLC28A1

<400>  82
agacgaagag agtccatctc tctcacacct gtggccaagg gtctggagaa catgggggct      60

gatttcttgg aaagcctggt ctgtaccctt ccccatcagt catcctgacc cctcagcctc      120


<210>  83
<211>  120
<212>  DNA
<213>  Artificial Sequence

<220>
<223>  SLC28A1

<400>  83
ggtgggtaag gggcaggccg acctgacggc tccctgcggg ctgtaggagg aaggccagct      60

ccctaggagt gacttgagcc ccgcagagat caggagcagc tggagcgagg cggcgccgaa      120


<210>  84
<211>  120
<212>  DNA
<213>  Artificial Sequence

<220>
<223>  SLC28A1

<400>  84
ccctaggagt gacttgagcc ccgcagagat caggagcagc tggagcgagg cggcgccgaa      60

gcccttctcc agatggtagg tgatctctgg agagacaagg gcgggcctgg ggtggaggct      120


<210>  85
<211>  120
<212>  DNA
<213>  Artificial Sequence

<220>
<223>  SLC28A1

<400>  85
gggtctgctc atgcactcat ggaccctgct ggtcttgttc ccaggaggaa cctgcagcca      60

gccctgagag ccagaagctt ctgcagggag cacatgcagc tgtttcgatg gatcggcaca     120


<210>  86
<211>  120
<212>  DNA
<213>  Artificial Sequence

<220>
<223>  SLC28A1

<400>  86
gccctgagag ccagaagctt ctgcagggag cacatgcagc tgtttcgatg gatcggcaca      60

ggcctgctct gcactggtga gcctggggcc cagcactacc caggacacaa tgactcctgc     120


<210>  87
<211>  120
<212>  DNA
<213>  Artificial Sequence

<220>
<223>  SLC28A1

<400>  87
ttgacccctc ctctgtctca tcccccaggg ctctctgcct tcctgctggt ggcctgcctc      60

ctggatttcc agagggccct ggctctgttt gtcctcacct gtgtggtcct caccttcctg     120


<210>  88
<211>  120
<212>  DNA
<213>  Artificial Sequence

<220>
<223>  SLC28A1

<400>  88
ctggatttcc agagggccct ggctctgttt gtcctcacct gtgtggtcct caccttcctg      60

ggccaccgcc tgctgaaacg gcttctgggg ccaaagctga ggaggtttct caagcctcag     120


<210>  89
<211>  120
<212>  DNA
<213>  Artificial Sequence

<220>
<223>  SLC28A1

<400>  89
ggccaccgcc tgctgaaacg gcttctgggg ccaaagctga ggaggtttct caagcctcag      60

ggccatcccc gcctgctgct ctggtttaag aggtgagtga gctcacagcc ccgaggcagg     120

<210>  90
<211>  120
<212>  DNA
<213>  Artificial Sequence

<220>
<223>  SLC28A1

<400>  90
cagtgggtgg cttcaaacaa agcagcatct ttgtggtgtt tcaccagttc ttagtcccag      60

ttacagcagg tgactgtggt ggacgaaaac tggactcaac agtttcctcc attcagggat      120


<210>  91
<211>  120
<212>  DNA
<213>  Artificial Sequence

<220>
<223>  SLC28A1

<400>  91
ttacagcagg tgactgtggt ggacgaaaac tggactcaac agtttcctcc attcagggat      60

cccaggccat ggagcaagga gggcccgaat cagtacctcc ctcagatcac ctggacagtg      120


<210>  92
<211>  120
<212>  DNA
<213>  Artificial Sequence

<220>
<223>  SLC28A1

<400>  92
ggtgggggtg caatgctgag ggtaatggct ttctgtctct tgcctgcagg ggtctagctc      60

ttgctgcttt cctgggcctg gtcctgtggc tgtctctgga cacctcccag cggcctgagc      120


<210>  93
<211>  120
<212>  DNA
<213>  Artificial Sequence

<220>
<223>  SLC28A1

<400>  93
ttgctgcttt cctgggcctg gtcctgtggc tgtctctgga cacctcccag cggcctgagc      60

aactggtgtc cttcgcagga atctgcgtgt tcgtcgctct cctctttgcc tgctcaaagc      120


<210>  94
<211>  120
<212>  DNA
<213>  Artificial Sequence

<220>
<223>  SLC28A1

<400>  94
aactggtgtc cttcgcagga atctgcgtgt tcgtcgctct cctctttgcc tgctcaaagc      60

atcattgcgc agtgagtgct agttgtgggg cccagggctg gaagtgtttg cctctctctt      120


<210>  95
<211>  120
<212>  DNA
<213>  Artificial Sequence

<220>
<223>  SLC28A1

<400>  95
cacccggctc cctgcccatc cctcaaggaa ctgaactcag ctttctgttg ggtggggtgg      60

taggtgtcct ggagggccgt gtcttgggga cttggactgc agtttgtact tggactcctc      120


<210>  96
<211>  120
<212>  DNA
<213>  Artificial Sequence

<220>
<223>  SLC28A1

<400>  96
taggtgtcct ggagggccgt gtcttgggga cttggactgc agtttgtact tggactcctc      60

gtcatcagaa cagaaccagg attcattgcg ttcgagtggc tgggcgagca gatccgggta      120


<210>  97
<211>  120
<212>  DNA
<213>  Artificial Sequence

<220>
<223>  SLC28A1

<400>  97
gtcatcagaa cagaaccagg attcattgcg ttcgagtggc tgggcgagca gatccgggta      60

ggtatgtggg gtctggctgc ccagagcatc ttagattact gggagtaggg gagaagccac      120


<210>  98
<211>  120
<212>  DNA
<213>  Artificial Sequence

<220>
<223>  SLC28A1

<400>  98
cccttcccag cctcactgcc tgtttttgtt tgttttgctt ttttctttca gatcttcctg      60

agctacacga aggctggctc cagcttcgtg tttggggagg cgctggtcaa ggatgtcttt      120

<210>  99
<211>  120
<212>  DNA
<213>  Artificial Sequence

<220>
<223>  SLC28A1

<400>  99
agctacacga aggctggctc cagcttcgtg tttggggagg cgctggtcaa ggatgtcttt      60

gcctttcagg tcagcttgac tcagggtccc agaggccttt agcagccacc gcccagcgac      120


<210>  100
<211>  120
<212>  DNA
<213>  Artificial Sequence

<220>
<223>  SLC28A1

<400>  100
tgcatcctgc ctgcctctaa cctgcggtcc tatgatctcc ttcccggcag gttctgccca      60

tcattgtctt tttcagctgt gtcatatccg ttctctacca cgtgggcctc atgcagtggg      120


<210>  101
<211>  120
<212>  DNA
<213>  Artificial Sequence

<220>
<223>  SLC28A1

<400>  101
tcattgtctt tttcagctgt gtcatatccg ttctctacca cgtgggcctc atgcagtggg      60

tgatcctgaa ggtaagttcc cagtgcccat ggccagggct ctcccagagt caccagctca      120


<210>  102
<211>  120
<212>  DNA
<213>  Artificial Sequence

<220>
<223>  SLC28A1

<400>  102
tctggggttt gctgatgtca gcccacaaag aacattctgc ttccttctag attgcctggc      60

tgatgcaagt caccatgggc accacagcca ctgagaccct gagtgtggct ggaaacatct      120


<210>  103
<211>  120
<212>  DNA
<213>  Artificial Sequence

<220>
<223>  SLC28A1

<400>  103
tgatgcaagt caccatgggc accacagcca ctgagaccct gagtgtggct ggaaacatct      60

ttgtgagcca ggtgggtatg gaagcctcct accctcatgc tcatcagcag cttccccaaa     120


<210>  104
<211>  120
<212>  DNA
<213>  Artificial Sequence

<220>
<223>  SLC28A1

<400>  104
gagaggatgt gcccaatcac ccccaccctg cttgtctgac atctttcctg tttgcagacc      60

gaggctccat tactgatccg gccctacttg gcagacatga cactctctga agtccacgtt     120


<210>  105
<211>  120
<212>  DNA
<213>  Artificial Sequence

<220>
<223>  SLC28A1

<400>  105
gaggctccat tactgatccg gccctacttg gcagacatga cactctctga agtccacgtt      60

gtcatgaccg gaggttacgc caccattgct ggcagcctgc tgggtgccta catctccttt     120


<210>  106
<211>  120
<212>  DNA
<213>  Artificial Sequence

<220>
<223>  SLC28A1

<400>  106
gtcatgaccg gaggttacgc caccattgct ggcagcctgc tgggtgccta catctccttt      60

ggggtaggta gagccctcct tctgcttggc tatgttgagg acctgaagcc catctttgtg     120


<210>  107
<211>  120
<212>  DNA
<213>  Artificial Sequence

<220>
<223>  SLC28A1

<400>  107
gtcgccagca tttcttctga ctgtccacct agaacctgca ctctcactct tgcagatcga      60

tgccacctcg ttgattgcag cctctgtgat ggctgcccct tgtgccttgg ccctctccaa     120

<210>  108
<211>  120
<212>  DNA
<213>  Artificial Sequence

<220>
<223>  SLC28A1

<400>  108
tgccacctcg ttgattgcag cctctgtgat ggctgcccct tgtgccttgg ccctctccaa      60

gctggtctac ccggaggtgg aggagtccaa gtttaggagg gaggaaggag tgaaactgac      120


<210>  109
<211>  120
<212>  DNA
<213>  Artificial Sequence

<220>
<223>  SLC28A1

<400>  109
gctggtctac ccggaggtgg aggagtccaa gtttaggagg gaggaaggag tgaaactgac      60

ctatgggtga gcacagcagg aggtcctgca gacagggtag tggtacaagg tggggggagc      120


<210>  110
<211>  120
<212>  DNA
<213>  Artificial Sequence

<220>
<223>  SLC28A1

<400>  110
caggccagta atgatcattc ttgatcccaa caacagagat gctcagaacc tcatagaagc      60

agccagcact ggggccgcca tctccgtgaa ggtggtcgcc aacatcgctg ccaacctgat      120


<210>  111
<211>  120
<212>  DNA
<213>  Artificial Sequence

<220>
<223>  SLC28A1

<400>  111
agccagcact ggggccgcca tctccgtgaa ggtggtcgcc aacatcgctg ccaacctgat      60

tgcgttcctg gctgtgctgg actttatcaa tgctgccctc tcctggctgg gagacatggt      120


<210>  112
<211>  120
<212>  DNA
<213>  Artificial Sequence

<220>
<223>  SLC28A1

<400>  112
tgcgttcctg gctgtgctgg actttatcaa tgctgccctc tcctggctgg gagacatggt      60

ggacatccaa gggctcagct tccaggtgcg tttctggcca ccacactcag tctgtagaga     120


<210>  113
<211>  120
<212>  DNA
<213>  Artificial Sequence

<220>
<223>  SLC28A1

<400>  113
gccataccgt tgtgccctca gctcatctgc tcctacatcc tgcggcctgt agccttcttg      60

atgggtgtgg cgtgggagga ctgcccagtg gtagctgagc tgctggggat caagctgttt     120


<210>  114
<211>  120
<212>  DNA
<213>  Artificial Sequence

<220>
<223>  SLC28A1

<400>  114
atgggtgtgg cgtgggagga ctgcccagtg gtagctgagc tgctggggat caagctgttt      60

ctgaacgagt ttgtggccta tcaagacctc tccaagtaca agcaacgccg cctggcaggg     120


<210>  115
<211>  120
<212>  DNA
<213>  Artificial Sequence

<220>
<223>  SLC28A1

<400>  115
ctgaacgagt ttgtggccta tcaagacctc tccaagtaca agcaacgccg cctggcaggg      60

gccgaggagt gggtcggcga caggaagcag tggatctccg tgagtgtccc agtcccttcc     120


<210>  116
<211>  120
<212>  DNA
<213>  Artificial Sequence

<220>
<223>  SLC28A1

<400>  116
caggcccatc tgaggggagc ccctcctcat gcatcttctg tattttcagg tcagagctga      60

agtcctcacg acgtttgccc tctgtggatt tgccaatttc agctccattg ggatcatgct     120

<210>  117
<211>  120
<212>  DNA
<213>  Artificial Sequence

<220>
<223>  SLC28A1

<400>  117
agtcctcacg acgtttgccc tctgtggatt tgccaatttc agctccattg ggatcatgct      60

gggaggcttg agtgagtcta atcagggcct caccagtgtc taggagagtc tgggacttga     120


<210>  118
<211>  120
<212>  DNA
<213>  Artificial Sequence

<220>
<223>  SLC28A1

<400>  118
acacagcttc ccaggccctg agcacttctg tccccttgca gcctccatgg tcccccaacg      60

gaagagcgac ttctcccaga tagtgctccg ggcgctcttc acgggagcct gtgtgtccct     120


<210>  119
<211>  120
<212>  DNA
<213>  Artificial Sequence

<220>
<223>  SLC28A1

<400>  119
gaagagcgac ttctcccaga tagtgctccg ggcgctcttc acgggagcct gtgtgtccct      60

ggtgaacgcc tgtatggcag gtgagtgcag gcctggcagg ctcagaaggt ggaaccctga     120


<210>  120
<211>  120
<212>  DNA
<213>  Artificial Sequence

<220>
<223>  SLC28A1

<400>  120
ggtggaaccc tgactttctg gctagcccgg gggccctgcc ccacccacca ctttccctct      60

acagggatcc tctacatgcc caggggggct gaagttgact gcatgtccct cttgaacacg     120


<210>  121
<211>  120
<212>  DNA
<213>  Artificial Sequence

<220>
<223>  SLC28A1

<400>  121
acagggatcc tctacatgcc caggggggct gaagttgact gcatgtccct cttgaacacg        60

accctcagca gcagtagctt tgagatttac cagtgctgcc gtgaggcctt ccagaggtga       120


<210>  122
<211>  120
<212>  DNA
<213>  Artificial Sequence

<220>
<223>  SLC28A1

<400>  122
accctcagca gcagtagctt tgagatttac cagtgctgcc gtgaggcctt ccagaggtga        60

gggcctgggc tgtgggacct gcagggcacc cacagtgata gacagaatgc ctgagcgctg       120


<210>  123
<211>  120
<212>  DNA
<213>  Artificial Sequence

<220>
<223>  SLC28A1

<400>  123
gcccgcagaa ccaggcctgg agctcccact gcgatctttg ctttcttttt agcgtcaatc        60

cagagttcag cccagaggcc ctggacaact gctgtcggtt ttacaaccac acgatctgtg       120


<210>  124
<211>  120
<212>  DNA
<213>  Artificial Sequence

<220>
<223>  SLC28A1

<400>  124
cagagttcag cccagaggcc ctggacaact gctgtcggtt ttacaaccac acgatctgtg        60

cacagtgagg acagaacatg cttgtgcttc tgcgcttctg agggctgttc tcccccggga       120


<210>  125
<211>  120
<212>  DNA
<213>  Artificial Sequence

<220>
<223>  SLC28A1

<400>  125
cgatggttgt tgcccctcat tttatttcta gttccattag ttatgttcca gtaacgtgtt        60

tcttgctctt aggatattca aaagtacacc tgccgagtcc cagccctgag gagtgttttc       120

<210>  126
<211>  120
<212>  DNA
<213>  Artificial Sequence

<220>
<223>  SLC28A1

<400>  126
tcttgctctt aggatattca aaagtacacc tgccgagtcc cagccctgag gagtgttttc      60

gtgcttcatt gttaagtatc ttggaggctc tcagctggaa ataaatgctc ttcatttatt      120


<210>  127
<211>  120
<212>  DNA
<213>  Artificial Sequence

<220>
<223>  KDR-E11

<400>  127
tgtaccatcc ttccattaaa gagagagaga ttttcaaatt ttaaaacttc aaactcactt      60

tgttttttcc ttcaattaga gcaaattgat ttttattaac ttcaatttta tttcctccct      120


<210>  128
<211>  120
<212>  DNA
<213>  Artificial Sequence

<220>
<223>  KDR-E11

<400>  128
gagagatttt caaattttaa aacttcaaac tcactttgtt ttttccttca attagagcaa      60

attgattttt attaacttca attttatttc ctccctggaa gtcctccaca cttctccatt      120


<210>  129
<211>  120
<212>  DNA
<213>  Artificial Sequence

<220>
<223>  KDR-E11

<400>  129
tcaaactcac tttgtttttt ccttcaatta gagcaaattg atttttatta acttcaattt      60

tatttcctcc ctggaagtcc tccacacttc tccattcttc acaagggtat gggtttgtca      120


<210>  130
<211>  120
<212>  DNA
<213>  Artificial Sequence

<220>
<223>  KDR-E11

<400>  130
caattagagc aaattgattt ttattaactt caattttatt tcctccctgg aagtcctcca      60

cacttctcca ttcttcacaa gggtatgggt ttgtcactga gacagcttgg ctataagaaa     120


<210>  131
<211>  120
<212>  DNA
<213>  Artificial Sequence

<220>
<223>  KDR-E11

<400>  131
ggaagtcctc cacacttctc cattcttcac aagggtatgg gtttgtcact gagacagctt      60

ggctataaga aagagataac agcgcatatt atgatttaat ttttctttaa ttagagtcaa     120


<210>  132
<211>  120
<212>  DNA
<213>  Artificial Sequence

<220>
<223>  TP53-E4

<400>  132
agacttggct gtcccagaat gcaagaagcc cagacggaaa ccgtagctgc cctggtaggt      60

tttctgggaa gggacagaag atgacagggg ccaggagggg gctggtgcag gggccgccgg     120


<210>  133
<211>  120
<212>  DNA
<213>  Artificial Sequence

<220>
<223>  TP53-E4

<400>  133
gaagcccaga cggaaaccgt agctgccctg gtaggttttc tgggaaggga cagaagatga      60

caggggccag gagggggctg gtgcaggggc cgccggtgta ggagctgctg gtgcaggggc     120


<210>  134
<211>  120
<212>  DNA
<213>  Artificial Sequence

<220>
<223>  TP53-E4

<400>  134
gccctggtag gttttctggg aagggacaga agatgacagg ggccaggagg gggctggtgc      60

aggggccgcc ggtgtaggag ctgctggtgc aggggccacg gggggagcag cctctggcat     120

<210>  135
<211>  120
<212>  DNA
<213>  Artificial Sequence

<220>
<223>  TP53-E4

<400>  135
gacagaagat gacaggggcc aggagggggc tggtgcaggg gccgccggtg taggagctgc      60

tggtgcaggg gccacggggg gagcagcctc tggcattctg ggagcttcat ctggacctgg      120


<210>  136
<211>  120
<212>  DNA
<213>  Artificial Sequence

<220>
<223>  TP53-E4

<400>  136
tgtaggagct gctggtgcag gggccacggg gggagcagcc tctggcattc tgggagcttc      60

atctggacct gggtcttcag tgaaccattg ttcaatatcg tccggggaca gcatcaaatc      120


<210>  137
<211>  120
<212>  DNA
<213>  Artificial Sequence

<220>
<223>  TP53-E4

<400>  137
cacgggggga gcagcctctg gcattctggg agcttcatct ggacctgggt cttcagtgaa      60

ccattgttca atatcgtccg gggacagcat caaatcatcc attgcttggg acggcaaggg      120


<210>  138
<211>  120
<212>  DNA
<213>  Artificial Sequence

<220>
<223>  TP53-E4

<400>  138
tctgggagct tcatctggac ctgggtcttc agtgaaccat tgttcaatat cgtccgggga      60

cagcatcaaa tcatccattg cttgggacgg caagggggac tgtagatggg tgaaaagagc      120


<210>  139
<211>  120
<212>  DNA
<213>  Artificial Sequence

<220>
<223>  ALK

<400>  139
tgcgaccgag ctcagggccc aggctggttc atgctattct tgcttttcag aatggtatcc      60

tcgtaatgac cagctccagg ggcagtagcg gcttctaagg gcaagccctg ttgctggtag     120


<210>  140
<211>  120
<212>  DNA
<213>  Artificial Sequence

<220>
<223>  ALK

<400>  140
tcgtaatgac cagctccagg ggcagtagcg gcttctaagg gcaagccctg ttgctggtag      60

ccgtaattga cattcccaca agggaagtga cgtagcctga acagaggtac ctccttcata     120


<210>  141
<211>  120
<212>  DNA
<213>  Artificial Sequence

<220>
<223>  ALK

<400>  141
ccgtaattga cattcccaca agggaagtga cgtagcctga acagaggtac ctccttcata      60

ttggcagtca gcgaagaggg ctctaggagc agtgaggccc ccggaagtct cccagttgca     120


<210>  142
<211>  120
<212>  DNA
<213>  Artificial Sequence

<220>
<223>  ALK

<400>  142
ttggcagtca gcgaagaggg ctctaggagc agtgaggccc ccggaagtct cccagttgca      60

acgttaggtg ggacagtaca gcttccctcc agccccaggt tacccctgtc gtgtggctcc     120


<210>  143
<211>  120
<212>  DNA
<213>  Artificial Sequence

<220>
<223>  ALK

<400>  143
acgttaggtg ggacagtaca gcttccctcc agccccaggt tacccctgtc gtgtggctcc      60

ttctttgcta taggattatt ctttttggtg ggtttctctg taaaccagga gccgtacgtt     120

<210>  144
<211>  120
<212>  DNA
<213>  Artificial Sequence

<220>
<223>  ALK

<400>  144
ttctttgcta taggattatt ctttttggtg ggtttctctg taaaccagga gccgtacgtt      60

gggttccaca agctggtggg cttgtttctg gatccgtgga ccttgtgcaa ctccgaagga      120


<210>  145
<211>  120
<212>  DNA
<213>  Artificial Sequence

<220>
<223>  ALK

<400>  145
gggttccaca agctggtggg cttgtttctg gatccgtgga ccttgtgcaa ctccgaagga      60

gggttggact gagagaatgc catattcacg tgtccccctt ccacggccgg ccctctaggg      120


<210>  146
<211>  120
<212>  DNA
<213>  Artificial Sequence

<220>
<223>  ALK

<400>  146
gggttggact gagagaatgc catattcacg tgtccccctt ccacggccgg ccctctaggg      60

actcgaacag agatctctgc agctgtgggt ttctttgcag ccttgccaga ggaggtggta      120


<210>  147
<211>  120
<212>  DNA
<213>  Artificial Sequence

<220>
<223>  ALK

<400>  147
actcgaacag agatctctgc agctgtgggt ttctttgcag ccttgccaga ggaggtggta      60

ggcagaggtg gtggggcagc tgggctgcgc tcctcctccc gttttgcctg ttgagagacc      120


<210>  148
<211>  120
<212>  DNA
<213>  Artificial Sequence

<220>
<223>  ALK

<400>  148
ggcagaggtg gtggggcagc tgggctgcgc tcctcctccc gttttgcctg ttgagagacc      60

aggagaggag gaaccccctc agggtccttg ggcctcacag gcactttctc ttcctcttcc     120


<210>  149
<211>  120
<212>  DNA
<213>  Artificial Sequence

<220>
<223>  ALK

<400>  149
aggagaggag gaaccccctc agggtccttg ggcctcacag gcactttctc ttcctcttcc      60

acaagtggac catattctat cggcaaagcg gtgttgatta catccgggtc ctgccgtagg     120


<210>  150
<211>  120
<212>  DNA
<213>  Artificial Sequence

<220>
<223>  ALK

<400>  150
gtttcatata gagtaaatgt tgaccaaagg gagaaaatgt tttacctggg tgcagtattc      60

aatcctctcc aaaatgatgg caaagttggg cctgtcttca ggctgatgtt gccagcactg     120


<210>  151
<211>  120
<212>  DNA
<213>  Artificial Sequence

<220>
<223>  ALK

<400>  151
aatcctctcc aaaatgatgg caaagttggg cctgtcttca ggctgatgtt gccagcactg      60

agtcattatc cggtatctaa aagaagaagc acattaatta aaataaggag aagcacaatg     120


<210>  152
<211>  120
<212>  DNA
<213>  Artificial Sequence

<220>
<223>  ALK

<400>  152
tgaaaagaaa aactgcttag taactagcag aagtgttcct aaaagagtca tacacaggcc      60

cagggcagtt cttgggtggg tccatccggc ctccactggt gacaaactcc agaacttcct     120

<210>  153
<211>  120
<212>  DNA
<213>  Artificial Sequence

<220>
<223>  ALK

<400>  153
cagggcagtt cttgggtggg tccatccggc ctccactggt gacaaactcc agaacttcct      60

ggttgctttt gctggggtat ggcatatatc caagagaaaa gatttcccat agcagcactc      120


<210>  154
<211>  120
<212>  DNA
<213>  Artificial Sequence

<220>
<223>  ALK

<400>  154
ggttgctttt gctggggtat ggcatatatc caagagaaaa gatttcccat agcagcactc      60

caaaggacct gggcatggga cagaggacat ggagatggat atagacacac ccacccacat      120


<210>  155
<211>  120
<212>  DNA
<213>  Artificial Sequence

<220>
<223>  ALK

<400>  155
tagagtcctt tggcccagga gcaccacctt atggctgcag ggatacctgg aggatgatgg      60

ctgacttacc atgtgtctgt tttagaagtg aatattcctt ccatgaaggc ctctgggggc      120


<210>  156
<211>  120
<212>  DNA
<213>  Artificial Sequence

<220>
<223>  ALK

<400>  156
ctgacttacc atgtgtctgt tttagaagtg aatattcctt ccatgaaggc ctctgggggc      60

atccacttaa ctggcagcat ggcacagcct ccctttctat agtagctcgc cctgtgggga      120


<210>  157
<211>  120
<212>  DNA
<213>  Artificial Sequence

<220>
<223>  ALK

<400>  157
atccacttaa ctggcagcat ggcacagcct ccctttctat agtagctcgc cctgtgggga        60

aggagaggaa aaccaaacta ggatctggag atggcattaa gcatctgccc tgctgggaaa       120


<210>  158
<211>  120
<212>  DNA
<213>  Artificial Sequence

<220>
<223>  ALK

<400>  158
ggtggaagag acaggcccgg aggggtgagg cagtctttac tcacctgtag atgtctcggg        60

ccatcccgaa gtctccaatc ttggccactc ttccagggcc tggacaggtc aagaggcagt       120


<210>  159
<211>  120
<212>  DNA
<213>  Artificial Sequence

<220>
<223>  ALK

<400>  159
ccatcccgaa gtctccaatc ttggccactc ttccagggcc tggacaggtc aagaggcagt        60

ttctggcagc aatgtctctg ggaagaaagg aaatgcattt cctaatttta tccctaggaa       120


<210>  160
<211>  120
<212>  DNA
<213>  Artificial Sequence

<220>
<223>  ALK

<400>  160
agcgacagga tgacaggaag agcacagtca ctttgactca ccggtggatg aagtggtttt        60

cctccaaata ctgacagcca caggcaatgt cccgagccac gtgcagaagg tccagcatgg       120


<210>  161
<211>  120
<212>  DNA
<213>  Artificial Sequence

<220>
<223>  ALK

<400>  161
cctccaaata ctgacagcca caggcaatgt cccgagccac gtgcagaagg tccagcatgg        60

ccagggagga gggctggctc tgtggggaga cagaagcggg ccactgacga ggagcttgtc       120

<210>  162
<211>  120
<212>  DNA
<213>  Artificial Sequence

<220>
<223>  ALK

<400>  162
atccttgctc ctgtccttgg cacaacaact gcagcaaaga ctggttctca ctcaccgggc      60

gagggcgggt ctctcggagg aaggacttga ggtctccccc cgccatgagc tccagcagga      120


<210>  163
<211>  120
<212>  DNA
<213>  Artificial Sequence

<220>
<223>  ALK

<400>  163
gagggcgggt ctctcggagg aaggacttga ggtctccccc cgccatgagc tccagcagga      60

tgaaccgggg cagggattgc aggctcaccc caatgcagcg aacaatgttc tggtggttga      120


<210>  164
<211>  120
<212>  DNA
<213>  Artificial Sequence

<220>
<223>  ALK

<400>  164
tgaaccgggg cagggattgc aggctcaccc caatgcagcg aacaatgttc tggtggttga      60

atttgctgca gagcagagag ggatgtaacc aaaattaact gagctgagtc tgggcaaatc      120


<210>  165
<211>  120
<212>  DNA
<213>  Artificial Sequence

<220>
<223>  ALK

<400>  165
gggcagcagg ggtcccgggc tgagcctaaa cccagaatct tgctggtgag caggtgggaa      60

gaaccacagc aggctccaca ggagttccat ttgcaggaga gtggctggag ctgtgaggat      120


<210>  166
<211>  120
<212>  DNA
<213>  Artificial Sequence

<220>
<223>  ALK

<400>  166
gaaccacagc aggctccaca ggagttccat ttgcaggaga gtggctggag ctgtgaggat      60

gttgctcagg cacttgggtg aggaagtgtc tcagggggca ggagagtgtc tttctcagat     120


<210>  167
<211>  120
<212>  DNA
<213>  Artificial Sequence

<220>
<223>  ALK

<400>  167
gttgctcagg cacttgggtg aggaagtgtc tcagggggca ggagagtgtc tttctcagat      60

actggtgcgt ggactgggtg ctagaattca tggtcgattt ctcccaggaa ggcctcaggc     120


<210>  168
<211>  120
<212>  DNA
<213>  Artificial Sequence

<220>
<223>  ALK

<400>  168
actggtgcgt ggactgggtg ctagaattca tggtcgattt ctcccaggaa ggcctcaggc      60

agaaggtgaa gcaaaacaac tgcttccaag agagactggg tgagacttgc caagtgacag     120


<210>  169
<211>  120
<212>  DNA
<213>  Artificial Sequence

<220>
<223>  ALK

<400>  169
tccaggttct ttgggggcag aggggagttg gggtgagggt gtctctctgt ggctttacct      60

gatgatcagg gcttccatga ggaaatccag ttcgtcctgt tcagagcaca cttcaggcag     120


<210>  170
<211>  120
<212>  DNA
<213>  Artificial Sequence

<220>
<223>  ALK

<400>  170
gatgatcagg gcttccatga ggaaatccag ttcgtcctgt tcagagcaca cttcaggcag      60

cgtctgggca gagaagggga gggtggggag gaggaggagg ctgtgagctg agaactgcag     120

<210>  171
<211>  120
<212>  DNA
<213>  Artificial Sequence

<220>
<223>  ALK

<400>  171
gagtccgcaa gccaagggca ggctcaagag tgagccactt cttaccttca cagccacttg        60

cagggggctt gggtcgttgg gcattccgga cacctggcct tcatacacct ccccaaaggc       120


<210>  172
<211>  120
<212>  DNA
<213>  Artificial Sequence

<220>
<223>  ALK

<400>  172
cagggggctt gggtcgttgg gcattccgga cacctggcct tcatacacct ccccaaaggc        60

gccatggccc agacccctgt gcaaaggaga agacaagagg agacagagtc aaacaggcca       120


<210>  173
<211>  120
<212>  DNA
<213>  Artificial Sequence

<220>
<223>  ALK

<400>  173
ctcccaggac ggcagcaggg cgctcaccga atgagggtga tgtttttccg cggcacctcc        60

ttcaggtcac tgatggagga ggtcttgcca gcaaagcagt agttggggtt gtagtcggtc       120


<210>  174
<211>  120
<212>  DNA
<213>  Artificial Sequence

<220>
<223>  ALK

<400>  174
ttcaggtcac tgatggagga ggtcttgcca gcaaagcagt agttggggtt gtagtcggtc        60

atgatggtcg aggtgcggag cttgctcagc ttgtactcag ggctctgcag ctccatctgc       120


<210>  175
<211>  120
<212>  DNA
<213>  Artificial Sequence

<220>
<223>  ALK

<400>  175
atgatggtcg aggtgcggag cttgctcagc ttgtactcag ggctctgcag ctccatctgc      60

atggcttgca gctcctggtg cttccggcgg tacactgcag gtgggtggtc agctgcaaca     120


<210>  176
<211>  120
<212>  DNA
<213>  Artificial Sequence

<220>
<223>  ALK

<400>  176
cagcctgaga cactattcag tcctgccttc ctgccccctt gggagtccct ggggctctgt      60

gcactcacca atcatgatgc cggagaaagc caggaccagg gcggccacga gggcagaggt     120


<210>  177
<211>  120
<212>  DNA
<213>  Artificial Sequence

<220>
<223>  ALK

<400>  177
gcactcacca atcatgatgc cggagaaagc caggaccagg gcggccacga gggcagaggt      60

caccacagag aggatcagcg agagtggcag gtgtggctcc ggggtgggtg acactggaag     120


<210>  178
<211>  120
<212>  DNA
<213>  Artificial Sequence

<220>
<223>  ALK

<400>  178
caccacagag aggatcagcg agagtggcag gtgtggctcc ggggtgggtg acactggaag      60

acaggtccca ctggggtatt gacaaccaca ccaggtctcc tttgagttgg tcccaagtca     120


<210>  179
<211>  120
<212>  DNA
<213>  Artificial Sequence

<220>
<223>  ALK

<400>  179
tcaggctatg ggcccctctg cctcccctgg ccctgccccc ttaccaatgc aggagacgcc      60

atcctcagcc agcaccgtcc cgtggtcaca gaagcagatg accttgtggc tttcagggtc     120

<210>  180
<211>  120
<212>  DNA
<213>  Artificial Sequence

<220>
<223>  ALK

<400>  180
atcctcagcc agcaccgtcc cgtggtcaca gaagcagatg accttgtggc tttcagggtc        60

catgtgacat tcgtctacct cacagtgact gcagtttaga taatgcttaa tattcacttc       120


<210>  181
<211>  120
<212>  DNA
<213>  Artificial Sequence

<220>
<223>  ALK

<400>  181
catgtgacat tcgtctacct cacagtgact gcagtttaga taatgcttaa tattcacttc        60

cccgtggcct tccatcacta gtgacaagga gggagggtca gtcttgggcc gagcctgcct       120


<210>  182
<211>  120
<212>  DNA
<213>  Artificial Sequence

<220>
<223>  ALK

<400>  182
gacctaagca agtttgttct gctgcctggc agagaagcta ccttttaaag ctggggtgta        60

caggatgccc agtggactga tgaaggaaac cccatcttcc ccatccattt cggggtcatt       120


<210>  183
<211>  120
<212>  DNA
<213>  Artificial Sequence

<220>
<223>  ALK

<400>  183
caggatgccc agtggactga tgaaggaaac cccatcttcc ccatccattt cggggtcatt        60

gtttgaggct gcattgccgc ctgagtagca aaccagagca gagtttaaca tggggggtgg       120


<210>  184
<211>  120
<212>  DNA
<213>  Artificial Sequence

<220>
<223>  ALK

<400>  184
gtgacacctt gaacacgaat catctttacc tatatatcct ccgcctcctc cacctgagga      60

gcacccccct ccaccccctc cgaaaccccc tcttgtctcc cacccccact tcttcatggc      120


<210>  185
<211>  120
<212>  DNA
<213>  Artificial Sequence

<220>
<223>  ALK

<400>  185
gcacccccct ccaccccctc cgaaaccccc tcttgtctcc cacccccact tcttcatggc      60

ctgggggcag gaatgtcctc cggtggcacc ctcctgcaaa gattttccgg cccagagcaa      120


<210>  186
<211>  120
<212>  DNA
<213>  Artificial Sequence

<220>
<223>  ALK

<400>  186
ctgggggcag gaatgtcctc cggtggcacc ctcctgcaaa gattttccgg cccagagcaa      60

ggaagtgtta tcattccagc cacctccacc acctgcggga agagataggg aacctgcgtg      120


<210>  187
<211>  120
<212>  DNA
<213>  Artificial Sequence

<220>
<223>  ALK

<400>  187
gaaggcctgg gagaggttct gggagagggc acgcttgcag cgctttacct gcggctccgg      60

aattgccgtt tagccctaga accgaggagt tattctccag tctctctggg tggaacgtgt      120


<210>  188
<211>  120
<212>  DNA
<213>  Artificial Sequence

<220>
<223>  ALK

<400>  188
aattgccgtt tagccctaga accgaggagt tattctccag tctctctggg tggaacgtgt      60

ctgtcttggc cccgtaggcc ctgccaccac ctccggctgc aatgatcagg ggcaccggca      120

<210>  189
<211>  120
<212>  DNA
<213>  Artificial Sequence

<220>
<223>  ALK

<400>  189
ctgtcttggc cccgtaggcc ctgccaccac ctccggctgc aatgatcagg ggcaccggca      60

ctccatcctt catctgacca ggggagacat tcagacattg agaaaccgag ctgtgcttcc      120


<210>  190
<211>  120
<212>  DNA
<213>  Artificial Sequence

<220>
<223>  ALK

<400>  190
ctgacattgc agatgcacag gaacctggtg gaaatctggc agcacacacc ataccttaaa      60

tacgtaggtg gctccacccc ctcctcctcc gcctcctgcc cactcatgca cgcttctgtt      120


<210>  191
<211>  120
<212>  DNA
<213>  Artificial Sequence

<220>
<223>  ALK

<400>  191
tacgtaggtg gctccacccc ctcctcctcc gcctcctgcc cactcatgca cgcttctgtt      60

cacacggatt tcttcttcta tcacattgtt ctctccaatg cagactttct ggattaactg      120


<210>  192
<211>  120
<212>  DNA
<213>  Artificial Sequence

<220>
<223>  ALK

<400>  192
cacacggatt tcttcttcta tcacattgtt ctctccaatg cagactttct ggattaactg      60

gtttgtctgt agaaacaaaa agcacgttag gtttgtggcc aaaccagagt tctccacttt      120


<210>  193
<211>  120
<212>  DNA
<213>  Artificial Sequence

<220>
<223>  ALK

<400>  193
ggcctgacag agtgcagacg agaaacccct gctctgggca cttacactgg ggcaggcgtc        60

ctctccctgc tgcccaacca ggatgtacag catgtcatcc ttctccaggt tgaagatgcc       120


<210>  194
<211>  120
<212>  DNA
<213>  Artificial Sequence

<220>
<223>  ALK

<400>  194
ctctccctgc tgcccaacca ggatgtacag catgtcatcc ttctccaggt tgaagatgcc        60

cagcacagac acgccgtggg accgcatcat ggtgttcttc ccgcctttcc cgccagcagc       120


<210>  195
<211>  120
<212>  DNA
<213>  Artificial Sequence

<220>
<223>  ALK

<400>  195
cagcacagac acgccgtggg accgcatcat ggtgttcttc ccgcctttcc cgccagcagc        60

tccgtagccc gagatgctgc aatgggacaa agaacgttgg ctcccgctgt ggtatgaaga       120


<210>  196
<211>  120
<212>  DNA
<213>  Artificial Sequence

<220>
<223>  ALK

<400>  196
gggtggtctg cccctcccct ccccctcttc catacgcacc tgtaggtgtc ggtggctggc        60

accttccaga tctggatgcc tttcaggggg ccctcgctcc ccacctccac gctcaggttg       120


<210>  197
<211>  120
<212>  DNA
<213>  Artificial Sequence

<220>
<223>  ALK

<400>  197
accttccaga tctggatgcc tttcaggggg ccctcgctcc ccacctccac gctcaggttg        60

gagttctggt aggcgttgtt gcactgtgcc tgggtggggc catggggccc gctggcccca       120

<210>  198
<211>  120
<212>  DNA
<213>  Artificial Sequence

<220>
<223>  ALK

<400>  198
gagttctggt aggcgttgtt gcactgtgcc tgggtggggc catggggccc gctggcccca      60

catgtggtga acagccaatg aactgtggca caagaggaga ggcagtcact catgtggcca      120


<210>  199
<211>  120
<212>  DNA
<213>  Artificial Sequence

<220>
<223>  ALK

<400>  199
ttctgccttt tgcaacaaga agttactgtg ctcacatttg tgagctgaac ccttacctgt      60

agggtcaaag atgggggtct gtcttggtga attttccccg ggtttcagct ccttgtttgg      120


<210>  200
<211>  120
<212>  DNA
<213>  Artificial Sequence

<220>
<223>  ALK

<400>  200
agggtcaaag atgggggtct gtcttggtga attttccccg ggtttcagct ccttgtttgg      60

gtttctctca aacaggtttc ttgatttggg tgctgtattc tgcaggatct tgtcctctcc      120


<210>  201
<211>  120
<212>  DNA
<213>  Artificial Sequence

<220>
<223>  ALK

<400>  201
gtttctctca aacaggtttc ttgatttggg tgctgtattc tgcaggatct tgtcctctcc      60

gctaactgca atagagaaga ccccacgggc tgagttaggt gagggttgat ttcagggtga      120


<210>  202
<211>  120
<212>  DNA
<213>  Artificial Sequence

<220>
<223>  ALK

<400> 202
atgggggttg ggggacagag tgctggggtc agagtgaact cactggtgag gtagcagtcc 60

aggctgatgg agatattgtc aaaagccacg atggctctgg atccttgtcc ccaccatgcg 120


<210> 203
<211> 120
<212> DNA
<213> Artificial Sequence

<220>
<223> ALK

<400> 203
aggctgatgg agatattgtc aaaagccacg atggctctgg atccttgtcc ccaccatgcg 60

accatctgca gccagaacct gtacacatca agaggaatgt gtgtgaggag caaactgggg 120


<210> 204
<211> 120
<212> DNA
<213> Artificial Sequence

<220>
<223> ALK

<400> 204
ggagaagggt attgggggag atgcatagag cctacctgtc agacacatcg aggagaggca 60

acaccatcca ctgccacagg ctcaagcctt cataggcggc gacatgccag accatcctgc 120


<210> 205
<211> 120
<212> DNA
<213> Artificial Sequence

<220>
<223> ALK

<400> 205
acaccatcca ctgccacagg ctcaagcctt cataggcggc gacatgccag accatcctgc 60

cttgctcctt cccggttttg ttctccacta gcaccaagga cacgtttccc ctcaagactc 120


<210> 206
<211> 120
<212> DNA
<213> Artificial Sequence

<220>
<223> ALK

<400> 206
cttgctcctt cccggttttg ttctccacta gcaccaagga cacgtttccc ctcaagactc 60

cacgaatgag ccaggacatt cggagctgtg agggcgagaa gagtcagagg acaaggtatg 120

<210>  207
<211>  120
<212>  DNA
<213>  Artificial Sequence

<220>
<223>  ALK

<400>  207
taatctgggt tccggaagtg acaagaggtg ggaggagaaa ttagagaact agagaaacaa      60

ggagacttgc ctcacatgga gagctcttga tcggtgcagg aaacgtagca ctggtcactg      120


<210>  208
<211>  120
<212>  DNA
<213>  Artificial Sequence

<220>
<223>  ALK

<400>  208
ggagacttgc ctcacatgga gagctcttga tcggtgcagg aaacgtagca ctggtcactg      60

tagcactttc agaagcgggg acatcagtgg tactgagcaa tagagcatgg tctaggagag      120


<210>  209
<211>  120
<212>  DNA
<213>  Artificial Sequence

<220>
<223>  ALK

<400>  209
tagcactttc agaagcgggg acatcagtgg tactgagcaa tagagcatgg tctaggagag      60

aggaaaagaa tcacaagcac gccattatca ggaactgggg gaccaggggt gcagggttaa      120


<210>  210
<211>  120
<212>  DNA
<213>  Artificial Sequence

<220>
<223>  ALK

<400>  210
gtctatgtgg gcatgaagat gggcaccaga gagaaggcag gagagcagta gtaccttggt      60

ggtcctggaa ccgggcatcc tttagggtcc tgacctgcca ttgaggagtg tggggtgaca      120


<210>  211
<211>  120
<212>  DNA
<213>  Artificial Sequence

<220>
<223>  ALK

<400>  211
ggtcctggaa ccgggcatcc tttagggtcc tgacctgcca ttgaggagtg tggggtgaca      60

gtgtgccttg ggtccagcca cagaagccat cttcaaagtt gcagtaaaaa cccacaggca     120


<210>  212
<211>  120
<212>  DNA
<213>  Artificial Sequence

<220>
<223>  ALK

<400>  212
gtgtgccttg ggtccagcca cagaagccat cttcaaagtt gcagtaaaaa cccacaggca      60

gtttccctat ggagagagca gagaggcacc atcattttca ggaccactaa aggcaaaata     120


<210>  213
<211>  120
<212>  DNA
<213>  Artificial Sequence

<220>
<223>  ALK

<400>  213
gggttatgag catgggctgg gctcaggcag ggtggggcag ccccatctac tcacggcaca      60

tctggctctc atcttctccc tgggcacagt cctggtggaa gtcacaggcc tgcccaagct     120


<210>  214
<211>  120
<212>  DNA
<213>  Artificial Sequence

<220>
<223>  ALK

<400>  214
tctggctctc atcttctccc tgggcacagt cctggtggaa gtcacaggcc tgcccaagct      60

ggaggactgt cccattccaa caagtgaagg agctctgcag ggccatcttg gagcctgggg     120


<210>  215
<211>  120
<212>  DNA
<213>  Artificial Sequence

<220>
<223>  ALK

<400>  215
ggaggactgt cccattccaa caagtgaagg agctctgcag ggccatcttg gagcctgggg      60

atgttcctgg agagcacaca gacacacaac catggtaagt ttgcatggcc ccaggcagca     120

<210>  216
<211>  120
<212>  DNA
<213>  Artificial Sequence

<220>
<223>  ALK

<400>  216
tgacacatct aacacaatag gctaccaagg agcgtgggaa agccagattc agataccttc      60

actgcagttc ttcagggcaa agaagtccac tgcagacaag ctgcggtttc cactggagat      120


<210>  217
<211>  120
<212>  DNA
<213>  Artificial Sequence

<220>
<223>  ALK

<400>  217
actgcagttc ttcagggcaa agaagtccac tgcagacaag ctgcggtttc cactggagat      60

gtattccagg gccactcgaa atgggttgtc tggacgcccg attcttccct ggagcactgt      120


<210>  218
<211>  120
<212>  DNA
<213>  Artificial Sequence

<220>
<223>  ALK

<400>  218
gtattccagg gccactcgaa atgggttgtc tggacgcccg attcttccct ggagcactgt      60

ccaactggtt gcattggaaa acagaggaga aaagcataga gaaacagata tgagaattag      120


<210>  219
<211>  120
<212>  DNA
<213>  Artificial Sequence

<220>
<223>  ALK

<400>  219
gacatggaga agtacttacc catgcttccc tggagtgggc atcaggagga tctctcttgc      60

agcctcgttg tggggcagca gctgggcaat gtaccttcca gagggctgca ggtgcctgtg      120


<210>  220
<211>  120
<212>  DNA
<213>  Artificial Sequence

<220>
<223>  ALK

<400>  220
agcctcgttg tggggcagca gctgggcaat gtaccttcca gagggctgca ggtgcctgtg        60

caccgagacg gccagtgtgc agtgctcact gctgctcctc atccacggac tcaggatggt       120


<210>  221
<211>  120
<212>  DNA
<213>  Artificial Sequence

<220>
<223>  ALK

<400>  221
caccgagacg gccagtgtgc agtgctcact gctgctcctc atccacggac tcaggatggt        60

gtgcttggag tcagctgagg tgttgagaag gagaaaggag cctggaaaga gacagggaaa       120


<210>  222
<211>  120
<212>  DNA
<213>  Artificial Sequence

<220>
<223>  ALK

<400>  222
tgacccaccc aggacatcac cagcagcctc tcccttacct ctgggcatct ccttagaacg        60

ctctgcccca ggcccatcca gcaagtccat ctgggaggcc tcctcggagg ggatgcggcg       120


<210>  223
<211>  120
<212>  DNA
<213>  Artificial Sequence

<220>
<223>  ALK

<400>  223
ctctgcccca ggcccatcca gcaagtccat ctgggaggcc tcctcggagg ggatgcggcg        60

ccaggaccag ctctggttcc tgaggtcatg cagtggaggg gaatactcca gctcacaggg       120


<210>  224
<211>  120
<212>  DNA
<213>  Artificial Sequence

<220>
<223>  ALK

<400>  224
ccaggaccag ctctggttcc tgaggtcatg cagtggaggg gaatactcca gctcacaggg        60

gaagtcaaag ctgcactcca gacctgcaat aatagccaag ggtcaatgga aaaaaccatt       120

<210>  225
<211>  120
<212>  DNA
<213>  Artificial Sequence

<220>
<223>  ALK

<400>  225
acagagtcct tattatgaga tagtgacagt gtatctcaag taaatattaa acatatactt      60

accatatcgg ctgcgatgag acaggaaagg gaaggagtct ttcattatcc aggtgagatt      120


<210>  226
<211>  120
<212>  DNA
<213>  Artificial Sequence

<220>
<223>  ALK

<400>  226
accatatcgg ctgcgatgag acaggaaagg gaaggagtct ttcattatcc aggtgagatt      60

ccatgtaaaa taatcaggag aaggagaagg catgtttgtt ggtgattcca aggagctatg      120


<210>  227
<211>  120
<212>  DNA
<213>  Artificial Sequence

<220>
<223>  ALK

<400>  227
ccatgtaaaa taatcaggag aaggagaagg catgtttgtt ggtgattcca aggagctatg      60

acctggacat aaaaataaag aaaacactga tccatgtgct tggggtgtgt cttttagctg      120


<210>  228
<211>  120
<212>  DNA
<213>  Artificial Sequence

<220>
<223>  ALK

<400>  228
cggcacactc aggcgggagc tgctcaccag tcccgaagat ctggaagaga aggcggggct      60

gggaggcgcg aattgccgcg gacagccttc cctctctgcc cacttccgac gccttcttct      120


<210>  229
<211>  120
<212>  DNA
<213>  Artificial Sequence

<220>
<223>  ALK

<400>  229
gggaggcgcg aattgccgcg gacagccttc cctctctgcc cacttccgac gccttcttct      60

cgggcatcag gcggatcctc agtcgccctt cgccttggcg aatccaccaa ctgaacagct     120


<210>  230
<211>  120
<212>  DNA
<213>  Artificial Sequence

<220>
<223>  ALK

<400>  230
cgggcatcag gcggatcctc agtcgccctt cgccttggcg aatccaccaa ctgaacagct      60

cgctgagatt gaactggagc agccccacag ccgcctcccc ggggggcccg acgcaaccct     120


<210>  231
<211>  120
<212>  DNA
<213>  Artificial Sequence

<220>
<223>  ALK

<400>  231
cgctgagatt gaactggagc agccccacag ccgcctcccc ggggggcccg acgcaaccct      60

ccaagatcgc ctcctcgccc agctccagca ccaactgctt ggcacgccgg agcttgcgca     120


<210>  232
<211>  120
<212>  DNA
<213>  Artificial Sequence

<220>
<223>  ALK

<400>  232
ccaagatcgc ctcctcgccc agctccagca ccaactgctt ggcacgccgg agcttgcgca      60

cggagccgcc cttcagcacc ctggacagcg tccgggcctc tgccggggct ggtgaaccgg     120


<210>  233
<211>  120
<212>  DNA
<213>  Artificial Sequence

<220>
<223>  ALK

<400>  233
cggagccgcc cttcagcacc ctggacagcg tccgggcctc tgccggggct ggtgaaccgg      60

cggtccagga gacccccggc gccggcccca gcaacctgag cagcggggcg cagtccagag     120

<210>  234
<211>  120
<212>  DNA
<213>  Artificial Sequence

<220>
<223>  ALK

<400>  234
cggtccagga gacccccggc gccggcccca gcaacctgag cagcggggcg cagtccagag      60

ctagcgagcc gcgggcctcg ggcctgccag ccttcagctc cgaggaggat ggtggcagca      120


<210>  235
<211>  120
<212>  DNA
<213>  Artificial Sequence

<220>
<223>  ALK

<400>  235
ctagcgagcc gcgggcctcg ggcctgccag ccttcagctc cgaggaggat ggtggcagca      60

gtaggtcccg ggcgtagaca cggaagagcg agggcaccac gaagtcaact gccagactct      120


<210>  236
<211>  120
<212>  DNA
<213>  Artificial Sequence

<220>
<223>  ALK

<400>  236
gtaggtcccg ggcgtagaca cggaagagcg agggcaccac gaagtcaact gccagactct      60

tcctctgcag gcgcgagtag ctgagtggct cccggggctg cagcggcggc cccgcagctg      120


<210>  237
<211>  120
<212>  DNA
<213>  Artificial Sequence

<220>
<223>  ALK

<400>  237
tcctctgcag gcgcgagtag ctgagtggct cccggggctg cagcggcggc cccgcagctg      60

gggagcccgc gcgctggccg gtccccatcc cggagcccac agctgccgtg gaaagcagca      120


<210>  238
<211>  120
<212>  DNA
<213>  Artificial Sequence

<220>
<223>  ALK

<400>  238
gggagcccgc gcgctggccg gtccccatcc cggagcccac agctgccgtg gaaagcagca      60

gcggcaggag ccacaggagc ccgatggctc ccatcccgcc ggaggaggcc gtttacactg      120


<210>  239
<211>  120
<212>  DNA
<213>  Artificial Sequence

<220>
<223>  ALK-E23

<400>  239
atccttgctc ctgtccttgg cacaacaact gcagcaaaga ctggttctca ctcaccgggc      60

gagggcgggt ctctcggagg aaggacttga ggtctccccc cgccatgagc tccagcagga      120


<210>  240
<211>  120
<212>  DNA
<213>  Artificial Sequence

<220>
<223>  ALK-E23

<400>  240
ctcggaggaa ggacttgagg tctccccccg ccatgagctc cagcaggatg aaccggggca      60

gggattgcag gctcacccca atgcagcgaa caatgttctg gtggttgaat ttgctgcaga      120


<210>  241
<211>  120
<212>  DNA
<213>  Artificial Sequence

<220>
<223>  ALK-E23

<400>  241
cccccgccat gagctccagc aggatgaacc ggggcaggga ttgcaggctc accccaatgc      60

agcgaacaat gttctggtgg ttgaatttgc tgcagagcag agagggatgt aaccaaaatt      120


<210>  242
<211>  120
<212>  DNA
<213>  Artificial Sequence

<220>
<223>  ALK-E23

<400>  242
tgaaccgggg cagggattgc aggctcaccc caatgcagcg aacaatgttc tggtggttga      60

atttgctgca gagcagagag ggatgtaacc aaaattaact gagctgagtc tgggcaaatc      120

<210>  243
<211>  120
<212>  DNA
<213>  Artificial Sequence

<220>
<223>  ALK-E24

<400>  243
gacctagtat tctgctctga agggggaaat gtgagccctt gagatctgcg gggaagcaca        60

cagatcagcg acaggatgac aggaagagca cagtcacttt gactcaccgg tggatgaagt       120


<210>  244
<211>  120
<212>  DNA
<213>  Artificial Sequence

<220>
<223>  ALK-E24

<400>  244
ggaaatgtga gcccttgaga tctgcgggga agcacacaga tcagcgacag gatgacagga        60

agagcacagt cactttgact caccggtgga tgaagtggtt ttcctccaaa tactgacagc       120


<210>  245
<211>  120
<212>  DNA
<213>  Artificial Sequence

<220>
<223>  ALK-E24

<400>  245
cggggaagca cacagatcag cgacaggatg acaggaagag cacagtcact ttgactcacc        60

ggtggatgaa gtggttttcc tccaaatact gacagccaca ggcaatgtcc cgagccacgt       120


<210>  246
<211>  120
<212>  DNA
<213>  Artificial Sequence

<220>
<223>  ALK-E24

<400>  246
aggatgacag gaagagcaca gtcactttga ctcaccggtg gatgaagtgg ttttcctcca        60

aatactgaca gccacaggca atgtcccgag ccacgtgcag aaggtccagc atggccaggg       120


<210>  247
<211>  120
<212>  DNA
<213>  Artificial Sequence

<220>
<223>  ALK-E24

<400>  247
ggttttcctc caaatactga cagccacagg caatgtcccg agccacgtgc agaaggtcca      60

gcatggccag ggaggagggc tggctctgtg gggagacaga agcgggccac tgacgaggag      120


<210>  248
<211>  120
<212>  DNA
<213>  Artificial Sequence

<220>
<223>  ALK-E24

<400>  248
cacaggcaat gtcccgagcc acgtgcagaa ggtccagcat ggccagggag gagggctggc      60

tctgtgggga gacagaagcg ggccactgac gaggagcttg tcagtgagag gagggaaatc      120


<210>  249
<211>  120
<212>  DNA
<213>  Artificial Sequence

<220>
<223>  ALK-E24

<400>  249
gcagaaggtc cagcatggcc agggaggagg gctggctctg tggggagaca gaagcgggcc      60

actgacgagg agcttgtcag tgagaggagg gaaatctgaa atgctggctt ccagtgctca      120


<210>  250
<211>  120
<212>  DNA
<213>  Artificial Sequence

<220>
<223>  ALK-E24

<400>  250
aggagggctg gctctgtggg gagacagaag cgggccactg acgaggagct tgtcagtgag      60

aggagggaaa tctgaaatgc tggcttccag tgctcacaag gaggcagacc gtgaacatgc      120


<210>  251
<211>  120
<212>  DNA
<213>  Artificial Sequence

<220>
<223>  ALK-E25

<400>  251
ctcgggccat cccgaagtct ccaatcttgg ccactcttcc agggcctgga caggtcaaga      60

ggcagtttct ggcagcaatg tctctgggaa gaaaggaaat gcatttccta attttatccc      120

<210>  252
<211>  120
<212>  DNA
<213>  Artificial Sequence

<220>
<223>  ALK-E25

<400>  252
tcttggccac tcttccaggg cctggacagg tcaagaggca gtttctggca gcaatgtctc      60

tgggaagaaa ggaaatgcat ttcctaattt tatccctagg aagatgagtg tacaacggcc     120


<210>  253
<211>  120
<212>  DNA
<213>  Artificial Sequence

<220>
<223>  ALK-E25

<400>  253
gacaggtcaa gaggcagttt ctggcagcaa tgtctctggg aagaaaggaa atgcatttcc      60

taattttatc cctaggaaga tgagtgtaca acggccatca ctaggatttt atctccaagc     120


<210>  254
<211>  120
<212>  DNA
<213>  Artificial Sequence

<220>
<223>  ALK-E25

<400>  254
cagcaatgtc tctgggaaga aaggaaatgc atttcctaat tttatcccta ggaagatgag      60

tgtacaacgg ccatcactag gattttatct ccaagctgaa gtttaaatag ttccttccct     120


<210>  255
<211>  120
<212>  DNA
<213>  Artificial Sequence

<220>
<223>  ALK-E25

<400>  255
atgtaaggga caagcagcca caccccattc ttgaggggct gaggtggaag agacaggccc      60

ggaggggtga ggcagtcttt actcacctgt agatgtctcg ggccatcccg aagtctccaa     120


<210>  256
<211>  120
<212>  DNA
<213>  Artificial Sequence

<220>
<223>  ALK-E25

<400>  256
ccattcttga ggggctgagg tggaagagac aggcccggag gggtgaggca gtctttactc        60

acctgtagat gtctcgggcc atcccgaagt ctccaatctt ggccactctt ccagggcctg       120


<210>  257
<211>  120
<212>  DNA
<213>  Artificial Sequence

<220>
<223>  ALK-E25

<400>  257
agagacaggc ccggaggggt gaggcagtct ttactcacct gtagatgtct cgggccatcc        60

cgaagtctcc aatcttggcc actcttccag ggcctggaca ggtcaagagg cagtttctgg       120


<210>  258
<211>  120
<212>  DNA
<213>  Artificial Sequence

<220>
<223>  ALK-E25

<400>  258
aagttgacag ggtaccagga gatgatgtaa gggacaagca gccacacccc attcttgagg        60

ggctgaggtg gaagagacag gcccggaggg gtgaggcagt ctttactcac ctgtagatgt       120


<210>  259
<211>  120
<212>  DNA
<213>  Artificial Sequence

<220>
<223>  BRAF

<400>  259
acagaactgt gttttgatgt taacaaattg tacgaacaca agacttaaga aataagagca        60

gatgctgcca tgatggtggc tacttgaagg ctgcaaattc tcctgtagag ggaggacaag       120


<210>  260
<211>  120
<212>  DNA
<213>  Artificial Sequence

<220>
<223>  BRAF

<400>  260
gatgctgcca tgatggtggc tacttgaagg ctgcaaattc tcctgtagag ggaggacaag        60

agctaatttt aaaaaagtca tctcaaataa cctagagaca cagaaaagcc tcatttgagc       120

<210>  261
<211>  120
<212>  DNA
<213>  Artificial Sequence

<220>
<223>  BRAF

<400>  261
ttccttttgt tgctactctc ctgaactctc tcactcattt gtttcagtgg acaggaaacg        60

caccatatcc ccctgcctgg atgggtgttt ttggagaagc acaagcatat agactaaaat       120


<210>  262
<211>  120
<212>  DNA
<213>  Artificial Sequence

<220>
<223>  BRAF

<400>  262
caccatatcc ccctgcctgg atgggtgttt ttggagaagc acaagcatat agactaaaat       60

cctctgtttg gaaaccagcc cgattcaagg agggttctga tgcactgcgg tgaatttttg       120


<210>  263
<211>  120
<212>  DNA
<213>  Artificial Sequence

<220>
<223>  BRAF

<400>  263
cctctgtttg gaaaccagcc cgattcaagg agggttctga tgcactgcgg tgaatttttg       60

gcaatgagcg ggccagcagc tcaatagagg cgagaatcta caaaaaaaaa aagaaaaaaa       120


<210>  264
<211>  120
<212>  DNA
<213>  Artificial Sequence

<220>
<223>  BRAF

<400>  264
tctgtctatg aatgttagtc tgttcttttg gatagcatga agcttttact tacttgggga       60

aagagtggtc tctcatctct tttctttttg aggcactctg ccattaatct cttcatggct       120


<210>  265
<211>  120
<212>  DNA
<213>  Artificial Sequence

<220>
<223>  BRAF

<400>  265
   aagagtggtc tctcatctct tttctttttg aggcactctg ccattaatct cttcatggct      60
   tttggacagt tactccgtac cttactgaga tctggagaca ggtatcctcg tcccaccata     120


<210>  266
<211>  120
<212>  DNA
<213>  Artificial Sequence

<220>
<223>  BRAF

<400>  266
tttggacagt tactccgtac cttactgaga tctggagaca ggtatcctcg tcccaccata      60

aaaattatct ggagagagaa aaaaaaggga aataattcaa ccttgtagat aagttgaaaa     120


<210>  267
<211>  120
<212>  DNA
<213>  Artificial Sequence

<220>
<223>  BRAF

<400>  267
aagaattaac tagtaaaggc cttacccttc tgttggtcac acattcctct catggagagt      60

aagcccttgc cacatcattt attatactcc catggaggac aaagcaccta aaatgccaag     120


<210>  268
<211>  120
<212>  DNA
<213>  Artificial Sequence

<220>
<223>  BRAF

<400>  268
aagcccttgc cacatcattt attatactcc catggaggac aaagcaccta aaatgccaag      60

aagtggatga gagaagaact ctgggatgct tttatctgta ttttggagat aatacttgtt     120


<210>  269
<211>  120
<212>  DNA
<213>  Artificial Sequence

<220>
<223>  BRAF

<400>  269
tggagccttg tatatagacg gtaaaataaa caccaagacg tggtaaatat ttacctggtc      60

cctgttgttg atgtttgaat aaggtaactg tccagtcatc aattcataca gaacaattcc     120

<210>  270
<211>  120
<212>  DNA
<213>  Artificial Sequence

<220>
<223>  BRAF

<400>  270
cctgttgttg atgtttgaat aaggtaactg tccagtcatc aattcataca gaacaattcc      60

aaatgcatat acatctgact gaaagctgta tggattttta tcttgcattc tgatgacttc      120


<210>  271
<211>  120
<212>  DNA
<213>  Artificial Sequence

<220>
<223>  BRAF

<400>  271
aaatgcatat acatctgact gaaagctgta tggattttta tcttgcattc tgatgacttc      60

tggtgcctgt tagaacatac aaagaaaaat attcttcact tcaattgaat aaagactgaa      120


<210>  272
<211>  120
<212>  DNA
<213>  Artificial Sequence

<220>
<223>  BRAF

<400>  272
taactcagca gcatctcagg gccaaaaatt taatcagtgg aaaaatagcc tcaattctta      60

ccatccacaa aatggatcca gacaactgtt caaactgatg ggacccactc catcgagatt      120


<210>  273
<211>  120
<212>  DNA
<213>  Artificial Sequence

<220>
<223>  BRAF

<400>  273
ccatccacaa aatggatcca gacaactgtt caaactgatg ggacccactc catcgagatt      60

tcactgtagc tagaccaaaa tcacctattt ttactgtgag gtcttcatga agaaatatat      120


<210>  274
<211>  120
<212>  DNA
<213>  Artificial Sequence

<220>
<223>  BRAF

<400>  274
tcactgtagc tagaccaaaa tcacctattt ttactgtgag gtcttcatga agaaatatat        60

ctgaggtgta gtaagtaaag gaaaacagta gatctcattt tcctatcaga gcaagcatta       120


<210>  275
<211>  120
<212>  DNA
<213>  Artificial Sequence

<220>
<223>  BRAF

<400>  275
aagacaaaat gcagaagaaa aagtcaggat gttttcaaac ttcgcagaca aatttcagga        60

aggatactat tactcttgag gtctctgtgg atgattgact tggcgtgtaa gtaactgaaa       120


<210>  276
<211>  120
<212>  DNA
<213>  Artificial Sequence

<220>
<223>  BRAF

<400>  276
aggatactat tactcttgag gtctctgtgg atgattgact tggcgtgtaa gtaactgaaa        60

aacaaaacat cattttaacc tgagtagggc taaaggactc tggcctcgaa atctacagaa       120


<210>  277
<211>  120
<212>  DNA
<213>  Artificial Sequence

<220>
<223>  BRAF

<400>  277
aatttacaag acatttaacg aatggaactt actccatgcc ctgtgcagtc tgtcgtgcaa        60

tatctataag tttgatcatc tcaaatttgg tctcaatgat atggagatgg tgatacaagc       120


<210>  278
<211>  120
<212>  DNA
<213>  Artificial Sequence

<220>
<223>  BRAF

<400>  278
tatctataag tttgatcatc tcaaatttgg tctcaatgat atggagatgg tgatacaagc        60

tggagccctc acaccactgg gtaacaatag ccagttgtgg ctttgtggaa tagcccatga       120

<210>  279
<211>  120
<212>  DNA
<213>  Artificial Sequence

<220>
<223>  BRAF

<400>  279
tggagccctc acaccactgg gtaacaatag ccagttgtgg ctttgtggaa tagcccatga      60

agagtaggat attcacatgt cgtgttttcc tgtacaaaga aatgtgacag taaacattaa      120


<210>  280
<211>  120
<212>  DNA
<213>  Artificial Sequence

<220>
<223>  BRAF

<400>  280
ataaaaataa cttctttctc tggaaaagag taattcacac aagctcacct gagtactcct      60

acttcatttt tgaaggcttg taactgctga ggtgtaggtg ctgtcacatt caacattttc      120


<210>  281
<211>  120
<212>  DNA
<213>  Artificial Sequence

<220>
<223>  BRAF

<400>  281
acttcatttt tgaaggcttg taactgctga ggtgtaggtg ctgtcacatt caacattttc      60

actgccacat cacctaaaag gcaattgtta ctccaagtgt catttcaatt tttaaaattt      120


<210>  282
<211>  120
<212>  DNA
<213>  Artificial Sequence

<220>
<223>  BRAF

<400>  282
taaaagttgt taaacatatc ctattatgac ttgtcacaat gtcaccacat tacatactta      60

ccatgccact ttcccttgta gactgttcca aatgatccag atccaattct ttgtcccact      120


<210>  283
<211>  120
<212>  DNA
<213>  Artificial Sequence

<220>
<223>  BRAF

<400>  283
ccatgccact ttcccttgta gactgttcca aatgatccag atccaattct ttgtcccact        60

gtaatctgcc catcaggaat ctcccaatca tcactcgagt cccgtctacc aagtgttttc       120


<210>  284
<211>  120
<212>  DNA
<213>  Artificial Sequence

<220>
<223>  BRAF

<400>  284
gtaatctgcc catcaggaat ctcccaatca tcactcgagt cccgtctacc aagtgttttc        60

ttgataaaaa cagtaaaaaa gtcaagtcaa gccaaacaga aaaagaaaac cttatgtttc       120


<210>  285
<211>  120
<212>  DNA
<213>  Artificial Sequence

<220>
<223>  BRAF

<400>  285
tgacttctaa gaagaaagaa ttcagagaaa aaaagatatc atatactctt accattcgat        60

tcctgtcttc tgaggatgaa gatgacttcc tttctcgctg aggtcctgga gatttctgta       120


<210>  286
<211>  120
<212>  DNA
<213>  Artificial Sequence

<220>
<223>  BRAF

<400>  286
tcctgtcttc tgaggatgaa gatgacttcc tttctcgctg aggtcctgga gatttctgta        60

aggctttcac gttagttagt gagccaggta atgaggcagg gggggtagca gacaaacctg       120


<210>  287
<211>  120
<212>  DNA
<213>  Artificial Sequence

<220>
<223>  BRAF

<400>  287
aggctttcac gttagttagt gagccaggta atgaggcagg gggggtagca gacaaacctg        60

tggttgatcc taaattagtg aaaagaaaaa tgtatacatt aaggaggagc aagtatgtta       120

<210>  288
<211>  120
<212>  DNA
<213>  Artificial Sequence

<220>
<223>  BRAF

<400>  288
tataaaggaa ataagcagca aagcaattgc agtttccttg agtttttaaa aaaacctgaa      60

atcactactt acctccatca ccacgaaatc cttggtctct aatcaagtcc tacaaataaa      120


<210>  289
<211>  120
<212>  DNA
<213>  Artificial Sequence

<220>
<223>  BRAF

<400>  289
atcactactt acctccatca ccacgaaatc cttggtctct aatcaagtcc tacaaataaa      60

tagtaatgta tatttattcc aagcaagcat ataatcagag agtagcgata acactgaatt      120


<210>  290
<211>  120
<212>  DNA
<213>  Artificial Sequence

<220>
<223>  BRAF

<400>  290
acttggtttt tttttagttc tagcaatgct ggatacttac atcaatattg acaggttcta      60

ttgtgtttat atgcacattg ggagctgatg aggatcggtc tcgttgccca aattgatttc      120


<210>  291
<211>  120
<212>  DNA
<213>  Artificial Sequence

<220>
<223>  BRAF

<400>  291
ttgtgtttat atgcacattg ggagctgatg aggatcggtc tcgttgccca aattgatttc      60

gatgatcttc atctgctggt cggaagggct gtggaattgg aatggatttt gaaggagacg      120


<210>  292
<211>  120
<212>  DNA
<213>  Artificial Sequence

<220>
<223>  BRAF

<400>  292
gatgatcttc atctgctggt cggaagggct gtggaattgg aatggatttt gaaggagacg        60

gactggtgag aatttggggc ctggaaaaat gaagtcattg gaagataaga ttcagagtaa       120


<210>  293
<211>  120
<212>  DNA
<213>  Artificial Sequence

<220>
<223>  BRAF

<400>  293
gagatccaaa agaaagcggt tcaagtagca tgtcgcccaa gagcagaagt caaaccatac        60

ccaatagagt ccgaggcggg tgcggaaggg gatgatccag atgttagggc agtctctgct       120


<210>  294
<211>  120
<212>  DNA
<213>  Artificial Sequence

<220>
<223>  BRAF

<400>  294
ccaatagagt ccgaggcggg tgcggaaggg gatgatccag atgttagggc agtctctgct        60

aaggacgcct cttcctgtgg tattgggtgg tgttcaaaga acttggagac aaacagcaaa       120


<210>  295
<211>  120
<212>  DNA
<213>  Artificial Sequence

<220>
<223>  BRAF

<400>  295
aaggacgcct cttcctgtgg tattgggtgg tgttcaaaga acttggagac aaacagcaaa        60

ctgtgaggca aaacaaaaca aacctaactt gtgcaaaacc cagaagcttc atataccaaa       120


<210>  296
<211>  120
<212>  DNA
<213>  Artificial Sequence

<220>
<223>  BRAF

<400>  296
aagtgtaaaa tggtaggtag aaaagagata tttttggatt acttactcaa gttggtcata        60

attaacacac atcagtggaa cttctgtact acaacgctgg tgaaatttat aaccacatgt       120

<210>  297
<211>  120
<212>  DNA
<213>  Artificial Sequence

<220>
<223>  BRAF

<400>  297
attaacacac atcagtggaa cttctgtact acaacgctgg tgaaatttat aaccacatgt      60

ttgacagcgg aaaccctgga aaagcagctt tcgacaaaag tcacaaaatg ctaaggtgaa      120


<210>  298
<211>  120
<212>  DNA
<213>  Artificial Sequence

<220>
<223>  BRAF

<400>  298
ttgacagcgg aaaccctgga aaagcagctt tcgacaaaag tcacaaaatg ctaaggtgaa      60

aaacgttttt cgtacctgca aagtaaaaaa tcacagagat ttcaaaaact cacaagaaaa      120


<210>  299
<211>  120
<212>  DNA
<213>  Artificial Sequence

<220>
<223>  BRAF

<400>  299
taaataaaaa ttcattcatt aaaatctaaa catttttgac atttcaaaaa aaaatgtaaa      60

gatacataca aagttgtgtg ttgtaagtgg aacattctcc aacacttcca catgcaattc      120


<210>  300
<211>  120
<212>  DNA
<213>  Artificial Sequence

<220>
<223>  BRAF

<400>  300
gatacataca aagttgtgtg ttgtaagtgg aacattctcc aacacttcca catgcaattc      60

ttctccagta agccaggaaa tatcagtgtc ccaaccaatt ggtttcttct ctctgaaaaa      120


<210>  301
<211>  120
<212>  DNA
<213>  Artificial Sequence

<220>
<223>  BRAF

<400>  301
ttctccagta agccaggaaa tatcagtgtc ccaaccaatt ggtttcttct ctctgaaaaa      60

tgtagacaca agcctttctt ggttattaca cctaaaaata ttcatatacc tcatgctgaa     120


<210>  302
<211>  120
<212>  DNA
<213>  Artificial Sequence

<220>
<223>  BRAF

<400>  302
tccatcgtta gaaatttaaa tggcaataaa ttaattttaa gtttacccca aataaatgta      60

tcactcttgg acaaacagca gggtaaagag atgtactctt ttcttcaagt cagctccaga     120


<210>  303
<211>  120
<212>  DNA
<213>  Artificial Sequence

<220>
<223>  BRAF

<400>  303
tcactcttgg acaaacagca gggtaaagag atgtactctt ttcttcaagt cagctccaga      60

aaagacctac aacagcagaa aatggattgt tcctggggtc caaaaaaaaa aaaaaaaaaa     120


<210>  304
<211>  120
<212>  DNA
<213>  Artificial Sequence

<220>
<223>  BRAF

<400>  304
agaaaacttc aaagtttaat gtgtgatttt ctttttaaac aaaatttcac gtcacataca      60

aaccataccc atcctgaatt ctgtaaacag cacagcactc tgggattaga cctctcatca     120


<210>  305
<211>  120
<212>  DNA
<213>  Artificial Sequence

<220>
<223>  BRAF

<400>  305
aaccataccc atcctgaatt ctgtaaacag cacagcactc tgggattaga cctctcatca      60

tcagtgcttt ctttagactg tctcggactg taactccaca ccttgcaggt acctatggta     120

<210>  306
<211>  120
<212>  DNA
<213>  Artificial Sequence

<220>
<223>  BRAF

<400>  306
tcagtgcttt ctttagactg tctcggactg taactccaca ccttgcaggt acctatggta      60

tcataaatat attgataaga ggtaaaggga gcaaattaca ttttttcata tcattaaaaa      120


<210>  307
<211>  120
<212>  DNA
<213>  Artificial Sequence

<220>
<223>  BRAF

<400>  307
atattaaaac tgactcacca ctgtcctctg tttgttgggc aggaagactc taacgatagg      60

tttttgtggt gacttggggt tgctccgtgc cacatctgtg ggattttgaa aaactgaaag      120


<210>  308
<211>  120
<212>  DNA
<213>  Artificial Sequence

<220>
<223>  BRAF

<400>  308
tttttgtggt gacttggggt tgctccgtgc cacatctgtg ggattttgaa aaactgaaag      60

agatgaaggt agcactgaaa ggctagaaga ggaagaagat gtaacggtat ccattgatgc      120


<210>  309
<211>  120
<212>  DNA
<213>  Artificial Sequence

<220>
<223>  BRAF

<400>  309
agatgaaggt agcactgaaa ggctagaaga ggaagaagat gtaacggtat ccattgatgc      60

agagctagaa acagaaaaat cagttccgtt ccccagagat tccaataact gttgttctct      120


<210>  310
<211>  120
<212>  DNA
<213>  Artificial Sequence

<220>
<223>  BRAF

<400>  310
agagctagaa acagaaaaat cagttccgtt ccccagagat tccaataact gttgttctct      60

ttgttggagt gcatctagct tgctggtgta ttcttcatag gcctataaaa taaagcagac     120


<210>  311
<211>  120
<212>  DNA
<213>  Artificial Sequence

<220>
<223>  BRAF

<400>  311
gtacaaatgt ttttataagt tcattttttt tcttttcaaa attactagat atgatactca      60

aaagcttacc tccagatata ttgatggtgg attatgctcc ccaccaaatt tgtccaatag     120


<210>  312
<211>  120
<212>  DNA
<213>  Artificial Sequence

<220>
<223>  BRAF

<400>  312
aaagcttacc tccagatata ttgatggtgg attatgctcc ccaccaaatt tgtccaatag      60

ggcctctata tgttcctgtg tcaacttaat catttgtttg atattccaca cctaaaaaat     120


<210>  313
<211>  120
<212>  DNA
<213>  Artificial Sequence

<220>
<223>  BRAF

<400>  313
ggcctctata tgttcctgtg tcaacttaat catttgtttg atattccaca cctaaaaaat      60

atttcaaaag aatttaaata aaaatcactt agtatatgaa ttagaaatat tttaacatag     120


<210>  314
<211>  120
<212>  DNA
<213>  Artificial Sequence

<220>
<223>  BRAF

<400>  314
gcccgcggag ctggcccgag aaggtggctg agggcatcaa gcccccaccg ccgcctcttt      60

ccaaaataaa caccagccag ccgccgagcc cggagtcggg agggcggcag ggtggcgcca     120

<210>  315
<211>  120
<212>  DNA
<213>  Artificial Sequence

<220>
<223>  BRAF

<400>  315
ccaaaataaa caccagccag ccgccgagcc cggagtcggg agggcggcag ggtggcgcca      60

gcactcacct cctccggaat ggcagggtcc gcagccgaag aggccgcggc gccggcgccg      120


<210>  316
<211>  120
<212>  DNA
<213>  Artificial Sequence

<220>
<223>  BRAF-E11

<400>  316
taaaagttgt taaacatatc ctattatgac ttgtcacaat gtcaccacat tacatactta      60

ccatgccact ttcccttgta gactgttcca aatgatccag atccaattct ttgtcccact      120


<210>  317
<211>  120
<212>  DNA
<213>  Artificial Sequence

<220>
<223>  BRAF-E11

<400>  317
tatgacttgt cacaatgtca ccacattaca tacttaccat gccactttcc cttgtagact      60

gttccaaatg atccagatcc aattctttgt cccactgtaa tctgcccatc aggaatctcc      120


<210>  318
<211>  120
<212>  DNA
<213>  Artificial Sequence

<220>
<223>  BRAF-E11

<400>  318
attacatact taccatgcca ctttcccttg tagactgttc caaatgatcc agatccaatt      60

ctttgtccca ctgtaatctg cccatcagga atctcccaat catcactcga gtcccgtcta      120


<210>  319
<211>  120
<212>  DNA
<213>  Artificial Sequence

<220>
<223>  BRAF-E11

<400>  319
cccttgtaga ctgttccaaa tgatccagat ccaattcttt gtcccactgt aatctgccca      60

tcaggaatct cccaatcatc actcgagtcc cgtctaccaa gtgttttctt gataaaaaca     120


<210>  320
<211>  120
<212>  DNA
<213>  Artificial Sequence

<220>
<223>  BRAF-E11

<400>  320
ccagatccaa ttctttgtcc cactgtaatc tgcccatcag gaatctccca atcatcactc      60

gagtcccgtc taccaagtgt tttcttgata aaaacagtaa aaaagtcaag tcaagccaaa     120


<210>  321
<211>  120
<212>  DNA
<213>  Artificial Sequence

<220>
<223>  BRAF-E11

<400>  321
gtaatctgcc catcaggaat ctcccaatca tcactcgagt cccgtctacc aagtgttttc      60

ttgataaaaa cagtaaaaaa gtcaagtcaa gccaaacaga aaaagaaaac cttatgtttc     120


<210>  322
<211>  120
<212>  DNA
<213>  Artificial Sequence

<220>
<223>  BRAF-E15

<400>  322
taactcagca gcatctcagg gccaaaaatt taatcagtgg aaaaatagcc tcaattctta      60

ccatccacaa aatggatcca gacaactgtt caaactgatg ggacccactc catcgagatt     120


<210>  323
<211>  120
<212>  DNA
<213>  Artificial Sequence

<220>
<223>  BRAF-E15

<400>  323
aaaatttaat cagtggaaaa atagcctcaa ttcttaccat ccacaaaatg gatccagaca      60

actgttcaaa ctgatgggac ccactccatc gagatttcac tgtagctaga ccaaaatcac     120

<210>  324
<211>  120
<212>  DNA
<213>  Artificial Sequence

<220>
<223>  BRAF-E15

<400>  324
cctcaattct taccatccac aaaatggatc cagacaactg ttcaaactga tgggacccac      60

tccatcgaga tttcactgta gctagaccaa aatcacctat ttttactgtg aggtcttcat      120


<210>  325
<211>  120
<212>  DNA
<213>  Artificial Sequence

<220>
<223>  BRAF-E15

<400>  325
gatgggaccc actccatcga gatttcactg tagctagacc aaaatcacct atttttactg      60

tgaggtcttc atgaagaaat atatctgagg tgtagtaagt aaaggaaaac agtagatctc      120


<210>  326
<211>  120
<212>  DNA
<213>  Artificial Sequence

<220>
<223>  BRAF-E15

<400>  326
tcactgtagc tagaccaaaa tcacctattt ttactgtgag gtcttcatga agaaatatat      60

ctgaggtgta gtaagtaaag gaaaacagta gatctcattt tcctatcaga gcaagcatta      120


<210>  327
<211>  120
<212>  DNA
<213>  Artificial Sequence

<220>
<223>  EGFR

<400>  327
cccctgactc cgtccagtat tgatcgggag agccggagcg agctcttcgg ggagcagcga      60

tgcgaccctc cgggacggcc ggggcagcgc tcctggcgct gctggctgcg ctctgcccgg      120


<210>  328
<211>  120
<212>  DNA
<213>  Artificial Sequence

<220>
<223>  EGFR

<400>  328
tgcgaccctc cgggacggcc ggggcagcgc tcctggcgct gctggctgcg ctctgcccgg      60

cgagtcgggc tctggaggaa aagaaaggta agggcgtgtc tcgccggctc ccgcgccgcc     120


<210>  329
<211>  120
<212>  DNA
<213>  Artificial Sequence

<220>
<223>  EGFR

<400>  329
tgcatttctc agtatttcat gtgatatctg tctttttctt ccagtttgcc aaggcacgag      60

taacaagctc acgcagttgg gcacttttga agatcatttt ctcagcctcc agaggatgtt     120


<210>  330
<211>  120
<212>  DNA
<213>  Artificial Sequence

<220>
<223>  EGFR

<400>  330
taacaagctc acgcagttgg gcacttttga agatcatttt ctcagcctcc agaggatgtt      60

caataactgt gaggtggtcc ttgggaattt ggaaattacc tatgtgcaga ggaattatga     120


<210>  331
<211>  120
<212>  DNA
<213>  Artificial Sequence

<220>
<223>  EGFR

<400>  331
caataactgt gaggtggtcc ttgggaattt ggaaattacc tatgtgcaga ggaattatga      60

tctttccttc ttaaaggttg gtgactttga ttttcctaca caaataaaat tggagaaaat     120


<210>  332
<211>  120
<212>  DNA
<213>  Artificial Sequence

<220>
<223>  EGFR

<400>  332
atcacgcatt tatgttttct cttcttagac catccaggag gtggctggtt atgtcctcat      60

tgccctcaac acagtggagc gaattccttt ggaaaacctg cagatcatca gaggaaatat     120

<210>  333
<211>  120
<212>  DNA
<213>  Artificial Sequence

<220>
<223>  EGFR

<400>  333
tgccctcaac acagtggagc gaattccttt ggaaaacctg cagatcatca gaggaaatat      60

gtactacgaa aattcctatg ccttagcagt cttatctaac tatgatgcaa ataaaaccgg      120


<210>  334
<211>  120
<212>  DNA
<213>  Artificial Sequence

<220>
<223>  EGFR

<400>  334
gtactacgaa aattcctatg ccttagcagt cttatctaac tatgatgcaa ataaaaccgg      60

actgaaggag ctgcccatga gaaatttaca gggtgagagg ctgggatgcc aaggctgggg      120


<210>  335
<211>  120
<212>  DNA
<213>  Artificial Sequence

<220>
<223>  EGFR

<400>  335
gggaatttaa aggagctgga aagagtgctc accgcagttc cattctcccg cagaaatcct      60

gcatggcgcc gtgcggttca gcaacaaccc tgccctgtgc aacgtggaga gcatccagtg      120


<210>  336
<211>  120
<212>  DNA
<213>  Artificial Sequence

<220>
<223>  EGFR

<400>  336
gcatggcgcc gtgcggttca gcaacaaccc tgccctgtgc aacgtggaga gcatccagtg      60

gcgggacata gtcagcagtg actttctcag caacatgtcg atggacttcc agaaccacct      120


<210>  337
<211>  120
<212>  DNA
<213>  Artificial Sequence

<220>
<223>  EGFR

<400>  337
gcgggacata gtcagcagtg actttctcag caacatgtcg atggacttcc agaaccacct      60

gggcagctgt aagtgtcgca tacacactat ctctgcctcc agctcctatg ggggacagct     120


<210>  338
<211>  120
<212>  DNA
<213>  Artificial Sequence

<220>
<223>  EGFR

<400>  338
gtcatcagtt tctcatcatt tcactgagat atgcatctat tacttttaca tttcaggcca      60

aaagtgtgat ccaagctgtc ccaatgggag ctgctggggt gcaggagagg agaactgcca     120


<210>  339
<211>  120
<212>  DNA
<213>  Artificial Sequence

<220>
<223>  EGFR

<400>  339
aaagtgtgat ccaagctgtc ccaatgggag ctgctggggt gcaggagagg agaactgcca      60

gaaacgtaag tcagtgaaca gcctcagacc catgtgtgac cgcccctctc ttccttcact     120


<210>  340
<211>  120
<212>  DNA
<213>  Artificial Sequence

<220>
<223>  EGFR

<400>  340
tccctgggaa atgatcctac cctcactctt cagctcacag ggaacctttg ctctttttca      60

gtgaccaaaa tcatctgtgc ccagcagtgc tccgggcgct gccgtggcaa gtcccccagt     120


<210>  341
<211>  120
<212>  DNA
<213>  Artificial Sequence

<220>
<223>  EGFR

<400>  341
gtgaccaaaa tcatctgtgc ccagcagtgc tccgggcgct gccgtggcaa gtcccccagt      60

gactgctgcc acaaccagtg tgctgcaggc tgcacaggcc cccgggagag cgactgcctg     120

<210>  342
<211>  120
<212>  DNA
<213>  Artificial Sequence

<220>
<223>  EGFR

<400>  342
gactgctgcc acaaccagtg tgctgcaggc tgcacaggcc cccgggagag cgactgcctg      60

gtaagatgcc cctccagcag cctccctgga gcaggctggg gctgcacccg ccccacccac      120


<210>  343
<211>  120
<212>  DNA
<213>  Artificial Sequence

<220>
<223>  EGFR

<400>  343
gctgagtgta cttacctcac ttgcccagcg tgtcctctct cctccatagg tctgccgcaa      60

attccgagac gaagccacgt gcaaggacac ctgcccccca ctcatgctct acaaccccac      120


<210>  344
<211>  120
<212>  DNA
<213>  Artificial Sequence

<220>
<223>  EGFR

<400>  344
attccgagac gaagccacgt gcaaggacac ctgcccccca ctcatgctct acaaccccac      60

cacgtaccag atggatgtga accccgaggg caaatacagc tttggtgcca cctgcgtgaa      120


<210>  345
<211>  120
<212>  DNA
<213>  Artificial Sequence

<220>
<223>  EGFR

<400>  345
cacgtaccag atggatgtga accccgaggg caaatacagc tttggtgcca cctgcgtgaa      60

gaagtgtccc cgtgagtcct cctctgtggg ccctctaact ggtcaggcat ccttgtcccg      120


<210>  346
<211>  120
<212>  DNA
<213>  Artificial Sequence

<220>
<223>  EGFR

<400>  346
tgtgtgaggc ccgagcacct ggtgccaccg tcatcacctt cctttcatgc tctcttcccc      60

aggtaattat gtggtgacag atcacggctc gtgcgtccga gcctgtgggg ccgacagcta     120


<210>  347
<211>  120
<212>  DNA
<213>  Artificial Sequence

<220>
<223>  EGFR

<400>  347
aggtaattat gtggtgacag atcacggctc gtgcgtccga gcctgtgggg ccgacagcta      60

tgagatggag gaagacggcg tccgcaagtg taagaagtgc gaagggcctt gccgcaaagg     120


<210>  348
<211>  120
<212>  DNA
<213>  Artificial Sequence

<220>
<223>  EGFR

<400>  348
tgagatggag gaagacggcg tccgcaagtg taagaagtgc gaagggcctt gccgcaaagg      60

taggaagccc gccggtgtgc ggacgaggct tgttctcggc tgctgaggct gggctctcat     120


<210>  349
<211>  120
<212>  DNA
<213>  Artificial Sequence

<220>
<223>  EGFR

<400>  349
tagctattct taatccaaca aatgtgaacg gaatacacgt ctctcttatc tctgcagtgt      60

gtaacggaat aggtattggt gaatttaaag actcactctc cataaatgct acgaatatta     120


<210>  350
<211>  120
<212>  DNA
<213>  Artificial Sequence

<220>
<223>  EGFR

<400>  350
gtaacggaat aggtattggt gaatttaaag actcactctc cataaatgct acgaatatta      60

aacacttcaa aaactgcacc tccatcagtg gcgatctcca catcctgccg gtggcattta     120

<210>  351
<211>  120
<212>  DNA
<213>  Artificial Sequence

<220>
<223>  EGFR

<400>  351
aacacttcaa aaactgcacc tccatcagtg gcgatctcca catcctgccg gtggcattta      60

gggggtgagt cacaggttca gttgcttgta taaagaaaaa caaaatctgc ctttttaact      120


<210>  352
<211>  120
<212>  DNA
<213>  Artificial Sequence

<220>
<223>  EGFR

<400>  352
taactggtag agattggtga tcaataatca ccctgttgtt tgtttcagtg actccttcac      60

acatactcct cctctggatc cacaggaact ggatattctg aaaaccgtaa aggaaatcac      120


<210>  353
<211>  120
<212>  DNA
<213>  Artificial Sequence

<220>
<223>  EGFR

<400>  353
acatactcct cctctggatc cacaggaact ggatattctg aaaaccgtaa aggaaatcac      60

aggtttgagc tgaattatca catgaatata aatgggaaat cagtgtttta gagagagaac      120


<210>  354
<211>  120
<212>  DNA
<213>  Artificial Sequence

<220>
<223>  EGFR

<400>  354
aactcctacg tggtgtgtgt ctgaagtctt tcatctgcct tacagggttt ttgctgattc      60

aggcttggcc tgaaaacagg acggacctcc atgcctttga gaacctagaa atcatacgcg      120


<210>  355
<211>  120
<212>  DNA
<213>  Artificial Sequence

<220>
<223>  EGFR

<400>  355
aggcttggcc tgaaaacagg acggacctcc atgcctttga gaacctagaa atcatacgcg      60

gcaggaccaa gcaacagtaa gttgaccaca gccaaagcct ggtagattac atttgccttt     120


<210>  356
<211>  120
<212>  DNA
<213>  Artificial Sequence

<220>
<223>  EGFR

<400>  356
ttttcttctc tccaatgtag tggtcagttt tctcttgcag tcgtcagcct gaacataaca      60

tccttgggat tacgctccct caaggagata agtgatggag atgtgataat ttcaggaaac     120


<210>  357
<211>  120
<212>  DNA
<213>  Artificial Sequence

<220>
<223>  EGFR

<400>  357
tccttgggat tacgctccct caaggagata agtgatggag atgtgataat ttcaggaaac      60

aaaaatttgt gctatgcaaa tacaataaac tggaaaaaac tgtttgggac ctccggtcag     120


<210>  358
<211>  120
<212>  DNA
<213>  Artificial Sequence

<220>
<223>  EGFR

<400>  358
aaaaatttgt gctatgcaaa tacaataaac tggaaaaaac tgtttgggac ctccggtcag      60

aaaaccaaaa ttataagcaa cagaggtgaa aacagctgca gtaagtcacc gctttctgtt     120


<210>  359
<211>  120
<212>  DNA
<213>  Artificial Sequence

<220>
<223>  EGFR

<400>  359
cccctcgggt ccctgctctg tcactgactg ctgtgaccca ctctgtctcc gcagaggcca      60

caggccaggt ctgccatgcc ttgtgctccc ccgagggctg ctggggcccg gagcccaggg     120

<210>  360
<211>  120
<212>  DNA
<213>  Artificial Sequence

<220>
<223>  EGFR

<400>  360
caggccaggt ctgccatgcc ttgtgctccc ccgagggctg ctggggcccg gagcccaggg      60

actgcgtctc ttgccggaat gtcagccgag gcagggaatg cgtggacaag tgcaaccttc      120


<210>  361
<211>  120
<212>  DNA
<213>  Artificial Sequence

<220>
<223>  EGFR

<400>  361
actgcgtctc ttgccggaat gtcagccgag gcagggaatg cgtggacaag tgcaaccttc      60

tggaggggta ggaggttatt tctttaatcc ccttgcgttg atcaaaaata aggctccagg      120


<210>  362
<211>  120
<212>  DNA
<213>  Artificial Sequence

<220>
<223>  EGFR

<400>  362
aatgtctcag gggtgggctg acgggtttcc tcttcctcct ctcagtgagc caaggagtt      60

tgtggagaac tctgagtgca tacagtgcca cccagagtgc ctgcctcagg ccatgaacat      120


<210>  363
<211>  120
<212>  DNA
<213>  Artificial Sequence

<220>
<223>  EGFR

<400>  363
tgtggagaac tctgagtgca tacagtgcca cccagagtgc ctgcctcagg ccatgaacat      60

cacctgcaca ggacgggtaa gagccccttg ctgctatcca cgtccatttc atgggaaggg      120


<210>  364
<211>  120
<212>  DNA
<213>  Artificial Sequence

<220>
<223>  EGFR

<400>  364
gaaagagaca tgcatgaaca tttttctcca ccttggtgca gggaccagac aactgtatcc      60

agtgtgccca ctacattgac ggcccccact gcgtcaagac ctgcccggca ggagtcatgg     120


<210>  365
<211>  120
<212>  DNA
<213>  Artificial Sequence

<220>
<223>  EGFR

<400>  365
agtgtgccca ctacattgac ggcccccact gcgtcaagac ctgcccggca ggagtcatgg      60

gagaaaacaa caccctggtc tggaagtacg cagacgccgg ccatgtgtgc cacctgtgcc     120


<210>  366
<211>  120
<212>  DNA
<213>  Artificial Sequence

<220>
<223>  EGFR

<400>  366
gagaaaacaa caccctggtc tggaagtacg cagacgccgg ccatgtgtgc cacctgtgcc      60

atccaaactg cacctacggg tgagtggaaa gtgaaggaga acagaacatt tcctctcttg     120


<210>  367
<211>  120
<212>  DNA
<213>  Artificial Sequence

<220>
<223>  EGFR

<400>  367
gccaaatata gaaagagggg atttagtcaa gatttaaatt aaaaatgtta gtggtcattt      60

ttctaatgtc tttctatttt ttcccaggtc ctaataaatc ttcactgtct gactttagtc     120


<210>  368
<211>  120
<212>  DNA
<213>  Artificial Sequence

<220>
<223>  EGFR

<400>  368
ttctaatgtc tttctatttt ttcccaggtc ctaataaatc ttcactgtct gactttagtc      60

tcccactaaa actgcatttc ctttctacaa tttcaatttc tccctttgct tcaaataaag     120

<210>  369
<211>  120
<212>  DNA
<213>  Artificial Sequence

<220>
<223>  EGFR

<400>  369
tattgaaatg tacttgtcca tctttctcca ggccaggaaa tgagagtctc aaagccatgt      60

tattctgcct ttttaaacta tcatcctgta atcaaagtaa tgatggcagc gtgtcccacc      120


<210>  370
<211>  120
<212>  DNA
<213>  Artificial Sequence

<220>
<223>  EGFR

<400>  370
tattctgcct ttttaaacta tcatcctgta atcaaagtaa tgatggcagc gtgtcccacc      60

agagcgggag cccagctgct caggagtcat gcttaggatg gatcccttct cttctgccgt      120


<210>  371
<211>  120
<212>  DNA
<213>  Artificial Sequence

<220>
<223>  EGFR

<400>  371
agagcgggag cccagctgct caggagtcat gcttaggatg gatcccttct cttctgccgt      60

cagagtttca gctgggttgg ggtggatgca gccacctcca tgcctggcct tctgcatctg      120


<210>  372
<211>  120
<212>  DNA
<213>  Artificial Sequence

<220>
<223>  EGFR

<400>  372
cagagtttca gctgggttgg ggtggatgca gccacctcca tgcctggcct tctgcatctg      60

tgatcatcac ggcctcctcc tgccactgag cctcatgcct tcacgtgtct gttccccccg      120


<210>  373
<211>  120
<212>  DNA
<213>  Artificial Sequence

<220>
<223>  EGFR

<400>  373
taaaaatctc caaaatatat gccaaagaag tagaatgaga aaaatgtata tttctctttc      60

acttcctaca gatgcactgg gccaggtctt gaaggctgtc caacgaatgg gtaagtgttc     120


<210>  374
<211>  120
<212>  DNA
<213>  Artificial Sequence

<220>
<223>  EGFR

<400>  374
acttcctaca gatgcactgg gccaggtctt gaaggctgtc caacgaatgg gtaagtgttc      60

acagctctgt gtcacatgga cctcgtcaag aatgaccaca ctgctgtggg tgaagatgct     120


<210>  375
<211>  120
<212>  DNA
<213>  Artificial Sequence

<220>
<223>  EGFR

<400>  375
gactggggag agcttgagaa agttggaaac gttgccttag aagcctgttt tttctccttt      60

tagaagctac atagtgtctc actttccaag atcattctac aagatgtcag tgcactgaaa     120


<210>  376
<211>  120
<212>  DNA
<213>  Artificial Sequence

<220>
<223>  EGFR

<400>  376
tagaagctac atagtgtctc actttccaag atcattctac aagatgtcag tgcactgaaa      60

catgcagggg cgtgttgagt gccaaggcca tggaatctgt cagcaacctc acccttcctt     120


<210>  377
<211>  120
<212>  DNA
<213>  Artificial Sequence

<220>
<223>  EGFR

<400>  377
ggaatctgtc agcaacctca cccttccttg ttcctccacc tcattccagg cctaagatcc      60

cgtccatcgc cactgggatg gtgggggccc tcctcttgct gctggtggtg gccctgggga     120

<210>  378
<211>  120
<212>  DNA
<213>  Artificial Sequence

<220>
<223>  EGFR

<400>  378
cgtccatcgc cactgggatg gtgggggccc tcctcttgct gctggtggtg gccctgggga      60

tcggcctctt catgcgaagg cgccacatcg ttcggaagcg cacgctgcgg aggctgctgc      120


<210>  379
<211>  120
<212>  DNA
<213>  Artificial Sequence

<220>
<223>  EGFR

<400>  379
tcggcctctt catgcgaagg cgccacatcg ttcggaagcg cacgctgcgg aggctgctgc      60

aggagaggga ggtgagtgcc agtcctgggt gggctcagga gccctcgcac cccgacagga      120


<210>  380
<211>  120
<212>  DNA
<213>  Artificial Sequence

<220>
<223>  EGFR

<400>  380
gctttccagc atggtgaggg ctgaggtgac ccttgtctct gtgttcttgt cccccccagc      60

ttgtggagcc tcttacaccc agtggagaag ctcccaacca agctctcttg aggatcttga      120


<210>  381
<211>  120
<212>  DNA
<213>  Artificial Sequence

<220>
<223>  EGFR

<400>  381
ttgtggagcc tcttacaccc agtggagaag ctcccaacca agctctcttg aggatcttga      60

aggaaactga attcaaaaag atcaaagtgc tgggctccgg tgcgttcggc acggtgtata      120


<210>  382
<211>  120
<212>  DNA
<213>  Artificial Sequence

<220>
<223>  EGFR

<400>  382
aggaaactga attcaaaaag atcaaagtgc tgggctccgg tgcgttcggc acggtgtata      60

aggtaaggtc cctggcacag gcctctgggc tgggccgcag ggcctctcat ggtctggtgg     120


<210>  383
<211>  120
<212>  DNA
<213>  Artificial Sequence

<220>
<223>  EGFR

<400>  383
cacaattgcc agttaacgtc ttccttctct ctctgtcata gggactctgg atcccagaag      60

gtgagaaagt taaaattccc gtcgctatca aggaattaag agaagcaaca tctccgaaag     120


<210>  384
<211>  120
<212>  DNA
<213>  Artificial Sequence

<220>
<223>  EGFR

<400>  384
gtgagaaagt taaaattccc gtcgctatca aggaattaag agaagcaaca tctccgaaag      60

ccaacaagga aatcctcgat gtgagtttct gctttgctgt gtgggggtcc atggctctga     120


<210>  385
<211>  120
<212>  DNA
<213>  Artificial Sequence

<220>
<223>  EGFR

<400>  385
cactgacgtg cctctccctc cctccaggaa gcctacgtga tggccagcgt ggacaacccc      60

cacgtgtgcc gcctgctggg catctgcctc acctccaccg tgcagctcat cacgcagctc     120


<210>  386
<211>  120
<212>  DNA
<213>  Artificial Sequence

<220>
<223>  EGFR

<400>  386
cacgtgtgcc gcctgctggg catctgcctc acctccaccg tgcagctcat cacgcagctc      60

atgcccttcg gctgcctcct ggactatgtc cgggaacaca aagacaatat tggctcccag     120

<210>  387
<211>  120
<212>  DNA
<213>  Artificial Sequence

<220>
<223>  EGFR

<400>  387
atgcccttcg gctgcctcct ggactatgtc cgggaacaca aagacaatat tggctcccag      60

tacctgctca actggtgtgt gcagatcgca aaggtaatca gggaagggag atacggggag      120


<210>  388
<211>  120
<212>  DNA
<213>  Artificial Sequence

<220>
<223>  EGFR

<400>  388
catgatgatc tgtccctcac agcagggtct tctctgtttc agggcatgaa ctacttggag      60

gaccgtcgct tggtgcaccg cgacctggca gccaggaacg tactggtgaa aacaccgcag      120


<210>  389
<211>  120
<212>  DNA
<213>  Artificial Sequence

<220>
<223>  EGFR

<400>  389
gaccgtcgct tggtgcaccg cgacctggca gccaggaacg tactggtgaa aacaccgcag      60

catgtcaaga tcacagattt tgggctggcc aaactgctgg gtgcggaaga gaaagaatac      120


<210>  390
<211>  120
<212>  DNA
<213>  Artificial Sequence

<220>
<223>  EGFR

<400>  390
catgtcaaga tcacagattt tgggctggcc aaactgctgg gtgcggaaga gaaagaatac      60

catgcagaag gaggcaaagt aaggaggtgg ctttaggtca gccagcattt tcctgacacc      120


<210>  391
<211>  120
<212>  DNA
<213>  Artificial Sequence

<220>
<223>  EGFR

<400>  391
aacagaggga aactaatagt tgtctcactg cctcatctct caccatccca aggtgcctat      60

caagtggatg gcattggaat caattttaca cagaatctat acccaccaga gtgatgtctg     120


<210>  392
<211>  120
<212>  DNA
<213>  Artificial Sequence

<220>
<223>  EGFR

<400>  392
caagtggatg gcattggaat caattttaca cagaatctat acccaccaga gtgatgtctg      60

gagctacggt gagtcataat cctgatgcta atgagtttgt actgaggcca agctggcttt     120


<210>  393
<211>  120
<212>  DNA
<213>  Artificial Sequence

<220>
<223>  EGFR

<400>  393
gttcattcat gatcccactg ccttcttttc ttgcttcatc ctctcagggg tgactgtttg      60

ggagttgatg acctttggat ccaagccata tgacggaatc cctgccagcg agatctcctc     120


<210>  394
<211>  120
<212>  DNA
<213>  Artificial Sequence

<220>
<223>  EGFR

<400>  394
ggagttgatg acctttggat ccaagccata tgacggaatc cctgccagcg agatctcctc      60

catcctggag aaaggagaac gcctccctca gccacccata tgtaccatcg atgtctacat     120


<210>  395
<211>  120
<212>  DNA
<213>  Artificial Sequence

<220>
<223>  EGFR

<400>  395
catcctggag aaaggagaac gcctccctca gccacccata tgtaccatcg atgtctacat      60

gatcatggtc aagtgtgagt gactggtggg tctgtccaca ctgcctagct gagccttggt     120

<210>  396
<211>  120
<212>  DNA
<213>  Artificial Sequence

<220>
<223>  EGFR

<400>  396
cagtgttcta attgcactgt tttttctcat tccttcccca ggctggatga tagacgcaga      60

tagtcgccca aagttccgtg agttgatcat cgaattctcc aaaatggccc gagaccccca      120


<210>  397
<211>  120
<212>  DNA
<213>  Artificial Sequence

<220>
<223>  EGFR

<400>  397
tagtcgccca aagttccgtg agttgatcat cgaattctcc aaaatggccc gagaccccca      60

gcgctacctt gtcattcagg tacaaattgc agtctgtgct tccattggga agagtccctc      120


<210>  398
<211>  120
<212>  DNA
<213>  Artificial Sequence

<220>
<223>  EGFR

<400>  398
cgggccattc taatagcctc aaaatctctg caccaggggg atgaaagaat gcatttgcca      60

agtcctacag actccaactt ctaccgtgcc ctgatggatg aagaagacat ggacgacgtg      120


<210>  399
<211>  120
<212>  DNA
<213>  Artificial Sequence

<220>
<223>  EGFR

<400>  399
agtcctacag actccaactt ctaccgtgcc ctgatggatg aagaagacat ggacgacgtg      60

gtggatgccg acgagtacct catcccacag cagggcttct tcagcagccc ctccacgtca      120


<210>  400
<211>  120
<212>  DNA
<213>  Artificial Sequence

<220>
<223>  EGFR

<400>  400
gtggatgccg acgagtacct catcccacag cagggcttct tcagcagccc ctccacgtca      60

cggactcccc tcctgagctc tctggtatga aatctctgtc tctctctctc tctcaagctg     120


<210>  401
<211>  120
<212>  DNA
<213>  Artificial Sequence

<220>
<223>  EGFR

<400>  401
attcaggaaa agtggatgag atgtggtaca agcattccat gggcaacttc tctgtttctt      60

tttcagagtg caaccagcaa caattccacc gtggcttgca ttgatagaaa tggggtatgt     120


<210>  402
<211>  120
<212>  DNA
<213>  Artificial Sequence

<220>
<223>  EGFR

<400>  402
tttcagagtg caaccagcaa caattccacc gtggcttgca ttgatagaaa tggggtatgt      60

atgaacacct tataagccag aatttacagc tctccactat ggctctattt tacatggaaa     120


<210>  403
<211>  120
<212>  DNA
<213>  Artificial Sequence

<220>
<223>  EGFR

<400>  403
taaccttccc tcatttcctc ctgcagctgc aaagctgtcc catcaaggaa gacagcttct      60

tgcagcgata cagctcagac cccacaggcg ccttgactga ggacagcata gacgacacct     120


<210>  404
<211>  120
<212>  DNA
<213>  Artificial Sequence

<220>
<223>  EGFR

<400>  404
tgcagcgata cagctcagac cccacaggcg ccttgactga ggacagcata gacgacacct      60

tcctcccagt gcctggtgag tggcttgtct ggaaacagtc ctgctcctca acctcctcga     120

<210>  405
<211>  120
<212>  DNA
<213>  Artificial Sequence

<220>
<223>  EGFR

<400>  405
tcctcccagt gcctggtgag tggcttgtct ggaaacagtc ctgctcctca acctcctcga      60

cccactcagc agcagccagt ctccagtgtc caagccaggt gctccctcca gcatctccag      120


<210>  406
<211>  120
<212>  DNA
<213>  Artificial Sequence

<220>
<223>  EGFR

<400>  406
cccactcagc agcagccagt ctccagtgtc caagccaggt gctccctcca gcatctccag      60

agggggaaac agtggcagat ttgcagacac agtgaagggc gtaaggagca gataaacaca      120


<210>  407
<211>  120
<212>  DNA
<213>  Artificial Sequence

<220>
<223>  EGFR

<400>  407
atttctttcc actttcagaa tacataaacc agtccgttcc caaaaggccc gctggctctg      60

tgcagaatcc tgtctatcac aatcagcctc tgaaccccgc gcccagcaga gacccacact      120


<210>  408
<211>  120
<212>  DNA
<213>  Artificial Sequence

<220>
<223>  EGFR

<400>  408
tgcagaatcc tgtctatcac aatcagcctc tgaaccccgc gcccagcaga gacccacact      60

accaggaccc ccacagcact gcagtgggca accccgagta tctcaacact gtccagccca      120


<210>  409
<211>  120
<212>  DNA
<213>  Artificial Sequence

<220>
<223>  EGFR

<400>  409
accaggaccc ccacagcact gcagtgggca accccgagta tctcaacact gtccagccca      60

cctgtgtcaa cagcacattc gacagccctg cccactgggc ccagaaaggc agccaccaaa     120


<210>  410
<211>  120
<212>  DNA
<213>  Artificial Sequence

<220>
<223>  EGFR

<400>  410
cctgtgtcaa cagcacattc gacagccctg cccactgggc ccagaaaggc agccaccaaa      60

ttagcctgga caaccctgac taccagcagg acttctttcc caaggaagcc aagccaaatg     120


<210>  411
<211>  120
<212>  DNA
<213>  Artificial Sequence

<220>
<223>  EGFR

<400>  411
ttagcctgga caaccctgac taccagcagg acttctttcc caaggaagcc aagccaaatg      60

gcatctttaa gggctccaca gctgaaaatg cagaatacct aagggtcgcg ccacaaagca     120


<210>  412
<211>  120
<212>  DNA
<213>  Artificial Sequence

<220>
<223>  EGFR

<400>  412
gcatctttaa gggctccaca gctgaaaatg cagaatacct aagggtcgcg ccacaaagca      60

gtgaatttat tggagcatga ccacggagga tagtatgagc cctaaaaatc cagactcttt     120


<210>  413
<211>  120
<212>  DNA
<213>  Artificial Sequence

<220>
<223>  EGFR-E18

<400>  413
gctttccagc atggtgaggg ctgaggtgac ccttgtctct gtgttcttgt cccccccagc      60

ttgtggagcc tcttacaccc agtggagaag ctcccaacca agctctcttg aggatcttga     120

<210>  414
<211>  120
<212>  DNA
<213>  Artificial Sequence

<220>
<223>  EGFR-E18

<400>  414
ggtgaccctt gtctctgtgt tcttgtcccc cccagcttgt ggagcctctt acacccagtg      60

gagaagctcc caaccaagct ctcttgagga tcttgaagga aactgaattc aaaaagatca      120


<210>  415
<211>  120
<212>  DNA
<213>  Artificial Sequence

<220>
<223>  EGFR-E18

<400>  415
accaagctct cttgaggatc ttgaaggaaa ctgaattcaa aaagatcaaa gtgctgggct      60

ccggtgcgtt cggcacggtg tataaggtaa ggtccctggc acaggcctct gggctgggcc      120


<210>  416
<211>  120
<212>  DNA
<213>  Artificial Sequence

<220>
<223>  EGFR-E18

<400>  416
aggaaactga attcaaaaag atcaaagtgc tgggctccgg tgcgttcggc acggtgtata      60

aggtaaggtc cctggcacag gcctctgggc tgggccgcag ggcctctcat ggtctggtgg      120


<210>  417
<211>  120
<212>  DNA
<213>  Artificial Sequence

<220>
<223>  EGFR-E19

<400>  417
cctcaccttc ggggtgcatc gctggtaaca tccacccaga tcactgggca gcatgtggca      60

ccatctcaca attgccagtt aacgtcttcc ttctctctct gtcataggga ctctggatcc      120


<210>  418
<211>  120
<212>  DNA
<213>  Artificial Sequence

<220>
<223>  EGFR-E19

<400> 418
gtaacatcca cccagatcac tgggcagcat gtggcaccat ctcacaattg ccagttaacg 60

tcttccttct ctctctgtca tagggactct ggatcccaga aggtgagaaa gttaaaattc 120


<210> 419
<211> 120
<212> DNA
<213> Artificial Sequence

<220>
<223> EGFR-E19

<400> 419
cagcatgtgg caccatctca caattgccag ttaacgtctt ccttctctct ctgtcatagg 60

gactctggat cccagaaggt gagaaagtta aaattcccgt cgctatcaag gaattaagag 120


<210> 420
<211> 120
<212> DNA
<213> Artificial Sequence

<220>
<223> EGFR-E19

<400> 420
tgccagttaa cgtcttcctt ctctctctgt catagggact ctggatccca gaaggtgaga 60

aagttaaaat tcccgtcgct atcaaggaat taagagaagc aacatctccg aaagccaaca 120


<210> 421
<211> 120
<212> DNA
<213> Artificial Sequence

<220>
<223> EGFR-E19

<400> 421
cagaaggtga gaaagttaaa attcccgtcg ctatcaagga attaagagaa gcaacatctc 60

cgaaagccaa caaggaaatc ctcgatgtga gtttctgctt tgctgtgtgg gggtccatgg 120


<210> 422
<211> 120
<212> DNA
<213> Artificial Sequence

<220>
<223> EGFR-E19

<400> 422
ccgtcgctat caaggaatta agagaagcaa catctccgaa agccaacaag gaaatcctcg 60

atgtgagttt ctgctttgct gtgtgggggt ccatggctct gaacctcagg cccacctttt 120

<210>  423
<211>  120
<212>  DNA
<213>  Artificial Sequence

<220>
<223>  EGFR-E19

<400>  423
   aagcaacatc tccgaaagcc aacaaggaaa tcctcgatgt gagtttctgc tttgctgtgt      60
   gggggtccat ggctctgaac ctcaggccca ccttttctca tgtctggcag ctgctctgct      120


<210>  424
<211>  120
<212>  DNA
<213>  Artificial Sequence

<220>
<223>  EGFR-E19

<400>  424
aggaaatcct cgatgtgagt ttctgctttg ctgtgtgggg gtccatggct ctgaacctca      60

ggcccacctt ttctcatgtc tggcagctgc tctgctctag accctgctca tctccacatc      120


<210>  425
<211>  120
<212>  DNA
<213>  Artificial Sequence

<220>
<223>  EGFR-E20

<400>  425
ccttctggcc accatgcgaa gccacactga cgtgcctctc cctccctcca ggaagcctac      60

gtgatggcca gcgtggacaa cccccacgtg tgccgcctgc tgggcatctg cctcacctcc      120


<210>  426
<211>  120
<212>  DNA
<213>  Artificial Sequence

<220>
<223>  EGFR-E20

<400>  426
ccttctggcc accatgcgaa gccacactga cgtgcctctc cctccctcca ggaagcctac      60

gtgatggcca gcgtggacaa cccccacgtg tgccgcctgc tgggcatctg cctcacctcc      120


<210>  427
<211>  120
<212>  DNA
<213>  Artificial Sequence

<220>
<223>  EGFR-E20

<400>  427
gtggacaacc cccacgtgtg ccgcctgctg ggcatctgcc tcacctccac cgtgcagctc        60

atcacgcagc tcatgccctt cggctgcctc ctggactatg tccgggaaca caaagacaat       120


<210>  428
<211>  120
<212>  DNA
<213>  Artificial Sequence

<220>
<223>  EGFR-E20

<400>  428
ctgctgggca tctgcctcac ctccaccgtg cagctcatca cgcagctcat gcccttcggc        60

tgcctcctgg actatgtccg ggaacacaaa gacaatattg gctcccagta cctgctcaac       120


<210>  429
<211>  120
<212>  DNA
<213>  Artificial Sequence

<220>
<223>  EGFR-E20

<400>  429
atgcccttcg gctgcctcct ggactatgtc cgggaacaca aagacaatat tggctcccag        60

tacctgctca actggtgtgt gcagatcgca aaggtaatca gggaagggag atacggggag       120


<210>  430
<211>  120
<212>  DNA
<213>  Artificial Sequence

<220>
<223>  EGFR-E20

<400>  430
tatgtccggg aacacaaaga caatattggc tcccagtacc tgctcaactg gtgtgtgcag        60

atcgcaaagg taatcaggga agggagatac ggggagggga gataaggagc caggatcctc       120


<210>  431
<211>  120
<212>  DNA
<213>  Artificial Sequence

<220>
<223>  EGFR-E21

<400>  431
agagcttctt cccatgatga tctgtccctc acagcagggt cttctctgtt tcagggcatg        60

aactacttgg aggaccgtcg cttggtgcac cgcgacctgg cagccaggaa cgtactggtg       120

<210>  432
<211>  120
<212>  DNA
<213>  Artificial Sequence

<220>
<223>  EGFR-E21

<400>  432
tccctcacag cagggtcttc tctgtttcag ggcatgaact acttggagga ccgtcgcttg        60

gtgcaccgcg acctggcagc caggaacgta ctggtgaaaa caccgcagca tgtcaagatc       120


<210>  433
<211>  120
<212>  DNA
<213>  Artificial Sequence

<220>
<223>  EGFR-E21

<400>  433
gaccgtcgct tggtgcaccg cgacctggca gccaggaacg tactggtgaa aacaccgcag        60

catgtcaaga tcacagattt tgggctggcc aaactgctgg gtgcggaaga gaaagaatac       120


<210>  434
<211>  120
<212>  DNA
<213>  Artificial Sequence

<220>
<223>  EGFR-E21

<400>  434
aaaacaccgc agcatgtcaa gatcacagat tttgggctgg ccaaactgct gggtgcggaa        60

gagaaagaat accatgcaga aggaggcaaa gtaaggaggt ggctttaggt cagccagcat       120


<210>  435
<211>  120
<212>  DNA
<213>  Artificial Sequence

<220>
<223>  EGFR-E21

<400>  435
acagattttg ggctggccaa actgctgggt gcggaagaga aagaatacca tgcagaagga        60

ggcaaagtaa ggaggtggct ttaggtcagc cagcattttc ctgacaccag ggaccaggct       120


<210>  436
<211>  120
<212>  DNA
<213>  Artificial Sequence

<220>
<223>  ERBB2

<400>  436
gccctcccag ccgggtccag ccggagccat ggggccggag ccgcagtgag caccatggag      60

ctggcggcct tgtgccgctg ggggctcctc ctcgccctct tgccccccgg agccgcgagc      120


<210>  437
<211>  120
<212>  DNA
<213>  Artificial Sequence

<220>
<223>  ERBB2

<400>  437
ctggcggcct tgtgccgctg ggggctcctc ctcgccctct tgccccccgg agccgcgagc      60

acccaaggtg ggtctggtgt ggggagggga cggagcagcg gcgggaccct gccctgtgga      120


<210>  438
<211>  120
<212>  DNA
<213>  Artificial Sequence

<220>
<223>  ERBB2

<400>  438
catctgctct ctcctgccag tgtgcaccgg cacagacatg aagctgcggc tccctgccag      60

tcccgagacc cacctggaca tgctccgcca cctctaccag ggctgccagg tggtgcaggg      120


<210>  439
<211>  120
<212>  DNA
<213>  Artificial Sequence

<220>
<223>  ERBB2

<400>  439
tcccgagacc cacctggaca tgctccgcca cctctaccag ggctgccagg tggtgcaggg      60

aaacctggaa ctcacctacc tgcccaccaa tgccagcctg tccttcctgc aggtgaggcc      120


<210>  440
<211>  120
<212>  DNA
<213>  Artificial Sequence

<220>
<223>  ERBB2

<400>  440
aaacctggaa ctcacctacc tgcccaccaa tgccagcctg tccttcctgc aggtgaggcc      60

cgtgggcaac ccagccaggc cctgcctcca gctgggctga gccctctgtt tacaggtggg      120

<210>  441
<211>  120
<212>  DNA
<213>  Artificial Sequence

<220>
<223>  ERBB2

<400>  441
ttccccctcc caggatatcc aggaggtgca gggctacgtg ctcatcgctc acaaccaagt      60

gaggcaggtc ccactgcaga ggctgcggat tgtgcgaggc acccagctct ttgaggacaa      120


<210>  442
<211>  120
<212>  DNA
<213>  Artificial Sequence

<220>
<223>  ERBB2

<400>  442
gaggcaggtc ccactgcaga ggctgcggat tgtgcgaggc acccagctct ttgaggacaa      60

ctatgccctg gccgtgctag acaatggaga cccgctgaac aataccaccc ctgtcacagg      120


<210>  443
<211>  120
<212>  DNA
<213>  Artificial Sequence

<220>
<223>  ERBB2

<400>  443
ctatgccctg gccgtgctag acaatggaga cccgctgaac aataccaccc ctgtcacagg      60

ggcctcccca ggaggcctgc gggagctgca gcttcgaagc ctcacaggtg gccttcaccg      120


<210>  444
<211>  120
<212>  DNA
<213>  Artificial Sequence

<220>
<223>  ERBB2

<400>  444
agcagaaggt gacagaaggg gaaagggtcc tctgatcatt gctcacccca cagagatctt      60

gaaaggaggg gtcttgatcc agcggaaccc ccagctctgc taccaggaca cgattttgtg      120


<210>  445
<211>  120
<212>  DNA
<213>  Artificial Sequence

<220>
<223>  ERBB2

<400>  445
gaaaggaggg gtcttgatcc agcggaaccc ccagctctgc taccaggaca cgattttgtg      60

gaaggacatc ttccacaaga acaaccagct ggctctcaca ctgatagaca ccaaccgctc     120


<210>  446
<211>  120
<212>  DNA
<213>  Artificial Sequence

<220>
<223>  ERBB2

<400>  446
gaaggacatc ttccacaaga acaaccagct ggctctcaca ctgatagaca ccaaccgctc      60

tcgggcctgt aagccatgcc cctccctgct gcctcttctc tcagacagcc tgaccccagc     120


<210>  447
<211>  120
<212>  DNA
<213>  Artificial Sequence

<220>
<223>  ERBB2

<400>  447
ccgtcctctc gctgttagac atctctctca ctgcctgtct ctggttctgt cctcaggcca      60

cccctgttct ccgatgtgta agggctcccg ctgctgggga gagagttctg aggattgtca     120


<210>  448
<211>  120
<212>  DNA
<213>  Artificial Sequence

<220>
<223>  ERBB2

<400>  448
cccctgttct ccgatgtgta agggctcccg ctgctgggga gagagttctg aggattgtca      60

gagccgtgag tctcagggag gcctggagtc agggaagggg agggctgggg ccgggtggaa     120


<210>  449
<211>  120
<212>  DNA
<213>  Artificial Sequence

<220>
<223>  ERBB2

<400>  449
tgctgatgag ggtctggtgc ccagggcgcc actcagccct catcctgccc tttgcccaac      60

agtgacgcgc actgtctgtg ccggtggctg tgcccgctgc aaggggccac tgcccactga     120

<210>  450
<211>  120
<212>  DNA
<213>  Artificial Sequence

<220>
<223>  ERBB2

<400>  450
agtgacgcgc actgtctgtg ccggtggctg tgcccgctgc aaggggccac tgcccactga      60

ctgctgccat gagcagtgtg ctgccggctg cacgggcccc aagcactctg actgcctggt      120


<210>  451
<211>  120
<212>  DNA
<213>  Artificial Sequence

<220>
<223>  ERBB2

<400>  451
ctgctgccat gagcagtgtg ctgccggctg cacgggcccc aagcactctg actgcctggt      60

atgtgcctct gctttgtgcc caatgtgctc taccccccag gatgcaaggg gtgggcaccc      120


<210>  452
<211>  120
<212>  DNA
<213>  Artificial Sequence

<220>
<223>  ERBB2

<400>  452
cacaccaggg caaaacagca cagtgaaagc cagccacctg tccccccagg cctgcctcca      60

cttcaaccac agtggcatct gtgagctgca ctgcccagcc ctggtcacct acaacacaga      120


<210>  453
<211>  120
<212>  DNA
<213>  Artificial Sequence

<220>
<223>  ERBB2

<400>  453
cttcaaccac agtggcatct gtgagctgca ctgcccagcc ctggtcacct acaacacaga      60

cacgtttgag tccatgccca atcccgaggg ccggtataca ttcggcgcca gctgtgtgac      120


<210>  454
<211>  120
<212>  DNA
<213>  Artificial Sequence

<220>
<223>  ERBB2

<400>  454
cacgtttgag tccatgccca atcccgaggg ccggtataca ttcggcgcca gctgtgtgac      60

tgcctgtccc tgtgagtgcc agggagaaac acagttttct cattttggtg gggaggtttg      120


<210>  455
<211>  120
<212>  DNA
<213>  Artificial Sequence

<220>
<223>  ERBB2

<400>  455
ctgctcatgg tggtgcacga agggccaggg tatgtggcta catgttcctg atctccttag      60

acaactacct ttctacggac gtgggatcct gcaccctcgt ctgccccctg cacaaccaag      120


<210>  456
<211>  120
<212>  DNA
<213>  Artificial Sequence

<220>
<223>  ERBB2

<400>  456
acaactacct ttctacggac gtgggatcct gcaccctcgt ctgccccctg cacaaccaag      60

aggtgacagc agaggatgga acacagcggt gtgagaagtg cagcaagccc tgtgcccgag      120


<210>  457
<211>  120
<212>  DNA
<213>  Artificial Sequence

<220>
<223>  ERBB2

<400>  457
aggtgacagc agaggatgga acacagcggt gtgagaagtg cagcaagccc tgtgcccgag      60

gtacccactc actgcccccg aggccagctg cagttcctgt ccctctgcgc atgcagcctg      120


<210>  458
<211>  120
<212>  DNA
<213>  Artificial Sequence

<220>
<223>  ERBB2

<400>  458
cctgtcccgg tatgaaggct gagacggccc cttccccacc cacccccacc tcctcagtgt      60

gctatggtct gggcatggag cacttgcgag aggtgagggc agttaccagt gccaatatcc      120

<210> 459
<211> 120
<212> DNA
<213> Artificial Sequence

<220>
<223> ERBB2

<400> 459
gctatggtct gggcatggag cacttgcgag aggtgagggc agttaccagt gccaatatcc 60

aggagtttgc tggctgcaag aagatctttg ggagcctggc atttctgccg gagagctttg 120


<210> 460
<211> 120
<212> DNA
<213> Artificial Sequence

<220>
<223> ERBB2

<400> 460
aggagtttgc tggctgcaag aagatctttg ggagcctggc atttctgccg gagagctttg 60

atgggtaaga gtgggcacga tgacctgaga cagtgtcagg gcagacagag tcctgaggat 120


<210> 461
<211> 120
<212> DNA
<213> Artificial Sequence

<220>
<223> ERBB2

<400> 461
agtgatgtcc accctgttcc tggccctgct gactcctctc ctgacccctc cagggaccca 60

gcctccaaca ctgccccgct ccagccagag cagctccaag tgtttgagac tctggaagag 120


<210> 462
<211> 120
<212> DNA
<213> Artificial Sequence

<220>
<223> ERBB2

<400> 462
gcctccaaca ctgccccgct ccagccagag cagctccaag tgtttgagac tctggaagag 60

atcacaggtg ggctctgtct ctgcatcctg ttctgcaggg gctgggagtc cttgtcctgt 120


<210> 463
<211> 120
<212> DNA
<213> Artificial Sequence

<220>
<223> ERBB2

<400>  463
tccttgtcct gtccccactc ctttaatctc accctctgcc tgcaggttac ctatacatct      60

cagcatggcc ggacagcctg cctgacctca gcgtcttcca gaacctgcaa gtaatccggg      120


<210>  464
<211>  120
<212>  DNA
<213>  Artificial Sequence

<220>
<223>  ERBB2

<400>  464
cagcatggcc ggacagcctg cctgacctca gcgtcttcca gaacctgcaa gtaatccggg      60

gacgaattct gcacaagtga gcactgagaa agagggggcc tgatggggag gagtcccagg      120


<210>  465
<211>  120
<212>  DNA
<213>  Artificial Sequence

<220>
<223>  ERBB2

<400>  465
tgcccacctt tctcccatag tggcgcctac tcgctgaccc tgcaagggct gggcatcagc      60

tggctggggc tgcgctcact gagggaactg ggcagtggac tggccctcat ccaccataac      120


<210>  466
<211>  120
<212>  DNA
<213>  Artificial Sequence

<220>
<223>  ERBB2

<400>  466
tggctggggc tgcgctcact gagggaactg ggcagtggac tggccctcat ccaccataac      60

acccacctct gcttcgtgca cacggtgccc tgggaccagc tctttcggaa cccgcaccaa      120


<210>  467
<211>  120
<212>  DNA
<213>  Artificial Sequence

<220>
<223>  ERBB2

<400>  467
acccacctct gcttcgtgca cacggtgccc tgggaccagc tctttcggaa cccgcaccaa      60

gctctgctcc acactgccaa ccggccagag gacgagtgtg gtaagacagg gagcccagtg      120

<210>  468
<211>  120
<212>  DNA
<213>  Artificial Sequence

<220>
<223>  ERBB2

<400>  468
tggacctggg gcctccccta aaagtcccct gcggtccctt cctcctcact gcagtgggcg      60

agggcctggc ctgccaccag ctgtgcgccc gagggcactg ctggggtcca gggcccaccc     120


<210>  469
<211>  120
<212>  DNA
<213>  Artificial Sequence

<220>
<223>  ERBB2

<400>  469
agggcctggc ctgccaccag ctgtgcgccc gagggcactg ctggggtcca gggcccaccc      60

agtgtgtcaa ctgcagccag ttccttcggg gccaggagtg cgtggaggaa tgccgagtac     120


<210>  470
<211>  120
<212>  DNA
<213>  Artificial Sequence

<220>
<223>  ERBB2

<400>  470
agtgtgtcaa ctgcagccag ttccttcggg gccaggagtg cgtggaggaa tgccgagtac      60

tgcaggggta tgaggggcgg aggagagggt ggctggaggg gtgcatgggg ctcctctcag     120


<210>  471
<211>  120
<212>  DNA
<213>  Artificial Sequence

<220>
<223>  ERBB2

<400>  471
catggggctc ctctcagacc ccctcaccac tgtcccttct ctcaggctcc ccagggagta      60

tgtgaatgcc aggcactgtt tgccgtgcca ccctgagtgt cagccccaga atggctcagt     120


<210>  472
<211>  120
<212>  DNA
<213>  Artificial Sequence

<220>
<223>  ERBB2

<400>  472
tgtgaatgcc aggcactgtt tgccgtgcca ccctgagtgt cagccccaga atggctcagt        60

gacctgtttt ggaccggtga gctgctggcg ggctcagagc tgggtggagg ggggcagcga       120


<210>  473
<211>  120
<212>  DNA
<213>  Artificial Sequence

<220>
<223>  ERBB2

<400>  473
agtgccagcc ccccacaaat cttttctgcc ccccccagga ggctgaccag tgtgtggcct        60

gtgcccacta taaggaccct cccttctgcg tggcccgctg ccccagcggt gtgaaacctg       120


<210>  474
<211>  120
<212>  DNA
<213>  Artificial Sequence

<220>
<223>  ERBB2

<400>  474
gtgcccacta taaggaccct cccttctgcg tggcccgctg ccccagcggt gtgaaacctg        60

acctctccta catgcccatc tggaagtttc cagatgagga gggcgcatgc cagccttgcc       120


<210>  475
<211>  120
<212>  DNA
<213>  Artificial Sequence

<220>
<223>  ERBB2

<400>  475
acctctccta catgcccatc tggaagtttc cagatgagga gggcgcatgc cagccttgcc        60

ccatcaactg cacccactcg tgagtccaac ggtcttttct gcagaaagga ggactttcct       120


<210>  476
<211>  120
<212>  DNA
<213>  Artificial Sequence

<220>
<223>  ERBB2

<400>  476
aggctggaaa ggtggttccc aagagggtgg ttcccagaat tgttgatgag actgtttctc        60

ctgcagctgt gtggacctgg atgacaaggg ctgccccgcc gagcagagag ccaggttggc       120

<210>  477
<211>  120
<212>  DNA
<213>  Artificial Sequence

<220>
<223>  ERBB2

<400>  477
ctgcagctgt gtggacctgg atgacaaggg ctgccccgcc gagcagagag ccaggttggc      60

ctggacccca ggatgtaccc ttcattgccc ttcactcccc cactggatgc tgggtggtca      120


<210>  478
<211>  120
<212>  DNA
<213>  Artificial Sequence

<220>
<223>  ERBB2

<400>  478
cctcccaccc caaactagcc ctcaatccct gaccctggct tccgccccca gccctctgac      60

gtccatcatc tctgcggtgg ttggcattct gctggtcgtg gtcttggggg tggtctttgg      120


<210>  479
<211>  120
<212>  DNA
<213>  Artificial Sequence

<220>
<223>  ERBB2

<400>  479
gtccatcatc tctgcggtgg ttggcattct gctggtcgtg gtcttggggg tggtctttgg      60

gatcctcatc aagcgacggc agcagaagat ccggaagtac acgatgcgga gactgctgca      120


<210>  480
<211>  120
<212>  DNA
<213>  Artificial Sequence

<220>
<223>  ERBB2

<400>  480
gatcctcatc aagcgacggc agcagaagat ccggaagtac acgatgcgga gactgctgca      60

ggaaacggag gtgaggcggg gtgaagtcct cccagcccgc gtggggtctg caccggcccc      120


<210>  481
<211>  120
<212>  DNA
<213>  Artificial Sequence

<220>
<223>  ERBB2

<400>  481
ccagcccgcg tggggtctgc accggccccc ggcactgacc caccaccccc tcaccccagc      60

tggtggagcc gctgacacct agcggagcga tgcccaacca ggcgcagatg cggatcctga     120


<210>  482
<211>  120
<212>  DNA
<213>  Artificial Sequence

<220>
<223>  ERBB2

<400>  482
tggtggagcc gctgacacct agcggagcga tgcccaacca ggcgcagatg cggatcctga      60

aagagacgga gctgaggaag gtgaaggtgc ttggatctgg cgcttttggc acagtctaca     120


<210>  483
<211>  120
<212>  DNA
<213>  Artificial Sequence

<220>
<223>  ERBB2

<400>  483
aagagacgga gctgaggaag gtgaaggtgc ttggatctgg cgcttttggc acagtctaca      60

aggtcagggc caggtcctgg ggtgggcggc cccagaggat gggggcggtg cctggagggg     120


<210>  484
<211>  120
<212>  DNA
<213>  Artificial Sequence

<220>
<223>  ERBB2

<400>  484
gcccacgctc ttctcactca tatcctcctc tttctgccca gggcatctgg atccctgatg      60

gggagaatgt gaaaattcca gtggccatca aagtgttgag ggaaaacaca tcccccaaag     120


<210>  485
<211>  120
<212>  DNA
<213>  Artificial Sequence

<220>
<223>  ERBB2

<400>  485
gggagaatgt gaaaattcca gtggccatca aagtgttgag ggaaaacaca tcccccaaag      60

ccaacaaaga aatcttagac gtaagcccct ccaccctctc ctgctaggag gacaggaagg     120

<210>  486
<211>  120
<212>  DNA
<213>  Artificial Sequence

<220>
<223>  ERBB2

<400>  486
accctctcag cgtacccttg tccccaggaa gcatacgtga tggctggtgt gggctcccca      60

tatgtctccc gccttctggg catctgcctg acatccacgg tgcagctggt gacacagctt      120


<210>  487
<211>  120
<212>  DNA
<213>  Artificial Sequence

<220>
<223>  ERBB2

<400>  487
tatgtctccc gccttctggg catctgcctg acatccacgg tgcagctggt gacacagctt      60

atgccctatg gctgcctctt agaccatgtc cgggaaaacc gcggacgcct gggctcccag      120


<210>  488
<211>  120
<212>  DNA
<213>  Artificial Sequence

<220>
<223>  ERBB2

<400>  488
atgccctatg gctgcctctt agaccatgtc cgggaaaacc gcggacgcct gggctcccag      60

gacctgctga actggtgtat gcagattgcc aaggtatgca cctgggctct ttgcaggtct      120


<210>  489
<211>  120
<212>  DNA
<213>  Artificial Sequence

<220>
<223>  ERBB2

<400>  489
caggccctcc cagaaggtct acatgggtgc ttcccattcc aggggatgag ctacctggag      60

gatgtgcggc tcgtacacag ggacttggcc gctcggaacg tgctggtcaa gagtcccaac      120


<210>  490
<211>  120
<212>  DNA
<213>  Artificial Sequence

<220>
<223>  ERBB2

<400>  490
gatgtgcggc tcgtacacag ggacttggcc gctcggaacg tgctggtcaa gagtcccaac        60

catgtcaaaa ttacagactt cgggctggct cggctgctgg acattgacga gacagagtac       120


<210>  491
<211>  120
<212>  DNA
<213>  Artificial Sequence

<220>
<223>  ERBB2

<400>  491
catgtcaaaa ttacagactt cgggctggct cggctgctgg acattgacga gacagagtac        60

catgcagatg ggggcaaggt taggtgaagg accaaggagc agaggaggct gggtggagtg       120


<210>  492
<211>  120
<212>  DNA
<213>  Artificial Sequence

<220>
<223>  ERBB2

<400>  492
ggccacctcc ccacaacaca cagttggagg acttcctctt ctgccctccc aggtgcccat        60

caagtggatg gcgctggagt ccattctccg ccggcggttc acccaccaga gtgatgtgtg       120


<210>  493
<211>  120
<212>  DNA
<213>  Artificial Sequence

<220>
<223>  ERBB2

<400>  493
caagtggatg gcgctggagt ccattctccg ccggcggttc acccaccaga gtgatgtgtg        60

gagttatggt gtgtgatggg gggtgttggg aggggtgggt gaggagccat ggctggaggg       120


<210>  494
<211>  120
<212>  DNA
<213>  Artificial Sequence

<220>
<223>  ERBB2

<400>  494
taaagctccc tctggccctc ccactcctga ccctgtctct gccttaggtg tgactgtgtg        60

ggagctgatg acttttgggg ccaaacctta cgatgggatc ccagcccggg agatccctga       120

<210>  495
<211>  120
<212>  DNA
<213>  Artificial Sequence

<220>
<223>  ERBB2

<400>  495
ggagctgatg acttttgggg ccaaacctta cgatgggatc ccagcccggg agatccctga      60

cctgctggaa aagggggagc ggctgcccca gccccccatc tgcaccattg atgtctacat      120


<210>  496
<211>  120
<212>  DNA
<213>  Artificial Sequence

<220>
<223>  ERBB2

<400>  496
cctgctggaa aagggggagc ggctgcccca gccccccatc tgcaccattg atgtctacat      60

gatcatggtc aaatgtgcgt ggctgagctg tgctggctgc ctggaggagg gtgggaggtc      120


<210>  497
<211>  120
<212>  DNA
<213>  Artificial Sequence

<220>
<223>  ERBB2

<400>  497
cagggcctgg gactagcatg ctgacctccc tcctgcccca ggttggatga ttgactctga      60

atgtcggcca agattccggg agttggtgtc tgaattctcc cgcatggcca gggaccccca      120


<210>  498
<211>  120
<212>  DNA
<213>  Artificial Sequence

<220>
<223>  ERBB2

<400>  498
atgtcggcca agattccggg agttggtgtc tgaattctcc cgcatggcca gggaccccca      60

gcgctttgtg gtcatccagg tactgggcct ctgtgcccca tccctgcctg tggctaagag      120


<210>  499
<211>  120
<212>  DNA
<213>  Artificial Sequence

<220>
<223>  ERBB2

<400>  499
ccatcctgcc tctccttcct ccacagaatg aggacttggg cccagccagt cccttggaca      60

gcaccttcta ccgctcactg ctggaggacg atgacatggg ggacctggtg gatgctgagg     120


<210>  500
<211>  120
<212>  DNA
<213>  Artificial Sequence

<220>
<223>  ERBB2

<400>  500
gcaccttcta ccgctcactg ctggaggacg atgacatggg ggacctggtg gatgctgagg      60

agtatctggt accccagcag ggcttcttct gtccagaccc tgccccgggc gctgggggca     120


<210>  501
<211>  120
<212>  DNA
<213>  Artificial Sequence

<220>
<223>  ERBB2

<400>  501
agtatctggt accccagcag ggcttcttct gtccagaccc tgccccgggc gctgggggca      60

tggtccacca caggcaccgc agctcatcta ccagggtcag tgccctcggt cacactgtgt     120


<210>  502
<211>  120
<212>  DNA
<213>  Artificial Sequence

<220>
<223>  ERBB2

<400>  502
catgactttc tttcttgtcc ccagagtggc ggtggggacc tgacactagg gctggagccc      60

tctgaagagg aggcccccag gtctccactg gcaccctccg aaggggctgg ctccgatgta     120


<210>  503
<211>  120
<212>  DNA
<213>  Artificial Sequence

<220>
<223>  ERBB2

<400>  503
tctgaagagg aggcccccag gtctccactg gcaccctccg aaggggctgg ctccgatgta      60

tttgatggtg acctgggaat gggggcagcc aaggggctgc aaagcctccc cacacatgac     120

<210>  504
<211>  120
<212>  DNA
<213>  Artificial Sequence

<220>
<223>  ERBB2

<400>  504
tttgatggtg acctgggaat gggggcagcc aaggggctgc aaagcctccc cacacatgac      60

cccagccctc tacagcggta cagtgaggac cccacagtac ccctgccctc tgagactgat      120


<210>  505
<211>  120
<212>  DNA
<213>  Artificial Sequence

<220>
<223>  ERBB2

<400>  505
cccagccctc tacagcggta cagtgaggac cccacagtac ccctgccctc tgagactgat      60

ggctacgttg cccccctgac ctgcagcccc cagcctggta tggagtccag tctaagcaga      120


<210>  506
<211>  120
<212>  DNA
<213>  Artificial Sequence

<220>
<223>  ERBB2

<400>  506
ccctaatggg tcaccttctc ttgacctttc agaatatgtg aaccagccag atgttcggcc      60

ccagccccct tcgccccgag agggccctct gcctgctgcc cgacctgctg gtgccactct      120


<210>  507
<211>  120
<212>  DNA
<213>  Artificial Sequence

<220>
<223>  ERBB2

<400>  507
ccagccccct tcgccccgag agggccctct gcctgctgcc cgacctgctg gtgccactct      60

ggaaaggccc aagactctct ccccagggaa gaatggggtc gtcaaagacg tttttgcctt      120


<210>  508
<211>  120
<212>  DNA
<213>  Artificial Sequence

<220>
<223>  ERBB2

<400>  508
ggaaaggccc aagactctct ccccagggaa gaatggggtc gtcaaagacg tttttgcctt      60

tgggggtgcc gtggagaacc ccgagtactt gacaccccag ggaggagctg cccctcagcc     120


<210>  509
<211>  120
<212>  DNA
<213>  Artificial Sequence

<220>
<223>  ERBB2

<400>  509
tgggggtgcc gtggagaacc ccgagtactt gacaccccag ggaggagctg cccctcagcc      60

ccaccctcct cctgccttca gcccagcctt cgacaacctc tattactggg accaggaccc     120


<210>  510
<211>  120
<212>  DNA
<213>  Artificial Sequence

<220>
<223>  ERBB2

<400>  510
ccaccctcct cctgccttca gcccagcctt cgacaacctc tattactggg accaggaccc      60

accagagcgg ggggctccac ccagcacctt caaagggaca cctacggcag agaacccaga     120


<210>  511
<211>  120
<212>  DNA
<213>  Artificial Sequence

<220>
<223>  ERBB2

<400>  511
accagagcgg ggggctccac ccagcacctt caaagggaca cctacggcag agaacccaga      60

gtacctgggt ctggacgtgc cagtgtgaac cagaaggcca agtccgcaga agccctgatg     120


<210>  512
<211>  120
<212>  DNA
<213>  Artificial Sequence

<220>
<223>  ERBB4

<400>  512
aaaaaccaca actgagctta caccacagta ttccggtgtc tgtaaggtgg aggcggcagc      60

acagtgcctg gcttcaggga gaactcagag aggtattcag gattctctgc cacaataggc     120

<210>  513
<211>  120
<212>  DNA
<213>  Artificial Sequence

<220>
<223>  ERBB4

<400>  513
acagtgcctg gcttcaggga gaactcagag aggtattcag gattctctgc cacaataggc      60

cggatccgcc cattctgttt ataaaaatat tttgtgctgt actcctgcag gtagtctggg      120


<210>  514
<211>  120
<212>  DNA
<213>  Artificial Sequence

<220>
<223>  ERBB4

<400>  514
cggatccgcc cattctgttt ataaaaatat tttgtgctgt actcctgcag gtagtctggg      60

tgctgaaggg tgctccgagg tggcaggctg tggttccagt agtcagggtt gtcaaacgct      120


<210>  515
<211>  120
<212>  DNA
<213>  Artificial Sequence

<220>
<223>  ERBB4

<400>  515
tgctgaaggg tgctccgagg tggcaggctg tggttccagt agtcagggtt gtcaaacgct      60

ttcttggcct tctctggcat tgacagtatg ttgttcttca ggtactcagc ttttcccaag      120


<210>  516
<211>  120
<212>  DNA
<213>  Artificial Sequence

<220>
<223>  ERBB4

<400>  516
ttcttggcct tctctggcat tgacagtatg ttgttcttca ggtactcagc ttttcccaag      60

gtgttggcaa aggtgttgag gtacagtggc tcattcacat actcatcctc ggccttgggt      120


<210>  517
<211>  120
<212>  DNA
<213>  Artificial Sequence

<220>
<223>  ERBB4

<400>  517
gtgttggcaa aggtgttgag gtacagtggc tcattcacat actcatcctc ggccttgggt      60

ggaccattgg atgcattgtg atattcggga ttatccaatg cttgaaggtc tccatttttt     120


<210>  518
<211>  120
<212>  DNA
<213>  Artificial Sequence

<220>
<223>  ERBB4

<400>  518
ggaccattgg atgcattgtg atattcggga ttatccaatg cttgaaggtc tccatttttt      60

ctccgagaaa caaaagggtt ctcctccact ggattcaggt attctaaagg aataaaaaaa     120


<210>  519
<211>  120
<212>  DNA
<213>  Artificial Sequence

<220>
<223>  ERBB4

<400>  519
tggcgatcgt ttctgaataa tcagttcata ccttgtttgg gtttgtctcg cataggagtc      60

atgtaacctt cctcatccag ctctcctcgt gggctccgtt ctggggcaaa cacggtgggg     120


<210>  520
<211>  120
<212>  DNA
<213>  Artificial Sequence

<220>
<223>  ERBB4

<400>  520
atgtaacctt cctcatccag ctctcctcgt gggctccgtt ctggggcaaa cacggtgggg      60

tcagcactgt acctctgggt gctactgtcc tcttggacat ggggtgccac tggcttgcgt     120


<210>  521
<211>  120
<212>  DNA
<213>  Artificial Sequence

<220>
<223>  ERBB4

<400>  521
tcagcactgt acctctgggt gctactgtcc tcttggacat ggggtgccac tggcttgcgt      60

agggtgccat tacagcagga gtcatcaaaa atctcagcag tagcaccctg tgccacagga     120

<210>  522
<211>  120
<212>  DNA
<213>  Artificial Sequence

<220>
<223>  ERBB4

<400>  522
agggtgccat tacagcagga gtcatcaaaa atctcagcag tagcaccctg tgccacagga      60

gcttctggaa ttgtgctagt tggggctctg tagggcacag acactccttg ttcagcagca     120


<210>  523
<211>  120
<212>  DNA
<213>  Artificial Sequence

<220>
<223>  ERBB4

<400>  523
gcttctggaa ttgtgctagt tggggctctg tagggcacag acactccttg ttcagcagca      60

aaacctccat ctcggtatac aaactggttc tgttaataag agaaacatat gtggagagaa     120


<210>  524
<211>  120
<212>  DNA
<213>  Artificial Sequence

<220>
<223>  ERBB4

<400>  524
gagaggaaac atggtaagca aagaccgaaa atcctaaaag atgaaggttg attgtgaaat      60

acttactcct gacatggggg tgtaggcagg aggagggctg tgtccaattt cactctaata     120


<210>  525
<211>  120
<212>  DNA
<213>  Artificial Sequence

<220>
<223>  ERBB4

<400>  525
acttactcct gacatggggg tgtaggcagg aggagggctg tgtccaattt cactctaata      60

ggaaagaaaa atggaatgat ggatataata agaggcaata tgaatgaaaa aattaaaaaa     120


<210>  526
<211>  120
<212>  DNA
<213>  Artificial Sequence

<220>
<223>  ERBB4

<400>  526
aaagaatatg atatgtgtat ataattattt cttaccctat tcgagtcaat tcttgctctg        60

gaagtataga tgggaggtgg gatgttgaaa gcctgaggga ccaagtactc ctcagcatcc       120


<210>  527
<211>  120
<212>  DNA
<213>  Artificial Sequence

<220>
<223>  ERBB4

<400>  527
gaagtataga tgggaggtgg gatgttgaaa gcctgaggga ccaagtactc ctcagcatcc        60

atcatatctt ccaaatcctc ttcatccaag agattctgaa agaacttgct gtcatttgga       120


<210>  528
<211>  120
<212>  DNA
<213>  Artificial Sequence

<220>
<223>  ERBB4

<400>  528
atcatatctt ccaaatcctc ttcatccaag agattctgaa agaacttgct gtcatttgga        60

ctgggaagct tcatacgatc atcaccctaa aagaaagatt gcccatcaga cacaaatatg       120


<210>  529
<211>  120
<212>  DNA
<213>  Artificial Sequence

<220>
<223>  ERBB4

<400>  529
ccattggcct agtcttaaag gcataagtca aatgtactca cctgaataac taggtatctt        60

tgagggtctc gagccatcct tgaaaactca gcagccagtt ccttaaattt aggtctactg       120


<210>  530
<211>  120
<212>  DNA
<213>  Artificial Sequence

<220>
<223>  ERBB4

<400>  530
tgagggtctc gagccatcct tgaaaactca gcagccagtt ccttaaattt aggtctactg        60

tcagcatcaa tcatccaacc tggaaattta cacagtgaaa atgtcactat attcgtaact       120

<210>  531
<211>  120
<212>  DNA
<213>  Artificial Sequence

<220>
<223>  ERBB4

<400>  531
actttactaa gaatgatgat ggtgataaca ttattttgca gtcttacatt tgaccatgac      60

catgtaaacg tcaatagtgc agatgggagg ctgaggcaaa cgttctcctt tctctaataa      120


<210>  532
<211>  120
<212>  DNA
<213>  Artificial Sequence

<220>
<223>  ERBB4

<400>  532
catgtaaacg tcaatagtgc agatgggagg ctgaggcaaa cgttctcctt tctctaataa      60

atcagggatt tctcgcgttg gaattccatc atagggtttt cctccaaagg tcatcagttc      120


<210>  533
<211>  120
<212>  DNA
<213>  Artificial Sequence

<220>
<223>  ERBB4

<400>  533
atcagggatt tctcgcgttg gaattccatc atagggtttt cctccaaagg tcatcagttc      60

ccatatagta actcctatat tggagaaaaa attcttactt aagcatatta acaacatatg      120


<210>  534
<211>  120
<212>  DNA
<213>  Artificial Sequence

<220>
<223>  ERBB4

<400>  534
acatatgtat ttttgcttta caagctttaa ttcgcaaaga agatttattt accatagctc      60

caaacgtcac tctgatgggt gaatttcctg taatgtatac actccagagc catccattta      120


<210>  535
<211>  120
<212>  DNA
<213>  Artificial Sequence

<220>
<223>  ERBB4

<400>  535
caaacgtcac tctgatgggt gaatttcctg taatgtatac actccagagc catccattta      60

attggcatct atagagaagt aagaagtaaa agttttaaaa ttacattaaa aattcatttc     120


<210>  536
<211>  120
<212>  DNA
<213>  Artificial Sequence

<220>
<223>  ERBB4

<400>  536
tactatattt tcaagcaaga ttgctctcaa aaagataccc acctttcctc catcagcatt      60

gtactctttt tcatctcctt ccaagagtct ggctagccca aaatctgtga ttttcacatg     120


<210>  537
<211>  120
<212>  DNA
<213>  Artificial Sequence

<220>
<223>  ERBB4

<400>  537
gtactctttt tcatctcctt ccaagagtct ggctagccca aaatctgtga ttttcacatg      60

gtttggagat ttcactaaga cattacgggc tgccaaatcc cgatgaacga gtcgtctttc     120


<210>  538
<211>  120
<212>  DNA
<213>  Artificial Sequence

<220>
<223>  ERBB4

<400>  538
gtttggagat ttcactaaga cattacgggc tgccaaatcc cgatgaacga gtcgtctttc      60

ttccaggtac atcattccct gaaaaatatc aagttcctta atgatattca gttaatgccc     120


<210>  539
<211>  120
<212>  DNA
<213>  Artificial Sequence

<220>
<223>  ERBB4

<400>  539
tttccataga aattgacagg cacttacctt agctatctgg acacaccagt taagcagcag      60

ttgtgatcca atgttatcct tgtgctcgtg gacatactcc aacaggcagc catggggcat     120

<210>  540
<211>  120
<212>  DNA
<213>  Artificial Sequence

<220>
<223>  ERBB4

<400>  540
ttgtgatcca atgttatcct tgtgctcgtg gacatactcc aacaggcagc catggggcat      60

aagttgagta accagctgga tggttgggct cagacacaca cccagcaacc ggactaggtg      120


<210>  541
<211>  120
<212>  DNA
<213>  Artificial Sequence

<220>
<223>  ERBB4

<400>  541
aagttgagta accagctgga tggttgggct cagacacaca cccagcaacc ggactaggtg      60

tggatgatcc atacttgcca tgatcagagc ttcctgtaag aaaaaaatgc aataccatga      120


<210>  542
<211>  120
<212>  DNA
<213>  Artificial Sequence

<220>
<223>  ERBB4

<400>  542
ataatggaga aaaatgtgat tgcctgggtg tctgtactta catccatgaa ctccacattt      60

gccttgggac cagttgtctc attaagaatc ttaatagcca caggaatctt cacagtttct      120


<210>  543
<211>  120
<212>  DNA
<213>  Artificial Sequence

<220>
<223>  ERBB4

<400>  543
gccttgggac cagttgtctc attaagaatc ttaatagcca caggaatctt cacagtttct      60

ccttcaggta cccaaatacc ctttggggaa aaaaatttac attaaatatg acatctcaac      120


<210>  544
<211>  120
<212>  DNA
<213>  Artificial Sequence

<220>
<223>  ERBB4

<400>  544
ttattttcta aacatctaaa acaaaactta actaacgata tgcgttgttt tttacttact      60

ttataaaccg ttccaaaagc acctgagcca aggactttta ccctcttcag ctcagtttct     120


<210>  545
<211>  120
<212>  DNA
<213>  Artificial Sequence

<220>
<223>  ERBB4

<400>  545
ttataaaccg ttccaaaagc acctgagcca aggactttta ccctcttcag ctcagtttct      60

ttcaaaatac gaagttgagc ttgattgggt gctgtgccac tgggagttaa tggttccacc     120


<210>  546
<211>  120
<212>  DNA
<213>  Artificial Sequence

<220>
<223>  ERBB4

<400>  546
ttcaaaatac gaagttgagc ttgattgggt gctgtgccac tgggagttaa tggttccacc      60

aactgcaaag cggaaagaag aaggttatac tttcagtccg atagtcagtc ctctctctct     120


<210>  547
<211>  120
<212>  DNA
<213>  Artificial Sequence

<220>
<223>  ERBB4

<400>  547
tctaaacctg atgaaaatcc ccaaacacat gaagaggaga aagaaatacc tcacctctgt      60

ttccaagaat cttctcaagg ctcttttctt tttgatgctc ttccttctaa cataaacagc     120


<210>  548
<211>  120
<212>  DNA
<213>  Artificial Sequence

<220>
<223>  ERBB4

<400>  548
ttccaagaat cttctcaagg ctcttttctt tttgatgctc ttccttctaa cataaacagc      60

aaatgtcaga cccacaatga ccagaatgaa gagcccacca attactccag ctgcaatcag     120

<210>  549
<211>  120
<212>  DNA
<213>  Artificial Sequence

<220>
<223>  ERBB4

<400>  549
aaatgtcaga cccacaatga ccagaatgaa gagcccacca attactccag ctgcaatcag      60

gggagttctg acaaccagaa tgagaaaaaa aaaaataaaa agtatgaaga gagagaagac      120


<210>  550
<211>  120
<212>  DNA
<213>  Artificial Sequence

<220>
<223>  ERBB4

<400>  550
atgataacta ggaaaggatt tgagcgacaa aatggaaaca tggtagatgt tacctagcat      60

gttgtggtaa agtggaatgg cccgtccatg ggtagtaaat gcagtcatga ctagtgggac      120


<210>  551
<211>  120
<212>  DNA
<213>  Artificial Sequence

<220>
<223>  ERBB4

<400>  551
gttgtggtaa agtggaatgg cccgtccatg ggtagtaaat gcagtcatga ctagtgggac      60

cgttacacct gcaggcaatt acagaacaga aaacatcatt ctccatccac agtgacatgc      120


<210>  552
<211>  120
<212>  DNA
<213>  Artificial Sequence

<220>
<223>  ERBB4

<400>  552
cacacatgca ccagtgctca cacatggagc cttggaggaa gaacatggga agcaagcaca      60

ccaagtacct atccatcagg ccgatgcagt cttcaatact tgagcctatg cacctgcaga      120


<210>  553
<211>  120
<212>  DNA
<213>  Artificial Sequence

<220>
<223>  ERBB4

<400>  553
ccaagtacct atccatcagg ccgatgcagt cttcaatact tgagcctatg cacctgcaga      60

aaatgcaaat ttaaaaaaat acaaggggaa aaaattagtc atttaaaaaa tcagtaaaat     120


<210>  554
<211>  120
<212>  DNA
<213>  Artificial Sequence

<220>
<223>  ERBB4

<400>  554
ataacacata ccaggtgagc ccttggccag caagaatgct tacccttggg tgcagtttgg      60

atggcatggg tggcactccc gatctggatc agcatacttg aaaatgaaac tgtttgcccc     120


<210>  555
<211>  120
<212>  DNA
<213>  Artificial Sequence

<220>
<223>  ERBB4

<400>  555
atggcatggg tggcactccc gatctggatc agcatacttg aaaatgaaac tgtttgcccc      60

ctgtaagcca tctggacatt tttccacaca gtttgggcca tctttaaaat gagagcactt     120


<210>  556
<211>  120
<212>  DNA
<213>  Artificial Sequence

<220>
<223>  ERBB4

<400>  556
ctgtaagcca tctggacatt tttccacaca gtttgggcca tctttaaaat gagagcactt      60

tgtacagttg tcaggaccct gaaatgtgaa aacgaaaaaa aaagaaaaaa gaaaagtggt     120


<210>  557
<211>  120
<212>  DNA
<213>  Artificial Sequence

<220>
<223>  ERBB4

<400>  557
gatactaaat aagccaacac accacagatg tcttcaggct taccggtcca tggcatgtga      60

ggaggccatc ttccatcttc tcacactggg ggtcacactc cacacagatg gagccattct     120

<210>  558
<211>  120
<212>  DNA
<213>  Artificial Sequence

<220>
<223>  ERBB4

<400>  558
ggaggccatc ttccatcttc tcacactggg ggtcacactc cacacagatg gagccattct      60

caaactcccg aaattcactg tgaaaacatc agccacatga ggaggtgtaa gcaaacaagc     120


<210>  559
<211>  120
<212>  DNA
<213>  Artificial Sequence

<220>
<223>  ERBB4

<400>  559
ctaatcagtc cttcaaagct catgaggtga aggcaaccct agaagaagcc ttacccatca      60

tagaggttac aagactctat gcagatcctt cctctactga agcggcgaca cgacagacat     120


<210>  560
<211>  120
<212>  DNA
<213>  Artificial Sequence

<220>
<223>  ERBB4

<400>  560
tagaggttac aagactctat gcagatcctt cctctactga agcggcgaca cgacagacat      60

tggtctggcc caggtcccca acagccatca ctggaacaca gatggttgca caccattcct     120


<210>  561
<211>  120
<212>  DNA
<213>  Artificial Sequence

<220>
<223>  ERBB4

<400>  561
tggtctggcc caggtcccca acagccatca ctggaacaca gatggttgca caccattcct      60

tcagcagctg tgaaacacca aaatcaaggg gaaataaaac agaggattgt gtcaaaactt     120


<210>  562
<211>  120
<212>  DNA
<213>  Artificial Sequence

<220>
<223>  ERBB4

<400>  562
tgtctgagta atgtacttac tacaattttc agcttttctg ttgtcccgga ttactattct      60

ctggttgatt gtgctgaaga gtgttgtcca gttaatggta tgataataac acaggttgct     120


<210>  563
<211>  120
<212>  DNA
<213>  Artificial Sequence

<220>
<223>  ERBB4

<400>  563
ctggttgatt gtgctgaaga gtgttgtcca gttaatggta tgataataac acaggttgct      60

gttgtcagta atatagatgt ttcctgcgct gatttccttc agggactgga actgtagaga     120


<210>  564
<211>  120
<212>  DNA
<213>  Artificial Sequence

<220>
<223>  ERBB4

<400>  564
gttgtcagta atatagatgt ttcctgcgct gatttccttc agggactgga actgtagaga      60

ggtgatgccc tgttgcttga ggataagcaa ggacaggcca ctaaggaggg ggaagtgaga     120


<210>  565
<211>  120
<212>  DNA
<213>  Artificial Sequence

<220>
<223>  ERBB4

<400>  565
ctcttgaaat ctgtgagccc tgcagcttta aacatatcca cttacctata gagtactctt      60

ccaccaatgg tcaccaggtt agaaaaaaca ctgaagtcag tcatgtttgg tggccatgac     120


<210>  566
<211>  120
<212>  DNA
<213>  Artificial Sequence

<220>
<223>  ERBB4

<400>  566
ccaccaatgg tcaccaggtt agaaaaaaca ctgaagtcag tcatgtttgg tggccatgac      60

tgtatgttca ggaaacctac aagtgaagag tagaaaaaat aaatcagaat atcattgtct     120

<210>  567
<211>  120
<212>  DNA
<213>  Artificial Sequence

<220>
<223>  ERBB4

<400>  567
ttttaaaaaa attatattgt tcatagcgca acagttgcag tttaaaaaat tacctgttat      60

ctctctgact gtccgaaaga cgttcagttt ctctgggtct atggcttcaa ttgcattgta      120


<210>  568
<211>  120
<212>  DNA
<213>  Artificial Sequence

<220>
<223>  ERBB4

<400>  568
ctctctgact gtccgaaaga cgttcagttt ctctgggtct atggcttcaa ttgcattgta      60

agggtcccta gaaaatcaag aagagatgta gccaaattta aattttacta aaggattgaa      120


<210>  569
<211>  120
<212>  DNA
<213>  Artificial Sequence

<220>
<223>  ERBB4

<400>  569
tctcttcagt tttgtctaca ctttgtaaaa taacttgcac aaaaatttaa tactgaccca      60

tgaataccag tgactagaaa gatcaaattc ccattgatct tggtacagtt tatgaatttg      120


<210>  570
<211>  120
<212>  DNA
<213>  Artificial Sequence

<220>
<223>  ERBB4

<400>  570
tgaataccag tgactagaaa gatcaaattc ccattgatct tggtacagtt tatgaatttg      60

tcaatgttac tggaatccac agtctgagct gacatcaatg atcctgtgcc aatgccatca      120


<210>  571
<211>  120
<212>  DNA
<213>  Artificial Sequence

<220>
<223>  ERBB4

<400>  571
tcaatgttac tggaatccac agtctgagct gacatcaatg atcctgtgcc aatgccatca      60

caagctgtag aaacaagact cagagttagg ggattgagaa acttattttt ggccaataaa     120


<210>  572
<211>  120
<212>  DNA
<213>  Artificial Sequence

<220>
<223>  ERBB4

<400>  572
agtgaattaa aaaccttgtt atataggccc agttctaact aaataatctg agctaccact      60

caccttttgg gcaaatgtca gtgcaaggtt tacacatttt aatcccattt tcttctactt     120


<210>  573
<211>  120
<212>  DNA
<213>  Artificial Sequence

<220>
<223>  ERBB4

<400>  573
caccttttgg gcaaatgtca gtgcaaggtt tacacatttt aatcccattt tcttctactt      60

ccatcttgga actagggcag gcacgcacac aagaactgga atctaccaca aagttatctg     120


<210>  574
<211>  120
<212>  DNA
<213>  Artificial Sequence

<220>
<223>  ERBB4

<400>  574
ccatcttgga actagggcag gcacgcacac aagaactgga atctaccaca aagttatctg      60

attaaaaaaa aaaaaaaggt aaaataagca ttaatgttaa cattcagcaa acaagctcaa     120


<210>  575
<211>  120
<212>  DNA
<213>  Artificial Sequence

<220>
<223>  ERBB4

<400>  575
tttcaaataa tgacctaatt aatttggtta ttcttattct gttacttacg tggacatttc      60

ttgacacaga atgctccata tgtgtacttt gcattgaaat tgtgctccag ttgaaaggtg     120

<210>  576
<211>  120
<212>  DNA
<213>  Artificial Sequence

<220>
<223>  ERBB4

<400>  576
ttgacacaga atgctccata tgtgtacttt gcattgaaat tgtgctccag ttgaaaggtg      60

gttggattgt agacaaaggt ttggggacac tgagtaacac atgctccact gtcattgaaa      120


<210>  577
<211>  120
<212>  DNA
<213>  Artificial Sequence

<220>
<223>  ERBB4

<400>  577
gttggattgt agacaaaggt ttggggacac tgagtaacac atgctccact gtcattgaaa      60

ttcatgcagg cctgcaacac agcaaatatt actttcattt acaaataaac tcataatact      120


<210>  578
<211>  120
<212>  DNA
<213>  Artificial Sequence

<220>
<223>  ERBB4

<400>  578
ctgaatcaaa tagggaagga aaggagagca ggataataaa agagagaaat cacagacata      60

caaagcagtc tgtgtcctta ggtcctgagc agcctccagc acattctcga tggcagcagt      120


<210>  579
<211>  120
<212>  DNA
<213>  Artificial Sequence

<220>
<223>  ERBB4

<400>  579
caaagcagtc tgtgtcctta ggtcctgagc agcctccagc acattctcga tggcagcagt      60

cactgacgta aggtccgtag catctgccgt cacattgttc tgcacacacc gtccttgtca      120


<210>  580
<211>  120
<212>  DNA
<213>  Artificial Sequence

<220>
<223>  ERBB4

<400>  580
cactgacgta aggtccgtag catctgccgt cacattgttc tgcacacacc gtccttgtca        60

ctgcagaaga cagagatagg accatgatca aagtctgcca ccaggagaaa ctcataagct       120


<210>  581
<211>  120
<212>  DNA
<213>  Artificial Sequence

<220>
<223>  ERBB4

<400>  581
atcattaaaa ttccttgacc acatcaaacc tgtgtgctct cactgatgaa cacttacaag        60

tctggcaatg attttctgtg ggtccccagc aacggccagt acaggactta tggcaacgtc       120


<210>  582
<211>  120
<212>  DNA
<213>  Artificial Sequence

<220>
<223>  ERBB4

<400>  582
tctggcaatg attttctgtg ggtccccagc aacggccagt acaggactta tggcaacgtc        60

cacctgcaga acacgaaaag ggaaaaagga catgcacgtt ataatctttc actccttgac       120


<210>  583
<211>  120
<212>  DNA
<213>  Artificial Sequence

<220>
<223>  ERBB4

<400>  583
tcgccacata gggtagaaca ttttgagaaa ttaaaaagaa taattctact tacatcctga        60

actaccattt gttgacacaa gagtcaagtt ggaaggccat gggttccgaa caatatcttg       120


<210>  584
<211>  120
<212>  DNA
<213>  Artificial Sequence

<220>
<223>  ERBB4

<400>  584
actaccattt gttgacacaa gagtcaagtt ggaaggccat gggttccgaa caatatcttg        60

ccaatgaatg gtgtctgcat aacaaaggaa tttgttctgg tctacataga ctccaccatt       120

<210>  585
<211>  120
<212>  DNA
<213>  Artificial Sequence

<220>
<223>  ERBB4

<400>  585
ccaatgaatg gtgtctgcat aacaaaggaa tttgttctgg tctacataga ctccaccatt      60

taggatttct gtattaaaaa acaaataaac aaattttttg tcaaactgct tgttgatgaa     120


<210>  586
<211>  120
<212>  DNA
<213>  Artificial Sequence

<220>
<223>  ERBB4

<400>  586
atttgccatt ttggatatat tccttacctg tcaagttctt taatccaagt tcttgaagtc      60

caaagtttcc atcttttctg tagtttaaaa atattgccaa ggcatatcga tcctcataaa     120


<210>  587
<211>  120
<212>  DNA
<213>  Artificial Sequence

<220>
<223>  ERBB4

<400>  587
caaagtttcc atcttttctg tagtttaaaa atattgccaa ggcatatcga tcctcataaa      60

gttttgtccc acgaataatg cgtaaattct ccagaggcag gtaacgaaac tgattaagag     120


<210>  588
<211>  120
<212>  DNA
<213>  Artificial Sequence

<220>
<223>  ERBB4

<400>  588
gttttgtccc acgaataatg cgtaaattct ccagaggcag gtaacgaaac tgattaagag      60

ccactaacac gtagcctgtg acttctcgaa cagactgaaa agacacaaac agttgcctgt     120


<210>  589
<211>  120
<212>  DNA
<213>  Artificial Sequence

<220>
<223>  ERBB4

<400>  589
ccactgtcca ttcacaaaga agagaaagaa agccacagct ttacccgcag gaaggagagg      60

tcccggttgt gctcaatgct ggttatctcc aggttgccca tgacaacctc acagttttca     120


<210>  590
<211>  120
<212>  DNA
<213>  Artificial Sequence

<220>
<223>  ERBB4

<400>  590
tcccggttgt gctcaatgct ggttatctcc aggttgccca tgacaacctc acagttttca      60

tagtacttgc gcaaggctcg gtactgctgt tccaggtcag agagagagct cagtttattc     120


<210>  591
<211>  120
<212>  DNA
<213>  Artificial Sequence

<220>
<223>  ERBB4

<400>  591
tagtacttgc gcaaggctcg gtactgctgt tccaggtcag agagagagct cagtttattc      60

tccgttcctg cacacactgc aaagacaaga agatacacgt gaaattacat aacctttata     120


<210>  592
<211>  120
<212>  DNA
<213>  Artificial Sequence

<220>
<223>  ERBB4

<400>  592
cccgccggcg gctgcaggtt cggcgaccgg agtgccagaa ggaacccacc tgactgagaa      60

tcgctgggct ggacggtccc cgccgccacg agaaggctca cccagaccca aagtcctgtc     120


<210>  593
<211>  120
<212>  DNA
<213>  Artificial Sequence

<220>
<223>  ERBB4

<400>  593
tcgctgggct ggacggtccc cgccgccacg agaaggctca cccagaccca aagtcctgtc      60

gccggcttca ttttttggaa gtctcagatc ccgtgctgac aattacatgt ccaaatggca     120

<210>  594
<211>  120
<212>  DNA
<213>  Artificial Sequence

<220>
<223>  HER2-E20

<400>  594
gtttgggggt gtgtggtctc ccataccctc tcagcgtacc cttgtcccca ggaagcatac      60

gtgatggctg gtgtgggctc cccatatgtc tcccgccttc tgggcatctg cctgacatcc      120


<210>  595
<211>  120
<212>  DNA
<213>  Artificial Sequence

<220>
<223>  HER2-E20

<400>  595
accctctcag cgtacccttg tccccaggaa gcatacgtga tggctggtgt gggctcccca      60

tatgtctccc gccttctggg catctgcctg acatccacgg tgcagctggt gacacagctt      120


<210>  596
<211>  120
<212>  DNA
<213>  Artificial Sequence

<220>
<223>  HER2-E20

<400>  596
gtgggctccc catatgtctc ccgccttctg ggcatctgcc tgacatccac ggtgcagctg      60

gtgacacagc ttatgcccta tggctgcctc ttagaccatg tccgggaaaa ccgcggacgc      120


<210>  597
<211>  120
<212>  DNA
<213>  Artificial Sequence

<220>
<223>  HER2-E20

<400>  597
cttctgggca tctgcctgac atccacggtg cagctggtga cacagcttat gccctatggc      60

tgcctcttag accatgtccg ggaaaaccgc ggacgcctgg gctcccagga cctgctgaac      120


<210>  598
<211>  120
<212>  DNA
<213>  Artificial Sequence

<220>
<223>  HER2-E20

<400>  598
atgccctatg gctgcctctt agaccatgtc cgggaaaacc gcggacgcct gggctcccag      60

gacctgctga actggtgtat gcagattgcc aaggtatgca cctgggctct ttgcaggtct     120


<210>  599
<211>  120
<212>  DNA
<213>  Artificial Sequence

<220>
<223>  HER2-E20

<400>  599
catgtccggg aaaaccgcgg acgcctgggc tcccaggacc tgctgaactg gtgtatgcag      60

attgccaagg tatgcacctg ggctctttgc aggtctctcc ggagcaaacc cctatgtcca     120


<210>  600
<211>  120
<212>  DNA
<213>  Artificial Sequence

<220>
<223>  HER4-E10

<400>  600
atattgttca tagcgcaaca gttgcagttt aaaaaattac ctgttatctc tctgactgtc      60

cgaaagacgt tcagtttctc tgggtctatg gcttcaattg cattgtaagg gtccctagaa     120


<210>  601
<211>  120
<212>  DNA
<213>  Artificial Sequence

<220>
<223>  HER4-E10

<400>  601
tctctgactg tccgaaagac gttcagtttc tctgggtcta tggcttcaat tgcattgtaa      60

gggtccctag aaaatcaaga agagatgtag ccaaatttaa attttactaa aggattgaaa     120


<210>  602
<211>  120
<212>  DNA
<213>  Artificial Sequence

<220>
<223>  HER4-E10

<400>  602
agtttctctg ggtctatggc ttcaattgca ttgtaagggt ccctagaaaa tcaagaagag      60

atgtagccaa atttaaattt tactaaagga ttgaaaatat gagaaatgtg acaatatttt     120

<210>  603
<211>  120
<212>  DNA
<213>  Artificial Sequence

<220>
<223>  HER4-E10

<400>  603
atattgttca tagcgcaaca gttgcagttt aaaaaattac ctgttatctc tctgactgtc      60

cgaaagacgt tcagtttctc tgggtctatg gcttcaattg cattgtaagg gtccctagaa      120


<210>  604
<211>  120
<212>  DNA
<213>  Artificial Sequence

<220>
<223>  HER4-E10

<400>  604
cagtttaaaa aattacctgt tatctctctg actgtccgaa agacgttcag tttctctggg      60

tctatggctt caattgcatt gtaagggtcc ctagaaaatc aagaagagat gtagccaaat      120


<210>  605
<211>  120
<212>  DNA
<213>  Artificial Sequence

<220>
<223>  HER4-E10

<400>  605
tctctgactg tccgaaagac gttcagtttc tctgggtcta tggcttcaat tgcattgtaa      60

gggtccctag aaaatcaaga agagatgtag ccaaatttaa attttactaa aggattgaaa      120


<210>  606
<211>  120
<212>  DNA
<213>  Artificial Sequence

<220>
<223>  HER4-E10

<400>  606
agtttctctg ggtctatggc ttcaattgca ttgtaagggt ccctagaaaa tcaagaagag      60

atgtagccaa atttaaattt tactaaagga ttgaaaatat gagaaatgtg acaatatttt      120


<210>  607
<211>  120
<212>  DNA
<213>  Artificial Sequence

<220>
<223>  HER4-E12

<400>  607
aagaatggga aaaaatttaa gtttctatgt tttaaatgtc tgagtaatgt acttactaca      60

attttcagct tttctgttgt cccggattac tattctctgg ttgattgtgc tgaagagtgt      120


<210>  608
<211>  120
<212>  DNA
<213>  Artificial Sequence

<220>
<223>  HER4-E12

<400>  608
ctatgtttta aatgtctgag taatgtactt actacaattt tcagcttttc tgttgtcccg      60

gattactatt ctctggttga ttgtgctgaa gagtgttgtc cagttaatgg tatgataata      120


<210>  609
<211>  120
<212>  DNA
<213>  Artificial Sequence

<220>
<223>  HER4-E12

<400>  609
tctgttgtcc cggattacta ttctctggtt gattgtgctg aagagtgttg tccagttaat      60

ggtatgataa taacacaggt tgctgttgtc agtaatatag atgtttcctg cgctgatttc      120


<210>  610
<211>  120
<212>  DNA
<213>  Artificial Sequence

<220>
<223>  HER4-E12

<400>  610
ctggttgatt gtgctgaaga gtgttgtcca gttaatggta tgataataac acaggttgct      60

gttgtcagta atatagatgt ttcctgcgct gatttccttc agggactgga actgtagaga      120


<210>  611
<211>  120
<212>  DNA
<213>  Artificial Sequence

<220>
<223>  HER4-E12

<400>  611
tgtccagtta atggtatgat aataacacag gttgctgttg tcagtaatat agatgtttcc      60

tgcgctgatt tccttcaggg actggaactg tagagaggtg atgccctgtt gcttgaggat      120

<210>  612
<211>  120
<212>  DNA
<213>  Artificial Sequence

<220>
<223>  HER4-E12

<400>  612
atagatgttt cctgcgctga tttccttcag ggactggaac tgtagagagg tgatgccctg        60

ttgcttgagg ataagcaagg acaggccact aaggaggggg aagtgagaaa acggaaccat       120


<210>  613
<211>  120
<212>  DNA
<213>  Artificial Sequence

<220>
<223>  HER4-E12

<400>  613
cttcagggac tggaactgta gagaggtgat gccctgttgc ttgaggataa gcaaggacag        60

gccactaagg agggggaagt gagaaaacgg aaccatgaaa cgcattccgg agtaaaataa       120


<210>  614
<211>  120
<212>  DNA
<213>  Artificial Sequence

<220>
<223>  HER4-E12

<400>  614
aagaatggga aaaaatttaa gtttctatgt tttaaatgtc tgagtaatgt acttactaca        60

attttcagct tttctgttgt cccggattac tattctctgg ttgattgtgc tgaagagtgt       120


<210>  615
<211>  120
<212>  DNA
<213>  Artificial Sequence

<220>
<223>  HER4-E12

<400>  615
ctatgtttta aatgtctgag taatgtactt actacaattt tcagcttttc tgttgtcccg        60

gattactatt ctctggttga ttgtgctgaa gagtgttgtc cagttaatgg tatgataata       120


<210>  616
<211>  120
<212>  DNA
<213>  Artificial Sequence

<220>
<223>  HER4-E12

<400>  616
gtacttacta caattttcag cttttctgtt gtcccggatt actattctct ggttgattgt      60

gctgaagagt gttgtccagt taatggtatg ataataacac aggttgctgt tgtcagtaat      120


<210>  617
<211>  120
<212>  DNA
<213>  Artificial Sequence

<220>
<223>  HER4-E12

<400>  617
tctgttgtcc cggattacta ttctctggtt gattgtgctg aagagtgttg tccagttaat      60

ggtatgataa taacacaggt tgctgttgtc agtaatatag atgtttcctg cgctgatttc      120


<210>  618
<211>  120
<212>  DNA
<213>  Artificial Sequence

<220>
<223>  HER4-E12

<400>  618
ctggttgatt gtgctgaaga gtgttgtcca gttaatggta tgataataac acaggttgct      60

gttgtcagta atatagatgt ttcctgcgct gatttccttc agggactgga actgtagaga      120


<210>  619
<211>  120
<212>  DNA
<213>  Artificial Sequence

<220>
<223>  HER4-E12

<400>  619
tgtccagtta atggtatgat aataacacag gttgctgttg tcagtaatat agatgtttcc      60

tgcgctgatt tccttcaggg actggaactg tagagaggtg atgccctgtt gcttgaggat      120


<210>  620
<211>  120
<212>  DNA
<213>  Artificial Sequence

<220>
<223>  HER4-E12

<400>  620
acacaggttg ctgttgtcag taatatagat gtttcctgcg ctgatttcct tcagggactg      60

gaactgtaga gaggtgatgc cctgttgctt gaggataagc aaggacaggc cactaaggag      120

<210>  621
<211>  120
<212>  DNA
<213>  Artificial Sequence

<220>
<223>  HER4-E12

<400>  621
atagatgttt cctgcgctga tttccttcag ggactggaac tgtagagagg tgatgccctg      60

ttgcttgagg ataagcaagg acaggccact aaggaggggg aagtgagaaa acggaaccat      120


<210>  622
<211>  120
<212>  DNA
<213>  Artificial Sequence

<220>
<223>  HER4-E12

<400>  622
cttcagggac tggaactgta gagaggtgat gccctgttgc ttgaggataa gcaaggacag      60

gccactaagg agggggaagt gagaaaacgg aaccatgaaa cgcattccgg agtaaaataa      120


<210>  623
<211>  120
<212>  DNA
<213>  Artificial Sequence

<220>
<223>  KDR

<400>  623
catgtgatgt ccaggagttg ggggtgtgga tgcttccttt taaacaggag gagagctcag      60

tgtggtcccc gagtcaggct ggagaatctg ggctgtgcta ccggtttgca ctccaatctc      120


<210>  624
<211>  120
<212>  DNA
<213>  Artificial Sequence

<220>
<223>  KDR

<400>  624
tgtggtcccc gagtcaggct ggagaatctg ggctgtgcta ccggtttgca ctccaatctc      60

tatcagcttt aaaagttctg cttcctcact ggagtacacg gtggtgtctg tgtcatcgga      120


<210>  625
<211>  120
<212>  DNA
<213>  Artificial Sequence

<220>
<223>  KDR

<400>  625
tatcagcttt aaaagttctg cttcctcact ggagtacacg gtggtgtctg tgtcatcgga      60

gtgatatccg gactggtagc cgcttgtctg gtttgagcct tcagatgcca cagactccct     120


<210>  626
<211>  120
<212>  DNA
<213>  Artificial Sequence

<220>
<223>  KDR

<400>  626
gtgatatccg gactggtagc cgcttgtctg gtttgagcct tcagatgcca cagactccct      60

gcttttgctg ggcaccattc cactgcagaa gaaatggcaa acaaaggagt tggcagagag     120


<210>  627
<211>  120
<212>  DNA
<213>  Artificial Sequence

<220>
<223>  KDR

<400>  627
caaaaattcc tattgaaatt tgtcttttta atatggctga gtcttaccca aaagatggag      60

ataatttggt tctgtcttcc aaagttttca gctcttctga ggcaagaacc ataccactgt     120


<210>  628
<211>  120
<212>  DNA
<213>  Artificial Sequence

<220>
<223>  KDR

<400>  628
ataatttggt tctgtcttcc aaagttttca gctcttctga ggcaagaacc ataccactgt      60

ccgtctggtt gtccttttta agagacaatg agatggcaca gttaattgag catataaaat     120


<210>  629
<211>  120
<212>  DNA
<213>  Artificial Sequence

<220>
<223>  KDR

<400>  629
ttgtattatt tctaagacta tttttaaaag acgtacttac atctgggatt acttttactt      60

ctggttcttc taacgggata tcttcaaatg tttttacact cacaggccgg ctctttcgct     120

<210>  630
<211>  120
<212>  DNA
<213>  Artificial Sequence

<220>
<223>  KDR

<400>  630
ctggttcttc taacgggata tcttcaaatg tttttacact cacaggccgg ctctttcgct      60

tactgttctg cagatactga ctgcaaaaga acaaatattt atattttagt ggtggtatat      120


<210>  631
<211>  120
<212>  DNA
<213>  Artificial Sequence

<220>
<223>  KDR

<400>  631
ttgaagtcac cctcccccgg gggatgttag gccatataca gtacctgatt cctgctgtgt      60

tgtcataatg gaatttgggg tcacatactt cctcctcctc catacaggaa acaggtgagg      120


<210>  632
<211>  120
<212>  DNA
<213>  Artificial Sequence

<220>
<223>  KDR

<400>  632
tgtcataatg gaatttgggg tcacatactt cctcctcctc catacaggaa acaggtgagg      60

taggcagaga gagtccagaa tcctcttcca tgctcaaagt ctctgatatc ggaagaacaa      120


<210>  633
<211>  120
<212>  DNA
<213>  Artificial Sequence

<220>
<223>  KDR

<400>  633
taggcagaga gagtccagaa tcctcttcca tgctcaaagt ctctgatatc ggaagaacaa      60

tgtagtcttt gccatcctga aacaataaac acagaagact gttgttatgg cttcagttct      120


<210>  634
<211>  120
<212>  DNA
<213>  Artificial Sequence

<220>
<223>  KDR

<400>  634
aggcagtagg aaagtccttg acatctaagt actctttgta ggtgcttctt ggatggaggt      60

gacaaacctg ctgagcatta gcttgcaaga gatttcccaa atgttccacc aactctgaaa     120


<210>  635
<211>  120
<212>  DNA
<213>  Artificial Sequence

<220>
<223>  KDR

<400>  635
gacaaacctg ctgagcatta gcttgcaaga gatttcccaa atgttccacc aactctgaaa      60

acgtgggtct ctgactgggc tccccgtgcc agcagtccag catggtctgg tacctagaga     120


<210>  636
<211>  120
<212>  DNA
<213>  Artificial Sequence

<220>
<223>  KDR

<400>  636
acgtgggtct ctgactgggc tccccgtgcc agcagtccag catggtctgg tacctagaga      60

agcaaaacac tgatttcatt aaatgcctct ttcttcctga atgctgaaaa taagcactta     120


<210>  637
<211>  120
<212>  DNA
<213>  Artificial Sequence

<220>
<223>  KDR

<400>  637
gcaaagaaag agaacacagg aatacttctt aaagtcttac atttctggtg tagtataatc      60

aggggccctc attctagttc cttctttcaa tcgcctacaa aattcttcat caatctttac     120


<210>  638
<211>  120
<212>  DNA
<213>  Artificial Sequence

<220>
<223>  KDR

<400>  638
aggggccctc attctagttc cttctttcaa tcgcctacaa aattcttcat caatctttac      60

cccaggatat ggagaagcac ctagaataaa acagggagga gacattcttt gatttgattt     120

<210>  639
<211>  120
<212>  DNA
<213>  Artificial Sequence

<220>
<223>  KDR

<400>  639
ggaaaagaca acttttgtct tcctctttgg agtctggatg gaaggacaaa aagaaatgac      60

ttacctaagg aaaatatttc ccacagcaaa acaccaaaag accagacgtc actctggatt      120


<210>  640
<211>  120
<212>  DNA
<213>  Artificial Sequence

<220>
<223>  KDR

<400>  640
ttacctaagg aaaatatttc ccacagcaaa acaccaaaag accagacgtc actctggatt      60

gtgtacactc tgtcaaaaat tgtttctggg gccatccatt tcaaagggag gcgagcctac      120


<210>  641
<211>  120
<212>  DNA
<213>  Artificial Sequence

<220>
<223>  KDR

<400>  641
gtgtacactc tgtcaaaaat tgtttctggg gccatccatt tcaaagggag gcgagcctac      60

aggggtagaa gacaaggatt cagaacccaa attagcaaat atctgaagaa aaaaacttcc      120


<210>  642
<211>  120
<212>  DNA
<213>  Artificial Sequence

<220>
<223>  KDR

<400>  642
ctacgaggag ttttaattct gttacttaac caagcactgg gttcatattt gaaacttaca      60

tctccttttc tgacataatc tggatcttta taaatatccc gggccaagcc aaagtcacag      120


<210>  643
<211>  120
<212>  DNA
<213>  Artificial Sequence

<220>
<223>  KDR

<400>  643
tctccttttc tgacataatc tggatcttta taaatatccc gggccaagcc aaagtcacag      60

attttaacca cgttcttctc cgataagagg atatttcgtg ccgccaggtc cctgtggata     120


<210>  644
<211>  120
<212>  DNA
<213>  Artificial Sequence

<220>
<223>  KDR

<400>  644
attttaacca cgttcttctc cgataagagg atatttcgtg ccgccaggtc cctgtggata      60

cactgcaaag agaatcatgt gtgcattagg tctcggcaca gctgatctct ttcacatctg     120


<210>  645
<211>  120
<212>  DNA
<213>  Artificial Sequence

<220>
<223>  KDR

<400>  645
tgatctgaaa agatagctga tttcccctca acctttctta cctttcgcga tgccaagaac      60

tccatgccct tagccacttg gaagctgtaa cagatgagat gctccaaggt caggaagtcc     120


<210>  646
<211>  120
<212>  DNA
<213>  Artificial Sequence

<220>
<223>  KDR

<400>  646
tccatgccct tagccacttg gaagctgtaa cagatgagat gctccaaggt caggaagtcc      60

ttatacagat cttcaggagc tgtccaaaga ggcaggagga tggagatcag tatttccatg     120


<210>  647
<211>  120
<212>  DNA
<213>  Artificial Sequence

<220>
<223>  KDR

<400>  647
tgacaagagc atgccatagc atgcaggaag cactagccag taccttcctc ttcttctaca      60

tcactgaggg acttctcctc cacaaatcca gagctggctg agctctggct actggtgatg     120

<210>  648
<211>  120
<212>  DNA
<213>  Artificial Sequence

<220>
<223>  KDR

<400>  648
tcactgaggg acttctcctc cacaaatcca gagctggctg agctctggct actggtgatg      60

ctgtccaagc gccgtttcag atccacaggg attgctccaa cgtagtcttt cccttgacgg     120


<210>  649
<211>  120
<212>  DNA
<213>  Artificial Sequence

<220>
<223>  KDR

<400>  649
ctgtccaagc gccgtttcag atccacaggg attgctccaa cgtagtcttt cccttgacgg      60

aatcgtgccc ctttggtcta taaaaaagca aaggaacaaa caaactcctt gaatacaaaa     120


<210>  650
<211>  120
<212>  DNA
<213>  Artificial Sequence

<220>
<223>  KDR

<400>  650
ccttcatttt ataacatggc cagagcagga ttaggagatg acataccttg taggggacaa      60

attcatttct cttgctcctc aggtaagtgg acaggtttcc aaatttgcag aattccacaa     120


<210>  651
<211>  120
<212>  DNA
<213>  Artificial Sequence

<220>
<223>  KDR

<400>  651
attcatttct cttgctcctc aggtaagtgg acaggtttcc aaatttgcag aattccacaa      60

tcaccatgag tggccctgca ggcagcatgt ccaggaagga aaatgggttt tcactcatat     120


<210>  652
<211>  120
<212>  DNA
<213>  Artificial Sequence

<220>
<223>  KDR

<400>  652
cctcaaacac tatcagagag gcatgttaaa attgggtgac caaaaccacc cacagttact      60

cacctcctgg cttggtacag gcacctagaa ggttgaccac attgagatgg tgaccaatat     120


<210>  653
<211>  120
<212>  DNA
<213>  Artificial Sequence

<220>
<223>  KDR

<400>  653
cacctcctgg cttggtacag gcacctagaa ggttgaccac attgagatgg tgaccaatat      60

gaatgaggat cttgagttca gacatgagag ctcgatgctc actgtgtgtt gctccttcta     120


<210>  654
<211>  120
<212>  DNA
<213>  Artificial Sequence

<220>
<223>  KDR

<400>  654
gaatgaggat cttgagttca gacatgagag ctcgatgctc actgtgtgtt gctccttcta      60

caaatacagt acaaagaggg aaatcatagg tatggacatt tccttcaata attggggtcc     120


<210>  655
<211>  120
<212>  DNA
<213>  Artificial Sequence

<220>
<223>  KDR

<400>  655
aagccttgca acatatttaa agactagata aaacagaaag atagatcacc acataatttt      60

gcttttacct ttcaacattt tgactgctac tgtcctgcaa gttgctgtct tgtcaattcc     120


<210>  656
<211>  120
<212>  DNA
<213>  Artificial Sequence

<220>
<223>  KDR

<400>  656
gcttttacct ttcaacattt tgactgctac tgtcctgcaa gttgctgtct tgtcaattcc      60

aaaggcatct gcttcaatca cttggccaaa ggcaccacgg ccaagaggct tacctagagt     120

<210>  657
<211>  120
<212>  DNA
<213>  Artificial Sequence

<220>
<223>  KDR

<400>  657
aaaggcatct gcttcaatca cttggccaaa ggcaccacgg ccaagaggct tacctagagt      60

caacaacaac agcaacaaga aaacagactt ggattactat acaaccaaag tgatacgcag      120


<210>  658
<211>  120
<212>  DNA
<213>  Artificial Sequence

<220>
<223>  KDR

<400>  658
aagagatggc ctggtaaaca caatatcaaa ttaatagcaa ttgaaaatgc acctagcttc      60

agccggtctc tggggaattc ccatttgctg gcatcataag gcagtcgttc acaatgttca      120


<210>  659
<211>  120
<212>  DNA
<213>  Artificial Sequence

<220>
<223>  KDR

<400>  659
agccggtctc tggggaattc ccatttgctg gcatcataag gcagtcgttc acaatgttca      60

tccaatggga gttcatctgg atccatgacg atggacaagt agcctgtctt cagttcccct      120


<210>  660
<211>  120
<212>  DNA
<213>  Artificial Sequence

<220>
<223>  KDR

<400>  660
tccaatggga gttcatctgg atccatgacg atggacaagt agcctgtctt cagttcccct      60

ccattggcct ggaaagcatc aatccttcca gtcataaaca cactgttgtt tggctgttgt      120


<210>  661
<211>  120
<212>  DNA
<213>  Artificial Sequence

<220>
<223>  KDR

<400>  661
accccagtca gttttcatta atcatgaaaa ccaatgtgca tgggcagaag ggaaattatt      60

tttttacccg cttaacggtc cgtaggatga tgacaagaag tagccagaag aacatggcaa     120


<210>  662
<211>  120
<212>  DNA
<213>  Artificial Sequence

<220>
<223>  KDR

<400>  662
tttttacccg cttaacggtc cgtaggatga tgacaagaag tagccagaag aacatggcaa      60

tcaccgccgt gcctactaga ataatgattt ccaagttcgt cttttcctgg gcacctggaa     120


<210>  663
<211>  120
<212>  DNA
<213>  Artificial Sequence

<220>
<223>  KDR

<400>  663
tcaccgccgt gcctactaga ataatgattt ccaagttcgt cttttcctgg gcacctggaa      60

agacacaatt gaatgagtat caacagttgg aaacttatac tttttgtttg taagatgaca     120


<210>  664
<211>  120
<212>  DNA
<213>  Artificial Sequence

<220>
<223>  KDR

<400>  664
accacatttt tttttatccc actgaccttc tattatgaaa aatgcctcca cttttgcaca      60

gccaagaaca ctgcatgcct ggcaggtgta gaggccttcg tcctccttcc tcactctgcg     120


<210>  665
<211>  120
<212>  DNA
<213>  Artificial Sequence

<220>
<223>  KDR

<400>  665
gccaagaaca ctgcatgcct ggcaggtgta gaggccttcg tcctccttcc tcactctgcg      60

gatagtgagg ttccggttcc catccttcaa tacaatgcct aacagaggaa gaaaacgatc     120

<210>  666
<211>  120
<212>  DNA
<213>  Artificial Sequence

<220>
<223>  KDR

<400>  666
gatagtgagg ttccggttcc catccttcaa tacaatgcct aacagaggaa gaaaacgatc      60

attctcattt atgatgatgt agtctgtgaa gtttttcagg gaaatttaga aaatataaat     120


<210>  667
<211>  120
<212>  DNA
<213>  Artificial Sequence

<220>
<223>  KDR

<400>  667
cctgggaaag ttctgcaacc caagagtgat agccaaattc tatttacctg agtcttctac      60

aagggtctca ttatctttaa accacatgat ctgtggaggg ggattcccag atgccgtgca     120


<210>  668
<211>  120
<212>  DNA
<213>  Artificial Sequence

<220>
<223>  KDR

<400>  668
aagggtctca ttatctttaa accacatgat ctgtggaggg ggattcccag atgccgtgca      60

tgagacttcg atgctttccc caatacttgt cgtctgattc tccaggtttc ctgtgatcgt     120


<210>  669
<211>  120
<212>  DNA
<213>  Artificial Sequence

<220>
<223>  KDR

<400>  669
tgagacttcg atgctttccc caatacttgt cgtctgattc tccaggtttc ctgtgatcgt      60

gggtgccaca cgctctagac acacaaaaag aaaatcacag aacatggaat tataactttt     120


<210>  670
<211>  120
<212>  DNA
<213>  Artificial Sequence

<220>
<223>  KDR

<400>  670
caactgcctc tgcacaatga tccagaattg tctccctacc taggactgtg agctgcctga      60

ccacgcaatg tcttttcttg gtcttcctgt cttgagcaag gcagacatag tctccttggt     120


<210>  671
<211>  120
<212>  DNA
<213>  Artificial Sequence

<220>
<223>  KDR

<400>  671
ccacgcaatg tcttttcttg gtcttcctgt cttgagcaag gcagacatag tctccttggt      60

cctgcaagga tgcattctta agctccatga tcaaaatgtc atttgtgcta ttagagaaca     120


<210>  672
<211>  120
<212>  DNA
<213>  Artificial Sequence

<220>
<223>  KDR

<400>  672
cctgcaagga tgcattctta agctccatga tcaaaatgtc atttgtgcta ttagagaaca      60

tggtggcatt caatttccaa agagtatcca agttcttgca aacaggtgtg ggcaactctc     120


<210>  673
<211>  120
<212>  DNA
<213>  Artificial Sequence

<220>
<223>  KDR

<400>  673
tggtggcatt caatttccaa agagtatcca agttcttgca aacaggtgtg ggcaactctc      60

ccacatggat tggcagaggc tgtgggccaa gcttgtacca tgtgaggttc tcaaacgtag     120


<210>  674
<211>  120
<212>  DNA
<213>  Artificial Sequence

<220>
<223>  KDR

<400>  674
ccacatggat tggcagaggc tgtgggccaa gcttgtacca tgtgaggttc tcaaacgtag      60

atctgtctgc agtgcaccac aaagacacgc tctcctgctc agtgggctgc atgtcaggtt     120

<210>  675
<211>  120
<212>  DNA
<213>  Artificial Sequence

<220>
<223>  KDR

<400>  675
atctgtctgc agtgcaccac aaagacacgc tctcctgctc agtgggctgc atgtcaggtt      60

gcaaagtaat ttcaggaccc cctaaaatga agaggccata gttattgaat ggtgatttaa      120


<210>  676
<211>  120
<212>  DNA
<213>  Artificial Sequence

<220>
<223>  KDR

<400>  676
gaattacata gcttagtacc cactgtgtga gtgatccaac ccaaacctcc agagaagagt      60

acttactggt cacgtggaag gagatcaccc tctctcctct cccgactttg ttgaccgctt      120


<210>  677
<211>  120
<212>  DNA
<213>  Artificial Sequence

<220>
<223>  KDR

<400>  677
acttactggt cacgtggaag gagatcaccc tctctcctct cccgactttg ttgaccgctt      60

cacatttgta caaagctgac acatttgccg cttggataac aagggtactt acagtctgtg      120


<210>  678
<211>  120
<212>  DNA
<213>  Artificial Sequence

<220>
<223>  KDR

<400>  678
cacatttgta caaagctgac acatttgccg cttggataac aagggtactt acagtctgtg      60

cggggaaaaa acaaatccca ggccataaac aacgcggctg tttgtgcaca agcactcagt      120


<210>  679
<211>  120
<212>  DNA
<213>  Artificial Sequence

<220>
<223>  KDR

<400>  679
tgtaccatcc ttccattaaa gagagagaga ttttcaaatt ttaaaacttc aaactcactt      60

tgttttttcc ttcaattaga gcaaattgat ttttattaac ttcaatttta tttcctccct      120


<210>  680
<211>  120
<212>  DNA
<213>  Artificial Sequence

<220>
<223>  KDR

<400>  680
tgttttttcc ttcaattaga gcaaattgat ttttattaac ttcaatttta tttcctccct      60

ggaagtcctc cacacttctc cattcttcac aagggtatgg gtttgtcact gagacagctt      120


<210>  681
<211>  120
<212>  DNA
<213>  Artificial Sequence

<220>
<223>  KDR

<400>  681
ggaagtcctc cacacttctc cattcttcac aagggtatgg gtttgtcact gagacagctt      60

ggctataaga aagagataac agcgcatatt atgatttaat ttttctttaa ttagagtcaa      120


<210>  682
<211>  120
<212>  DNA
<213>  Artificial Sequence

<220>
<223>  KDR

<400>  682
tgcaagatgg caggaaagca aagagcatgt ggccttactc acctgggctc gttggcgcac      60

tcttcctcca actgccaata ccagtggatg tgatgcgggg gaggaatggc atagaccgta      120


<210>  683
<211>  120
<212>  DNA
<213>  Artificial Sequence

<220>
<223>  KDR

<400>  683
tcttcctcca actgccaata ccagtggatg tgatgcgggg gaggaatggc atagaccgta      60

catgtcagcg tttgagtggt gccgtactgg taggaatcca caggagagat tagagatttc      120

<210>  684
<211>  120
<212>  DNA
<213>  Artificial Sequence

<220>
<223>  KDR

<400>  684
catgtcagcg tttgagtggt gccgtactgg taggaatcca caggagagat tagagatttc      60

tcaccaatct ggggtgggac tgaagatggg aaaaacaact tttgaattgt cagtcagctt      120


<210>  685
<211>  120
<212>  DNA
<213>  Artificial Sequence

<220>
<223>  KDR

<400>  685
ataaatcttg ggcagagagg aaaattgaat ggactcacca tacacaacca gagagaccac      60

atggctctgc ttctcctttg aaatgggatt ggtaaggatg acagtgtaat ttcctgtgtc      120


<210>  686
<211>  120
<212>  DNA
<213>  Artificial Sequence

<220>
<223>  KDR

<400>  686
atggctctgc ttctcctttg aaatgggatt ggtaaggatg acagtgtaat ttcctgtgtc      60

tctttcactc acttccataa tcgtcagtac atgccccgct ttaattgtgt gattggactc      120


<210>  687
<211>  120
<212>  DNA
<213>  Artificial Sequence

<220>
<223>  KDR

<400>  687
tctttcactc acttccataa tcgtcagtac atgccccgct ttaattgtgt gattggactc      60

aaggggtatt ccatttttat acctatgaaa aaaaattctc aggaattagt atagtcaaag      120


<210>  688
<211>  120
<212>  DNA
<213>  Artificial Sequence

<220>
<223>  KDR

<400>  688
tagtatagtc aaaggatttg ctctcacacg aaatgatgct ttgcatttat ttccagtagt        60

taccatttta tttctggggg tgggtaacca aggtacttcg cagggattct gacacgctcc       120


<210>  689
<211>  120
<212>  DNA
<213>  Artificial Sequence

<220>
<223>  KDR

<400>  689
taccatttta tttctggggg tgggtaacca aggtacttcg cagggattct gacacgctcc        60

cccaccgtgg cttccaccag agattccatg ccacttccaa aagcaacaaa aggtttttct       120


<210>  690
<211>  120
<212>  DNA
<213>  Artificial Sequence

<220>
<223>  KDR

<400>  690
cccaccgtgg cttccaccag agattccatg ccacttccaa aagcaacaaa aggtttttct        60

ggaagaaaat aaaaaaaaaa aaaggtcaac ttactgtaaa tggtcatttg atttagtttt       120


<210>  691
<211>  120
<212>  DNA
<213>  Artificial Sequence

<220>
<223>  KDR

<400>  691
gcacagaata atttccaaga ccatagctta ccatggaccc tgacaaatgt gctgttcttc        60

ttggtcatca gcccactgga tgctgcacag gtgtacaatc cttggtcact ccgggttaca       120


<210>  692
<211>  120
<212>  DNA
<213>  Artificial Sequence

<220>
<223>  KDR

<400>  692
ttggtcatca gcccactgga tgctgcacag gtgtacaatc cttggtcact ccgggttaca        60

ccatctatag ttaaggtgct caaaaatttc ttcatctcac tcccagactg ggtttttagg       120

<210>  693
<211>  120
<212>  DNA
<213>  Artificial Sequence

<220>
<223>  KDR

<400>  693
ccatctatag ttaaggtgct caaaaatttc ttcatctcac tcccagactg ggtttttagg      60

tctcggttta caagtttctt atgctgatgc tgaaaaaaag agttgactga acttccaaag     120


<210>  694
<211>  120
<212>  DNA
<213>  Artificial Sequence

<220>
<223>  KDR

<400>  694
ggcttattat ctaagtattt ggaggtctgg ctttgaatca ttagcgttac cttcgaagaa      60

gggtattccc agttgaagtc aatccccaca tttagttcag ttcttgctgt acaatttaag     120


<210>  695
<211>  120
<212>  DNA
<213>  Artificial Sequence

<220>
<223>  KDR

<400>  695
gggtattccc agttgaagtc aatccccaca tttagttcag ttcttgctgt acaatttaag      60

acaagctttt ctccaacaga tagttcaatt ccatgagacg gactcagaac cacatcataa     120


<210>  696
<211>  120
<212>  DNA
<213>  Artificial Sequence

<220>
<223>  KDR

<400>  696
acaagctttt ctccaacaga tagttcaatt ccatgagacg gactcagaac cacatcataa      60

atcctatacc ctagagcaag taaattgaaa aaacagaaca tgagagagca aataagccta     120


<210>  697
<211>  120
<212>  DNA
<213>  Artificial Sequence

<220>
<223>  KDR

<400>  697
tgttattaat gatatggaaa ggaaatgtcc tcttacctac aacgacaact atgtacataa      60

tagactggta actttcatca ttaatttttg cttcacagaa gaccatgcca gcatagctga      120


<210>  698
<211>  120
<212>  DNA
<213>  Artificial Sequence

<220>
<223>  KDR

<400>  698
tagactggta actttcatca ttaatttttg cttcacagaa gaccatgcca gcatagctga      60

tcatgtagct gggaatagta aagcccttct tgctgtccca ggaaattctg ttaccatcag      120


<210>  699
<211>  120
<212>  DNA
<213>  Artificial Sequence

<220>
<223>  KDR

<400>  699
tcatgtagct gggaatagta aagcccttct tgctgtccca ggaaattctg ttaccatcag      60

gaacaaatct cttttctggg tatctctggg taataaaaag acatatcaaa tatttaatcc      120


<210>  700
<211>  120
<212>  DNA
<213>  Artificial Sequence

<220>
<223>  KDR

<400>  700
atattagctt aaaattataa aaacttaaga gacgattgga ggagatgcaa cttactgcac      60

aaagtgacac gttgagattt gaaatggacc cgagacatgg aatcaccaca gttttgtttt      120


<210>  701
<211>  120
<212>  DNA
<213>  Artificial Sequence

<220>
<223>  KDR

<400>  701
aaagtgacac gttgagattt gaaatggacc cgagacatgg aatcaccaca gttttgtttt      60

tgttctcagt aatgtacacg actccatgtt ggtcactaac agaagcaata aatggagatc      120

<210>  702
<211>  120
<212>  DNA
<213>  Artificial Sequence

<220>
<223>  KDR

<400>  702
tgttctcagt aatgtacacg actccatgtt ggtcactaac agaagcaata aatggagatc      60

tgtaatctag aagaaaattt agttttatta atgagttaat agtatttact agaaaagttg      120


<210>  703
<211>  120
<212>  DNA
<213>  Artificial Sequence

<220>
<223>  KDR

<400>  703
tgaattttat ttcaccactt accttgaaca tagacataaa tgaccgaggc caagtcagtt      60

tcccggtaga agcacttgta ggctccagtg tcatttccga tcacttttgg aattgtgagt      120


<210>  704
<211>  120
<212>  DNA
<213>  Artificial Sequence

<220>
<223>  KDR

<400>  704
tcccggtaga agcacttgta ggctccagtg tcatttccga tcacttttgg aattgtgagt      60

gtcttacaga agaggccatc gctgcactca gtcacctcca ccctttgctc actgccactc      120


<210>  705
<211>  120
<212>  DNA
<213>  Artificial Sequence

<220>
<223>  KDR

<400>  705
gtcttacaga agaggccatc gctgcactca gtcacctcca ccctttgctc actgccactc      60

tgattattgg gccaaagcca gtccaagtcc ctctgtcccc tgaaaaatta atttcaggga      120


<210>  706
<211>  120
<212>  DNA
<213>  Artificial Sequence

<220>
<223>  KDR

<400>  706
agttacttaa cacaagaaat ctagatctag aatgaatcct tacctgcaag taatttgaag      60

agttgtatta gccttaattg taagtatgtc tttttgtatg ctgagcctgg gcagatcaag     120


<210>  707
<211>  120
<212>  DNA
<213>  Artificial Sequence

<220>
<223>  KDR

<400>  707
agttgtatta gccttaattg taagtatgtc tttttgtatg ctgagcctgg gcagatcaag      60

agaaacacta ggcaaaccta gaaacaaatt aaataaatga atgtagttgc cactgagtta     120


<210>  708
<211>  120
<212>  DNA
<213>  Artificial Sequence

<220>
<223>  KDR

<400>  708
gtcctctccg ccctcacccg acctgtctgc cttcctcctc cagagtgggc tccttaccca      60

cagaggcggc ccgggtctcc acgcagagcc acagggcgac ggccagcagc accttgctct     120


<210>  709
<211>  120
<212>  DNA
<213>  Artificial Sequence

<220>
<223>  KDR

<400>  709
cagaggcggc ccgggtctcc acgcagagcc acagggcgac ggccagcagc accttgctct      60

gcatcctgca cctcgagccg ggcgaaatgc ccagaactcg ggagccggtt ctttctccca     120


<210>  710
<211>  120
<212>  DNA
<213>  Artificial Sequence

<220>
<223>  KIT

<400>  710
cacttgggcg agagctggaa cgtggaccag agctcggatc ccatcgcagc taccgcgatg      60

agaggcgctc gcggcgcctg ggattttctc tgcgttctgc tcctactgct tcgcgtccag     120

<210>  711
<211>  120
<212>  DNA
<213>  Artificial Sequence

<220>
<223>  KIT

<400>  711
agaggcgctc gcggcgcctg ggattttctc tgcgttctgc tcctactgct tcgcgtccag      60

acaggtggga caccgcggct ggcaccccga ccgtgcgact actcggcgaa gcctgtgcct      120


<210>  712
<211>  120
<212>  DNA
<213>  Artificial Sequence

<220>
<223>  KIT

<400>  712
tgtttttctt ggcaggctct tctcaaccat ctgtgagtcc aggggaaccg tctccaccat      60

ccatccatcc aggaaaatca gacttaatag tccgcgtggg cgacgagatt aggctgttat      120


<210>  713
<211>  120
<212>  DNA
<213>  Artificial Sequence

<220>
<223>  KIT

<400>  713
ccatccatcc aggaaaatca gacttaatag tccgcgtggg cgacgagatt aggctgttat      60

gcactgatcc gggctttgtc aaatggactt ttgagatcct ggatgaaacg aatgagaata      120


<210>  714
<211>  120
<212>  DNA
<213>  Artificial Sequence

<220>
<223>  KIT

<400>  714
gcactgatcc gggctttgtc aaatggactt ttgagatcct ggatgaaacg aatgagaata      60

agcagaatga atggatcacg gaaaaggcag aagccaccaa caccggcaaa tacacgtgca      120


<210>  715
<211>  120
<212>  DNA
<213>  Artificial Sequence

<220>
<223>  KIT

<400>  715
agcagaatga atggatcacg gaaaaggcag aagccaccaa caccggcaaa tacacgtgca      60

ccaacaaaca cggcttaagc aattccattt atgtgtttgt tagaggtaaa tgcttggctt     120


<210>  716
<211>  120
<212>  DNA
<213>  Artificial Sequence

<220>
<223>  KIT

<400>  716
cagccattcc aactactgat tttggatatg cttctataga tcctgccaag cttttccttg      60

ttgaccgctc cttgtatggg aaagaagaca acgacacgct ggtccgctgt cctctcacag     120


<210>  717
<211>  120
<212>  DNA
<213>  Artificial Sequence

<220>
<223>  KIT

<400>  717
ttgaccgctc cttgtatggg aaagaagaca acgacacgct ggtccgctgt cctctcacag      60

acccagaagt gaccaattat tccctcaagg ggtgccaggg gaagcctctt cccaaggact     120


<210>  718
<211>  120
<212>  DNA
<213>  Artificial Sequence

<220>
<223>  KIT

<400>  718
acccagaagt gaccaattat tccctcaagg ggtgccaggg gaagcctctt cccaaggact      60

tgaggtttat tcctgacccc aaggcgggca tcatgatcaa aagtgtgaaa cgcgcctacc     120


<210>  719
<211>  120
<212>  DNA
<213>  Artificial Sequence

<220>
<223>  KIT

<400>  719
tgaggtttat tcctgacccc aaggcgggca tcatgatcaa aagtgtgaaa cgcgcctacc      60

atcggctctg tctgcattgt tctgtggacc aggagggcaa gtcagtgctg tcggaaaaat     120

<210>  720
<211>  120
<212>  DNA
<213>  Artificial Sequence

<220>
<223>  KIT

<400>  720
atcggctctg tctgcattgt tctgtggacc aggagggcaa gtcagtgctg tcggaaaaat      60

tcatcctgaa agtgaggcca ggtactggct ctttcttatc tgcctctggg agttgagaac      120


<210>  721
<211>  120
<212>  DNA
<213>  Artificial Sequence

<220>
<223>  KIT

<400>  721
tttcatgcta taatacaaat tatttgaggg gccacatttc ttttcattct agccttcaaa      60

gctgtgcctg ttgtgtctgt gtccaaagca agctatcttc ttagggaagg ggaagaattc      120


<210>  722
<211>  120
<212>  DNA
<213>  Artificial Sequence

<220>
<223>  KIT

<400>  722
gctgtgcctg ttgtgtctgt gtccaaagca agctatcttc ttagggaagg ggaagaattc      60

acagtgacgt gcacaataaa agatgtgtct agttctgtgt actcaacgtg gaaaagagaa      120


<210>  723
<211>  120
<212>  DNA
<213>  Artificial Sequence

<220>
<223>  KIT

<400>  723
acagtgacgt gcacaataaa agatgtgtct agttctgtgt actcaacgtg gaaaagagaa      60

aacagtcagg tgagtgaatc gcttcattct tctcatgttc tgtctctgtg ggagatgata      120


<210>  724
<211>  120
<212>  DNA
<213>  Artificial Sequence

<220>
<223>  KIT

<400>  724
tcttcatttt tttttctcct tttctgaaac cagcagacta aactacagga gaaatataat      60

agctggcatc acggtgactt caattatgaa cgtcaggcaa cgttgactat cagttcagcg      120


<210>  725
<211>  120
<212>  DNA
<213>  Artificial Sequence

<220>
<223>  KIT

<400>  725
agctggcatc acggtgactt caattatgaa cgtcaggcaa cgttgactat cagttcagcg      60

agagttaatg attctggagt gttcatgtgt tatgccaata atacttttgg atcagcaaat      120


<210>  726
<211>  120
<212>  DNA
<213>  Artificial Sequence

<220>
<223>  KIT

<400>  726
agagttaatg attctggagt gttcatgtgt tatgccaata atacttttgg atcagcaaat      60

gtcacaacaa ccttggaagt agtaggtaaa tacctctatg ggaatgttta aattactggc      120


<210>  727
<211>  120
<212>  DNA
<213>  Artificial Sequence

<220>
<223>  KIT

<400>  727
ttctgtctta tttcattcta attagataaa ggattcatta atatcttccc catgataaac      60

actacagtat ttgtaaacga tggagaaaat gtagatttga ttgttgaata tgaagcattc      120


<210>  728
<211>  120
<212>  DNA
<213>  Artificial Sequence

<220>
<223>  KIT

<400>  728
actacagtat ttgtaaacga tggagaaaat gtagatttga ttgttgaata tgaagcattc      60

cccaaacctg aacaccagca gtggatctat atgaacagaa ccttcactga taaatgggaa      120

<210>  729
<211>  120
<212>  DNA
<213>  Artificial Sequence

<220>
<223>  KIT

<400>  729
cccaaacctg aacaccagca gtggatctat atgaacagaa ccttcactga taaatgggaa      60

gattatccca agtctgagaa tgaaagtaat atcaggtaag aaatggacct tgccctgggg      120


<210>  730
<211>  120
<212>  DNA
<213>  Artificial Sequence

<220>
<223>  KIT

<400>  730
aaagacatgc cttccaaggc atgctatcca caggtgattg actagttgtc ttttctttgt      60

agatacgtaa gtgaacttca tctaacgaga ttaaaaggca ccgaaggagg cacttacaca      120


<210>  731
<211>  120
<212>  DNA
<213>  Artificial Sequence

<220>
<223>  KIT

<400>  731
agatacgtaa gtgaacttca tctaacgaga ttaaaaggca ccgaaggagg cacttacaca      60

ttcctagtgt ccaattctga cgtcaatgct gccatagcat ttaatgttta tgtgaatagt      120


<210>  732
<211>  120
<212>  DNA
<213>  Artificial Sequence

<220>
<223>  KIT

<400>  732
ttcctagtgt ccaattctga cgtcaatgct gccatagcat ttaatgttta tgtgaatagt      60

aagtaacatg aagggctcct tttaattttt tattctttta aagttgtggc tcgtgtttgt      120


<210>  733
<211>  120
<212>  DNA
<213>  Artificial Sequence

<220>
<223>  KIT

<400>  733
tgaagtgaat gttgctgagg ttttccagca ctctgacata tggccatttc tgttttcctg      60

tagcaaaacc agaaatcctg acttacgaca ggctcgtgaa tggcatgctc caatgtgtgg     120


<210>  734
<211>  120
<212>  DNA
<213>  Artificial Sequence

<220>
<223>  KIT

<400>  734
tagcaaaacc agaaatcctg acttacgaca ggctcgtgaa tggcatgctc caatgtgtgg      60

cagcaggatt cccagagccc acaatagatt ggtatttttg tccaggaact gagcagaggt     120


<210>  735
<211>  120
<212>  DNA
<213>  Artificial Sequence

<220>
<223>  KIT

<400>  735
cagcaggatt cccagagccc acaatagatt ggtatttttg tccaggaact gagcagaggt      60

gagatgatta tttttggcac tgcttataat gcagagggga aggactgcaa ttcacttgaa     120


<210>  736
<211>  120
<212>  DNA
<213>  Artificial Sequence

<220>
<223>  KIT

<400>  736
ggcttttgtt ttcttccctt tagatgctct gcttctgtac tgccagtgga tgtgcagaca      60

ctaaactcat ctgggccacc gtttggaaag ctagtggttc agagttctat agattctagt     120


<210>  737
<211>  120
<212>  DNA
<213>  Artificial Sequence

<220>
<223>  KIT

<400>  737
ctaaactcat ctgggccacc gtttggaaag ctagtggttc agagttctat agattctagt      60

gcattcaagc acaatggcac ggttgaatgt aaggcttaca acgatgtggg caagacttct     120

<210>  738
<211>  120
<212>  DNA
<213>  Artificial Sequence

<220>
<223>  KIT

<400>  738
gcattcaagc acaatggcac ggttgaatgt aaggcttaca acgatgtggg caagacttct      60

gcctatttta actttgcatt taaaggtaac aacaaaggta tatttctttt taatccaatt      120


<210>  739
<211>  120
<212>  DNA
<213>  Artificial Sequence

<220>
<223>  KIT

<400>  739
ctgagtggct gtggtagaga tcccatcctg ccaaagtttg tgattccaca tttctcttcc      60

attgtagagc aaatccatcc ccacaccctg ttcactcctt tgctgattgg tttcgtaatc      120


<210>  740
<211>  120
<212>  DNA
<213>  Artificial Sequence

<220>
<223>  KIT

<400>  740
attgtagagc aaatccatcc ccacaccctg ttcactcctt tgctgattgg tttcgtaatc      60

gtagctggca tgatgtgcat tattgtgatg attctgacct acaaatattt acaggtaacc      120


<210>  741
<211>  120
<212>  DNA
<213>  Artificial Sequence

<220>
<223>  KIT

<400>  741
gtagctggca tgatgtgcat tattgtgatg attctgacct acaaatattt acaggtaacc      60

atttatttgt tctctctcca gagtgctcta atgactgaga caataattat taaaaggtga      120


<210>  742
<211>  120
<212>  DNA
<213>  Artificial Sequence

<220>
<223>  KIT

<400>  742
taatgactga gacaataatt attaaaaggt gatctatttt tccctttctc cccacagaaa      60

cccatgtatg aagtacagtg gaaggttgtt gaggagataa atggaaacaa ttatgtttac     120


<210>  743
<211>  120
<212>  DNA
<213>  Artificial Sequence

<220>
<223>  KIT

<400>  743
cccatgtatg aagtacagtg gaaggttgtt gaggagataa atggaaacaa ttatgtttac      60

atagacccaa cacaacttcc ttatgatcac aaatgggagt ttcccagaaa caggctgagt     120


<210>  744
<211>  120
<212>  DNA
<213>  Artificial Sequence

<220>
<223>  KIT

<400>  744
atagacccaa cacaacttcc ttatgatcac aaatgggagt ttcccagaaa caggctgagt      60

tttggtcagt atgaaacagg ggctttccat gtcacctttt tgggtacaca taacagtgac     120


<210>  745
<211>  120
<212>  DNA
<213>  Artificial Sequence

<220>
<223>  KIT

<400>  745
gcacaaatgg tccttcaatt ccaccaccag caccatcacc acttaccttg ttgtcttcct      60

tcctacaggg aaaaccctgg gtgctggagc tttcgggaag gttgttgagg caactgctta     120


<210>  746
<211>  120
<212>  DNA
<213>  Artificial Sequence

<220>
<223>  KIT

<400>  746
tcctacaggg aaaaccctgg gtgctggagc tttcgggaag gttgttgagg caactgctta      60

tggcttaatt aagtcagatg cggccatgac tgtcgctgta aagatgctca agcgtaagtt     120

<210>  747
<211>  120
<212>  DNA
<213>  Artificial Sequence

<220>
<223>  KIT

<400>  747
tggcttaatt aagtcagatg cggccatgac tgtcgctgta aagatgctca agcgtaagtt      60

cctgtatggt actgcatgcg cttgacatca gtttgccagt tgtgcttttt gctaaaatgc      120


<210>  748
<211>  120
<212>  DNA
<213>  Artificial Sequence

<220>
<223>  KIT

<400>  748
ctgcatgcgc ttgacatcag tttgccagtt gtgctttttg ctaaaatgca tgtttccaat      60

tttagcgagt gcccatttga cagaacggga agccctcatg tctgaactca aagtcctgag      120


<210>  749
<211>  120
<212>  DNA
<213>  Artificial Sequence

<220>
<223>  KIT

<400>  749
tttagcgagt gcccatttga cagaacggga agccctcatg tctgaactca aagtcctgag      60

ttaccttggt aatcacatga atattgtgaa tctacttgga gcctgcacca ttggaggtaa      120


<210>  750
<211>  120
<212>  DNA
<213>  Artificial Sequence

<220>
<223>  KIT

<400>  750
ttaccttggt aatcacatga atattgtgaa tctacttgga gcctgcacca ttggaggtaa      60

agccgtgtcc aagctgcctt ttattgtctg tcaggttatc aaaacatgac attttaatat      120


<210>  751
<211>  120
<212>  DNA
<213>  Artificial Sequence

<220>
<223>  KIT

<400>  751
catgtcccac ttgattcagt catgacttgt ttcatctctc ccagcagcga tagtactaat      60

gagtacatgg acatgaaacc tggagtttct tatgttgtcc caaccaaggc cgacaaaagg     120


<210>  752
<211>  120
<212>  DNA
<213>  Artificial Sequence

<220>
<223>  KIT

<400>  752
gagtacatgg acatgaaacc tggagtttct tatgttgtcc caaccaaggc cgacaaaagg      60

agatctgtga gaataggtga gtacctacct atcaagcaac caagagtaac tttacagaga     120


<210>  753
<211>  120
<212>  DNA
<213>  Artificial Sequence

<220>
<223>  KIT

<400>  753
cttttccttt cctgaccttt atggttgtaa ttgctaagaa aaatcctctc ttcctcacag      60

gctcatacat agaaagagat gtgactcccg ccatcatgga ggatgacgag ttggccctag     120


<210>  754
<211>  120
<212>  DNA
<213>  Artificial Sequence

<220>
<223>  KIT

<400>  754
gctcatacat agaaagagat gtgactcccg ccatcatgga ggatgacgag ttggccctag      60

acttagaaga cttgctgagc ttttcttacc aggtggcaaa gggcatggct ttcctcgcct     120


<210>  755
<211>  120
<212>  DNA
<213>  Artificial Sequence

<220>
<223>  KIT

<400>  755
acttagaaga cttgctgagc ttttcttacc aggtggcaaa gggcatggct ttcctcgcct      60

ccaagaatgt aagtgggagt gattctctaa agagttttgt gttttgtttt tttgattttt     120

<210>  756
<211>  120
<212>  DNA
<213>  Artificial Sequence

<220>
<223>  KIT

<400>  756
tcactcttta caagttaaaa tgaatttaaa tggttttctt ttctcctcca acctaatagt      60

gtattcacag agacttggca gccagaaata tcctccttac tcatggtcgg atcacaaaga      120


<210>  757
<211>  120
<212>  DNA
<213>  Artificial Sequence

<220>
<223>  KIT

<400>  757
gtattcacag agacttggca gccagaaata tcctccttac tcatggtcgg atcacaaaga      60

tttgtgattt tggtctagcc agagacatca agaatgattc taattatgtg gttaaaggaa      120


<210>  758
<211>  120
<212>  DNA
<213>  Artificial Sequence

<220>
<223>  KIT

<400>  758
tttgtgattt tggtctagcc agagacatca agaatgattc taattatgtg gttaaaggaa      60

acgtgagtac ccattctctg cttgacagtc ctgcaaagga tttttagttt caactttcga      120


<210>  759
<211>  120
<212>  DNA
<213>  Artificial Sequence

<220>
<223>  KIT

<400>  759
cttctgttca attttgttga gcttctgaat taacattatt gactctgttg tgcttctatt      60

acaggctcga ctacctgtga agtggatggc acctgaaagc attttcaact gtgtatacac      120


<210>  760
<211>  120
<212>  DNA
<213>  Artificial Sequence

<220>
<223>  KIT

<400>  760
acaggctcga ctacctgtga agtggatggc acctgaaagc attttcaact gtgtatacac      60

gtttgaaagt gacgtctggt cctatgggat ttttctttgg gagctgttct ctttaggtaa      120


<210>  761
<211>  120
<212>  DNA
<213>  Artificial Sequence

<220>
<223>  KIT

<400>  761
gtttgaaagt gacgtctggt cctatgggat ttttctttgg gagctgttct ctttaggtaa      60

aatgatcctt gccaaagaca acttcattag actcagagca tcttcttgaa gtttcattgg      120


<210>  762
<211>  120
<212>  DNA
<213>  Artificial Sequence

<220>
<223>  KIT

<400>  762
cttccttgtg attaacactg ctttgcaaac tgtgtctcag gaagcagccc ctatcctgga      60

atgccggtcg attctaagtt ctacaagatg atcaaggaag gcttccggat gctcagccct      120


<210>  763
<211>  120
<212>  DNA
<213>  Artificial Sequence

<220>
<223>  KIT

<400>  763
atgccggtcg attctaagtt ctacaagatg atcaaggaag gcttccggat gctcagccct      60

gaacacgcac ctgctgaaat gtaagagcca aaaaattttt cctttaggtc acgttttccc      120


<210>  764
<211>  120
<212>  DNA
<213>  Artificial Sequence

<220>
<223>  KIT

<400>  764
atgcccatac atttgaaaac aagctgaggg cattgaggag ggatagtaaa tggcccttgt      60

cttgcaggta tgacataatg aagacttgct gggatgcaga tcccctaaaa agaccaacat      120

<210>  765
<211>  120
<212>  DNA
<213>  Artificial Sequence

<220>
<223>  KIT

<400>  765
cttgcaggta tgacataatg aagacttgct gggatgcaga tcccctaaaa agaccaacat      60

tcaagcaaat tgttcagcta attgagaagc agatttcaga gagcaccaat catgtgagta      120


<210>  766
<211>  120
<212>  DNA
<213>  Artificial Sequence

<220>
<223>  KIT

<400>  766
tcaagcaaat tgttcagcta attgagaagc agatttcaga gagcaccaat catgtgagta      60

taccctggcc aggcatagaa tcccccttct cccagttcca ggtgtgtcct cctcctcagg      120


<210>  767
<211>  120
<212>  DNA
<213>  Artificial Sequence

<220>
<223>  KIT

<400>  767
tgttgctatg ttcgttgtag ggactgctgt attgactatg ggcttgtttt ctccagattt      60

actccaactt agcaaactgc agccccaacc gacagaagcc cgtggtagac cattctgtgc      120


<210>  768
<211>  120
<212>  DNA
<213>  Artificial Sequence

<220>
<223>  KIT

<400>  768
actccaactt agcaaactgc agccccaacc gacagaagcc cgtggtagac cattctgtgc      60

ggatcaattc tgtcggcagc accgcttcct cctcccagcc tctgcttgtg cacgacgatg      120


<210>  769
<211>  120
<212>  DNA
<213>  Artificial Sequence

<220>
<223>  KIT

<400>  769
ggatcaattc tgtcggcagc accgcttcct cctcccagcc tctgcttgtg cacgacgatg      60

tctgagcaga atcagtgttt gggtcacccc tccaggaatg atctcttctt ttggcttcca     120


<210>  770
<211>  120
<212>  DNA
<213>  Artificial Sequence

<220>
<223>  KIT-E11

<400>  770
taatgactga gacaataatt attaaaaggt gatctatttt tccctttctc cccacagaaa      60

cccatgtatg aagtacagtg gaaggttgtt gaggagataa atggaaacaa ttatgtttac     120


<210>  771
<211>  120
<212>  DNA
<213>  Artificial Sequence

<220>
<223>  KIT-E11

<400>  771
aaaggtgatc tatttttccc tttctcccca cagaaaccca tgtatgaagt acagtggaag      60

gttgttgagg agataaatgg aaacaattat gtttacatag acccaacaca acttccttat     120


<210>  772
<211>  120
<212>  DNA
<213>  Artificial Sequence

<220>
<223>  KIT-E11

<400>  772
ataaatggaa acaattatgt ttacatagac ccaacacaac ttccttatga tcacaaatgg      60

gagtttccca gaaacaggct gagttttggt cagtatgaaa caggggcttt ccatgtcacc     120


<210>  773
<211>  120
<212>  DNA
<213>  Artificial Sequence

<220>
<223>  KIT-E11

<400>  773
atagacccaa cacaacttcc ttatgatcac aaatgggagt ttcccagaaa caggctgagt      60

tttggtcagt atgaaacagg ggctttccat gtcacctttt tgggtacaca taacagtgac     120

<210>  774
<211>  120
<212>  DNA
<213>  Artificial Sequence

<220>
<223>  KIT-E13

<400>  774
ctgtaaagat gctcaagcgt aagttcctgt atggtactgc atgcgcttga catcagtttg      60

ccagttgtgc tttttgctaa aatgcatgtt tccaatttta gcgagtgccc atttgacaga      120


<210>  775
<211>  120
<212>  DNA
<213>  Artificial Sequence

<220>
<223>  KIT-E13

<400>  775
tcctgtatgg tactgcatgc gcttgacatc agtttgccag ttgtgctttt tgctaaaatg      60

catgtttcca attttagcga gtgcccattt gacagaacgg gaagccctca tgtctgaact      120


<210>  776
<211>  120
<212>  DNA
<213>  Artificial Sequence

<220>
<223>  KIT-E13

<400>  776
gacatcagtt tgccagttgt gctttttgct aaaatgcatg tttccaattt tagcgagtgc      60

ccatttgaca gaacgggaag ccctcatgtc tgaactcaaa gtcctgagtt accttggtaa      120


<210>  777
<211>  120
<212>  DNA
<213>  Artificial Sequence

<220>
<223>  KIT-E13

<400>  777
tttgctaaaa tgcatgtttc caattttagc gagtgcccat ttgacagaac gggaagccct      60

catgtctgaa ctcaaagtcc tgagttacct tggtaatcac atgaatattg tgaatctact      120


<210>  778
<211>  120
<212>  DNA
<213>  Artificial Sequence

<220>
<223>  KIT-E13

<400>  778
acgggaagcc ctcatgtctg aactcaaagt cctgagttac cttggtaatc acatgaatat      60

tgtgaatcta cttggagcct gcaccattgg aggtaaagcc gtgtccaagc tgccttttat     120


<210>  779
<211>  120
<212>  DNA
<213>  Artificial Sequence

<220>
<223>  KIT-E13

<400>  779
caaagtcctg agttaccttg gtaatcacat gaatattgtg aatctacttg gagcctgcac      60

cattggaggt aaagccgtgt ccaagctgcc ttttattgtc tgtcaggtta tcaaaacatg     120


<210>  780
<211>  120
<212>  DNA
<213>  Artificial Sequence

<220>
<223>  KIT-E13

<400>  780
tcacatgaat attgtgaatc tacttggagc ctgcaccatt ggaggtaaag ccgtgtccaa      60

gctgcctttt attgtctgtc aggttatcaa aacatgacat tttaatatga ttttggcaat     120


<210>  781
<211>  120
<212>  DNA
<213>  Artificial Sequence

<220>
<223>  KIT-E13

<400>  781
tggagcctgc accattggag gtaaagccgt gtccaagctg ccttttattg tctgtcaggt      60

tatcaaaaca tgacatttta atatgatttt ggcaatgcta gattataaac tgcttggaag     120


<210>  782
<211>  120
<212>  DNA
<213>  Artificial Sequence

<220>
<223>  KIT-E14

<400>  782
ttacagaata ttgttgctat ggtgatcttt tgaatttttt gagaagaaaa cgtgattcat      60

ttatttgttc aaagcaggaa gatcatgcag aagctgcact ttataagaat cttctgcatt     120

<210>  783
<211>  120
<212>  DNA
<213>  Artificial Sequence

<220>
<223>  KIT-E17

<400>  783
tcactcttta caagttaaaa tgaatttaaa tggttttctt ttctcctcca acctaatagt      60

gtattcacag agacttggca gccagaaata tcctccttac tcatggtcgg atcacaaaga      120


<210>  784
<211>  120
<212>  DNA
<213>  Artificial Sequence

<220>
<223>  KIT-E17

<400>  784
tttaaatggt tttcttttct cctccaacct aatagtgtat tcacagagac ttggcagcca      60

gaaatatcct ccttactcat ggtcggatca caaagatttg tgattttggt ctagccagag      120


<210>  785
<211>  120
<212>  DNA
<213>  Artificial Sequence

<220>
<223>  KIT-E17

<400>  785
ttactcatgg tcggatcaca aagatttgtg attttggtct agccagagac atcaagaatg      60

attctaatta tgtggttaaa ggaaacgtga gtacccattc tctgcttgac agtcctgcaa      120


<210>  786
<211>  120
<212>  DNA
<213>  Artificial Sequence

<220>
<223>  KIT-E17

<400>  786
tttgtgattt tggtctagcc agagacatca agaatgattc taattatgtg gttaaaggaa      60

acgtgagtac ccattctctg cttgacagtc ctgcaaagga tttttagttt caactttcga      120


<210>  787
<211>  120
<212>  DNA
<213>  Artificial Sequence

<220>
<223>  KIT-E18

<400>  787
tccacatttc agcaacagca gcatctataa gaatatcttc tgttcaattt tgttgagctt      60

ctgaattaac attattgact ctgttgtgct tctattacag gctcgactac ctgtgaagtg     120


<210>  788
<211>  120
<212>  DNA
<213>  Artificial Sequence

<220>
<223>  KIT-E18

<400>  788
ctataagaat atcttctgtt caattttgtt gagcttctga attaacatta ttgactctgt      60

tgtgcttcta ttacaggctc gactacctgt gaagtggatg gcacctgaaa gcattttcaa     120


<210>  789
<211>  120
<212>  DNA
<213>  Artificial Sequence

<220>
<223>  KIT-E18

<400>  789
tttgttgagc ttctgaatta acattattga ctctgttgtg cttctattac aggctcgact      60

acctgtgaag tggatggcac ctgaaagcat tttcaactgt gtatacacgt ttgaaagtga     120


<210>  790
<211>  120
<212>  DNA
<213>  Artificial Sequence

<220>
<223>  KIT-E18

<400>  790
tattgactct gttgtgcttc tattacaggc tcgactacct gtgaagtgga tggcacctga      60

aagcattttc aactgtgtat acacgtttga aagtgacgtc tggtcctatg ggatttttct     120


<210>  791
<211>  120
<212>  DNA
<213>  Artificial Sequence

<220>
<223>  KIT-E18

<400>  791
acaggctcga ctacctgtga agtggatggc acctgaaagc attttcaact gtgtatacac      60

gtttgaaagt gacgtctggt cctatgggat ttttctttgg gagctgttct ctttaggtaa     120

<210>  792
<211>  120
<212>  DNA
<213>  Artificial Sequence

<220>
<223>  KIT-E18

<400>  792
gatggcacct gaaagcattt tcaactgtgt atacacgttt gaaagtgacg tctggtccta      60

tgggattttt ctttgggagc tgttctcttt aggtaaaatg atccttgcca aagacaactt      120


<210>  793
<211>  120
<212>  DNA
<213>  Artificial Sequence

<220>
<223>  KIT-E18

<400>  793
ctgtgtatac acgtttgaaa gtgacgtctg gtcctatggg atttttcttt gggagctgtt      60

ctctttaggt aaaatgatcc ttgccaaaga caacttcatt agactcagag catcttcttg      120


<210>  794
<211>  120
<212>  DNA
<213>  Artificial Sequence

<220>
<223>  KIT-E18

<400>  794
cgtctggtcc tatgggattt ttctttggga gctgttctct ttaggtaaaa tgatccttgc      60

caaagacaac ttcattagac tcagagcatc ttcttgaagt ttcattggtg tcctgcttcc      120


<210>  795
<211>  120
<212>  DNA
<213>  Artificial Sequence

<220>
<223>  KIT-E18

<400>  795
ttgggagctg ttctctttag gtaaaatgat ccttgccaaa gacaacttca ttagactcag      60

agcatcttct tgaagtttca ttggtgtcct gcttccttgt gattaacact gctttgcaaa      120


<210>  796
<211>  120
<212>  DNA
<213>  Artificial Sequence

<220>
<223>  KIT-E8

<400>  796
gcagcctcag gaaggttgta gggattagag agggagtgaa gtgaatgttg ctgaggtttt      60

ccagcactct gacatatggc catttctgtt ttcctgtagc aaaaccagaa atcctgactt     120


<210>  797
<211>  120
<212>  DNA
<213>  Artificial Sequence

<220>
<223>  KIT-E8

<400>  797
ttagagaggg agtgaagtga atgttgctga ggttttccag cactctgaca tatggccatt      60

tctgttttcc tgtagcaaaa ccagaaatcc tgacttacga caggctcgtg aatggcatgc     120


<210>  798
<211>  120
<212>  DNA
<213>  Artificial Sequence

<220>
<223>  KIT-E8

<400>  798
tgctgaggtt ttccagcact ctgacatatg gccatttctg ttttcctgta gcaaaaccag      60

aaatcctgac ttacgacagg ctcgtgaatg gcatgctcca atgtgtggca gcaggattcc     120


<210>  799
<211>  120
<212>  DNA
<213>  Artificial Sequence

<220>
<223>  KIT-E8

<400>  799
catatggcca tttctgtttt cctgtagcaa aaccagaaat cctgacttac gacaggctcg      60

tgaatggcat gctccaatgt gtggcagcag gattcccaga gcccacaata gattggtatt     120


<210>  800
<211>  120
<212>  DNA
<213>  Artificial Sequence

<220>
<223>  KIT-E8

<400>  800
tagcaaaacc agaaatcctg acttacgaca ggctcgtgaa tggcatgctc caatgtgtgg      60

cagcaggatt cccagagccc acaatagatt ggtatttttg tccaggaact gagcagaggt     120

<210> 801
<211> 120
<212> DNA
<213> Artificial Sequence

<220>
<223> KIT-E8

<400> 801
acgacaggct cgtgaatggc atgctccaat gtgtggcagc aggattccca gagcccacaa 60

tagattggta tttttgtcca ggaactgagc agaggtgaga tgattatttt tggcactgct 120


<210> 802
<211> 120
<212> DNA
<213> Artificial Sequence

<220>
<223> KIT-E8

<400> 802
tccaatgtgt ggcagcagga ttcccagagc ccacaataga ttggtatttt tgtccaggaa 60

ctgagcagag gtgagatgat tatttttggc actgcttata atgcagaggg gaaggactgc 120


<210> 803
<211> 120
<212> DNA
<213> Artificial Sequence

<220>
<223> KIT-E8

<400> 803
cagagcccac aatagattgg tatttttgtc caggaactga gcagaggtga gatgattatt 60

tttggcactg cttataatgc agaggggaag gactgcaatt cacttgaatt tcaaatatgt 120


<210> 804
<211> 120
<212> DNA
<213> Artificial Sequence

<220>
<223> KIT-E8

<400> 804
tttgtccagg aactgagcag aggtgagatg attatttttg gcactgctta taatgcagag 60

gggaaggact gcaattcact tgaatttcaa atatgttttc tgattttttt taaaaaagct 120


<210> 805
<211> 120
<212> DNA
<213> Artificial Sequence

<220>
<223> KIT-E9

<400>  805
ttttatgtat ttatttattt tcctagagta agccagggct tttgttttct tccctttaga        60

tgctctgctt ctgtactgcc agtggatgtg cagacactaa actcatctgg gccaccgttt       120


<210>  806
<211>  120
<212>  DNA
<213>  Artificial Sequence

<220>
<223>  KIT-E9

<400>  806
agagtaagcc agggcttttg ttttcttccc tttagatgct ctgcttctgt actgccagtg        60

gatgtgcaga cactaaactc atctgggcca ccgtttggaa agctagtggt tcagagttct       120


<210>  807
<211>  120
<212>  DNA
<213>  Artificial Sequence

<220>
<223>  KIT-E9

<400>  807
gtactgccag tggatgtgca gacactaaac tcatctgggc caccgtttgg aaagctagtg        60

gttcagagtt ctatagattc tagtgcattc aagcacaatg gcacggttga atgtaaggct       120


<210>  808
<211>  120
<212>  DNA
<213>  Artificial Sequence

<220>
<223>  KIT-E9

<400>  808
ctaaactcat ctgggccacc gtttggaaag ctagtggttc agagttctat agattctagt        60

gcattcaagc acaatggcac ggttgaatgt aaggcttaca acgatgtggg caagacttct       120


<210>  809
<211>  120
<212>  DNA
<213>  Artificial Sequence

<220>
<223>  KIT-E9

<400>  809
ggaaagctag tggttcagag ttctatagat tctagtgcat tcaagcacaa tggcacggtt        60

gaatgtaagg cttacaacga tgtgggcaag acttctgcct attttaactt tgcatttaaa       120

<210>  810
<211>  120
<212>  DNA
<213>  Artificial Sequence

<220>
<223>  KIT-E9

<400>  810
aatggcacgg ttgaatgtaa ggcttacaac gatgtgggca agacttctgc ctattttaac      60

tttgcattta aaggtaacaa caaaggtata tttcttttta atccaattta aggggatgtt      120


<210>  811
<211>  120
<212>  DNA
<213>  Artificial Sequence

<220>
<223>  KIT-E9

<400>  811
tacaacgatg tgggcaagac ttctgcctat tttaactttg catttaaagg taacaacaaa      60

ggtatatttc tttttaatcc aatttaaggg gatgtttagg ctctgtctac catatcagtc      120


<210>  812
<211>  120
<212>  DNA
<213>  Artificial Sequence

<220>
<223>  KIT-E9

<400>  812
ttttatgtat ttatttattt tcctagagta agccagggct tttgttttct tccctttaga      60

tgctctgctt ctgtactgcc agtggatgtg cagacactaa actcatctgg gccaccgttt      120


<210>  813
<211>  120
<212>  DNA
<213>  Artificial Sequence

<220>
<223>  KIT-E9

<400>  813
agagtaagcc agggcttttg ttttcttccc tttagatgct ctgcttctgt actgccagtg      60

gatgtgcaga cactaaactc atctgggcca ccgtttggaa agctagtggt tcagagttct      120


<210>  814
<211>  120
<212>  DNA
<213>  Artificial Sequence

<220>
<223>  KIT-E9

<400>  814
cttcccttta gatgctctgc ttctgtactg ccagtggatg tgcagacact aaactcatct      60

gggccaccgt ttggaaagct agtggttcag agttctatag attctagtgc attcaagcac     120


<210>  815
<211>  120
<212>  DNA
<213>  Artificial Sequence

<220>
<223>  KIT-E9

<400>  815
gtactgccag tggatgtgca gacactaaac tcatctgggc caccgtttgg aaagctagtg      60

gttcagagtt ctatagattc tagtgcattc aagcacaatg gcacggttga atgtaaggct     120


<210>  816
<211>  120
<212>  DNA
<213>  Artificial Sequence

<220>
<223>  KIT-E9

<400>  816
ctaaactcat ctgggccacc gtttggaaag ctagtggttc agagttctat agattctagt      60

gcattcaagc acaatggcac ggttgaatgt aaggcttaca acgatgtggg caagacttct     120


<210>  817
<211>  120
<212>  DNA
<213>  Artificial Sequence

<220>
<223>  KIT-E9

<400>  817
ggaaagctag tggttcagag ttctatagat tctagtgcat tcaagcacaa tggcacggtt      60

gaatgtaagg cttacaacga tgtgggcaag acttctgcct attttaactt tgcatttaaa     120


<210>  818
<211>  120
<212>  DNA
<213>  Artificial Sequence

<220>
<223>  KIT-E9

<400>  818
atagattcta gtgcattcaa gcacaatggc acggttgaat gtaaggctta caacgatgtg      60

ggcaagactt ctgcctattt taactttgca tttaaaggta acaacaaagg tatatttctt     120

<210>  819
<211>  120
<212>  DNA
<213>  Artificial Sequence

<220>
<223>  KIT-E9

<400>  819
aatggcacgg ttgaatgtaa ggcttacaac gatgtgggca agacttctgc ctattttaac      60

tttgcattta aaggtaacaa caaaggtata tttcttttta atccaattta aggggatgtt      120


<210>  820
<211>  120
<212>  DNA
<213>  Artificial Sequence

<220>
<223>  KIT-E9

<400>  820
tacaacgatg tgggcaagac ttctgcctat tttaactttg catttaaagg taacaacaaa      60

ggtatatttc tttttaatcc aatttaaggg gatgtttagg ctctgtctac catatcagtc      120


<210>  821
<211>  120
<212>  DNA
<213>  Artificial Sequence

<220>
<223>  KRAS

<400>  821
gtgtaatgta caaaaattac cacttgtact agtatgcctt aagaaaaaag tacaaattgt      60

atttacataa ttacacactt tgtctttgac ttctttttct tctttttacc atctttgctc      120


<210>  822
<211>  120
<212>  DNA
<213>  Artificial Sequence

<220>
<223>  KRAS

<400>  822
atttacataa ttacacactt tgtctttgac ttctttttct tctttttacc atctttgctc      60

atcttttctt tatgttttcg aatttctcga actaatgtat agaaggcatc atcaacaccc      120


<210>  823
<211>  120
<212>  DNA
<213>  Artificial Sequence

<220>
<223>  KRAS

<400>  823
atcttttctt tatgttttcg aatttctcga actaatgtat agaaggcatc atcaacaccc      60

tgaaatacat aaaaagtatt aaaatgtgaa tatatacgat ggcttcatgt gtacaggtaa     120


<210>  824
<211>  120
<212>  DNA
<213>  Artificial Sequence

<220>
<223>  KRAS

<400>  824
tacctttaaa agacatctgc tttctgccaa aattaatgtg ctgaacttaa acttaccaga      60

ttacattata atgcattttt taattttcac acagccagga gtcttttctt ctttgctgat     120


<210>  825
<211>  120
<212>  DNA
<213>  Artificial Sequence

<220>
<223>  KRAS

<400>  825
ttacattata atgcattttt taattttcac acagccagga gtcttttctt ctttgctgat      60

ttttttcaat ctgtattgtc ggatctccct caccaatgta taaaaagcat cctccactct     120


<210>  826
<211>  120
<212>  DNA
<213>  Artificial Sequence

<220>
<223>  KRAS

<400>  826
ttttttcaat ctgtattgtc ggatctccct caccaatgta taaaaagcat cctccactct      60

ctgcattgta aaacacaact tctttaaagt ctgttgcatt ggtaagagta atttactggg     120


<210>  827
<211>  120
<212>  DNA
<213>  Artificial Sequence

<220>
<223>  KRAS

<400>  827
ttgcagaaaa cagatctgta tttatttcag tgttacttac ctgtcttgtc tttgctgatg      60

tttcaataaa aggaattcca taacttcttg ctaagtcctg agcctgtttt gtgtctactg     120

<210>  828
<211>  120
<212>  DNA
<213>  Artificial Sequence

<220>
<223>  KRAS

<400>  828
tttcaataaa aggaattcca taacttcttg ctaagtcctg agcctgtttt gtgtctactg      60

ttctagaagg caaatcacat ttatttccta ctaggaccat aggtacatct tcagagtcct      120


<210>  829
<211>  120
<212>  DNA
<213>  Artificial Sequence

<220>
<223>  KRAS

<400>  829
ttctagaagg caaatcacat ttatttccta ctaggaccat aggtacatct tcagagtcct      60

taactctttt aatttgttct ctgggaaaga aaaaaaagtt atagcacagt cattagtaac      120


<210>  830
<211>  120
<212>  DNA
<213>  Artificial Sequence

<220>
<223>  KRAS

<400>  830
tgtcagctta ttatattcaa tttaaaccca cctataatgg tgaatatctt caaatgattt      60

agtattattt atggcaaata cacaaagaaa gccctcccca gtcctcatgt actggtccct      120


<210>  831
<211>  120
<212>  DNA
<213>  Artificial Sequence

<220>
<223>  KRAS

<400>  831
agtattattt atggcaaata cacaaagaaa gccctcccca gtcctcatgt actggtccct      60

cattgcactg tactcctctt gacctgctgt gtcgagaata tccaagagac aggtttctcc      120


<210>  832
<211>  120
<212>  DNA
<213>  Artificial Sequence

<220>
<223>  KRAS

<400>  832
cattgcactg tactcctctt gacctgctgt gtcgagaata tccaagagac aggtttctcc        60

atcaattact acttgcttcc tgtaggaatc ctgagaaggg agaaacacag tctggattat       120


<210>  833
<211>  120
<212>  DNA
<213>  Artificial Sequence

<220>
<223>  KRAS

<400>  833
aaatgtcaat aatgtattac tcggggattt cctcttgaaa ttgtatgttt tcttaatgta        60

ttaagtattg taaggacttt ttacatactg cttaataaca cctgtaggaa aagatgagaa       120


<210>  834
<211>  120
<212>  DNA
<213>  Artificial Sequence

<220>
<223>  KRAS

<400>  834
ttaagtattg taaggacttt ttacatactg cttaataaca cctgtaggaa aagatgagaa        60

acttttactc aattaatgct caagtacttt acgttactct tccgaaaggt tatttaaatt       120


<210>  835
<211>  120
<212>  DNA
<213>  Artificial Sequence

<220>
<223>  KRAS

<400>  835
gaaaccttta tctgtatcaa agaatggtcc tgcaccagta atatgcatat taaaacaaga        60

tttacctcta ttgttggatc atattcgtcc acaaaatgat tctgaattag ctgtatcgtc       120


<210>  836
<211>  120
<212>  DNA
<213>  Artificial Sequence

<220>
<223>  KRAS

<400>  836
tttacctcta ttgttggatc atattcgtcc acaaaatgat tctgaattag ctgtatcgtc        60

aaggcactct tgcctacgcc accagctcca actaccacaa gtttatattc agtcattttc       120

<210>  837
<211>  120
<212>  DNA
<213>  Artificial Sequence

<220>
<223>  KRAS

<400>  837
aaggcactct tgcctacgcc accagctcca actaccacaa gtttatattc agtcattttc      60

agcaggcctt ataataaaaa taatgaaaat gtgactatat tagaacatgt cacacataag      120


<210>  838
<211>  120
<212>  DNA
<213>  Artificial Sequence

<220>
<223>  KRAS-E2

<400>  838
tttatctgta tcaaagaatg gtcctgcacc agtaatatgc atattaaaac aagatttacc      60

tctattgttg gatcatattc gtccacaaaa tgattctgaa ttagctgtat cgtcaaggca      120


<210>  839
<211>  120
<212>  DNA
<213>  Artificial Sequence

<220>
<223>  KRAS-E2

<400>  839
tgcaccagta atatgcatat taaaacaaga tttacctcta ttgttggatc atattcgtcc      60

acaaaatgat tctgaattag ctgtatcgtc aaggcactct tgcctacgcc accagctcca      120


<210>  840
<211>  120
<212>  DNA
<213>  Artificial Sequence

<220>
<223>  KRAS-E2

<400>  840
tcatattcgt ccacaaaatg attctgaatt agctgtatcg tcaaggcact cttgcctacg      60

ccaccagctc caactaccac aagtttatat tcagtcattt tcagcaggcc ttataataaa      120


<210>  841
<211>  120
<212>  DNA
<213>  Artificial Sequence

<220>
<223>  KRAS-E2

<400>  841
tgaattagct gtatcgtcaa ggcactcttg cctacgccac cagctccaac taccacaagt      60

ttatattcag tcattttcag caggccttat aataaaaata atgaaaatgt gactatatta     120


<210>  842
<211>  120
<212>  DNA
<213>  Artificial Sequence

<220>
<223>  KRAS-E2

<400>  842
ctcttgccta cgccaccagc tccaactacc acaagtttat attcagtcat tttcagcagg      60

ccttataata aaaataatga aaatgtgact atattagaac atgtcacaca taaggttaat     120


<210>  843
<211>  120
<212>  DNA
<213>  Artificial Sequence

<220>
<223>  KRAS-E3

<400>  843
ataaaaacta taattactcc ttaatgtcag cttattatat tcaatttaaa cccacctata      60

atggtgaata tcttcaaatg atttagtatt atttatggca aatacacaaa gaaagccctc     120


<210>  844
<211>  120
<212>  DNA
<213>  Artificial Sequence

<220>
<223>  KRAS-E3

<400>  844
tgtcagctta ttatattcaa tttaaaccca cctataatgg tgaatatctt caaatgattt      60

agtattattt atggcaaata cacaaagaaa gccctcccca gtcctcatgt actggtccct     120


<210>  845
<211>  120
<212>  DNA
<213>  Artificial Sequence

<220>
<223>  KRAS-E3

<400>  845
aacccaccta taatggtgaa tatcttcaaa tgatttagta ttatttatgg caaatacaca      60

aagaaagccc tccccagtcc tcatgtactg gtccctcatt gcactgtact cctcttgacc     120

<210>  846
<211>  120
<212>  DNA
<213>  Artificial Sequence

<220>
<223>  KRAS-E3

<400>  846
ttcaaatgat ttagtattat ttatggcaaa tacacaaaga aagccctccc cagtcctcat      60

gtactggtcc ctcattgcac tgtactcctc ttgacctgct gtgtcgagaa tatccaagag      120


<210>  847
<211>  120
<212>  DNA
<213>  Artificial Sequence

<220>
<223>  KRAS-E3

<400>  847
cccagtcctc atgtactggt ccctcattgc actgtactcc tcttgacctg ctgtgtcgag      60

aatatccaag agacaggttt ctccatcaat tactacttgc ttcctgtagg aatcctgaga      120


<210>  848
<211>  120
<212>  DNA
<213>  Artificial Sequence

<220>
<223>  KRAS-E3

<400>  848
cattgcactg tactcctctt gacctgctgt gtcgagaata tccaagagac aggtttctcc      60

atcaattact acttgcttcc tgtaggaatc ctgagaaggg agaaacacag tctggattat      120


<210>  849
<211>  120
<212>  DNA
<213>  Artificial Sequence

<220>
<223>  KRAS-E3

<400>  849
tgctgtgtcg agaatatcca agagacaggt ttctccatca attactactt gcttcctgta      60

ggaatcctga gaagggagaa acacagtctg gattattaca gtgcaccttt tacttcaaaa      120


<210>  850
<211>  120
<212>  DNA
<213>  Artificial Sequence

<220>
<223>  KRAS-E4

<400>  850
acagttatga ttttgcagaa aacagatctg tatttatttc agtgttactt acctgtcttg      60

tctttgctga tgtttcaata aaaggaattc cataacttct tgctaagtcc tgagcctgtt     120


<210>  851
<211>  120
<212>  DNA
<213>  Artificial Sequence

<220>
<223>  KRAS-E4

<400>  851
gatctgtatt tatttcagtg ttacttacct gtcttgtctt tgctgatgtt tcaataaaag      60

gaattccata acttcttgct aagtcctgag cctgttttgt gtctactgtt ctagaaggca     120


<210>  852
<211>  120
<212>  DNA
<213>  Artificial Sequence

<220>
<223>  KRAS-E4

<400>  852
tttcaataaa aggaattcca taacttcttg ctaagtcctg agcctgtttt gtgtctactg      60

ttctagaagg caaatcacat ttatttccta ctaggaccat aggtacatct tcagagtcct     120


<210>  853
<211>  120
<212>  DNA
<213>  Artificial Sequence

<220>
<223>  KRAS-E4

<400>  853
ttcttgctaa gtcctgagcc tgttttgtgt ctactgttct agaaggcaaa tcacatttat      60

ttcctactag gaccataggt acatcttcag agtccttaac tcttttaatt tgttctctgg     120


<210>  854
<211>  120
<212>  DNA
<213>  Artificial Sequence

<220>
<223>  KRAS-E4

<400>  854
ttgtgtctac tgttctagaa ggcaaatcac atttatttcc tactaggacc ataggtacat      60

cttcagagtc cttaactctt ttaatttgtt ctctgggaaa gaaaaaaaag ttatagcaca     120

<210>  855
<211>  120
<212>  DNA
<213>  Artificial Sequence

<220>
<223>  KRAS-E4

<400>  855
aatcacattt atttcctact aggaccatag gtacatcttc agagtcctta actcttttaa      60

tttgttctct gggaaagaaa aaaaagttat agcacagtca ttagtaacac aaatatcttt      120


<210>  856
<211>  120
<212>  DNA
<213>  Artificial Sequence

<220>
<223>  MAP2K1

<400>  856
agaggtgctg ccctcccccc ggagttggaa gcgcgttacc cgggtccaaa atgcccaaga      60

agaagccgac gcccatccag ctgaacccgg cccccgacgg ctctgcagtt aacgggacca      120


<210>  857
<211>  120
<212>  DNA
<213>  Artificial Sequence

<220>
<223>  MAP2K1

<400>  857
agaagccgac gcccatccag ctgaacccgg cccccgacgg ctctgcagtt aacgggacca      60

gctctgcgga gtaagtatgg ggcgggcggt gaacctcggg gcccggctgg ggaggcccga      120


<210>  858
<211>  120
<212>  DNA
<213>  Artificial Sequence

<220>
<223>  MAP2K1

<400>  858
cccactttgg aacaggacca acttggaggc cttgcagaag aagctggagg agctagagct      60

tgatgagcag cagcgaaagc gccttgaggc ctttcttacc cagaagcaga aggtgggaga      120


<210>  859
<211>  120
<212>  DNA
<213>  Artificial Sequence

<220>
<223>  MAP2K1

<400>  859
tgatgagcag cagcgaaagc gccttgaggc ctttcttacc cagaagcaga aggtgggaga      60

actgaaggat gacgactttg agaagatcag tgagctgggg gctggcaatg gcggtgtggt     120


<210>  860
<211>  120
<212>  DNA
<213>  Artificial Sequence

<220>
<223>  MAP2K1

<400>  860
actgaaggat gacgactttg agaagatcag tgagctgggg gctggcaatg gcggtgtggt      60

gttcaaggtc tcccacaagc cttctggcct ggtcatggcc agaaaggtga gtttgccttg     120


<210>  861
<211>  120
<212>  DNA
<213>  Artificial Sequence

<220>
<223>  MAP2K1

<400>  861
ctccctcttt ctttcataaa acctctcttt cttccacctt tctccagcta attcatctgg      60

agatcaaacc cgcaatccgg aaccagatca taagggagct gcaggttctg catgagtgca     120


<210>  862
<211>  120
<212>  DNA
<213>  Artificial Sequence

<220>
<223>  MAP2K1

<400>  862
agatcaaacc cgcaatccgg aaccagatca taagggagct gcaggttctg catgagtgca      60

actctccgta catcgtgggc ttctatggtg cgttctacag cgatggcgag atcagtatct     120


<210>  863
<211>  120
<212>  DNA
<213>  Artificial Sequence

<220>
<223>  MAP2K1

<400>  863
actctccgta catcgtgggc ttctatggtg cgttctacag cgatggcgag atcagtatct      60

gcatggagca catggtatgt gacaccctct cagcctctgg agcaatggcc ttaagagttg     120

<210>  864
<211>  120
<212>  DNA
<213>  Artificial Sequence

<220>
<223>  MAP2K1

<400>  864
aaagaatagt tagaacattg tcactaactg gtctggtatt ctcgatctta ggatggaggt      60

tctctggatc aagtcctgaa gaaagctgga agaattcctg aacaaatttt aggaaaagtt      120


<210>  865
<211>  120
<212>  DNA
<213>  Artificial Sequence

<220>
<223>  MAP2K1

<400>  865
tctctggatc aagtcctgaa gaaagctgga agaattcctg aacaaatttt aggaaaagtt      60

agcattgctg tgagtatgtt atgaagtttt tcttctaagt tcctcattga taagttaatg      120


<210>  866
<211>  120
<212>  DNA
<213>  Artificial Sequence

<220>
<223>  MAP2K1

<400>  866
aaatagattg agtatttttc ttatcaccag tattttcttt tcttttacat tccctttcct      60

ctaggtaata aaaggcctga catatctgag ggagaagcac aagatcatgc acagaggtaa      120


<210>  867
<211>  120
<212>  DNA
<213>  Artificial Sequence

<220>
<223>  MAP2K1

<400>  867
ctaggtaata aaaggcctga catatctgag ggagaagcac aagatcatgc acagaggtaa      60

gaagttattt gctagttatt ttgctttgaa ttttagatat aatccaaagg ctgttgcttc      120


<210>  868
<211>  120
<212>  DNA
<213>  Artificial Sequence

<220>
<223>  MAP2K1

<400>  868
cagactcctg ttgtttacaa gtcttctcca gaagccctgg aagaatgaac tgtaaggcct      60

ggactatgga atggtacctg tgcaccagca attcctgagc aagcagcaca gtgagtaggt     120


<210>  869
<211>  120
<212>  DNA
<213>  Artificial Sequence

<220>
<223>  MAP2K1

<400>  869
ggactatgga atggtacctg tgcaccagca attcctgagc aagcagcaca gtgagtaggt      60

gattcctgga aacctgagtt caggtgaaaa tgagagagag cagtctcctt gtccctgcct     120


<210>  870
<211>  120
<212>  DNA
<213>  Artificial Sequence

<220>
<223>  MAP2K1

<400>  870
tctcttcccc aatctacctg tgtcagttcc ctccttttct attttctctt ccctgcagat      60

gtcaagccct ccaacatcct agtcaactcc cgtggggaga tcaagctctg tgactttggg     120


<210>  871
<211>  120
<212>  DNA
<213>  Artificial Sequence

<220>
<223>  MAP2K1

<400>  871
gtcaagccct ccaacatcct agtcaactcc cgtggggaga tcaagctctg tgactttggg      60

gtcagcgggc agctcatcga ctccatggcc aactccttcg tgggcacaag gtcctacatg     120


<210>  872
<211>  120
<212>  DNA
<213>  Artificial Sequence

<220>
<223>  MAP2K1

<400>  872
gtcagcgggc agctcatcga ctccatggcc aactccttcg tgggcacaag gtcctacatg      60

tcggtatgaa cagaagtttc cattgcttga gcttcttgta cggtcaggga gaggagccca     120

<210>  873
<211>  120
<212>  DNA
<213>  Artificial Sequence

<220>
<223>  MAP2K1

<400>  873
ctgtctgtct ctcctgcagc cagaaagact ccaggggact cattactctg tgcagtcaga      60

catctggagc atgggactgt ctctggtaga gatggcggtt gggaggtatc ccatccctcc      120


<210>  874
<211>  120
<212>  DNA
<213>  Artificial Sequence

<220>
<223>  MAP2K1

<400>  874
catctggagc atgggactgt ctctggtaga gatggcggtt gggaggtatc ccatccctcc      60

tccagatgcc aaggagctgg agctgatgtt tgggtgccag gtggaaggag atgcggctga      120


<210>  875
<211>  120
<212>  DNA
<213>  Artificial Sequence

<220>
<223>  MAP2K1

<400>  875
tccagatgcc aaggagctgg agctgatgtt tgggtgccag gtggaaggag atgcggctga      60

gaccccaccc aggccaagga cccccgggag gccccttagc tgtgagtagc ctggtgtgtc      120


<210>  876
<211>  120
<212>  DNA
<213>  Artificial Sequence

<220>
<223>  MAP2K1

<400>  876
tcatattaac aagtaatctg tttctgagaa gtattttttc tttttataaa atttgtagca      60

tacggaatgg acagccgacc tcccatggca atttttgagt tgttggatta catagtcaac      120


<210>  877
<211>  120
<212>  DNA
<213>  Artificial Sequence

<220>
<223>  MAP2K1

<400>  877
tacggaatgg acagccgacc tcccatggca atttttgagt tgttggatta catagtcaac        60

gaggtaagta ctgcctggtt tccttcacct tggaatttac ttgctcatct taagaaaaca       120


<210>  878
<211>  120
<212>  DNA
<213>  Artificial Sequence

<220>
<223>  MAP2K1

<400>  878
agaggagatg gctggagcaa ggagccaggc atttttctta tctcaacatg tgtttgcagc        60

ctcctccaaa actgcccagt ggagtgttca gtctggaatt tcaagatttt gtgaataaat       120


<210>  879
<211>  120
<212>  DNA
<213>  Artificial Sequence

<220>
<223>  MAP2K1

<400>  879
ctcctccaaa actgcccagt ggagtgttca gtctggaatt tcaagatttt gtgaataaat        60

ggtaagttgg ctccttgttc tctggaagcg tatactctgg atttgtcagg ctccccaccc       120


<210>  880
<211>  120
<212>  DNA
<213>  Artificial Sequence

<220>
<223>  MAP2K1

<400>  880
caaacatgtt atttgagcta gaaccagtgc caggcaacag ctcttacctt gtctttcttc        60

ctttaagctt aataaaaaac cccgcagaga gagcagattt gaagcaactc atggtgagtc       120


<210>  881
<211>  120
<212>  DNA
<213>  Artificial Sequence

<220>
<223>  MAP2K1

<400>  881
ctttaagctt aataaaaaac cccgcagaga gagcagattt gaagcaactc atggtgagtc        60

tatttattcc ggattcttac agtacctgtt tattcatttg ttcttctctg tcagtcatct       120

<210>  882
<211>  120
<212>  DNA
<213>  Artificial Sequence

<220>
<223>  MAP2K1

<400>  882
cctggtgggt tttgtttttt tgtttctttt taacaccacg tcctctcgtt tccttacatg      60

caggttcatg cttttatcaa gagatctgat gctgaggaag tggattttgc aggttggctc      120


<210>  883
<211>  120
<212>  DNA
<213>  Artificial Sequence

<220>
<223>  MAP2K1

<400>  883
caggttcatg cttttatcaa gagatctgat gctgaggaag tggattttgc aggttggctc      60

tgctccacca tcggccttaa ccagcccagc acaccaaccc atgctgctgg cgtctaagtg      120


<210>  884
<211>  120
<212>  DNA
<213>  Artificial Sequence

<220>
<223>  MAP2K1

<400>  884
tgctccacca tcggccttaa ccagcccagc acaccaaccc atgctgctgg cgtctaagtg      60

tttgggaagc aacaaagagc gagtcccctg cccggtggtt tgccatgtcg cttttgggcc      120


<210>  885
<211>  120
<212>  DNA
<213>  Artificial Sequence

<220>
<223>  MAP2K1-E2

<400>  885
ggttgacttc tctggtgaca gtattgactt gtgctcccca ctttggaaca ggaccaactt      60

ggaggccttg cagaagaagc tggaggagct agagcttgat gagcagcagc gaaagcgcct      120


<210>  886
<211>  120
<212>  DNA
<213>  Artificial Sequence

<220>
<223>  MAP2K1-E2

<400>  886
tgacttgtgc tccccacttt ggaacaggac caacttggag gccttgcaga agaagctgga      60

ggagctagag cttgatgagc agcagcgaaa gcgccttgag gcctttctta cccagaagca     120


<210>  887
<211>  120
<212>  DNA
<213>  Artificial Sequence

<220>
<223>  MAP2K1-E2

<400>  887
caggaccaac ttggaggcct tgcagaagaa gctggaggag ctagagcttg atgagcagca      60

gcgaaagcgc cttgaggcct ttcttaccca gaagcagaag gtgggagaac tgaaggatga     120


<210>  888
<211>  120
<212>  DNA
<213>  Artificial Sequence

<220>
<223>  MAP2K1-E2

<400>  888
tgatgagcag cagcgaaagc gccttgaggc ctttcttacc cagaagcaga aggtgggaga      60

actgaaggat gacgactttg agaagatcag tgagctgggg gctggcaatg gcggtgtggt     120


<210>  889
<211>  120
<212>  DNA
<213>  Artificial Sequence

<220>
<223>  MAP2K1-E2

<400>  889
tgaggccttt cttacccaga agcagaaggt gggagaactg aaggatgacg actttgagaa      60

gatcagtgag ctgggggctg gcaatggcgg tgtggtgttc aaggtctccc acaagccttc     120


<210>  890
<211>  120
<212>  DNA
<213>  Artificial Sequence

<220>
<223>  MAP2K1-E2

<400>  890
gaaggtggga gaactgaagg atgacgactt tgagaagatc agtgagctgg gggctggcaa      60

tggcggtgtg gtgttcaagg tctcccacaa gccttctggc ctggtcatgg ccagaaaggt     120

<210>  891
<211>  120
<212>  DNA
<213>  Artificial Sequence

<220>
<223>  MAP2K1-E2

<400>  891
cgactttgag aagatcagtg agctgggggc tggcaatggc ggtgtggtgt tcaaggtctc      60

ccacaagcct tctggcctgg tcatggccag aaaggtgagt ttgccttgat taacaggtaa      120


<210>  892
<211>  120
<212>  DNA
<213>  Artificial Sequence

<220>
<223>  MAP2K1-E2

<400>  892
gggggctggc aatggcggtg tggtgttcaa ggtctcccac aagccttctg gcctggtcat      60

ggccagaaag gtgagtttgc cttgattaac aggtaattgg attatttctc agggtactta      120


<210>  893
<211>  120
<212>  DNA
<213>  Artificial Sequence

<220>
<223>  MAP2K1-E2

<400>  893
ggttgacttc tctggtgaca gtattgactt gtgctcccca ctttggaaca ggaccaactt      60

ggaggccttg cagaagaagc tggaggagct agagcttgat gagcagcagc gaaagcgcct      120


<210>  894
<211>  120
<212>  DNA
<213>  Artificial Sequence

<220>
<223>  MAP2K1-E2

<400>  894
tgacttgtgc tccccacttt ggaacaggac caacttggag gccttgcaga agaagctgga      60

ggagctagag cttgatgagc agcagcgaaa gcgccttgag gcctttctta cccagaagca      120


<210>  895
<211>  120
<212>  DNA
<213>  Artificial Sequence

<220>
<223>  MAP2K1-E2

<400>  895
caggaccaac ttggaggcct tgcagaagaa gctggaggag ctagagcttg atgagcagca      60

gcgaaagcgc cttgaggcct ttcttaccca gaagcagaag gtgggagaac tgaaggatga     120


<210>  896
<211>  120
<212>  DNA
<213>  Artificial Sequence

<220>
<223>  MAP2K1-E2

<400>  896
gaagaagctg gaggagctag agcttgatga gcagcagcga aagcgccttg aggcctttct      60

tacccagaag cagaaggtgg gagaactgaa ggatgacgac tttgagaaga tcagtgagct     120


<210>  897
<211>  120
<212>  DNA
<213>  Artificial Sequence

<220>
<223>  MAP2K1-E2

<400>  897
tgatgagcag cagcgaaagc gccttgaggc ctttcttacc cagaagcaga aggtgggaga      60

actgaaggat gacgactttg agaagatcag tgagctgggg gctggcaatg gcggtgtggt     120


<210>  898
<211>  120
<212>  DNA
<213>  Artificial Sequence

<220>
<223>  MAP2K1-E2

<400>  898
tgaggccttt cttacccaga agcagaaggt gggagaactg aaggatgacg actttgagaa      60

gatcagtgag ctgggggctg gcaatggcgg tgtggtgttc aaggtctccc acaagccttc     120


<210>  899
<211>  120
<212>  DNA
<213>  Artificial Sequence

<220>
<223>  MAP2K1-E2

<400>  899
gaaggtggga gaactgaagg atgacgactt tgagaagatc agtgagctgg gggctggcaa      60

tggcggtgtg gtgttcaagg tctcccacaa gccttctggc ctggtcatgg ccagaaaggt     120

<210>  900
<211>  120
<212>  DNA
<213>  Artificial Sequence

<220>
<223>  MAP2K1-E2

<400>  900
cgactttgag aagatcagtg agctgggggc tggcaatggc ggtgtggtgt tcaaggtctc      60

ccacaagcct tctggcctgg tcatggccag aaaggtgagt ttgccttgat taacaggtaa      120


<210>  901
<211>  120
<212>  DNA
<213>  Artificial Sequence

<220>
<223>  MAP2K1-E2

<400>  901
gggggctggc aatggcggtg tggtgttcaa ggtctcccac aagccttctg gcctggtcat      60

ggccagaaag gtgagtttgc cttgattaac aggtaattgg attatttctc agggtactta      120


<210>  902
<211>  120
<212>  DNA
<213>  Artificial Sequence

<220>
<223>  MET

<400>  902
tcgccttgaa cctgttttgg cagataaacc tctcataatg aaggcccccg ctgtgcttgc      60

acctggcatc ctcgtgctcc tgtttacctt ggtgcagagg agcaatgggg agtgtaaaga      120


<210>  903
<211>  120
<212>  DNA
<213>  Artificial Sequence

<220>
<223>  MET

<400>  903
acctggcatc ctcgtgctcc tgtttacctt ggtgcagagg agcaatgggg agtgtaaaga      60

ggcactagca aagtccgaga tgaatgtgaa tatgaagtat cagcttccca acttcaccgc      120


<210>  904
<211>  120
<212>  DNA
<213>  Artificial Sequence

<220>
<223>  MET

<400>  904
ggcactagca aagtccgaga tgaatgtgaa tatgaagtat cagcttccca acttcaccgc      60

ggaaacaccc atccagaatg tcattctaca tgagcatcac attttccttg gtgccactaa     120


<210>  905
<211>  120
<212>  DNA
<213>  Artificial Sequence

<220>
<223>  MET

<400>  905
ggaaacaccc atccagaatg tcattctaca tgagcatcac attttccttg gtgccactaa      60

ctacatttat gttttaaatg aggaagacct tcagaaggtt gctgagtaca agactgggcc     120


<210>  906
<211>  120
<212>  DNA
<213>  Artificial Sequence

<220>
<223>  MET

<400>  906
ctacatttat gttttaaatg aggaagacct tcagaaggtt gctgagtaca agactgggcc      60

tgtgctggaa cacccagatt gtttcccatg tcaggactgc agcagcaaag ccaatttatc     120


<210>  907
<211>  120
<212>  DNA
<213>  Artificial Sequence

<220>
<223>  MET

<400>  907
tgtgctggaa cacccagatt gtttcccatg tcaggactgc agcagcaaag ccaatttatc      60

aggaggtgtt tggaaagata acatcaacat ggctctagtt gtcgacacct actatgatga     120


<210>  908
<211>  120
<212>  DNA
<213>  Artificial Sequence

<220>
<223>  MET

<400>  908
aggaggtgtt tggaaagata acatcaacat ggctctagtt gtcgacacct actatgatga      60

tcaactcatt agctgtggca gcgtcaacag agggacctgc cagcgacatg tctttcccca     120

<210>  909
<211>  120
<212>  DNA
<213>  Artificial Sequence

<220>
<223>  MET

<400>  909
tcaactcatt agctgtggca gcgtcaacag agggacctgc cagcgacatg tctttcccca      60

caatcatact gctgacatac agtcggaggt tcactgcata ttctccccac agatagaaga      120


<210>  910
<211>  120
<212>  DNA
<213>  Artificial Sequence

<220>
<223>  MET

<400>  910
caatcatact gctgacatac agtcggaggt tcactgcata ttctccccac agatagaaga      60

gcccagccag tgtcctgact gtgtggtgag cgccctggga gccaaagtcc tttcatctgt      120


<210>  911
<211>  120
<212>  DNA
<213>  Artificial Sequence

<220>
<223>  MET

<400>  911
gcccagccag tgtcctgact gtgtggtgag cgccctggga gccaaagtcc tttcatctgt      60

aaaggaccgg ttcatcaact tctttgtagg caataccata aattcttctt atttcccaga      120


<210>  912
<211>  120
<212>  DNA
<213>  Artificial Sequence

<220>
<223>  MET

<400>  912
aaaggaccgg ttcatcaact tctttgtagg caataccata aattcttctt atttcccaga      60

tcatccattg cattcgatat cagtgagaag gctaaaggaa acgaaagatg gttttatgtt      120


<210>  913
<211>  120
<212>  DNA
<213>  Artificial Sequence

<220>
<223>  MET

<400>  913
tcatccattg cattcgatat cagtgagaag gctaaaggaa acgaaagatg gttttatgtt      60

tttgacggac cagtcctaca ttgatgtttt acctgagttc agagattctt accccattaa     120


<210>  914
<211>  120
<212>  DNA
<213>  Artificial Sequence

<220>
<223>  MET

<400>  914
tttgacggac cagtcctaca ttgatgtttt acctgagttc agagattctt accccattaa      60

gtatgtccat gcctttgaaa gcaacaattt tatttacttc ttgacggtcc aaagggaaac     120


<210>  915
<211>  120
<212>  DNA
<213>  Artificial Sequence

<220>
<223>  MET

<400>  915
gtatgtccat gcctttgaaa gcaacaattt tatttacttc ttgacggtcc aaagggaaac      60

tctagatgct cagacttttc acacaagaat aatcaggttc tgttccataa actctggatt     120


<210>  916
<211>  120
<212>  DNA
<213>  Artificial Sequence

<220>
<223>  MET

<400>  916
tctagatgct cagacttttc acacaagaat aatcaggttc tgttccataa actctggatt      60

gcattcctac atggaaatgc ctctggagtg tattctcaca gaaaagagaa aaaagagatc     120


<210>  917
<211>  120
<212>  DNA
<213>  Artificial Sequence

<220>
<223>  MET

<400>  917
gcattcctac atggaaatgc ctctggagtg tattctcaca gaaaagagaa aaaagagatc      60

cacaaagaag gaagtgttta atatacttca ggctgcgtat gtcagcaagc ctgggcccca     120

<210>  918
<211>  120
<212>  DNA
<213>  Artificial Sequence

<220>
<223>  MET

<400>  918
cacaaagaag gaagtgttta atatacttca ggctgcgtat gtcagcaagc ctggggccca      60

gcttgctaga caaataggag ccagcctgaa tgatgacatt cttttcgggg tgttcgcaca      120


<210>  919
<211>  120
<212>  DNA
<213>  Artificial Sequence

<220>
<223>  MET

<400>  919
gcttgctaga caaataggag ccagcctgaa tgatgacatt cttttcgggg tgttcgcaca      60

aagcaagcca gattctgccg aaccaatgga tcgatctgcc atgtgtgcat tccctatcaa      120


<210>  920
<211>  120
<212>  DNA
<213>  Artificial Sequence

<220>
<223>  MET

<400>  920
aagcaagcca gattctgccg aaccaatgga tcgatctgcc atgtgtgcat tccctatcaa      60

atatgtcaac gacttcttca acaagatcgt caacaaaaac aatgtgagat gtctccagca      120


<210>  921
<211>  120
<212>  DNA
<213>  Artificial Sequence

<220>
<223>  MET

<400>  921
atatgtcaac gacttcttca acaagatcgt caacaaaaac aatgtgagat gtctccagca      60

tttttacgga cccaatcatg agcactgctt taatagggta agtcacatca gttccccact      120


<210>  922
<211>  120
<212>  DNA
<213>  Artificial Sequence

<220>
<223>  MET

<400>  922
ctctatgacc atattttatt ccagacactt ctgagaaatt catcaggctg tgaagcgcgc      60

cgtgatgaat atcgaacaga gtttaccaca gctttgcagc gcgttgactt attcatgggt     120


<210>  923
<211>  120
<212>  DNA
<213>  Artificial Sequence

<220>
<223>  MET

<400>  923
cgtgatgaat atcgaacaga gtttaccaca gctttgcagc gcgttgactt attcatgggt      60

caattcagcg aagtcctctt aacatctata tccaccttca ttaaaggaga cctcaccata     120


<210>  924
<211>  120
<212>  DNA
<213>  Artificial Sequence

<220>
<223>  MET

<400>  924
caattcagcg aagtcctctt aacatctata tccaccttca ttaaaggaga cctcaccata      60

gctaatcttg ggacatcaga gggtcgcttc atgcaggtaa gtgctttctg agagtagctg     120


<210>  925
<211>  120
<212>  DNA
<213>  Artificial Sequence

<220>
<223>  MET

<400>  925
tatttaaact gagcttgttg gaataaggat gttataactt ttttgctgtt taggttgtgg      60

tttctcgatc aggaccatca acccctcatg tgaattttct cctggactcc catccagtgt     120


<210>  926
<211>  120
<212>  DNA
<213>  Artificial Sequence

<220>
<223>  MET

<400>  926
tttctcgatc aggaccatca acccctcatg tgaattttct cctggactcc catccagtgt      60

ctccagaagt gattgtggag catacattaa accaaaatgg ctacacactg gttatcactg     120

<210>  927
<211>  120
<212>  DNA
<213>  Artificial Sequence

<220>
<223>  MET

<400>  927
ctccagaagt gattgtggag catacattaa accaaaatgg ctacacactg gttatcactg      60

ggaagaaggt aagctgttcc cacagggaat ttccatagac gtggtttttc ccaaatgcat      120


<210>  928
<211>  120
<212>  DNA
<213>  Artificial Sequence

<220>
<223>  MET

<400>  928
tctggaagct ctttccaccc cttctcttca cagatcacga agatcccatt gaatggcttg      60

ggctgcagac atttccagtc ctgcagtcaa tgcctctctg ccccaccctt tgttcagtgt      120


<210>  929
<211>  120
<212>  DNA
<213>  Artificial Sequence

<220>
<223>  MET

<400>  929
ggctgcagac atttccagtc ctgcagtcaa tgcctctctg ccccaccctt tgttcagtgt      60

ggctggtgcc acgacaaatg tgtgcgatcg gaggaatgcc tgagcgggac atggactcaa      120


<210>  930
<211>  120
<212>  DNA
<213>  Artificial Sequence

<220>
<223>  MET

<400>  930
ggctggtgcc acgacaaatg tgtgcgatcg gaggaatgcc tgagcgggac atggactcaa      60

cagatctgtc tgcctgcaat ctacaaggta ggaatctcta acagctggca tacatgtttt      120


<210>  931
<211>  120
<212>  DNA
<213>  Artificial Sequence

<220>
<223>  MET

<400>  931
tatattgggt ttttttaaaa gttctatgtt gtccttgtag gttttcccaa atagtgcacc        60

ccttgaagga gggacaaggc tgaccatatg tggctgggac tttggatttc ggaggaataa       120


<210>  932
<211>  120
<212>  DNA
<213>  Artificial Sequence

<220>
<223>  MET

<400>  932
ccttgaagga gggacaaggc tgaccatatg tggctgggac tttggatttc ggaggaataa        60

taaatttgat ttaaagaaaa ctagagttct ccttggaaat gagagctgca ccttgacttt       120


<210>  933
<211>  120
<212>  DNA
<213>  Artificial Sequence

<220>
<223>  MET

<400>  933
taaatttgat ttaaagaaaa ctagagttct ccttggaaat gagagctgca ccttgacttt        60

aagtgagagc acgatgaata cgtaaggatc ttaaaatgct ttgctggggt gtgcttggaa       120


<210>  934
<211>  120
<212>  DNA
<213>  Artificial Sequence

<220>
<223>  MET

<400>  934
cctataaaac aacctaacca gaaaattcct tggatttgtc atgtattaaa ctttgggttt        60

tttttccaga ttgaaatgca cagttggtcc tgccatgaat aagcatttca atatgtccat       120


<210>  935
<211>  120
<212>  DNA
<213>  Artificial Sequence

<220>
<223>  MET

<400>  935
tttttccaga ttgaaatgca cagttggtcc tgccatgaat aagcatttca atatgtccat        60

aattatttca aatggccacg ggacaacaca atacagtaca ttctcctatg tggtaaggaa       120

<210>  936
<211>  120
<212>  DNA
<213>  Artificial Sequence

<220>
<223>  MET

<400>  936
aattatttca aatggccacg ggacaacaca atacagtaca ttctcctatg tggtaaggaa      60

gattctatcc tatcatgttt gatttttact taatctattt aaattataag atgaacaagt      120


<210>  937
<211>  120
<212>  DNA
<213>  Artificial Sequence

<220>
<223>  MET

<400>  937
ttaaattata agatgaacaa gttactttgt tttgttttta tctcccctcc aggatcctgt      60

aataacaagt atttcgccga aatacggtcc tatggctggt ggcactttac ttactttaac      120


<210>  938
<211>  120
<212>  DNA
<213>  Artificial Sequence

<220>
<223>  MET

<400>  938
aataacaagt atttcgccga aatacggtcc tatggctggt ggcactttac ttactttaac      60

tggaaattac ctaaacagtg ggaattctag acacatttca attggtggaa aaacatgtac      120


<210>  939
<211>  120
<212>  DNA
<213>  Artificial Sequence

<220>
<223>  MET

<400>  939
tggaaattac ctaaacagtg ggaattctag acacatttca attggtggaa aaacatgtac      60

tttaaaaagg tgttgtaaat ttattttttg ttgcatctgt caatttgaat taatatctgt      120


<210>  940
<211>  120
<212>  DNA
<213>  Artificial Sequence

<220>
<223>  MET

<400>  940
taatagctaa aattcacttc cttaattttt tttgttcagt gtgtcaaaca gtattcttga        60

atgttatacc ccagcccaaa ccatttcaac tgagtttgct gttaaattga aaattgactt       120


<210>  941
<211>  120
<212>  DNA
<213>  Artificial Sequence

<220>
<223>  MET

<400>  941
atgttatacc ccagcccaaa ccatttcaac tgagtttgct gttaaattga aaattgactt        60

agccaaccga gagacaagca tcttcagtta ccgtgaagat cccattgtct atgaaattca       120


<210>  942
<211>  120
<212>  DNA
<213>  Artificial Sequence

<220>
<223>  MET

<400>  942
agccaaccga gagacaagca tcttcagtta ccgtgaagat cccattgtct atgaaattca        60

tccaaccaaa tcttttatta ggtaagtaga agcttctgat gggtataaga aaacaatgaa       120


<210>  943
<211>  120
<212>  DNA
<213>  Artificial Sequence

<220>
<223>  MET

<400>  943
ctgtaatcag tgcaggtgat taaattgaat ccctctctta cagtacttgg tggaaagaac        60

ctctcaacat tgtcagtttt ctattttgct ttgccagtgg tgggagcaca ataacaggtg       120


<210>  944
<211>  120
<212>  DNA
<213>  Artificial Sequence

<220>
<223>  MET

<400>  944
ctctcaacat tgtcagtttt ctattttgct ttgccagtgg tgggagcaca ataacaggtg        60

ttgggaaaaa cctgaattca gttagtgtcc cgagaatggt cataaatgtg catgaagcag       120

<210>  945
<211>  120
<212>  DNA
<213>  Artificial Sequence

<220>
<223>  MET

<400>  945
ttgggaaaaa cctgaattca gttagtgtcc cgagaatggt cataaatgtg catgaagcag      60

gaaggaactt tacagtggta agtcctttga gcaatggttc tactcagagc tctgcatctt      120


<210>  946
<211>  120
<212>  DNA
<213>  Artificial Sequence

<220>
<223>  MET

<400>  946
tctttctcta ggcatgtcaa catcgctcta attcagagat aatctgttgt accactcctt      60

ccctgcaaca gctgaatctg caactccccc tgaaaaccaa agcctttttc atgttagatg      120


<210>  947
<211>  120
<212>  DNA
<213>  Artificial Sequence

<220>
<223>  MET

<400>  947
ccctgcaaca gctgaatctg caactccccc tgaaaaccaa agcctttttc atgttagatg      60

ggatcctttc caaatacttt gatctcattt atgtacataa tcctgtgttt aagccttttg      120


<210>  948
<211>  120
<212>  DNA
<213>  Artificial Sequence

<220>
<223>  MET

<400>  948
ggatcctttc caaatacttt gatctcattt atgtacataa tcctgtgttt aagccttttg      60

aaaagccagt gatgatctca atgggcaatg aaaatgtact ggaaattaag gtaagaaatg      120


<210>  949
<211>  120
<212>  DNA
<213>  Artificial Sequence

<220>
<223>  MET

<400>  949
tgaagtgtta acaacctttt tttttttttt tcctttcagg gaaatgatat tgaccctgaa      60

gcagttaaag gtgaagtgtt aaaagttgga aataagagct gtgagaatat acacttacat     120


<210>  950
<211>  120
<212>  DNA
<213>  Artificial Sequence

<220>
<223>  MET

<400>  950
gcagttaaag gtgaagtgtt aaaagttgga aataagagct gtgagaatat acacttacat      60

tctgaagccg ttttatgcac ggtccccaat gacctgctga aattgaacag cgagctaaat     120


<210>  951
<211>  120
<212>  DNA
<213>  Artificial Sequence

<220>
<223>  MET

<400>  951
tctgaagccg ttttatgcac ggtccccaat gacctgctga aattgaacag cgagctaaat      60

atagaggtgg gattcctgca ttcctctcat gatgtaaata aggaagccag tgtaattatg     120


<210>  952
<211>  120
<212>  DNA
<213>  Artificial Sequence

<220>
<223>  MET

<400>  952
atagaggtgg gattcctgca ttcctctcat gatgtaaata aggaagccag tgtaattatg      60

ttattctcag gcttaaaata aatcattaaa gctcatttat gtgtgggttt tggctcatca     120


<210>  953
<211>  120
<212>  DNA
<213>  Artificial Sequence

<220>
<223>  MET

<400>  953
atcatggcta aatgctgact tttctttatt tgtcattttt agtggaagca agcaatttct      60

tcaaccgtcc ttggaaaagt aatagttcaa ccagatcaga atttcacagg attgattgct     120

<210>  954
<211>  120
<212>  DNA
<213>  Artificial Sequence

<220>
<223>  MET

<400>  954
tcaaccgtcc ttggaaaagt aatagttcaa ccagatcaga atttcacagg attgattgct        60

ggtgttgtct caatatcaac agcactgtta ttactacttg ggtttttcct gtggctgaaa       120


<210>  955
<211>  120
<212>  DNA
<213>  Artificial Sequence

<220>
<223>  MET

<400>  955
ggtgttgtct caatatcaac agcactgtta ttactacttg ggtttttcct gtggctgaaa        60

aagagaaagc aaattaaagg tgcatttttg ttactgttca tttttagaag ttaccttaag       120


<210>  956
<211>  120
<212>  DNA
<213>  Artificial Sequence

<220>
<223>  MET

<400>  956
ggcccatgat agccgtcttt aacaagctct ttctttctct ctgttttaag atctgggcag        60

tgaattagtt cgctacgatg caagagtaca cactcctcat ttggataggc ttgtaagtgc       120


<210>  957
<211>  120
<212>  DNA
<213>  Artificial Sequence

<220>
<223>  MET

<400>  957
tgaattagtt cgctacgatg caagagtaca cactcctcat ttggataggc ttgtaagtgc        60

ccgaagtgta agcccaacta cagaaatggt ttcaaatgaa tctgtagact accgagctac       120


<210>  958
<211>  120
<212>  DNA
<213>  Artificial Sequence

<220>
<223>  MET

<400>  958
ccgaagtgta agcccaacta cagaaatggt ttcaaatgaa tctgtagact accgagctac      60

ttttccagaa ggtatatttc agtttattgt tctgagaaat acctatacat atacctcagt     120


<210>  959
<211>  120
<212>  DNA
<213>  Artificial Sequence

<220>
<223>  MET

<400>  959
ctgttgttct ttaataattt tccttcatct tacagatcag tttcctaatt catctcagaa      60

cggttcatgc cgacaagtgc agtatcctct gacagacatg tcccccatcc taactagtgg     120


<210>  960
<211>  120
<212>  DNA
<213>  Artificial Sequence

<220>
<223>  MET

<400>  960
cggttcatgc cgacaagtgc agtatcctct gacagacatg tcccccatcc taactagtgg      60

ggactctgat atatccagtc cattactgca aaatactgtc cacattgacc tcagtgctct     120


<210>  961
<211>  120
<212>  DNA
<213>  Artificial Sequence

<220>
<223>  MET

<400>  961
ggactctgat atatccagtc cattactgca aaatactgtc cacattgacc tcagtgctct      60

aaatccagag ctggtccagg cagtgcagca tgtagtgatt gggcccagta gcctgattgt     120


<210>  962
<211>  120
<212>  DNA
<213>  Artificial Sequence

<220>
<223>  MET

<400>  962
aaatccagag ctggtccagg cagtgcagca tgtagtgatt gggcccagta gcctgattgt      60

gcatttcaat gaagtcatag gaagaggtaa gtatttccac tcagcttttt gttaaatacg     120

<210>  963
<211>  120
<212>  DNA
<213>  Artificial Sequence

<220>
<223>  MET

<400>  963
ttcataatta aatgttacgc agtgctaacc aagttctttc ttttgcacag ggcattttgg      60

ttgtgtatat catgggactt tgttggacaa tgatggcaag aaaattcact gtgctgtgaa      120


<210>  964
<211>  120
<212>  DNA
<213>  Artificial Sequence

<220>
<223>  MET

<400>  964
ttgtgtatat catgggactt tgttggacaa tgatggcaag aaaattcact gtgctgtgaa      60

atccttgaac agtaagtggc attttattta accatggagt atacttttgt ggtttgcaac      120


<210>  965
<211>  120
<212>  DNA
<213>  Artificial Sequence

<220>
<223>  MET

<400>  965
gttaatgtct ccaccactgg atttctcagg aatcactgac ataggagaag tttcccaatt      60

tctgaccgag ggaatcatca tgaaagattt tagtcatccc aatgtcctct cgctcctggg      120


<210>  966
<211>  120
<212>  DNA
<213>  Artificial Sequence

<220>
<223>  MET

<400>  966
tctgaccgag ggaatcatca tgaaagattt tagtcatccc aatgtcctct cgctcctggg      60

aatctgcctg cgaagtgaag ggtctccgct ggtggtccta ccatacatga aacatggaga      120


<210>  967
<211>  120
<212>  DNA
<213>  Artificial Sequence

<220>
<223>  MET

<400>  967
aatctgcctg cgaagtgaag ggtctccgct ggtggtccta ccatacatga aacatggaga      60

tcttcgaaat ttcattcgaa atgagactca tgtaagttga ctgccaagct tactaactgg     120


<210>  968
<211>  120
<212>  DNA
<213>  Artificial Sequence

<220>
<223>  MET

<400>  968
ttctaaggtc aaaattagaa cagtagatgc ttagtttatg cttttctaac tctctttgac      60

tgcagaatcc aactgtaaaa gatcttattg gctttggtct tcaagtagcc aaaggcatga     120


<210>  969
<211>  120
<212>  DNA
<213>  Artificial Sequence

<220>
<223>  MET

<400>  969
tgcagaatcc aactgtaaaa gatcttattg gctttggtct tcaagtagcc aaaggcatga      60

aatatcttgc aagcaaaaag tttgtccaca gagacttggc tgcaagaaac tgtatgtaag     120


<210>  970
<211>  120
<212>  DNA
<213>  Artificial Sequence

<220>
<223>  MET

<400>  970
aatatcttgc aagcaaaaag tttgtccaca gagacttggc tgcaagaaac tgtatgtaag      60

tatcagaatc tctgtgccac aatccaaatt aagtgacaag gaggaatctg tttcccactg     120


<210>  971
<211>  120
<212>  DNA
<213>  Artificial Sequence

<220>
<223>  MET

<400>  971
tttcagccac gggtaataat ttttgtcctt tctgtaggct ggatgaaaaa ttcacagtca      60

aggttgctga ttttggtctt gccagagaca tgtatgataa agaatactat agtgtacaca     120

<210>  972
<211>  120
<212>  DNA
<213>  Artificial Sequence

<220>
<223>  MET

<400>  972
aggttgctga ttttggtctt gccagagaca tgtatgataa agaatactat agtgtacaca      60

acaaaacagg tgcaaagctg ccagtgaagt ggatggcttt ggaaagtctg caaactcaaa      120


<210>  973
<211>  120
<212>  DNA
<213>  Artificial Sequence

<220>
<223>  MET

<400>  973
acaaaacagg tgcaaagctg ccagtgaagt ggatggcttt ggaaagtctg caaactcaaa      60

agtttaccac caagtcagat gtggtaatgt attggttatc tctgagtttc tcctctttta      120


<210>  974
<211>  120
<212>  DNA
<213>  Artificial Sequence

<220>
<223>  MET

<400>  974
atatgtatgg tcacatctct cacctcatct gtcctgtttc ttgttttact agtggtcctt      60

tggcgtgctc ctctgggagc tgatgacaag aggagcccca ccttatcctg acgtaaacac      120


<210>  975
<211>  120
<212>  DNA
<213>  Artificial Sequence

<220>
<223>  MET

<400>  975
tggcgtgctc ctctgggagc tgatgacaag aggagcccca ccttatcctg acgtaaacac      60

ctttgatata actgtttact tgttgcaagg gagaagactc ctacaacccg aatactgccc      120


<210>  976
<211>  120
<212>  DNA
<213>  Artificial Sequence

<220>
<223>  MET

<400>  976
ctttgatata actgtttact tgttgcaagg gagaagactc ctacaacccg aatactgccc      60

agaccccttg taagtagtct ttctgtacct cttacgttct ttacttttac agaaatgcct     120


<210>  977
<211>  120
<212>  DNA
<213>  Artificial Sequence

<220>
<223>  MET

<400>  977
ctcttacagc atgtctttct ttttggaaca gatatgaagt aatgctaaaa tgctggcacc      60

ctaaagccga aatgcgccca tccttttctg aactggtgtc ccggatatca gcgatcttct     120


<210>  978
<211>  120
<212>  DNA
<213>  Artificial Sequence

<220>
<223>  MET

<400>  978
ctaaagccga aatgcgccca tccttttctg aactggtgtc ccggatatca gcgatcttct      60

ctactttcat tggggagcac tatgtccatg tgaacgctac ttatgtgaac gtaaaatgtg     120


<210>  979
<211>  120
<212>  DNA
<213>  Artificial Sequence

<220>
<223>  MET

<400>  979
ctactttcat tggggagcac tatgtccatg tgaacgctac ttatgtgaac gtaaaatgtg      60

tcgctccgta tccttctctg ttgtcatcag aagataacgc tgatgatgag gtggacacac     120


<210>  980
<211>  120
<212>  DNA
<213>  Artificial Sequence

<220>
<223>  MET

<400>  980
tcgctccgta tccttctctg ttgtcatcag aagataacgc tgatgatgag gtggacacac      60

gaccagcctc cttctgggag acatcatagt gctagtacta tgtcaaagca acagtccaca     120

<210>  981
<211>  120
<212>  DNA
<213>  Artificial Sequence

<220>
<223>  MET-E14

<400>  981
cccatgagtt ctgggcactg ggtcaaagtc tcctggggcc catgatagcc gtctttaaca      60

agctctttct ttctctctgt tttaagatct gggcagtgaa ttagttcgct acgatgcaag      120


<210>  982
<211>  120
<212>  DNA
<213>  Artificial Sequence

<220>
<223>  MET-E14

<400>  982
aaagtctcct ggggcccatg atagccgtct ttaacaagct ctttctttct ctctgtttta      60

agatctgggc agtgaattag ttcgctacga tgcaagagta cacactcctc atttggatag      120


<210>  983
<211>  120
<212>  DNA
<213>  Artificial Sequence

<220>
<223>  MET-E14

<400>  983
ccgtctttaa caagctcttt ctttctctct gttttaagat ctgggcagtg aattagttcg      60

ctacgatgca agagtacaca ctcctcattt ggataggctt gtaagtgccc gaagtgtaag      120


<210>  984
<211>  120
<212>  DNA
<213>  Artificial Sequence

<220>
<223>  MET-E14

<400>  984
ctctctgttt taagatctgg gcagtgaatt agttcgctac gatgcaagag tacacactcc      60

tcatttggat aggcttgtaa gtgcccgaag tgtaagccca actacagaaa tggtttcaaa      120


<210>  985
<211>  120
<212>  DNA
<213>  Artificial Sequence

<220>
<223>  MET-E14

<400>  985
tgaattagtt cgctacgatg caagagtaca cactcctcat ttggataggc ttgtaagtgc      60

ccgaagtgta agcccaacta cagaaatggt ttcaaatgaa tctgtagact accgagctac     120


<210>  986
<211>  120
<212>  DNA
<213>  Artificial Sequence

<220>
<223>  MET-E14

<400>  986
agtacacact cctcatttgg ataggcttgt aagtgcccga agtgtaagcc caactacaga      60

aatggtttca aatgaatctg tagactaccg agctactttt ccagaaggta tatttcagtt     120


<210>  987
<211>  120
<212>  DNA
<213>  Artificial Sequence

<220>
<223>  MET-E14

<400>  987
gcttgtaagt gcccgaagtg taagcccaac tacagaaatg gtttcaaatg aatctgtaga      60

ctaccgagct acttttccag aaggtatatt tcagtttatt gttctgagaa atacctatac     120


<210>  988
<211>  120
<212>  DNA
<213>  Artificial Sequence

<220>
<223>  MET-E14

<400>  988
cccaactaca gaaatggttt caaatgaatc tgtagactac cgagctactt ttccagaagg      60

tatatttcag tttattgttc tgagaaatac ctatacatat acctcagtgg gttgtgacat     120


<210>  989
<211>  120
<212>  DNA
<213>  Artificial Sequence

<220>
<223>  MET-E14

<400>  989
tgaatctgta gactaccgag ctacttttcc agaaggtata tttcagttta ttgttctgag      60

aaatacctat acatatacct cagtgggttg tgacattgtt gtttattttt ggttttgcat     120

<210>  990
<211>  120
<212>  DNA
<213>  Artificial Sequence

<220>
<223>  MET-E15

<400>  990
ccattaaatg aggttttact gttgttcttt aataattttc cttcatctta cagatcagtt      60

tcctaattca tctcagaacg gttcatgccg acaagtgcag tatcctctga cagacatgtc      120


<210>  991
<211>  120
<212>  DNA
<213>  Artificial Sequence

<220>
<223>  MET-E15

<400>  991
ttctttaata attttccttc atcttacaga tcagtttcct aattcatctc agaacggttc      60

atgccgacaa gtgcagtatc ctctgacaga catgtccccc atcctaacta gtggggactc      120


<210>  992
<211>  120
<212>  DNA
<213>  Artificial Sequence

<220>
<223>  MET-E15

<400>  992
tacagatcag tttcctaatt catctcagaa cggttcatgc cgacaagtgc agtatcctct      60

gacagacatg tcccccatcc taactagtgg ggactctgat atatccagtc cattactgca      120


<210>  993
<211>  120
<212>  DNA
<213>  Artificial Sequence

<220>
<223>  MET-E15

<400>  993
tcagaacggt tcatgccgac aagtgcagta tcctctgaca gacatgtccc ccatcctaac      60

tagtggggac tctgatatat ccagtccatt actgcaaaat actgtccaca ttgacctcag      120


<210>  994
<211>  120
<212>  DNA
<213>  Artificial Sequence

<220>
<223>  MET-E15

<400>  994
gcagtatcct ctgacagaca tgtcccccat cctaactagt ggggactctg atatatccag      60

tccattactg caaaatactg tccacattga cctcagtgct ctaaatccag agctggtcca     120


<210>  995
<211>  120
<212>  DNA
<213>  Artificial Sequence

<220>
<223>  MET-E15

<400>  995
ccccatccta actagtgggg actctgatat atccagtcca ttactgcaaa atactgtcca      60

cattgacctc agtgctctaa atccagagct ggtccaggca gtgcagcatg tagtgattgg     120


<210>  996
<211>  120
<212>  DNA
<213>  Artificial Sequence

<220>
<223>  MET-E15

<400>  996
tgatatatcc agtccattac tgcaaaatac tgtccacatt gacctcagtg ctctaaatcc      60

agagctggtc caggcagtgc agcatgtagt gattgggccc agtagcctga ttgtgcattt     120


<210>  997
<211>  120
<212>  DNA
<213>  Artificial Sequence

<220>
<223>  MET-E15

<400>  997
aaatactgtc cacattgacc tcagtgctct aaatccagag ctggtccagg cagtgcagca      60

tgtagtgatt gggcccagta gcctgattgt gcatttcaat gaagtcatag gaagaggtaa     120


<210>  998
<211>  120
<212>  DNA
<213>  Artificial Sequence

<220>
<223>  MET-E15

<400>  998
tgctctaaat ccagagctgg tccaggcagt gcagcatgta gtgattgggc ccagtagcct      60

gattgtgcat ttcaatgaag tcataggaag aggtaagtat ttccactcag ctttttgtta     120

<210>  999
<211>  120
<212>  DNA
<213>  Artificial Sequence

<220>
<223>  MET-E15

<400>  999
ggcagtgcag catgtagtga ttgggcccag tagcctgatt gtgcatttca atgaagtcat      60

aggaagaggt aagtatttcc actcagcttt ttgttaaata cgattttcca gtaagcattt      120


<210>  1000
<211>  120
<212>  DNA
<213>  Artificial Sequence

<220>
<223>  MET-E16

<400>  1000
atagaaagaa gaaagaataa aatgaagctc ataaagggtt tgataaataa ttatttcata      60

attaaatgtt acgcagtgct aaccaagttc tttcttttgc acagggcatt ttggttgtgt      120


<210>  1001
<211>  120
<212>  DNA
<213>  Artificial Sequence

<220>
<223>  MET-E16

<400>  1001
aagctcataa agggtttgat aaataattat ttcataatta aatgttacgc agtgctaacc      60

aagttctttc ttttgcacag ggcattttgg ttgtgtatat catgggactt tgttggacaa      120


<210>  1002
<211>  120
<212>  DNA
<213>  Artificial Sequence

<220>
<223>  MET-E16

<400>  1002
aattatttca taattaaatg ttacgcagtg ctaaccaagt tctttctttt gcacagggca      60

ttttggttgt gtatatcatg ggactttgtt ggacaatgat ggcaagaaaa ttcactgtgc      120


<210>  1003
<211>  120
<212>  DNA
<213>  Artificial Sequence

<220>
<223>  MET-E16

<400>  1003
gcagtgctaa ccaagttctt tcttttgcac agggcatttt ggttgtgtat atcatgggac      60

tttgttggac aatgatggca agaaaattca ctgtgctgtg aaatccttga acagtaagtg     120


<210>  1004
<211>  120
<212>  DNA
<213>  Artificial Sequence

<220>
<223>  MET-E16

<400>  1004
ttgcacaggg cattttggtt gtgtatatca tgggactttg ttggacaatg atggcaagaa      60

aattcactgt gctgtgaaat ccttgaacag taagtggcat tttatttaac catggagtat     120


<210>  1005
<211>  120
<212>  DNA
<213>  Artificial Sequence

<220>
<223>  MET-E16

<400>  1005
atatcatggg actttgttgg acaatgatgg caagaaaatt cactgtgctg tgaaatcctt      60

gaacagtaag tggcatttta tttaaccatg gagtatactt ttgtggtttg caacctaata     120


<210>  1006
<211>  120
<212>  DNA
<213>  Artificial Sequence

<220>
<223>  MET-E16

<400>  1006
tgatggcaag aaaattcact gtgctgtgaa atccttgaac agtaagtggc attttattta      60

accatggagt atacttttgt ggtttgcaac ctaataaata gcttataata aaacgttgat     120


<210>  1007
<211>  120
<212>  DNA
<213>  Artificial Sequence

<220>
<223>  MET-E16

<400>  1007
tgtgaaatcc ttgaacagta agtggcattt tatttaacca tggagtatac ttttgtggtt      60

tgcaacctaa taaatagctt ataataaaac gttgatttac actttcccct tgtggaaaaa     120

<210>  1008
<211>  120
<212>  DNA
<213>  Artificial Sequence

<220>
<223>  MET-E17

<400>  1008
aagtattcac tgttccataa tgaagttaat gtctccacca ctggatttct caggaatcac      60

tgacatagga gaagtttccc aatttctgac cgagggaatc atcatgaaag attttagtca      120


<210>  1009
<211>  120
<212>  DNA
<213>  Artificial Sequence

<220>
<223>  MET-E17

<400>  1009
gttaatgtct ccaccactgg atttctcagg aatcactgac ataggagaag tttcccaatt      60

tctgaccgag ggaatcatca tgaaagattt tagtcatccc aatgtcctct cgctcctggg      120


<210>  1010
<211>  120
<212>  DNA
<213>  Artificial Sequence

<220>
<223>  MET-E17

<400>  1010
agtttcccaa tttctgaccg agggaatcat catgaaagat tttagtcatc ccaatgtcct      60

ctcgctcctg ggaatctgcc tgcgaagtga agggtctccg ctggtggtcc taccatacat      120


<210>  1011
<211>  120
<212>  DNA
<213>  Artificial Sequence

<220>
<223>  MET-E17

<400>  1011
aatcatcatg aaagatttta gtcatcccaa tgtcctctcg ctcctgggaa tctgcctgcg      60

aagtgaaggg tctccgctgg tggtcctacc atacatgaaa catggagatc ttcgaaattt      120


<210>  1012
<211>  120
<212>  DNA
<213>  Artificial Sequence

<220>
<223>  MET-E17

<400>  1012
aatctgcctg cgaagtgaag ggtctccgct ggtggtccta ccatacatga aacatggaga      60

tcttcgaaat ttcattcgaa atgagactca tgtaagttga ctgccaagct tactaactgg     120


<210>  1013
<211>  120
<212>  DNA
<213>  Artificial Sequence

<220>
<223>  MET-E17

<400>  1013
tccgctggtg gtcctaccat acatgaaaca tggagatctt cgaaatttca ttcgaaatga      60

gactcatgta agttgactgc caagcttact aactggcaaa ctagctgtaa gccagccatc     120


<210>  1014
<211>  120
<212>  DNA
<213>  Artificial Sequence

<220>
<223>  MET-E18

<400>  1014
tttttataaa taaatatttc agaattctaa ggtcaaaatt agaacagtag atgcttagtt      60

tatgcttttc taactctctt tgactgcaga atccaactgt aaaagatctt attggctttg     120


<210>  1015
<211>  120
<212>  DNA
<213>  Artificial Sequence

<220>
<223>  MET-E18

<400>  1015
ttctaaggtc aaaattagaa cagtagatgc ttagtttatg cttttctaac tctctttgac      60

tgcagaatcc aactgtaaaa gatcttattg gctttggtct tcaagtagcc aaaggcatga     120


<210>  1016
<211>  120
<212>  DNA
<213>  Artificial Sequence

<220>
<223>  MET-E18

<400>  1016
agatgcttag tttatgcttt tctaactctc tttgactgca gaatccaact gtaaaagatc      60

ttattggctt tggtcttcaa gtagccaaag gcatgaaata tcttgcaagc aaaaagtttg     120

<210>  1017
<211>  120
<212>  DNA
<213>  Artificial Sequence

<220>
<223>  MET-E18

<400>  1017
gtcttcaagt agccaaaggc atgaaatatc ttgcaagcaa aaagtttgtc cacagagact      60

tggctgcaag aaactgtatg taagtatcag aatctctgtg ccacaatcca aattaagtga      120


<210>  1018
<211>  120
<212>  DNA
<213>  Artificial Sequence

<220>
<223>  MET-E18

<400>  1018
aatatcttgc aagcaaaaag tttgtccaca gagacttggc tgcaagaaac tgtatgtaag      60

tatcagaatc tctgtgccac aatccaaatt aagtgacaag gaggaatctg tttcccactg      120


<210>  1019
<211>  120
<212>  DNA
<213>  Artificial Sequence

<220>
<223>  MET-E18

<400>  1019
tccacagaga cttggctgca agaaactgta tgtaagtatc agaatctctg tgccacaatc      60

caaattaagt gacaaggagg aatctgtttc ccactgttca atgctagtta agctgttttc      120


<210>  1020
<211>  120
<212>  DNA
<213>  Artificial Sequence

<220>
<223>  MET-E18

<400>  1020
actgtatgta agtatcagaa tctctgtgcc acaatccaaa ttaagtgaca aggaggaatc      60

tgtttcccac tgttcaatgc tagttaagct gttttctctt cttatgcaaa agtcctttat      120


<210>  1021
<211>  120
<212>  DNA
<213>  Artificial Sequence

<220>
<223>  MET-E19

<400>  1021
tcagcatcat tgtaaattat tctatttcag ccacgggtaa taatttttgt cctttctgta      60

ggctggatga aaaattcaca gtcaaggttg ctgattttgg tcttgccaga gacatgtatg     120


<210>  1022
<211>  120
<212>  DNA
<213>  Artificial Sequence

<220>
<223>  MET-E19

<400>  1022
tttcagccac gggtaataat ttttgtcctt tctgtaggct ggatgaaaaa ttcacagtca      60

aggttgctga ttttggtctt gccagagaca tgtatgataa agaatactat agtgtacaca     120


<210>  1023
<211>  120
<212>  DNA
<213>  Artificial Sequence

<220>
<223>  MET-E19

<400>  1023
aattcacagt caaggttgct gattttggtc ttgccagaga catgtatgat aaagaatact      60

atagtgtaca caacaaaaca ggtgcaaagc tgccagtgaa gtggatggct ttggaaagtc     120


<210>  1024
<211>  120
<212>  DNA
<213>  Artificial Sequence

<220>
<223>  MET-E19

<400>  1024
ttggtcttgc cagagacatg tatgataaag aatactatag tgtacacaac aaaacaggtg      60

caaagctgcc agtgaagtgg atggctttgg aaagtctgca aactcaaaag tttaccacca     120


<210>  1025
<211>  120
<212>  DNA
<213>  Artificial Sequence

<220>
<223>  MET-E19

<400>  1025
acaaaacagg tgcaaagctg ccagtgaagt ggatggcttt ggaaagtctg caaactcaaa      60

agtttaccac caagtcagat gtggtaatgt attggttatc tctgagtttc tcctctttta     120

<210> 1026
<211> 120
<212> DNA
<213> Artificial Sequence

<220>
<223> MET-E19

<400> 1026
tgaagtggat ggctttggaa agtctgcaaa ctcaaaagtt taccaccaag tcagatgtgg 60

taatgtattg gttatctctg agtttctcct cttttacttt catatccaac tttttttgaa 120


<210> 1027
<211> 120
<212> DNA
<213> Artificial Sequence

<220>
<223> MET-E2

<400> 1027
ttctcttcat ttctgacaac tgaactgctc tcgccttgaa cctgttttgg cagataaacc 60

tctcataatg aaggcccccg ctgtgcttgc acctggcatc ctcgtgctcc tgtttacctt 120


<210> 1028
<211> 120
<212> DNA
<213> Artificial Sequence

<220>
<223> MET-E2

<400> 1028
ctgctctcgc cttgaacctg ttttggcaga taaacctctc ataatgaagg cccccgctgt 60

gcttgcacct ggcatcctcg tgctcctgtt taccttggtg cagaggagca atggggagtg 120


<210> 1029
<211> 120
<212> DNA
<213> Artificial Sequence

<220>
<223> MET-E2

<400> 1029
ggcagataaa cctctcataa tgaaggcccc cgctgtgctt gcacctggca tcctcgtgct 60

cctgtttacc ttggtgcaga ggagcaatgg ggagtgtaaa gaggcactag caaagtccga 120


<210> 1030
<211> 120
<212> DNA
<213> Artificial Sequence

<220>
<223> MET-E2

<400>  1030
ggcccccgct gtgcttgcac ctggcatcct cgtgctcctg tttaccttgg tgcagaggag      60

caatggggag tgtaaagagg cactagcaaa gtccgagatg aatgtgaata tgaagtatca     120


<210>  1031
<211>  120
<212>  DNA
<213>  Artificial Sequence

<220>
<223>  MET-E2

<400>  1031
catcctcgtg ctcctgttta ccttggtgca gaggagcaat ggggagtgta aagaggcact      60

agcaaagtcc gagatgaatg tgaatatgaa gtatcagctt cccaacttca ccgcggaaac     120


<210>  1032
<211>  120
<212>  DNA
<213>  Artificial Sequence

<220>
<223>  MET-E2

<400>  1032
ggtgcagagg agcaatgggg agtgtaaaga ggcactagca aagtccgaga tgaatgtgaa      60

tatgaagtat cagcttccca acttcaccgc ggaaacaccc atccagaatg tcattctaca     120


<210>  1033
<211>  120
<212>  DNA
<213>  Artificial Sequence

<220>
<223>  MET-E2

<400>  1033
taaagaggca ctagcaaagt ccgagatgaa tgtgaatatg aagtatcagc ttcccaactt      60

caccgcggaa acacccatcc agaatgtcat tctacatgag catcacattt tccttggtgc     120


<210>  1034
<211>  120
<212>  DNA
<213>  Artificial Sequence

<220>
<223>  MET-E2

<400>  1034
gatgaatgtg aatatgaagt atcagcttcc caacttcacc gcggaaacac ccatccagaa      60

tgtcattcta catgagcatc acattttcct tggtgccact aactacattt atgttttaaa     120

<210>  1035
<211>  120
<212>  DNA
<213>  Artificial Sequence

<220>
<223>  MET-E2

<400>  1035
gcttcccaac ttcaccgcgg aaacacccat ccagaatgtc attctacatg agcatcacat      60

tttccttggt gccactaact acatttatgt tttaaatgag gaagaccttc agaaggttgc      120


<210>  1036
<211>  120
<212>  DNA
<213>  Artificial Sequence

<220>
<223>  MET-E2

<400>  1036
acccatccag aatgtcattc tacatgagca tcacattttc cttggtgcca ctaactacat      60

ttatgtttta aatgaggaag accttcagaa ggttgctgag tacaagactg ggcctgtgct      120


<210>  1037
<211>  120
<212>  DNA
<213>  Artificial Sequence

<220>
<223>  MET-E2

<400>  1037
tgagcatcac attttccttg gtgccactaa ctacatttat gttttaaatg aggaagacct      60

tcagaaggtt gctgagtaca agactgggcc tgtgctggaa cacccagatt gtttcccatg      120


<210>  1038
<211>  120
<212>  DNA
<213>  Artificial Sequence

<220>
<223>  MET-E2

<400>  1038
cactaactac atttatgttt taaatgagga agaccttcag aaggttgctg agtacaagac      60

tgggcctgtg ctggaacacc cagattgttt cccatgtcag gactgcagca gcaaagccaa      120


<210>  1039
<211>  120
<212>  DNA
<213>  Artificial Sequence

<220>
<223>  MET-E2

<400>  1039
tgaggaagac cttcagaagg ttgctgagta caagactggg cctgtgctgg aacacccaga      60

ttgtttccca tgtcaggact gcagcagcaa agccaattta tcaggaggtg tttggaaaga     120


<210>  1040
<211>  120
<212>  DNA
<213>  Artificial Sequence

<220>
<223>  MET-E2

<400>  1040
tgagtacaag actgggcctg tgctggaaca cccagattgt ttcccatgtc aggactgcag      60

cagcaaagcc aatttatcag gaggtgtttg gaaagataac atcaacatgg ctctagttgt     120


<210>  1041
<211>  120
<212>  DNA
<213>  Artificial Sequence

<220>
<223>  MET-E2

<400>  1041
ggaacaccca gattgtttcc catgtcagga ctgcagcagc aaagccaatt tatcaggagg      60

tgtttggaaa gataacatca acatggctct agttgtcgac acctactatg atgatcaact     120


<210>  1042
<211>  120
<212>  DNA
<213>  Artificial Sequence

<220>
<223>  MET-E2

<400>  1042
tcaggactgc agcagcaaag ccaatttatc aggaggtgtt tggaaagata acatcaacat      60

ggctctagtt gtcgacacct actatgatga tcaactcatt agctgtggca gcgtcaacag     120


<210>  1043
<211>  120
<212>  DNA
<213>  Artificial Sequence

<220>
<223>  MET-E2

<400>  1043
tttatcagga ggtgtttgga aagataacat caacatggct ctagttgtcg acacctacta      60

tgatgatcaa ctcattagct gtggcagcgt caacagaggg acctgccagc gacatgtctt     120

<210>  1044
<211>  120
<212>  DNA
<213>  Artificial Sequence

<220>
<223>  MET-E2

<400>  1044
taacatcaac atggctctag ttgtcgacac ctactatgat gatcaactca ttagctgtgg      60

cagcgtcaac agagggacct gccagcgaca tgtctttccc cacaatcata ctgctgacat      120


<210>  1045
<211>  120
<212>  DNA
<213>  Artificial Sequence

<220>
<223>  MET-E2

<400>  1045
cgacacctac tatgatgatc aactcattag ctgtggcagc gtcaacagag ggacctgcca      60

gcgacatgtc tttccccaca atcatactgc tgacatacag tcggaggttc actgcatatt      120


<210>  1046
<211>  120
<212>  DNA
<213>  Artificial Sequence

<220>
<223>  MET-E2

<400>  1046
cattagctgt ggcagcgtca acagagggac ctgccagcga catgtctttc cccacaatca      60

tactgctgac atacagtcgg aggttcactg catattctcc ccacagatag aagagcccag      120


<210>  1047
<211>  120
<212>  DNA
<213>  Artificial Sequence

<220>
<223>  MET-E2

<400>  1047
agggacctgc cagcgacatg tctttcccca caatcatact gctgacatac agtcggaggt      60

tcactgcata ttctccccac agatagaaga gcccagccag tgtcctgact gtgtggtgag      120


<210>  1048
<211>  120
<212>  DNA
<213>  Artificial Sequence

<220>
<223>  MET-E2

<400>  1048
tccccacaat catactgctg acatacagtc ggaggttcac tgcatattct ccccacagat      60

agaagagccc agccagtgtc ctgactgtgt ggtgagcgcc ctgggagcca aagtcctttc     120


<210>  1049
<211>  120
<212>  DNA
<213>  Artificial Sequence

<220>
<223>  MET-E2

<400>  1049
acagtcggag gttcactgca tattctcccc acagatagaa gagcccagcc agtgtcctga      60

ctgtgtggtg agcgccctgg gagccaaagt cctttcatct gtaaaggacc ggttcatcaa     120


<210>  1050
<211>  120
<212>  DNA
<213>  Artificial Sequence

<220>
<223>  MET-E2

<400>  1050
ctccccacag atagaagagc ccagccagtg tcctgactgt gtggtgagcg ccctgggagc      60

caaagtcctt tcatctgtaa aggaccggtt catcaacttc tttgtaggca ataccataaa     120


<210>  1051
<211>  120
<212>  DNA
<213>  Artificial Sequence

<220>
<223>  MET-E2

<400>  1051
ccagtgtcct gactgtgtgg tgagcgccct gggagccaaa gtcctttcat ctgtaaagga      60

ccggttcatc aacttctttg taggcaatac cataaattct tcttatttcc cagatcatcc     120


<210>  1052
<211>  120
<212>  DNA
<213>  Artificial Sequence

<220>
<223>  MET-E2

<400>  1052
cgccctggga gccaaagtcc tttcatctgt aaaggaccgg ttcatcaact tctttgtagg      60

caataccata aattcttctt atttcccaga tcatccattg cattcgatat cagtgagaag     120

<210>  1053
<211>  120
<212>  DNA
<213>  Artificial Sequence

<220>
<223>  MET-E2

<400>  1053
atctgtaaag gaccggttca tcaacttctt tgtaggcaat accataaatt cttcttattt      60

cccagatcat ccattgcatt cgatatcagt gagaaggcta aaggaaacga aagatggttt      120


<210>  1054
<211>  120
<212>  DNA
<213>  Artificial Sequence

<220>
<223>  MET-E2

<400>  1054
cttctttgta ggcaatacca taaattcttc ttatttccca gatcatccat tgcattcgat      60

atcagtgaga aggctaaagg aaacgaaaga tggttttatg tttttgacgg accagtccta      120


<210>  1055
<211>  120
<212>  DNA
<213>  Artificial Sequence

<220>
<223>  MET-E2

<400>  1055
ttcttcttat ttcccagatc atccattgca ttcgatatca gtgagaaggc taaaggaaac      60

gaaagatggt tttatgtttt tgacggacca gtcctacatt gatgttttac ctgagttcag      120


<210>  1056
<211>  120
<212>  DNA
<213>  Artificial Sequence

<220>
<223>  MET-E2

<400>  1056
attgcattcg atatcagtga gaaggctaaa ggaaacgaaa gatggtttta tgtttttgac      60

ggaccagtcc tacattgatg ttttacctga gttcagagat tcttacccca ttaagtatgt      120


<210>  1057
<211>  120
<212>  DNA
<213>  Artificial Sequence

<220>
<223>  MET-E2

<400>  1057
gctaaaggaa acgaaagatg gttttatgtt tttgacggac cagtcctaca ttgatgtttt      60

acctgagttc agagattctt accccattaa gtatgtccat gcctttgaaa gcaacaattt     120


<210>  1058
<211>  120
<212>  DNA
<213>  Artificial Sequence

<220>
<223>  MET-E2

<400>  1058
tatgtttttg acggaccagt cctacattga tgttttacct gagttcagag attcttaccc      60

cattaagtat gtccatgcct ttgaaagcaa caattttatt tacttcttga cggtccaaag     120


<210>  1059
<211>  120
<212>  DNA
<213>  Artificial Sequence

<220>
<223>  MET-E2

<400>  1059
cattgatgtt ttacctgagt tcagagattc ttaccccatt aagtatgtcc atgcctttga      60

aagcaacaat tttatttact tcttgacggt ccaaagggaa actctagatg ctcagacttt     120


<210>  1060
<211>  120
<212>  DNA
<213>  Artificial Sequence

<220>
<223>  MET-E2

<400>  1060
agattcttac cccattaagt atgtccatgc ctttgaaagc aacaatttta tttacttctt      60

gacggtccaa agggaaactc tagatgctca gacttttcac acaagaataa tcaggttctg     120


<210>  1061
<211>  120
<212>  DNA
<213>  Artificial Sequence

<220>
<223>  MET-E2

<400>  1061
ccatgccttt gaaagcaaca attttattta cttcttgacg gtccaaaggg aaactctaga      60

tgctcagact tttcacacaa gaataatcag gttctgttcc ataaactctg gattgcattc     120

<210>  1062
<211>  120
<212>  DNA
<213>  Artificial Sequence

<220>
<223>  MET-E2

<400>  1062
tatttacttc ttgacggtcc aaagggaaac tctagatgct cagacttttc acacaagaat      60

aatcaggttc tgttccataa actctggatt gcattcctac atggaaatgc ctctggagtg      120


<210>  1063
<211>  120
<212>  DNA
<213>  Artificial Sequence

<220>
<223>  MET-E2

<400>  1063
ggaaactcta gatgctcaga cttttcacac aagaataatc aggttctgtt ccataaactc      60

tggattgcat tcctacatgg aaatgcctct ggagtgtatt ctcacagaaa agagaaaaaa      120


<210>  1064
<211>  120
<212>  DNA
<213>  Artificial Sequence

<220>
<223>  MET-E2

<400>  1064
tcacacaaga ataatcaggt tctgttccat aaactctgga ttgcattcct acatggaaat      60

gcctctggag tgtattctca cagaaaagag aaaaaagaga tccacaaaga aggaagtgtt      120


<210>  1065
<211>  120
<212>  DNA
<213>  Artificial Sequence

<220>
<223>  MET-E2

<400>  1065
ttccataaac tctggattgc attcctacat ggaaatgcct ctggagtgta ttctcacaga      60

aaagagaaaa aagagatcca caaagaagga agtgtttaat atacttcagg ctgcgtatgt      120


<210>  1066
<211>  120
<212>  DNA
<213>  Artificial Sequence

<220>
<223>  MET-E2

<400>  1066
ctacatggaa atgcctctgg agtgtattct cacagaaaag agaaaaaaga gatccacaaa      60

gaaggaagtg tttaatatac ttcaggctgc gtatgtcagc aagcctgggg cccagcttgc     120


<210>  1067
<211>  120
<212>  DNA
<213>  Artificial Sequence

<220>
<223>  MET-E2

<400>  1067
tattctcaca gaaaagagaa aaaagagatc cacaaagaag gaagtgttta atatacttca      60

ggctgcgtat gtcagcaagc ctggggccca gcttgctaga caaataggag ccagcctgaa     120


<210>  1068
<211>  120
<212>  DNA
<213>  Artificial Sequence

<220>
<223>  MET-E2

<400>  1068
gagatccaca aagaaggaag tgtttaatat acttcaggct gcgtatgtca gcaagcctgg      60

ggcccagctt gctagacaaa taggagccag cctgaatgat gacattcttt tcggggtgtt     120


<210>  1069
<211>  120
<212>  DNA
<213>  Artificial Sequence

<220>
<223>  MET-E2

<400>  1069
taatatactt caggctgcgt atgtcagcaa gcctggggcc cagcttgcta gacaaatagg      60

agccagcctg aatgatgaca ttcttttcgg ggtgttcgca caaagcaagc cagattctgc     120


<210>  1070
<211>  120
<212>  DNA
<213>  Artificial Sequence

<220>
<223>  MET-E2

<400>  1070
cagcaagcct ggggcccagc ttgctagaca aataggagcc agcctgaatg atgacattct      60

tttcggggtg ttcgcacaaa gcaagccaga ttctgccgaa ccaatggatc gatctgccat     120

<210>  1071
<211>  120
<212>  DNA
<213>  Artificial Sequence

<220>
<223>  MET-E2

<400>  1071
tagacaaata ggagccagcc tgaatgatga cattcttttc ggggtgttcg cacaaagcaa      60

gccagattct gccgaaccaa tggatcgatc tgccatgtgt gcattcccta tcaaatatgt      120


<210>  1072
<211>  120
<212>  DNA
<213>  Artificial Sequence

<220>
<223>  MET-E2

<400>  1072
tgatgacatt cttttcgggg tgttcgcaca aagcaagcca gattctgccg aaccaatgga      60

tcgatctgcc atgtgtgcat tccctatcaa atatgtcaac gacttcttca acaagatcgt      120


<210>  1073
<211>  120
<212>  DNA
<213>  Artificial Sequence

<220>
<223>  MET-E2

<400>  1073
cgcacaaagc aagccagatt ctgccgaacc aatggatcga tctgccatgt gtgcattccc      60

tatcaaatat gtcaacgact tcttcaacaa gatcgtcaac aaaaacaatg tgagatgtct      120


<210>  1074
<211>  120
<212>  DNA
<213>  Artificial Sequence

<220>
<223>  MET-E2

<400>  1074
cgaaccaatg gatcgatctg ccatgtgtgc attccctatc aaatatgtca acgacttctt      60

caacaagatc gtcaacaaaa acaatgtgag atgtctccag catttttacg gacccaatca      120


<210>  1075
<211>  120
<212>  DNA
<213>  Artificial Sequence

<220>
<223>  MET-E2

<400>  1075
gtgtgcattc cctatcaaat atgtcaacga cttcttcaac aagatcgtca acaaaaacaa      60

tgtgagatgt ctccagcatt tttacggacc caatcatgag cactgcttta atagggtaag     120


<210>  1076
<211>  120
<212>  DNA
<213>  Artificial Sequence

<220>
<223>  MET-E2

<400>  1076
caacgacttc ttcaacaaga tcgtcaacaa aaacaatgtg agatgtctcc agcattttta      60

cggacccaat catgagcact gctttaatag ggtaagtcac atcagttccc cacttataaa     120


<210>  1077
<211>  120
<212>  DNA
<213>  Artificial Sequence

<220>
<223>  MET-E2

<400>  1077
caacaaaaac aatgtgagat gtctccagca tttttacgga cccaatcatg agcactgctt      60

taatagggta agtcacatca gttccccact tataaactgt gaggtataaa ttagaaataa     120


<210>  1078
<211>  120
<212>  DNA
<213>  Artificial Sequence

<220>
<223>  MET-E20

<400>  1078
atatgtatgg tcacatctct cacctcatct gtcctgtttc ttgttttact agtggtcctt      60

tggcgtgctc ctctgggagc tgatgacaag aggagcccca ccttatcctg acgtaaacac     120


<210>  1079
<211>  120
<212>  DNA
<213>  Artificial Sequence

<220>
<223>  MET-E20

<400>  1079
tcatctgtcc tgtttcttgt tttactagtg gtcctttggc gtgctcctct gggagctgat      60

gacaagagga gccccacctt atcctgacgt aaacaccttt gatataactg tttacttgtt     120

<210>  1080
<211>  120
<212>  DNA
<213>  Artificial Sequence

<220>
<223>  MET-E20

<400>  1080
ctgggagctg atgacaagag gagccccacc ttatcctgac gtaaacacct ttgatataac      60

tgtttacttg ttgcaaggga gaagactcct acaacccgaa tactgcccag accccttgta      120


<210>  1081
<211>  120
<212>  DNA
<213>  Artificial Sequence

<220>
<223>  MET-E20

<400>  1081
cccaccttat cctgacgtaa acacctttga tataactgtt tacttgttgc aagggagaag      60

actcctacaa cccgaatact gcccagaccc cttgtaagta gtctttctgt acctcttacg      120


<210>  1082
<211>  120
<212>  DNA
<213>  Artificial Sequence

<220>
<223>  MET-E20

<400>  1082
ctttgatata actgtttact tgttgcaagg gagaagactc ctacaacccg aatactgccc      60

agaccccttg taagtagtct ttctgtacct cttacgttct ttacttttac agaaatgcct      120


<210>  1083
<211>  120
<212>  DNA
<213>  Artificial Sequence

<220>
<223>  NRAS

<400>  1083
attgcccaat actatatact aagatttgta attatgccaa gaaaccatat gctcaccttg      60

ttacatcacc acacatggca atcccataca accctgagtc ccatcatcac tgctgttgag      120


<210>  1084
<211>  120
<212>  DNA
<213>  Artificial Sequence

<220>
<223>  NRAS

<400>  1084
ttacatcacc acacatggca atcccataca accctgagtc ccatcatcac tgctgttgag      60

ttttttcatt cggtactggc gtatttctct taccagtgtg taaaaagcat cttcaacacc     120


<210>  1085
<211>  120
<212>  DNA
<213>  Artificial Sequence

<220>
<223>  NRAS

<400>  1085
ttttttcatt cggtactggc gtatttctct taccagtgtg taaaaagcat cttcaacacc      60

ctataaaagg aaaaaatgaa aaaaaatgag agagctagct caacggacac aatccaaatt     120


<210>  1086
<211>  120
<212>  DNA
<213>  Artificial Sequence

<220>
<223>  NRAS

<400>  1086
gatgcaaact cttgcacaaa tgctgaaagc tgtaccatac ctgtctggtc ttggctgagg      60

tttcaatgaa tggaatcccg taactcttgg ccagttcgtg ggcttgtttt gtatcaactg     120


<210>  1087
<211>  120
<212>  DNA
<213>  Artificial Sequence

<220>
<223>  NRAS

<400>  1087
tttcaatgaa tggaatcccg taactcttgg ccagttcgtg ggcttgtttt gtatcaactg      60

tccttgttgg caaatcacac ttgtttccca ctagcaccat aggtacatca tccgagtctt     120


<210>  1088
<211>  120
<212>  DNA
<213>  Artificial Sequence

<220>
<223>  NRAS

<400>  1088
tccttgttgg caaatcacac ttgtttccca ctagcaccat aggtacatca tccgagtctt      60

ttactcgctt aatctgctcc ctaaaaacgg gaatatatta tcagaacata agaaaaacaa     120

<210>  1089
<211>  120
<212>  DNA
<213>  Artificial Sequence

<220>
<223>  NRAS

<400>  1089
atcctttcag agaaaataat gctcctagta cctgtagagg ttaatatccg caaatgactt      60

gctattattg atggcaaata cacagaggaa gccttcgcct gtcctcatgt attggtctct      120


<210>  1090
<211>  120
<212>  DNA
<213>  Artificial Sequence

<220>
<223>  NRAS

<400>  1090
gctattattg atggcaaata cacagaggaa gccttcgcct gtcctcatgt attggtctct      60

catggcactg tactcttctt gtccagctgt atccagtatg tccaacaaac aggtttcacc      120


<210>  1091
<211>  120
<212>  DNA
<213>  Artificial Sequence

<220>
<223>  NRAS

<400>  1091
catggcactg tactcttctt gtccagctgt atccagtatg tccaacaaac aggtttcacc      60

atctataacc acttgttttc tgtaagaatc ctgggggtgt ggagggtaag gggcagggga      120


<210>  1092
<211>  120
<212>  DNA
<213>  Artificial Sequence

<220>
<223>  NRAS

<400>  1092
atgggtaaag atgatccgac aagtgagaga caggatcagg tcagcgggct accactgggc      60

ctcacctcta tggtgggatc atattcatct acaaagtggt tctggattag ctggattgtc      120


<210>  1093
<211>  120
<212>  DNA
<213>  Artificial Sequence

<220>
<223>  NRAS

<400>  1093
ctcacctcta tggtgggatc atattcatct acaaagtggt tctggattag ctggattgtc      60

agtgcgcttt tcccaacacc acctgctcca accaccacca gtttgtactc agtcatttca     120


<210>  1094
<211>  120
<212>  DNA
<213>  Artificial Sequence

<220>
<223>  NRAS

<400>  1094
agtgcgcttt tcccaacacc acctgctcca accaccacca gtttgtactc agtcatttca      60

caccagcaag aacctgttgg aaaccagtaa tcagggttaa ttggcgagcc acatctacag     120


<210>  1095
<211>  120
<212>  DNA
<213>  Artificial Sequence

<220>
<223>  NRAS-E2

<400>  1095
gatgatccga caagtgagag acaggatcag gtcagcgggc taccactggg cctcacctct      60

atggtgggat catattcatc tacaaagtgg ttctggatta gctggattgt cagtgcgctt     120


<210>  1096
<211>  120
<212>  DNA
<213>  Artificial Sequence

<220>
<223>  NRAS-E2

<400>  1096
gatcaggtca gcgggctacc actgggcctc acctctatgg tgggatcata ttcatctaca      60

aagtggttct ggattagctg gattgtcagt gcgcttttcc caacaccacc tgctccaacc     120


<210>  1097
<211>  120
<212>  DNA
<213>  Artificial Sequence

<220>
<223>  NRAS-E2

<400>  1097
tattcatcta caaagtggtt ctggattagc tggattgtca gtgcgctttt cccaacacca      60

cctgctccaa ccaccaccag tttgtactca gtcatttcac accagcaaga acctgttgga     120

<210>  1098
<211>  120
<212>  DNA
<213>  Artificial Sequence

<220>
<223>  NRAS-E2

<400>  1098
attagctgga ttgtcagtgc gcttttccca acaccacctg ctccaaccac caccagtttg      60

tactcagtca tttcacacca gcaagaacct gttggaaacc agtaatcagg gttaattggc      120


<210>  1099
<211>  120
<212>  DNA
<213>  Artificial Sequence

<220>
<223>  NRAS-E2

<400>  1099
ttcccaacac cacctgctcc aaccaccacc agtttgtact cagtcatttc acaccagcaa      60

gaacctgttg gaaaccagta atcagggtta attggcgagc cacatctaca gtactttaaa      120


<210>  1100
<211>  120
<212>  DNA
<213>  Artificial Sequence

<220>
<223>  NRAS-E3

<400>  1100
ataaagattc agaacacaaa gatcatcctt tcagagaaaa taatgctcct agtacctgta      60

gaggttaata tccgcaaatg acttgctatt attgatggca aatacacaga ggaagccttc      120


<210>  1101
<211>  120
<212>  DNA
<213>  Artificial Sequence

<220>
<223>  NRAS-E3

<400>  1101
atcctttcag agaaaataat gctcctagta cctgtagagg ttaatatccg caaatgactt      60

gctattattg atggcaaata cacagaggaa gccttcgcct gtcctcatgt attggtctct      120


<210>  1102
<211>  120
<212>  DNA
<213>  Artificial Sequence

<220>
<223>  NRAS-E3

<400>  1102
ctagtacctg tagaggttaa tatccgcaaa tgacttgcta ttattgatgg caaatacaca      60

gaggaagcct tcgcctgtcc tcatgtattg gtctctcatg gcactgtact cttcttgtcc     120


<210>  1103
<211>  120
<212>  DNA
<213>  Artificial Sequence

<220>
<223>  NRAS-E3

<400>  1103
cgcaaatgac ttgctattat tgatggcaaa tacacagagg aagccttcgc ctgtcctcat      60

gtattggtct ctcatggcac tgtactcttc ttgtccagct gtatccagta tgtccaacaa     120


<210>  1104
<211>  120
<212>  DNA
<213>  Artificial Sequence

<220>
<223>  NRAS-E3

<400>  1104
gcctgtcctc atgtattggt ctctcatggc actgtactct tcttgtccag ctgtatccag      60

tatgtccaac aaacaggttt caccatctat aaccacttgt tttctgtaag aatcctgggg     120


<210>  1105
<211>  120
<212>  DNA
<213>  Artificial Sequence

<220>
<223>  NRAS-E3

<400>  1105
catggcactg tactcttctt gtccagctgt atccagtatg tccaacaaac aggtttcacc      60

atctataacc acttgttttc tgtaagaatc ctgggggtgt ggagggtaag ggggcaggga     120


<210>  1106
<211>  120
<212>  DNA
<213>  Artificial Sequence

<220>
<223>  NRAS-E3

<400>  1106
agctgtatcc agtatgtcca acaaacaggt ttcaccatct ataaccactt gttttctgta      60

agaatcctgg gggtgtggag ggtaaggggg cagggaggga gggaagttca atttttatta     120

<210>  1107
<211>  120
<212>  DNA
<213>  Artificial Sequence

<220>
<223>  NRAS-E4

<400>  1107
cagagttaat caactgatgc aaactcttgc acaaatgctg aaagctgtac catacctgtc      60

tggtcttggc tgaggtttca atgaatggaa tcccgtaact cttggccagt tcgtgggctt      120


<210>  1108
<211>  120
<212>  DNA
<213>  Artificial Sequence

<220>
<223>  NRAS-E4

<400>  1108
tcttgcacaa atgctgaaag ctgtaccata cctgtctggt cttggctgag gtttcaatga      60

atggaatccc gtaactcttg gccagttcgt gggcttgttt tgtatcaact gtccttgttg      120


<210>  1109
<211>  120
<212>  DNA
<213>  Artificial Sequence

<220>
<223>  NRAS-E4

<400>  1109
aggtttcaat gaatggaatc ccgtaactct tggccagttc gtgggcttgt tttgtatcaa      60

ctgtccttgt tggcaaatca cacttgtttc ccactagcac cataggtaca tcatccgagt      120


<210>  1110
<211>  120
<212>  DNA
<213>  Artificial Sequence

<220>
<223>  NRAS-E4

<400>  1110
aactcttggc cagttcgtgg gcttgttttg tatcaactgt ccttgttggc aaatcacact      60

tgtttcccac tagcaccata ggtacatcat ccgagtcttt tactcgctta atctgctccc      120


<210>  1111
<211>  120
<212>  DNA
<213>  Artificial Sequence

<220>
<223>  NRAS-E4

<400>  1111
gttttgtatc aactgtcctt gttggcaaat cacacttgtt tcccactagc accataggta        60

catcatccga gtcttttact cgcttaatct gctccctaaa aacgggaata tattatcaga       120


<210>  1112
<211>  120
<212>  DNA
<213>  Artificial Sequence

<220>
<223>  NRAS-E4

<400>  1112
gcaaatcaca cttgtttccc actagcacca taggtacatc atccgagtct tttactcgct        60

taatctgctc cctaaaaacg ggaatatatt atcagaacat aagaaaaaca agattaggct       120


<210>  1113
<211>  120
<212>  DNA
<213>  Artificial Sequence

<220>
<223>  PDGFRA

<400>  1113
taggctccag ggttgtttct atttgctaat gctgtttctg ttgacttttg acttttctag        60

tttcccagag ctatggggac ttcccatccg gcgttcctgg tcttaggctg tcttctcaca       120


<210>  1114
<211>  120
<212>  DNA
<213>  Artificial Sequence

<220>
<223>  PDGFRA

<400>  1114
tttcccagag ctatggggac ttcccatccg gcgttcctgg tcttaggctg tcttctcaca        60

ggtacggagc ccagtcctct ctgagttcct tgtttgggtg tcttgttttt ttaagctttg       120


<210>  1115
<211>  120
<212>  DNA
<213>  Artificial Sequence

<220>
<223>  PDGFRA

<400>  1115
agagcgtgct tccttttgca gggctgagcc taatcctctg ccagctttca ttaccctcta        60

tccttccaaa tgaaaatgaa aaggttgtgc agctgaattc atccttttct ctgagatgct       120

<210>  1116
<211>  120
<212>  DNA
<213>  Artificial Sequence

<220>
<223>  PDGFRA

<400>  1116
tccttccaaa tgaaaatgaa aaggttgtgc agctgaattc atccttttct ctgagatgct      60

ttggggagag tgaagtgagc tggcagtacc ccatgtctga agaagagagc tccgatgtgg     120


<210>  1117
<211>  120
<212>  DNA
<213>  Artificial Sequence

<220>
<223>  PDGFRA

<400>  1117
ttggggagag tgaagtgagc tggcagtacc ccatgtctga agaagagagc tccgatgtgg      60

aaatcagaaa tgaagaaaac aacagcggcc tttttgtgac ggtcttggaa gtgagcagtg     120


<210>  1118
<211>  120
<212>  DNA
<213>  Artificial Sequence

<220>
<223>  PDGFRA

<400>  1118
aaatcagaaa tgaagaaaac aacagcggcc tttttgtgac ggtcttggaa gtgagcagtg      60

cctcggcggc ccacacaggg ttgtacactt gctattacaa ccacactcag acagaagaga     120


<210>  1119
<211>  120
<212>  DNA
<213>  Artificial Sequence

<220>
<223>  PDGFRA

<400>  1119
cctcggcggc ccacacaggg ttgtacactt gctattacaa ccacactcag acagaagaga      60

atgagcttga aggcaggcac atttacatct atgtgccagg tgagttggct gggtctccag     120


<210>  1120
<211>  120
<212>  DNA
<213>  Artificial Sequence

<220>
<223>  PDGFRA

<400>  1120
gtctaataga gtcttcattc ttttttaaac cacagaccca gatgtagcct ttgtacctct        60

aggaatgacg gattatttag tcatcgtgga ggatgatgat tctgccatta taccttgtcg       120


<210>  1121
<211>  120
<212>  DNA
<213>  Artificial Sequence

<220>
<223>  PDGFRA

<400>  1121
aggaatgacg gattatttag tcatcgtgga ggatgatgat tctgccatta taccttgtcg        60

cacaactgat cccgagactc ctgtaacctt acacaacagt gagggggtgg tacctgcctc       120


<210>  1122
<211>  120
<212>  DNA
<213>  Artificial Sequence

<220>
<223>  PDGFRA

<400>  1122
cacaactgat cccgagactc ctgtaacctt acacaacagt gagggggtgg tacctgcctc        60

ctacgacagc agacagggct ttaatgggac cttcactgta gggccctata tctgtgaggc       120


<210>  1123
<211>  120
<212>  DNA
<213>  Artificial Sequence

<220>
<223>  PDGFRA

<400>  1123
ctacgacagc agacagggct ttaatgggac cttcactgta gggccctata tctgtgaggc        60

caccgtcaaa ggaaagaagt tccagaccat cccatttaat gtttatgctt taaaaggtac       120


<210>  1124
<211>  120
<212>  DNA
<213>  Artificial Sequence

<220>
<223>  PDGFRA

<400>  1124
caccgtcaaa ggaaagaagt tccagaccat cccatttaat gtttatgctt taaaggtac        60

ttgtatcatc tccttccttc tttaaataag agtaacaggc aaaatcataa ggtgcgtgta       120

<210>  1125
<211>  120
<212>  DNA
<213>  Artificial Sequence

<220>
<223>  PDGFRA

<400>  1125
aacccaaact ttataagatc ctggctatcc tgtggatttt taggcccttg tatttgttct      60

tttttatagc aacatcagag ctggatctag aaatggaagc tcttaaaacc gtgtataagt      120


<210>  1126
<211>  120
<212>  DNA
<213>  Artificial Sequence

<220>
<223>  PDGFRA

<400>  1126
tttttatagc aacatcagag ctggatctag aaatggaagc tcttaaaacc gtgtataagt      60

caggggaaac gattgtggtc acctgtgctg tttttaacaa tgaggtggtt gaccttcaat      120


<210>  1127
<211>  120
<212>  DNA
<213>  Artificial Sequence

<220>
<223>  PDGFRA

<400>  1127
caggggaaac gattgtggtc acctgtgctg tttttaacaa tgaggtggtt gaccttcaat      60

ggacttaccc tggagaagtg gtaggtaccc tcaaaacgtg caatggcttg gagcagagca      120


<210>  1128
<211>  120
<212>  DNA
<213>  Artificial Sequence

<220>
<223>  PDGFRA

<400>  1128
tttaaaagtc ggttttcttc cccttttgct gtagaaaggc aaaggcatca caatgctgga      60

agaaatcaaa gtcccatcca tcaaattggt gtacactttg acggtccccg aggccacggt      120


<210>  1129
<211>  120
<212>  DNA
<213>  Artificial Sequence

<220>
<223>  PDGFRA

<400>  1129
agaaatcaaa gtcccatcca tcaaattggt gtacactttg acggtccccg aggccacggt      60

gaaagacagt ggagattacg aatgtgctgc ccgccaggct accagggagg tcaaagaaat     120


<210>  1130
<211>  120
<212>  DNA
<213>  Artificial Sequence

<220>
<223>  PDGFRA

<400>  1130
gaaagacagt ggagattacg aatgtgctgc ccgccaggct accagggagg tcaaagaaat      60

gaagaaagtc actatttctg tccatggtac attccgcttt ctaaaatgtc agttgtccat     120


<210>  1131
<211>  120
<212>  DNA
<213>  Artificial Sequence

<220>
<223>  PDGFRA

<400>  1131
ttaatggaaa ctcttccctg tacagagaaa ggtttcattg aaatcaaacc caccttcagc      60

cagttggaag ctgtcaacct gcatgaagtc aaacattttg ttgtagaggt gcgggcctac     120


<210>  1132
<211>  120
<212>  DNA
<213>  Artificial Sequence

<220>
<223>  PDGFRA

<400>  1132
cagttggaag ctgtcaacct gcatgaagtc aaacattttg ttgtagaggt gcgggcctac      60

ccacctccca ggatatcctg gctgaaaaac aatctgactc tgattgaaaa tctcactgag     120


<210>  1133
<211>  120
<212>  DNA
<213>  Artificial Sequence

<220>
<223>  PDGFRA

<400>  1133
ccacctccca ggatatcctg gctgaaaaac aatctgactc tgattgaaaa tctcactgag      60

atcaccactg atgtggaaaa gattcaggaa ataaggtaaa gaaactctct gcccaagtat     120

<210>  1134
<211>  120
<212>  DNA
<213>  Artificial Sequence

<220>
<223>  PDGFRA

<400>  1134
tgtttaaaca attggaactt acttagctac tgcttgttga aacaaaatcc tttttttaaa      60

aggtatcgaa gcaaattaaa gctgatccgt gctaaggaag aagacagtgg ccattatact      120


<210>  1135
<211>  120
<212>  DNA
<213>  Artificial Sequence

<220>
<223>  PDGFRA

<400>  1135
aggtatcgaa gcaaattaaa gctgatccgt gctaaggaag aagacagtgg ccattatact      60

attgtagctc aaaatgaaga tgctgtgaag agctatactt ttgaactgtt aactcaaggt      120


<210>  1136
<211>  120
<212>  DNA
<213>  Artificial Sequence

<220>
<223>  PDGFRA

<400>  1136
attgtagctc aaaatgaaga tgctgtgaag agctatactt ttgaactgtt aactcaaggt      60

atgtaaaggg agtataaaga taatgctagc tctgtagatg agtgtcttcc aaggaaagcc      120


<210>  1137
<211>  120
<212>  DNA
<213>  Artificial Sequence

<220>
<223>  PDGFRA

<400>  1137
tctcatatgt tctgggacac gagctattcc attctgactt ctttctgcct cttgcagttc      60

cttcatccat tctggacttg gtcgatgatc accatggctc aactggggga cagacggtga      120


<210>  1138
<211>  120
<212>  DNA
<213>  Artificial Sequence

<220>
<223>  PDGFRA

<400>  1138
cttcatccat tctggacttg gtcgatgatc accatggctc aactggggga cagacggtga      60

ggtgcacagc tgaaggcacg ccgcttcctg atattgagtg gatgatatgc aaagatatta      120


<210>  1139
<211>  120
<212>  DNA
<213>  Artificial Sequence

<220>
<223>  PDGFRA

<400>  1139
ggtgcacagc tgaaggcacg ccgcttcctg atattgagtg gatgatatgc aaagatatta      60

agaagtatgg aaaacagatg tgtcttcttc tttcgtggtc agaatatttc tcccttgaca      120


<210>  1140
<211>  120
<212>  DNA
<213>  Artificial Sequence

<220>
<223>  PDGFRA

<400>  1140
acttaggccc tttttctctc tagatgtaat aatgaaactt cctggactat tttggccaac      60

aatgtctcaa acatcatcac ggagatccac tcccgagaca ggagtaccgt ggagggccgt      120


<210>  1141
<211>  120
<212>  DNA
<213>  Artificial Sequence

<220>
<223>  PDGFRA

<400>  1141
aatgtctcaa acatcatcac ggagatccac tcccgagaca ggagtaccgt ggagggccgt      60

gtgactttcg ccaaagtgga ggagaccatc gccgtgcgat gcctggctaa gaatctcctt      120


<210>  1142
<211>  120
<212>  DNA
<213>  Artificial Sequence

<220>
<223>  PDGFRA

<400>  1142
gtgactttcg ccaaagtgga ggagaccatc gccgtgcgat gcctggctaa gaatctcctt      60

ggagctgaga accgagagct gaagctggtg gctcccagtg agttcctcaa cagtcaggac      120

<210>  1143
<211>  120
<212>  DNA
<213>  Artificial Sequence

<220>
<223>  PDGFRA

<400>  1143
tgccaggaaa cttttcattg tgcctctctc tcttgtcacg tagccctgcg ttctgaactc      60

acggtggctg ctgcagtcct ggtgctgttg gtgattgtga tcatctcact tattgtcctg      120


<210>  1144
<211>  120
<212>  DNA
<213>  Artificial Sequence

<220>
<223>  PDGFRA

<400>  1144
acggtggctg ctgcagtcct ggtgctgttg gtgattgtga tcatctcact tattgtcctg      60

gttgtcattt ggaaacaggt agatattttc tcataaaact aaagatcttt gaagccaatg      120


<210>  1145
<211>  120
<212>  DNA
<213>  Artificial Sequence

<220>
<223>  PDGFRA

<400>  1145
ctggtgcact gggactttgg taattcacca gttacctgtc ctggtcattt atagaaaccg      60

aggtatgaaa ttcgctggag ggtcattgaa tcaatcagcc cagatggaca tgaatatatt      120


<210>  1146
<211>  120
<212>  DNA
<213>  Artificial Sequence

<220>
<223>  PDGFRA

<400>  1146
aggtatgaaa ttcgctggag ggtcattgaa tcaatcagcc cagatggaca tgaatatatt      60

tatgtggacc cgatgcagct gccttatgac tcaagatggg agtttccaag agatggacta      120


<210>  1147
<211>  120
<212>  DNA
<213>  Artificial Sequence

<220>
<223>  PDGFRA

<400>  1147
tatgtggacc cgatgcagct gccttatgac tcaagatggg agtttccaag agatggacta      60

gtgcttggta agttccatgg ggtaacctcc caagactccc ttttcccttg cacacaactt     120


<210>  1148
<211>  120
<212>  DNA
<213>  Artificial Sequence

<220>
<223>  PDGFRA

<400>  1148
ggctacggtg cagaaagctg aggaggcgtc tggagttttt gggtgttaat gattctgcct      60

gcccacaggt cgggtcttgg ggtctggagc gtttgggaag gtggttgaag gaacagccta     120


<210>  1149
<211>  120
<212>  DNA
<213>  Artificial Sequence

<220>
<223>  PDGFRA

<400>  1149
gcccacaggt cgggtcttgg ggtctggagc gtttgggaag gtggttgaag gaacagccta      60

tggattaagc cggtcccaac ctgtcatgaa agttgcagtg aagatgctaa aacgtaagtg     120


<210>  1150
<211>  120
<212>  DNA
<213>  Artificial Sequence

<220>
<223>  PDGFRA

<400>  1150
   tggattaagc cggtcccaac ctgtcatgaa agttgcagtg aagatgctaa aacgtaagtg      60
   ctccttcctg ggattttttt gagcacgggg attttttgag catggggata ttaagggaat     120


<210>  1151
<211>  120
<212>  DNA
<213>  Artificial Sequence

<220>
<223>  PDGFRA

<400>  1151
tttttttctg agaacaggaa gttggtagct cagctggact gatatgtgat ttattctttc      60

aacagccacg gccagatcca gtgaaaaaca agctctcatg tctgaactga agataatgac     120

<210>  1152
<211>  120
<212>  DNA
<213>  Artificial Sequence

<220>
<223>  PDGFRA

<400>  1152
aacagccacg gccagatcca gtgaaaaaca agctctcatg tctgaactga agataatgac      60

tcacctgggg ccacatttga acattgtaaa cttgctggga gcctgcacca agtcaggtgg      120


<210>  1153
<211>  120
<212>  DNA
<213>  Artificial Sequence

<220>
<223>  PDGFRA

<400>  1153
tcacctgggg ccacatttga acattgtaaa cttgctggga gcctgcacca agtcaggtgg      60

gctcactgac ctggagtgag gattttcact ggacacatgt ggttgtgaaa actgttcaat      120


<210>  1154
<211>  120
<212>  DNA
<213>  Artificial Sequence

<220>
<223>  PDGFRA

<400>  1154
cttcattttc atacccatct cctaacggct tttgtcccca taggccccat ttacatcatc      60

acagagtatt gcttctatgg agatttggtc aactatttgc ataagaatag ggatagcttc      120


<210>  1155
<211>  120
<212>  DNA
<213>  Artificial Sequence

<220>
<223>  PDGFRA

<400>  1155
acagagtatt gcttctatgg agatttggtc aactatttgc ataagaatag ggatagcttc      60

ctgagccacc acccagagaa gccaaagaaa gagctggata tctttggatt gaaccctgct      120


<210>  1156
<211>  120
<212>  DNA
<213>  Artificial Sequence

<220>
<223>  PDGFRA

<400>  1156
ctgagccacc acccagagaa gccaaagaaa gagctggata tctttggatt gaaccctgct      60

gatgaaagca cacggaggtg ggtgcaaaga gagatgttgc tgtctatcat tatcttacag     120


<210>  1157
<211>  120
<212>  DNA
<213>  Artificial Sequence

<220>
<223>  PDGFRA

<400>  1157
tgggagctct tctgctattt acatgtgatc cacttagacc tataaaatgc agctctggcc      60

agggatgctt gagttctgga accttgcaag aactgtctgt ggatctccaa gctcgaggtc     120


<210>  1158
<211>  120
<212>  DNA
<213>  Artificial Sequence

<220>
<223>  PDGFRA

<400>  1158
agggatgctt gagttctgga accttgcaag aactgtctgt ggatctccaa gctcgaggtc      60

cttgctgaac ctggacctat aaatgacgtc aatgatagtg atccctactg cagaaatcta     120


<210>  1159
<211>  120
<212>  DNA
<213>  Artificial Sequence

<220>
<223>  PDGFRA

<400>  1159
ggcaccctgg gtaagatttc tctttctgtt tttacagcta tgttatttta tcttttgaaa      60

acaatggtga ctacatggac atgaagcagg ctgatactac acagtatgtc cccatgctag     120


<210>  1160
<211>  120
<212>  DNA
<213>  Artificial Sequence

<220>
<223>  PDGFRA

<400>  1160
acaatggtga ctacatggac atgaagcagg ctgatactac acagtatgtc cccatgctag      60

aaaggaaaga ggtttctaaa tattccgaca tccagagatc actctatgat cgtccagcct     120

<210>  1161
<211>  120
<212>  DNA
<213>  Artificial Sequence

<220>
<223>  PDGFRA

<400>  1161
aaaggaaaga ggtttctaaa tattccgaca tccagagatc actctatgat cgtccagcct      60

catataagaa gaaatctatg ttaggtaaaa gtgtctatac tcactctggg tgttgggact     120


<210>  1162
<211>  120
<212>  DNA
<213>  Artificial Sequence

<220>
<223>  PDGFRA

<400>  1162
tctgcaacct gatgatttcc tgctgcctgc cagcaccaat acatttaatt tcttttctgc      60

agactcagaa gtcaaaaacc tcctttcaga tgataactca gaaggcctta ctttattgga     120


<210>  1163
<211>  120
<212>  DNA
<213>  Artificial Sequence

<220>
<223>  PDGFRA

<400>  1163
agactcagaa gtcaaaaacc tcctttcaga tgataactca gaaggcctta ctttattgga      60

tttgttgagc ttcacctatc aagttgcccg aggaatggag tttttggctt caaaaaatgt     120


<210>  1164
<211>  120
<212>  DNA
<213>  Artificial Sequence

<220>
<223>  PDGFRA

<400>  1164
tttgttgagc ttcacctatc aagttgcccg aggaatggag tttttggctt caaaaaatgt      60

aagttcaagg aacacagacc tttttagacc cagatttcag tgagtggagt gtggacggag     120


<210>  1165
<211>  120
<212>  DNA
<213>  Artificial Sequence

<220>
<223>  PDGFRA

<400>  1165
gtgatgctat tcagctacag atggcttgat cctgagtcat ttcttccttt tccatgcagt      60

gtgtccaccg tgatctggct gctcgcaacg tcctcctggc acaaggaaaa attgtgaaga     120


<210>  1166
<211>  120
<212>  DNA
<213>  Artificial Sequence

<220>
<223>  PDGFRA

<400>  1166
gtgtccaccg tgatctggct gctcgcaacg tcctcctggc acaaggaaaa attgtgaaga      60

tctgtgactt tggcctggcc agagacatca tgcatgattc gaactatgtg tcgaaaggca     120


<210>  1167
<211>  120
<212>  DNA
<213>  Artificial Sequence

<220>
<223>  PDGFRA

<400>  1167
tctgtgactt tggcctggcc agagacatca tgcatgattc gaactatgtg tcgaaaggca      60

gtgtacgtcc tcacttccct cactggtcag gctcatcctc cttcacttta atctctaaag     120


<210>  1168
<211>  120
<212>  DNA
<213>  Artificial Sequence

<220>
<223>  PDGFRA

<400>  1168
tttccactgc tgtggatcat cagtgagtag acatgggttt aactgtctcc ctccttcctt      60

gcagaccttt ctgcccgtga agtggatggc tcctgagagc atctttgaca acctctacac     120


<210>  1169
<211>  120
<212>  DNA
<213>  Artificial Sequence

<220>
<223>  PDGFRA

<400>  1169
gcagaccttt ctgcccgtga agtggatggc tcctgagagc atctttgaca acctctacac      60

cacactgagt gatgtctggt cttatggcat tctgctctgg gagatctttt cccttggtat     120

<210>  1170
<211>  120
<212>  DNA
<213>  Artificial Sequence

<220>
<223>  PDGFRA

<400>  1170
cacactgagt gatgtctggt cttatggcat tctgctctgg gagatctttt cccttggtat      60

gggcctgaca ttgctgctta tttgggctgt tctgaaacac cactggaagg aaaatgtgtt      120


<210>  1171
<211>  120
<212>  DNA
<213>  Artificial Sequence

<220>
<223>  PDGFRA

<400>  1171
agcgtttgtt agtcctggtg ttttattgtt tggcttttag gtggcacccc ttaccccggc      60

atgatggtgg attctacttt ctacaataag atcaagagtg ggtaccggat ggccaagcct      120


<210>  1172
<211>  120
<212>  DNA
<213>  Artificial Sequence

<220>
<223>  PDGFRA

<400>  1172
atgatggtgg attctacttt ctacaataag atcaagagtg ggtaccggat ggccaagcct      60

gaccacgcta ccagtgaagt gtgagctcct tccccatccc gggggcctgt gttcacagtc      120


<210>  1173
<211>  120
<212>  DNA
<213>  Artificial Sequence

<220>
<223>  PDGFRA

<400>  1173
gggtctaggg ggagggaggg gccctgagac ttccccctgt gcccactctt gagttctgtc      60

cccacagcta cgagatcatg gtgaaatgct ggaacagtga gccggagaag agaccctcct      120


<210>  1174
<211>  120
<212>  DNA
<213>  Artificial Sequence

<220>
<223>  PDGFRA

<400>  1174
cccacagcta cgagatcatg gtgaaatgct ggaacagtga gccggagaag agaccctcct      60

tttaccacct gagtgagatt gtggagaatc tgctgcctgg acaatataaa aaggtgtgtt     120


<210>  1175
<211>  120
<212>  DNA
<213>  Artificial Sequence

<220>
<223>  PDGFRA

<400>  1175
tttaccacct gagtgagatt gtggagaatc tgctgcctgg acaatataaa aaggtgtgtt      60

tggatctgtg ggtggaaagg tctggataaa gctggaagtt ataccagtga gctgtgctgt     120


<210>  1176
<211>  120
<212>  DNA
<213>  Artificial Sequence

<220>
<223>  PDGFRA

<400>  1176
gattaaatat gttcaatgaa tgtttataga gttatgaaaa aattcacctg gacttcctga      60

agagtgacca tcctgctgtg gcacgcatgc gtgtggactc agacaatgca tacattggtg     120


<210>  1177
<211>  120
<212>  DNA
<213>  Artificial Sequence

<220>
<223>  PDGFRA

<400>  1177
agagtgacca tcctgctgtg gcacgcatgc gtgtggactc agacaatgca tacattggtg      60

tcacctacaa aaacgaggaa gacaagctga aggactggga gggtggtctg gatgagcaga     120


<210>  1178
<211>  120
<212>  DNA
<213>  Artificial Sequence

<220>
<223>  PDGFRA

<400>  1178
tcacctacaa aaacgaggaa gacaagctga aggactggga gggtggtctg gatgagcaga      60

gactgagcgc tgacagtggc tacatcattc ctctgcctga cattgaccct gtccctgagg     120

<210>  1179
<211>  120
<212>  DNA
<213>  Artificial Sequence

<220>
<223>  PDGFRA

<400>  1179
gactgagcgc tgacagtggc tacatcattc ctctgcctga cattgaccct gtccctgagg      60

aggaggacct gggcaagagg aacagacaca ggtagctgtg ggggcagcct cggtgtctca      120


<210>  1180
<211>  120
<212>  DNA
<213>  Artificial Sequence

<220>
<223>  PDGFRA

<400>  1180
gttctgtgca ggagttgtaa tatttgctct tctctccctc ctccagctcg cagacctctg      60

aagagagtgc cattgagacg ggttccagca gttccacctt catcaagaga gaggacgaga      120


<210>  1181
<211>  120
<212>  DNA
<213>  Artificial Sequence

<220>
<223>  PDGFRA

<400>  1181
aagagagtgc cattgagacg ggttccagca gttccacctt catcaagaga gaggacgaga      60

ccattgaaga catcgacatg atggatgaca tcggcataga ctcttcagac ctggtggaag      120


<210>  1182
<211>  120
<212>  DNA
<213>  Artificial Sequence

<220>
<223>  PDGFRA

<400>  1182
ccattgaaga catcgacatg atggatgaca tcggcataga ctcttcagac ctggtggaag      60

acagcttcct gtaactggcg gattcgaggg gttccttcca cttctggggc cacctctgga      120


<210>  1183
<211>  120
<212>  DNA
<213>  Artificial Sequence

<220>
<223>  PDGFRA-E12

<400>  1183
ctggtgcact gggactttgg taattcacca gttacctgtc ctggtcattt atagaaaccg      60

aggtatgaaa ttcgctggag ggtcattgaa tcaatcagcc cagatggaca tgaatatatt     120


<210>  1184
<211>  120
<212>  DNA
<213>  Artificial Sequence

<220>
<223>  PDGFRA-E12

<400>  1184
tcaccagtta cctgtcctgg tcatttatag aaaccgaggt atgaaattcg ctggagggtc      60

attgaatcaa tcagcccaga tggacatgaa tatatttatg tggacccgat gcagctgcct     120


<210>  1185
<211>  120
<212>  DNA
<213>  Artificial Sequence

<220>
<223>  PDGFRA-E12

<400>  1185
agcccagatg gacatgaata tatttatgtg gacccgatgc agctgcctta tgactcaaga      60

tgggagtttc caagagatgg actagtgctt ggtaagttcc atggggtaac ctcccaagac     120


<210>  1186
<211>  120
<212>  DNA
<213>  Artificial Sequence

<220>
<223>  PDGFRA-E12

<400>  1186
tatgtggacc cgatgcagct gccttatgac tcaagatggg agtttccaag agatggacta      60

gtgcttggta agttccatgg ggtaacctcc caagactccc ttttcccttg cacacaactt     120


<210>  1187
<211>  120
<212>  DNA
<213>  Artificial Sequence

<220>
<223>  PDGFRA-E14

<400>  1187
aatgatatcc aatcacagga ttagtcatat tcttggtttt tttctgagaa caggaagttg      60

gtagctcagc tggactgata tgtgatttat tctttcaaca gccacggcca gatccagtga     120

<210>  1188
<211>  120
<212>  DNA
<213>  Artificial Sequence

<220>
<223>  PDGFRA-E14

<400>  1188
tcatattctt ggtttttttc tgagaacagg aagttggtag ctcagctgga ctgatatgtg        60

atttattctt tcaacagcca cggccagatc cagtgaaaaa caagctctca tgtctgaact       120


<210>  1189
<211>  120
<212>  DNA
<213>  Artificial Sequence

<220>
<223>  PDGFRA-E14

<400>  1189
aacaggaagt tggtagctca gctggactga tatgtgattt attctttcaa cagccacggc        60

cagatccagt gaaaaacaag ctctcatgtc tgaactgaag ataatgactc acctggggcc       120


<210>  1190
<211>  120
<212>  DNA
<213>  Artificial Sequence

<220>
<223>  PDGFRA-E14

<400>  1190
gactgatatg tgatttattc tttcaacagc cacggccaga tccagtgaaa aacaagctct        60

catgtctgaa ctgaagataa tgactcacct ggggccacat ttgaacattg taaacttgct       120


<210>  1191
<211>  120
<212>  DNA
<213>  Artificial Sequence

<220>
<223>  PDGFRA-E14

<400>  1191
aaaacaagct ctcatgtctg aactgaagat aatgactcac ctggggccac atttgaacat        60

tgtaaacttg ctgggagcct gcaccaagtc aggtgggctc actgacctgg agtgaggatt       120


<210>  1192
<211>  120
<212>  DNA
<213>  Artificial Sequence

<220>
<223>  PDGFRA-E14

<400>  1192
gaagataatg actcacctgg ggccacattt gaacattgta aacttgctgg gagcctgcac      60

caagtcaggt gggctcactg acctggagtg aggattttca ctggacacat gtggttgtga     120


<210>  1193
<211>  120
<212>  DNA
<213>  Artificial Sequence

<220>
<223>  PDGFRA-E14

<400>  1193
acatttgaac attgtaaact tgctgggagc ctgcaccaag tcaggtgggc tcactgacct      60

ggagtgagga ttttcactgg acacatgtgg ttgtgaaaac tgttcaatca ggcttaaatc     120


<210>  1194
<211>  120
<212>  DNA
<213>  Artificial Sequence

<220>
<223>  PDGFRA-E14

<400>  1194
gggagcctgc accaagtcag gtgggctcac tgacctggag tgaggatttt cactggacac      60

atgtggttgt gaaaactgtt caatcaggct taaatcctcc actctccatc cccacacatg     120


<210>  1195
<211>  120
<212>  DNA
<213>  Artificial Sequence

<220>
<223>  PDGFRA-E18

<400>  1195
gtgatgctat tcagctacag atggcttgat cctgagtcat ttcttccttt tccatgcagt      60

gtgtccaccg tgatctggct gctcgcaacg tcctcctggc acaaggaaaa attgtgaaga     120


<210>  1196
<211>  120
<212>  DNA
<213>  Artificial Sequence

<220>
<223>  PDGFRA-E18

<400>  1196
cttgatcctg agtcatttct tccttttcca tgcagtgtgt ccaccgtgat ctggctgctc      60

gcaacgtcct cctggcacaa ggaaaaattg tgaagatctg tgactttggc ctggccagag     120

<210>  1197
<211>  120
<212>  DNA
<213>  Artificial Sequence

<220>
<223>  PDGFRA-E18

<400>  1197
tggcacaagg aaaaattgtg aagatctgtg actttggcct ggccagagac atcatgcatg      60

attcgaacta tgtgtcgaaa ggcagtgtac gtcctcactt ccctcactgg tcaggctcat      120


<210>  1198
<211>  120
<212>  DNA
<213>  Artificial Sequence

<220>
<223>  PDGFRA-E18

<400>  1198
tctgtgactt tggcctggcc agagacatca tgcatgattc gaactatgtg tcgaaaggca      60

gtgtacgtcc tcacttccct cactggtcag gctcatcctc cttcacttta atctctaaag      120


<210>  1199
<211>  120
<212>  DNA
<213>  Artificial Sequence

<220>
<223>  TP53

<400>  1199
gagagatggg ggtgggaggc tgtcagtggg gaacaagaag tggagaatgt cagtctgagt      60

caggcccttc tgtcttgaac atgagttttt tatggcggga ggtagactga ccctttttgg      120


<210>  1200
<211>  120
<212>  DNA
<213>  Artificial Sequence

<220>
<223>  TP53

<400>  1200
caggcccttc tgtcttgaac atgagttttt tatggcggga ggtagactga ccctttttgg      60

acttcaggtg gctgtaggag acagaagcag ggaggagaga tgacatcaca tgagtgagag      120


<210>  1201
<211>  120
<212>  DNA
<213>  Artificial Sequence

<220>
<223>  TP53

<400>  1201
gatgagaatg gaatcctatg gctttccaac ctaggaaggc aggggagtag ggccaggaag        60

gggctgaggt cactcacctg gagtgagccc tgctcccccc tggctccttc ccagcctggg       120


<210>  1202
<211>  120
<212>  DNA
<213>  Artificial Sequence

<220>
<223>  TP53

<400>  1202
gggctgaggt cactcacctg gagtgagccc tgctcccccc tggctccttc ccagcctggg        60

catccttgag ttccaaggcc tcattcagct ctcggaacat ctcgaagcgc tcacgcccac       120


<210>  1203
<211>  120
<212>  DNA
<213>  Artificial Sequence

<220>
<223>  TP53

<400>  1203
catccttgag ttccaaggcc tcattcagct ctcggaacat ctcgaagcgc tcacgcccac        60

ggatctgcag caacagagga gggggagaag taagtatata cacagtacct gagttaaaag       120


<210>  1204
<211>  120
<212>  DNA
<213>  Artificial Sequence

<220>
<223>  TP53

<400>  1204
gtaagtcaag tagcatctgt atcaggcaaa gtcatagaac cattttcatg ctctctttaa        60

caattttctt tttgaaagct ggtctggtcc tttaaaatat atattatggt ataagttggt       120


<210>  1205
<211>  120
<212>  DNA
<213>  Artificial Sequence

<220>
<223>  TP53

<400>  1205
caattttctt tttgaaagct ggtctggtcc tttaaaatat atattatggt ataagttggt        60

gttctgaagt tagttagcta caaccaggag ccattgtctt tgaggcatca ctgccccctg       120

<210>  1206
<211>  120
<212>  DNA
<213>  Artificial Sequence

<220>
<223>  TP53

<400>  1206
gtaagtcaag tagcatctgt atcaggcaaa gtcatagaac cattttcatg ctctctttaa      60

caattttctt tttgaaagct ggtctggtcc tttaaaatat atattatggt ataagttggt      120


<210>  1207
<211>  120
<212>  DNA
<213>  Artificial Sequence

<220>
<223>  TP53

<400>  1207
caattttctt tttgaaagct ggtctggtcc tttaaaatat atattatggt ataagttggt      60

gttctgaagt tagttagcta caaccaggag ccattgtctt tgaggcatca ctgccccctg      120


<210>  1208
<211>  120
<212>  DNA
<213>  Artificial Sequence

<220>
<223>  TP53

<400>  1208
tgagtgttag actggaaact ttccacttga taagaggtcc caagacttag tacctgaagg      60

gtgaaatatt ctccatccag tggtttcttc tttggctggg gagaggagct ggtgttgttg      120


<210>  1209
<211>  120
<212>  DNA
<213>  Artificial Sequence

<220>
<223>  TP53

<400>  1209
gtgaaatatt ctccatccag tggtttcttc tttggctggg gagaggagct ggtgttgttg      60

ggcagtgcta ggaaagaggc aaggaaaggt gataaaagtg aatctgaggc ataactgcac      120


<210>  1210
<211>  120
<212>  DNA
<213>  Artificial Sequence

<220>
<223>  TP53

<400>  1210
   ggcataactg cacccttggt ctcctccacc gcttcttgtc ctgcttgctt acctcgctta        60
   gtgctccctg ggggcagctc gtggtgaggc tcccctttct tgcggagatt ctcttcctct     120


<210>  1211
<211>  120
<212>  DNA
<213>  Artificial Sequence

<220>
<223>  TP53

<400>  1211
gtgctccctg ggggcagctc gtggtgaggc tcccctttct tgcggagatt ctcttcctct      60

gtgcgccggt ctctcccagg acaggcacaa acacgcacct caaagctgtt ccgtcccagt     120


<210>  1212
<211>  120
<212>  DNA
<213>  Artificial Sequence

<220>
<223>  TP53

<400>  1212
gtgcgccggt ctctcccagg acaggcacaa acacgcacct caaagctgtt ccgtcccagt      60

agattaccac tactcaggat aggaaaagag aagcaagagg cagtaaggaa atcaggtcct     120


<210>  1213
<211>  120
<212>  DNA
<213>  Artificial Sequence

<220>
<223>  TP53

<400>  1213
ggtcagaggc aagcagaggc tggggcacag caggccagtg tgcagggtgg caagtggctc      60

ctgacctgga gtcttccagt gtgatgatgg tgaggatggg cctccggttc atgccgccca     120


<210>  1214
<211>  120
<212>  DNA
<213>  Artificial Sequence

<220>
<223>  TP53

<400>  1214
ctgacctgga gtcttccagt gtgatgatgg tgaggatggg cctccggttc atgccgccca      60

tgcaggaact gttacacatg tagttgtagt ggatggtggt acagtcagag ccaacctagg     120

<210>  1215
<211>  120
<212>  DNA
<213>  Artificial Sequence

<220>
<223>  TP53

<400>  1215
tgcaggaact gttacacatg tagttgtagt ggatggtggt acagtcagag ccaacctagg      60

agataacaca ggcccaagat gaggccagtg cgccttgggg agacctgtgg caagcagggg     120


<210>  1216
<211>  120
<212>  DNA
<213>  Artificial Sequence

<220>
<223>  TP53

<400>  1216
cctggagggc cactgacaac cacccttaac ccctcctccc agagacccca gttgcaaacc      60

agacctcagg cggctcatag ggcaccacca cactatgtcg aaaagtgttt ctgtcatcca     120


<210>  1217
<211>  120
<212>  DNA
<213>  Artificial Sequence

<220>
<223>  TP53

<400>  1217
agacctcagg cggctcatag ggcaccacca cactatgtcg aaaagtgttt ctgtcatcca      60

aatactccac acgcaaattt ccttccactc ggataagatg ctgaggaggg gccagaccta     120


<210>  1218
<211>  120
<212>  DNA
<213>  Artificial Sequence

<220>
<223>  TP53

<400>  1218
aatactccac acgcaaattt ccttccactc ggataagatg ctgaggaggg gccagaccta      60

agagcaatca gtgaggaatc agaggcctgg ggaccctggg caaccagccc tgtcgtctct     120


<210>  1219
<211>  120
<212>  DNA
<213>  Artificial Sequence

<220>
<223>  TP53

<400>  1219
tgtcgtctct ccagccccag ctgctcacca tcgctatctg agcagcgctc atggtggggg      60

cagcgcctca caacctccgt catgtgctgt gactgcttgt agatggccat ggcgcggacg      120


<210>  1220
<211>  120
<212>  DNA
<213>  Artificial Sequence

<220>
<223>  TP53

<400>  1220
cagcgcctca caacctccgt catgtgctgt gactgcttgt agatggccat ggcgcggacg      60

cgggtgccgg gcgggggtgt ggaatcaacc cacagctgca cagggcaggt cttggccagt      120


<210>  1221
<211>  120
<212>  DNA
<213>  Artificial Sequence

<220>
<223>  TP53

<400>  1221
cgggtgccgg gcgggggtgt ggaatcaacc cacagctgca cagggcaggt cttggccagt      60

tggcaaaaca tcttgttgag ggcaggggag tactgtagga agaggaagga gacagagttg      120


<210>  1222
<211>  120
<212>  DNA
<213>  Artificial Sequence

<220>
<223>  TP53

<400>  1222
gggcaactga ccgtgcaagt cacagacttg gctgtcccag aatgcaagaa gcccagacgg      60

aaaccgtagc tgccctggta ggttttctgg gaagggacag aagatgacag gggccaggag      120


<210>  1223
<211>  120
<212>  DNA
<213>  Artificial Sequence

<220>
<223>  TP53

<400>  1223
gggcaactga ccgtgcaagt cacagacttg gctgtcccag aatgcaagaa gcccagacgg      60

aaaccgtagc tgccctggta ggttttctgg gaagggacag aagatgacag gggccaggag      120

<210>  1224
<211>  120
<212>  DNA
<213>  Artificial Sequence

<220>
<223>  TP53

<400>  1224
ggggctggtg caggggccgc cggtgtagga gctgctggtg caggggccac ggggggagca      60

gcctctggca ttctgggagc ttcatctgga cctgggtctt cagtgaacca ttgttcaata      120


<210>  1225
<211>  120
<212>  DNA
<213>  Artificial Sequence

<220>
<223>  TP53

<400>  1225
gcctctggca ttctgggagc ttcatctgga cctgggtctt cagtgaacca ttgttcaata      60

tcgtccgggg acagcatcaa atcatccatt gcttgggacg gcaaggggga ctgtagatgg      120


<210>  1226
<211>  120
<212>  DNA
<213>  Artificial Sequence

<220>
<223>  TP53

<400>  1226
ccctccaggt ccccagccca acccttgtcc ttaccagaac gttgttttca ggaagtctga      60

aagacaagag cagaaagtca gtcccatgga attttcgctt cccacaggtc tctgctaggg      120


<210>  1227
<211>  120
<212>  DNA
<213>  Artificial Sequence

<220>
<223>  TP53

<400>  1227
aagacaagag cagaaagtca gtcccatgga attttcgctt cccacaggtc tctgctaggg      60

ggctggggtt ggggtggggg tggtgggcct gcccttccaa tggatccact cacagtttcc      120


<210>  1228
<211>  120
<212>  DNA
<213>  Artificial Sequence

<220>
<223>  TP53

<400>  1228
ggctggggtt ggggtggggg tggtgggcct gcccttccaa tggatccact cacagtttcc      60

ataggtctga aaatgtttcc tgactcagag ggggctcgac gctaggatct gactgcggct     120

<210>  1229
<211>  120
<212>  DNA
<213>  Artificial Sequence

<220>
<223>  TP53

<400>  1229
ataggtctga aaatgtttcc tgactcagag ggggctcgac gctaggatct gactgcggct      60

cctccatggc agtgacccgg aaggcagtct ggctgctgca agaggaaaag tggggatcca     120